(19) Europäisches Patentamt
European Patent Office
Office européen des brevets

(11) EP 4 553 075 A1

(12) EUROPEAN PATENT APPLICATION
published in accordance with Art. 153(4) EPC

(43) Date of publication:
14.05.2025 Bulletin 2025/20

(21) Application number: 23834889.0

(22) Date of filing: 05.07.2023

(51) International Patent Classification (IPC):
*C07D 471/04* (2006.01) *C07D 487/04* (2006.01)
*C07D 413/12* (2006.01) *C07D 405/04* (2006.01)
*A61K 31/4433* (2006.01) *A61K 31/352* (2006.01)
*A61P 35/00* (2006.01)

(52) Cooperative Patent Classification (CPC):
**A61K 31/352; A61K 31/36; A61K 31/4035; A61K 31/4155; A61K 31/4375; A61K 31/4433; A61K 31/453; A61K 31/454; A61K 31/517; A61K 31/5377; A61P 9/00; A61P 29/00; A61P 35/00; C07D 239/90; C07D 239/91;** (Cont.)

(86) International application number:
PCT/CN2023/105915

(87) International publication number:
WO 2024/008122 (11.01.2024 Gazette 2024/02)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 07.07.2022 CN 202210794939

(71) Applicant: **Hinova Pharmaceuticals Inc.**
**Chengdu, Sichuan 610041 (CN)**

(72) Inventors:
- **FAN, Lei**
  **Chengdu, Sichuan 610041 (CN)**
- **YU, Hua**
  **Chengdu, Sichuan 610041 (CN)**
- **WANG, Fei**
  **Chengdu, Sichuan 610041 (CN)**
- **AI, Chaowu**
  **Chengdu, Sichuan 610041 (CN)**
- **XU, Kexin**
  **Chengdu, Sichuan 610041 (CN)**
- **LIU, Xingtai**
  **Chengdu, Sichuan 610041 (CN)**
- **DU, Jing**
  **Chengdu, Sichuan 610041 (CN)**
- **PENG, Ying**
  **Chengdu, Sichuan 610041 (CN)**
- **LUO, Tongchuan**
  **Chengdu, Sichuan 610041 (CN)**
- **TAN, Bin**
  **Chengdu, Sichuan 610041 (CN)**
- **WANG, Wenzhong**
  **Chengdu, Sichuan 610041 (CN)**
- **YAN, Dejun**
  **Chengdu, Sichuan 610041 (CN)**
- **WANG, Xuzhao**
  **Chengdu, Sichuan 610041 (CN)**
- **LI, Liang**
  **Chengdu, Sichuan 610041 (CN)**
- **XIAO, Daibiao**
  **Chengdu, Sichuan 610041 (CN)**
- **LIU, Chengcheng**
  **Chengdu, Sichuan 610041 (CN)**
- **LUO, Yao**
  **Chengdu, Sichuan 610041 (CN)**
- **LI, Xinghai**
  **Chengdu, Sichuan 610041 (CN)**
- **CHEN, Yuanwei**
  **Chengdu, Sichuan 610041 (CN)**

(74) Representative: **Altmann Stößel Dick Patentanwälte PartG mbB**
**Theodor-Heuss-Anlage 2**
**68165 Mannheim (DE)**

# (54) PI3K INHIBITOR, PREPARATION METHOD THEREFOR, AND USE THEREOF

(57) The present invention belongs to the field of chemical pharmaceuticals, and provided are a PI3K inhibitor, a preparation method therefor, and a use thereof. The PI3K inhibitor of the present invention is a compound as represented by formula I, or a salt thereof, a stereoisomer thereof, a solvate thereof, a hydrate thereof, or a



Processed by Luminess, 75001 PARIS (FR)

(Cont. next page)

prodrug thereof. The prepared compound can be used for preparing a PI3K selective inhibitor and for preparing a drug for preventing and/or treating a disease related to PI3K, such as a drug for preventing and/or treating cancer. The present invention provides a new choice for clinically treating cancer, and the present invention has good application prospects.

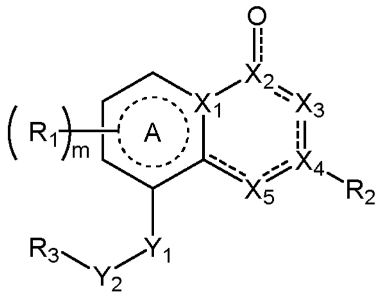

Formula I

(52) Cooperative Patent Classification (CPC): (Cont.)
**C07D 311/22; C07D 311/30; C07D 401/04; C07D 401/12; C07D 401/14; C07D 403/04; C07D 405/04; C07D 405/12; C07D 405/14; C07D 407/04; C07D 413/04; C07D 413/12; C07D 413/14; C07D 471/04; C07D 487/04**

## Description

### Field of the invention

The present invention belongs to the field of chemical medicaments, and specifically relates to a PI3K inhibitor, preparation methods therefor, and uses thereof.

### Background of the invention

Phosphatidylinositol 3-kinase (PI3K) is an intracellular phosphatidylinositol kinase, as an important signaling molecule in cells. PI3K is mainly involved in regulating physiological processes such as cell proliferation, apoptosis, and differentiation, and can specifically phosphorylate 3-hydroxy in the phosphatidylinositol ring. As the main downstream effector of receptor tyrosine kinase (RTK) and G protein-coupled receptor (GPCR), PI3K transduces signals from various growth factors and cytokines to intracellular messengers by generating phospholipids that can activate serine/threonine protein kinase (Akt) and other downstream effectors. The signal pathway composed of PI3K and its downstream molecule Akt can activate downstream signal molecules, which is closely related to the occurrence and development of breast cancer, gastric cancer, colon cancer, rectal cancer, ovarian cancer, prostate cancer and other tumors. It has been shown that excessive activation of PI3K is associated with various proliferative, inflammatory, or cardiovascular diseases, including cancer, inflammation, and cardiovascular diseases.

It has been demonstrated that PI3K is a promising drug target, and using selective PI3K inhibitors as anti-tumor medicaments can increase treatment selectivity, and reduce the occurrence of adverse reactions and toxic side effects. The number and structure types of selective PI3K inhibitors currently reported are still quite limited, and their effectiveness needs to be further improved. Studying a PI3K inhibitor with excellent efficacy, as well as minimal adverse reactions and toxic side effects is of great significance for the clinical treatment of cancer.

### Content of the invention

The object of the present invention is to provide a PI3K inhibitor, preparation methods therefor, and uses thereof.

The present invention provides a compound as represented by formula I, or a salt thereof, or a stereoisomer thereof, or a solvate thereof, or a hydrate thereof, or a prodrug thereof, or a deuterated compound thereof:

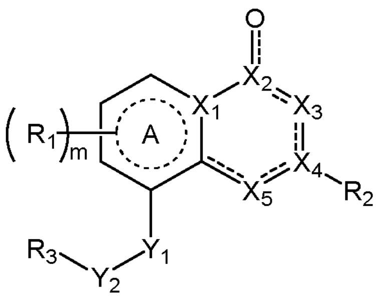

Formula I

wherein,

$R_1$ is a substituent in ring A, which has m substituents; each $R_1$ is independently selected from the group consisting of substituted or unsubstituted $C_1$-$C_8$ alkyl, $C_1$-$C_8$ alkoxy, halogen, cyano, nitro, hydroxy, carboxyl, $-NHR_{15}$, $-NHR_8$, $-C(O)NHR_8$, $-C(O)NHR_{15}$, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl; m is selected from the group consisting of 0, 1, 2, or 3;

$R_{15}$ is selected from the group consisting of H, substituted or unsubstituted $C_1$-$C_8$ alkyl, $C_1$-$C_8$ alkoxy, halogen, cyano, nitro, hydroxy, carboxyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl;

$Y_1$ and $Y_2$ are each independently selected from the group consisting of absence, $CR_4R_5$, and $NR_4$;

$R_4$ and $R_5$ are each independently selected from the group consisting of H and $C_1$-$C_8$ alkyl; alternatively, $R_4$ and $R_5$, together with a carbon, form a ketone group;

$R_3$ is selected from the group consisting of H, (6-10)-membered aryl substituted with n $R_6$, (5-10)-membered

heteroaryl substituted with n $R_6$, (4-10)-membered heterocycloalkyl substituted with n $R_6$ or

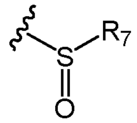

;

each $R_6$ is independently selected from the group consisting of substituted or unsubstituted $C_1$-$C_8$ alkyl, $C_1$-$C_8$ alkoxy, halogen, cyano, nitro, amino, hydroxy, -C(O)O$R_{81}$, -C(O)$R_{81}$,

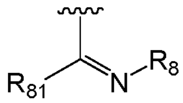

,

cyanomethyl, phosphonyl, sulfonic acid group, sulfonamido, boric acid group, - NH$R_8$ or -C(O)NH$R_8$; alternatively, two $R_6$ linked to the same atom form =O; n is selected from the group consisting of 0, 1, 2, 3, or 4;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

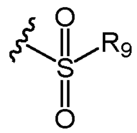

;

$R_7$ and $R_9$ are each independently selected from the group consisting of $C_1$-$C_8$ alkyl and (6-10)-membered aryl;
$X_1$ is selected from C or N; provided that $X_1$ is C, ring A is benzene ring; provided that $X_1$ is N, ring A is dihydropyridine;
$X_2$ is selected from C or N; provided that $X_2$ is C, the bond linking $X_2$ to O is a double bond, and the bond linking $X_2$ to $X_3$ is a single bond; provided that $X_2$ is N, the bond linking $X_2$ to O is a single bond, and the bond linking $X_2$ to $X_3$ is a double bond, with N carrying a positive charge and O carrying a negative charge;
$X_3$ is selected from the group consisting of N, $CR_{10}$ or $NR_{10}$;
$X_4$ is C;
$X_5$ is selected from the group consisting of O, N, $NR_{10}$, or $CR_{10}$;
$R_{10}$ is selected from the group consisting of H as well as substituted or unsubstituted $C_1$-$C_8$ alkyl;
provided that $X_3$ is selected from $CR_{10}$ and $X_5$ is O, the bond linking $X_3$ to $X_4$ is a double bond, the bond linking $X_4$ to $X_5$ is a single bond, and the bond linking $X_5$ to C is a single bond;
provided that $X_3$ is selected from $NR_{10}$ and $X_5$ is N, the bond linking $X_3$ to $X_4$ is a single bond, the bond linking $X_4$ to $X_5$ is a double bond, and the bond linking $X_5$ to C is a single bond;
provided that $X_3$ is selected from $CR_{10}$ and $X_5$ is selected from $CR_{10}$, the bond linking $X_3$ to $X_4$ is a single bond, the bond linking $X_4$ to $X_5$ is a double bond, and the bond linking $X_5$ to C is a single bond; alternatively, the bond linking $X_3$ to $X_4$ is a double bond, the bond linking $X_4$ to $X_5$ is a single bond, and the bond linking $X_5$ to C is a double bond;
provided that $X_3$ is selected from $CR_{10}$ and $X_5$ is N, the bond linking $X_3$ to $X_4$ is a double bond, the bond linking $X_4$ to $X_5$ is a single bond, and the bond linking $X_5$ to C is a double bond;
provided that $X_3$ is N and $X_5$ is selected from $NR_{10}$, the bond linking $X_3$ to $X_4$ is a double bond, the bond linking $X_4$ to $X_5$ is a single bond, and the bond linking $X_5$ to C is a single bond;
$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, -$NR_{11}R_{12}$,

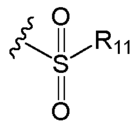

,

and -$SR_{11}$;
$R_{11}$ and $R_{12}$ are each independently selected from the group consisting of H, substituted or unsubstituted $C_1$-$C_8$ alkyl, and amino;

the substituent of the alkyl is selected from the group consisting of deuterium, halogen, hydroxy, cyano, nitro, carboxyl, (4-10)-membered heterocycloalkyl, or $-NR_{13}R_{14}$;

$R_{13}$ and $R_{14}$ are each independently selected from the group consisting of H and $C_1$-$C_8$ alkyl;

the substituent of the cycloalkyl, aryl, heteroaryl or heterocycloalkyl is selected from the group consisting of halogen, hydroxy, amino, carboxyl, nitro, cyano, $-C(O)NR_{16}R_{17}$, $-C(O)OR_{16}$, substituted or unsubstituted $C_1$-$C_8$ alkyl or $C_1$-$C_8$ alkoxy;

$R_{16}$ and $R_{17}$ are each independently selected from the group consisting of H, substituted or unsubstituted $C_1$-$C_8$ alkyl, and hydroxy;

The heteroatoms in the heteroaryl and heterocycloalkyl are selected from the group consisting of N, O, or S, and the number of heteroatoms is selected from the group consisting of 1, 2, or 3.

Further,

for said $R_1$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;

for said $R_{15}$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;

for said $R_3$, the aryl is phenyl; and the heteroaryl is selected from the group consisting of isoindolinyl, pyridyl, pyrimidyl, pyridazinyl, thienyl, furyl, pyrazolyl, imidazolyl or pyrrolyl; he heterocycloalkyl is piperidyl;

for $R_7$ and $R_9$, the aryl is selected from phenyl or naphthyl;

for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

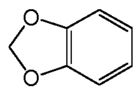
;

the substituent of the alkyl is selected from the group consisting of deuterium, halogen, hydroxy, cyano, nitro, carboxyl, piperidyl, morpholinyl or $-NR_{13}R_{14}$;

$R_{13}$ and $R_{14}$ are each independently selected from the group consisting of H and $C_1$-$C_8$ alkyl.

Further, the compound is as represented by formula II:

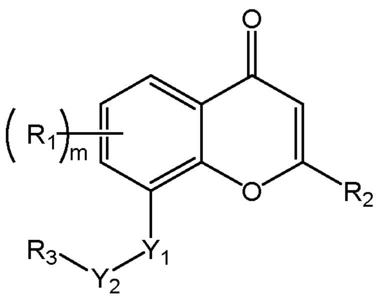

Formula II

wherein,

$R_1$ is a substituent in benzene ring, which has m substituents; each $R_1$ is independently selected from the group consisting of substituted or unsubstituted $C_1$-$C_8$ alkyl, $C_1$-$C_8$ alkoxy, halogen, cyano, nitro, hydroxy, carboxyl, $-NHR_{15}$, $-NHR_8$, $-C(O)NHR_8$, $-C(O)NHR_{15}$, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl; m is selected from the group consisting of 0, 1, 2, or 3;

$R_{15}$ is selected from the group consisting of H, substituted or unsubstituted $C_1$-$C_8$ alkyl, $C_1$-$C_8$ alkoxy, halogen, cyano, nitro, hydroxy, carboxyl, substituted or unsubstituted (3-8)-membered cycloalkyl, substituted or unsubstituted (6-10)-membered aryl, substituted or unsubstituted (4-10)-membered heterocycloalkyl, substituted or unsubstituted

(5-10)-membered heteroaryl;

$Y_1$ and $Y_2$ are each independently selected from the group consisting of absence, $CR_4R_5$, and $NR_4$;

$R_4$ and $R_5$ are each independently selected from the group consisting of H and $C_1$-$C_8$ alkyl; alternatively, $R_4$ and $R_5$, together with a carbon, form a ketone group;

$R_3$ is selected from the group consisting of (6-10)-membered aryl substituted with n $R_6$, (5-10)-membered heteroaryl substituted with n $R_6$, (4-10)-membered heterocycloalkyl substituted with n $R_6$ or

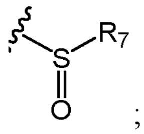

;

each $R_6$ is independently selected from the group consisting of substituted or unsubstituted $C_1$-$C_8$ alkyl, $C_1$-$C_8$ alkoxy, halogen, cyano, nitro, amino, hydroxy, -C(O)OR$_{81}$, -C(O)R$_{81}$,

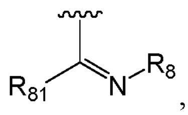

,

cyanomethyl, phosphonyl, sulfonic acid group, sulfonamido, boric acid group, - NHR$_8$ or -C(O)NHR$_8$; alternatively, two $R_6$ linked to the same atom form =O; n is selected from the group consisting of 0, 1, 2, 3, or 4;

$R_{81}$ is selected from the group consisting of H, substituted or unsubstituted $C_1$-$C_8$ alkyl, and amino;

$R_8$ is selected from the group consisting of substituted or unsubstituted $C_1$-$C_8$ alkyl, hydroxy, and

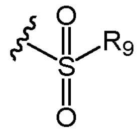

;

$R_7$ and $R_9$ are each independently selected from the group consisting of $C_1$-$C_8$ alkyl and (6-10)-membered aryl;

$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, -NR$_{11}$R$_{12}$,

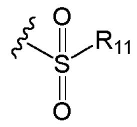

,

and -SR$_{11}$;

provided that $R_6$ is carboxyl, $R_2$ is not selected from the group consisting of substituted or unsubstituted (3-8)-membered cycloalkyl, substituted or unsubstituted (6-10)-membered aryl, and substituted or unsubstituted (5-10)-membered heteroaryl;

$R_{11}$ and $R_{12}$ are each independently selected from the group consisting of H, substituted or unsubstituted $C_1$-$C_8$ alkyl, and amino;

the substituent of the alkyl is selected from the group consisting of deuterium, halogen, hydroxy, cyano, nitro, carboxyl, (4-10)-membered heterocycloalkyl, or -NR$_{13}$R$_{14}$;

$R_{13}$ and $R_{14}$ are each independently selected from the group consisting of H and $C_1$-$C_8$ alkyl;

the substituent of the cycloalkyl, aryl, heteroaryl or heterocycloalkyl is selected from the group consisting of halogen, hydroxy, amino, carboxyl, nitro, cyano, -C(O)NR$_{16}$R$_{17}$, -C(O)OR$_{16}$, substituted or unsubstituted $C_1$-$C_8$ alkyl or $C_1$-$C_8$ alkoxy;

$R_{16}$ and $R_{17}$ are each independently selected from the group consisting of H, substituted or unsubstituted $C_1$-$C_8$ alkyl, and hydroxy;

The heteroatoms in the heteroaryl and heterocycloalkyl are selected from the group consisting of N, O, or S, and the number of heteroatoms is selected from the group consisting of 1, 2, or 3;

preferably,

for said $R_1$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;
for said $R_{15}$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;
for said $R_3$, the aryl is phenyl; and the heteroaryl is selected from the group consisting of isoindolinyl, pyridyl, pyrimidyl, pyridazinyl, thienyl, furyl, pyrazolyl, imidazolyl or pyrrolyl; the heterocycloalkyl is piperidyl;
for $R_7$ and $R_9$, the aryl is selected from phenyl or naphthyl;
for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

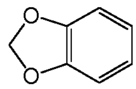
;

the substituent of the alkyl is selected from the group consisting of deuterium, halogen, hydroxy, cyano, nitro, carboxyl, piperidyl, morpholinyl or $-NR_{13}R_{14}$;
$R_{13}$ and $R_{14}$ are each independently selected from the group consisting of H and $C_1$-$C_8$ alkyl.

Further, the compound is as represented by formula II-1:

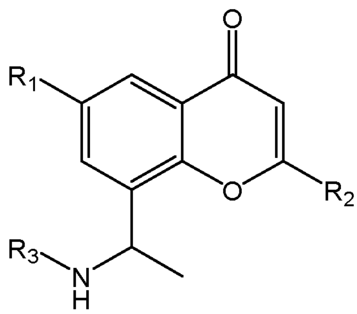

Formula II-1

wherein,

$R_1$ is selected from the group consisting of H, substituted or unsubstituted $C_1$-$C_8$ alkyl, $C_1$-$C_8$ alkoxy, halogen, cyano, nitro, hydroxy, carboxyl, $-NHR_{15}$, $-NHR_8$, $-C(O)NHR_8$, $-C(O)NHR_{15}$, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl;
$R_{15}$ is selected from the group consisting of H, substituted or unsubstituted $C_1$-$C_8$ alkyl, $C_1$-$C_8$ alkoxy, halogen, cyano, nitro, hydroxy, carboxyl, substituted or unsubstituted (3-8)-membered cycloalkyl, substituted or unsubstituted (6-10)-membered aryl, substituted or unsubstituted (4-10)-membered heterocycloalkyl, and substituted or unsubstituted (5-10)-membered heteroaryl;
$R_3$ is selected from the group consisting of (6-10)-membered aryl substituted with n $R_6$, (5-10)-membered heteroaryl substituted with n $R_6$, (4-10)-membered heterocycloalkyl substituted with n $R_6$ or

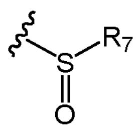
;

each $R_6$ is independently selected from the group consisting of substituted or unsubstituted $C_1$-$C_8$ alkyl, $C_1$-$C_8$ alkoxy, halogen, cyano, nitro, amino, hydroxy, $-C(O)OR_{81}$, $-C(O)R_{81}$,

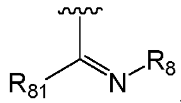
,

cyanomethyl, phosphonyl, sulfonic acid group, sulfonamido, boric acid group, - $NHR_8$ or $-C(O)NHR_8$; alternatively, two $R_6$ linked to the same atom form =O; n is selected from the group consisting of 0, 1, 2, 3, or 4;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

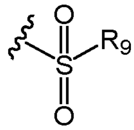
;

$R_7$ and $R_9$ are each independently selected from the group consisting of $C_1$-$C_8$ alkyl and (6-10)-membered aryl;
$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, $-NR_{11}R_{12}$,

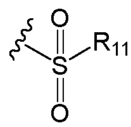
,

and $-SR_{11}$;
provided that $R_6$ is carboxyl, $R_2$ is not selected from the group consisting of substituted or unsubstituted (3-8)-membered cycloalkyl, substituted or unsubstituted (6-10)-membered aryl, and substituted or unsubstituted (5-10)-membered heteroaryl;
$R_{11}$ and $R_{12}$ are each independently selected from the group consisting of H, substituted or unsubstituted $C_1$-$C_8$ alkyl, and amino;
the substituent of the alkyl is selected from the group consisting of deuterium, halogen, hydroxy, cyano, nitro, carboxyl, (4-10)-membered heterocycloalkyl, or $-NR_{13}R_{14}$;
$R_{13}$ and $R_{14}$ are each independently selected from the group consisting of H and $C_1$-$C_8$ alkyl;
the substituent of the cycloalkyl, aryl, heteroaryl or heterocycloalkyl is selected from the group consisting of halogen, hydroxy, amino, carboxyl, nitro, cyano, $-C(O)NR_{16}R_{17}$, $-C(O)OR_{16}$, substituted or unsubstituted $C_1$-$C_8$ alkyl or $C_1$-$C_8$ alkoxy;
$R_{16}$ and $R_{17}$ are each independently selected from the group consisting of H, substituted or unsubstituted $C_1$-$C_8$ alkyl, and hydroxy;
The heteroatoms in the heteroaryl and heterocycloalkyl are selected from the group consisting of N, O, or S, and the number of heteroatoms is selected from the group consisting of 1, 2, or 3;
preferably,
for said $R_1$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;
for said $R_{15}$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;
for said $R_3$, the aryl is phenyl; and the heteroaryl is selected from the group consisting of isoindolinyl, pyridyl, pyrimidyl, pyridazinyl, thienyl, furyl, pyrazolyl, imidazolyl or pyrrolyl; he heterocycloalkyl is piperidyl;
for $R_7$ and $R_9$, the aryl is selected from phenyl or naphthyl;
for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

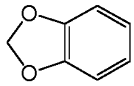;

the substituent of the alkyl is selected from the group consisting of deuterium, halogen, hydroxy, cyano, nitro, carboxyl, piperidyl, morpholinyl or $-NR_{13}R_{14}$;
$R_{13}$ and $R_{14}$ are each independently selected from the group consisting of H and $C_1$-$C_8$ alkyl.

Further, the compound is as represented by formula II-2:

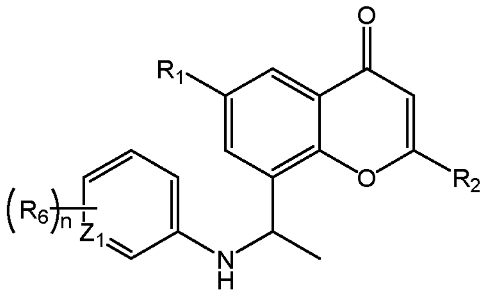

Formula II-2

wherein,

$Z_1$ is selected from CH or N;
$R_1$ is selected from the group consisting of H, substituted or unsubstituted $C_1$-$C_8$ alkyl, $C_1$-$C_8$ alkoxy, halogen, cyano, nitro, hydroxy, carboxyl, $-NHR_{15}$, $-NHR_8$, $-C(O)NHR_8$, $-C(O)NHR_{15}$, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl;
$R_{15}$ is selected from the group consisting of H, substituted or unsubstituted $C_1$-$C_8$ alkyl, $C_1$-$C_8$ alkoxy, halogen, cyano, nitro, hydroxy, carboxyl, substituted or unsubstituted (3-8)-membered cycloalkyl, substituted or unsubstituted (6-10)-membered aryl, substituted or unsubstituted (4-10)-membered heterocycloalkyl, and substituted or unsubstituted (5-10)-membered heteroaryl;
n is selected from the group consisting of 0, 1, 2, 3 or 4;
each $R_6$ is independently selected from the group consisting of substituted or unsubstituted $C_1$-$C_8$ alkyl, $C_1$-$C_8$ alkoxy, halogen, cyano, nitro, amino, hydroxy, $-C(O)OR_{81}$, $-C(O)R_{81}$,

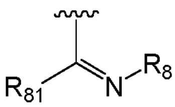,

cyanomethyl, phosphonyl, sulfonic acid group, sulfonamido, boric acid group, - $NHR_8$ or $-C(O)NHR_8$; alternatively, two $R_6$ linked to the same atom form =O;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

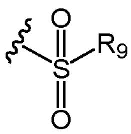;

$R_9$ is each independently selected from the group consisting of $C_1$-$C_8$ alkyl and (6-10)-membered aryl;
$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, $-NR_{11}R_{12}$,

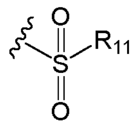

,

and $-SR_{11}$;

provided that $R_6$ is carboxyl, $R_2$ is not selected from the group consisting of substituted or unsubstituted (3-8)-membered cycloalkyl, substituted or unsubstituted (6-10)-membered aryl, and substituted or unsubstituted (5-10)-membered heteroaryl;

$R_{11}$ and $R_{12}$ are each independently selected from the group consisting of H, substituted or unsubstituted $C_1$-$C_8$ alkyl, and amino;

the substituent of the alkyl is selected from the group consisting of deuterium, halogen, hydroxy, cyano, nitro, carboxyl, (4-10)-membered heterocycloalkyl, or $-NR_{13}R_{14}$;

$R_{13}$ and $R_{14}$ are each independently selected from the group consisting of H and $C_1$-$C_8$ alkyl;

the substituent of the cycloalkyl, aryl, heteroaryl or heterocycloalkyl is selected from the group consisting of halogen, hydroxy, amino, carboxyl, nitro, cyano, $-C(O)NR_{16}R_{17}$, $-C(O)OR_{16}$, substituted or unsubstituted $C_1$-$C_8$ alkyl or $C_1$-$C_8$ alkoxy;

$R_{16}$ and $R_{17}$ are each independently selected from the group consisting of H, substituted or unsubstituted $C_1$-$C_8$ alkyl, and hydroxy;

The heteroatoms in the heteroaryl and heterocycloalkyl are selected from the group consisting of N, O, or S, and the number of heteroatoms is selected from the group consisting of 1, 2, or 3;

preferably,

for said $R_1$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;

for said $R_{15}$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;

for $R_9$, the aryl is selected from phenyl or naphthyl;

for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

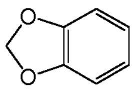

;

the substituent of the alkyl is selected from the group consisting of deuterium, halogen, hydroxy, cyano, nitro, carboxyl, piperidyl, morpholinyl or $-NR_{13}R_{14}$;

$R_{13}$ and $R_{14}$ are each independently selected from the group consisting of H and $C_1$-$C_8$ alkyl.

Further, the compound is as represented by formula II-3:

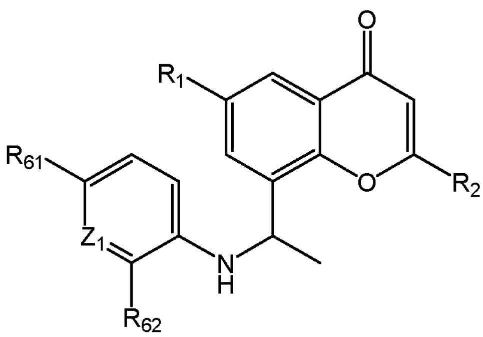

Formula II-3

wherein,

$Z_1$ is selected from CH or N;
$R_1$ is selected from the group consisting of substituted or unsubstituted $C_1$-$C_8$ alkyl and halogen;
$R_{61}$ and $R_{62}$ are each independently selected from the group consisting of H, substituted or unsubstituted $C_1$-$C_8$ alkyl, $C_1$-$C_8$ alkoxy, halogen, cyano, nitro, amino, hydroxy, -C(O)OR$_{81}$, - C(O)R$_{81}$,

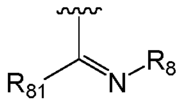
,

cyanomethyl, phosphonyl, sulfonic acid group, sulfonamido, boric acid group, -NHR$_8$ or -C(O)NHR$_8$;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

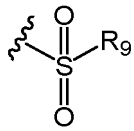
;

$R_9$ is each independently selected from the group consisting of $C_1$-$C_8$ alkyl and (6-10)-membered aryl;
$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, -NR$_{11}$R$_{12}$,

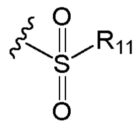
,

and -SR$_{11}$;
provided that $R_6$ is carboxyl, $R_2$ is not selected from the group consisting of substituted or unsubstituted (3-8)-membered cycloalkyl, substituted or unsubstituted (6-10)-membered aryl, and substituted or unsubstituted (5-10)-membered heteroaryl;
$R_{11}$ and $R_{12}$ are each independently selected from the group consisting of H, substituted or unsubstituted $C_1$-$C_8$ alkyl, and amino;
the substituent of the alkyl is selected from the group consisting of deuterium, halogen, hydroxy, cyano, nitro, carboxyl, (4-10)-membered heterocycloalkyl, or -NR$_{13}$R$_{14}$;
$R_{13}$ and $R_{14}$ are each independently selected from the group consisting of H and $C_1$-$C_8$ alkyl;
the substituent of the cycloalkyl, aryl, heteroaryl or heterocycloalkyl is selected from the group consisting of halogen, hydroxy, amino, carboxyl, nitro, cyano, -C(O)NR$_{16}$R$_{17}$, -C(O)OR$_{16}$, substituted or unsubstituted $C_1$-$C_8$ alkyl or $C_1$-$C_8$ alkoxy;
$R_{16}$ and $R_{17}$ are each independently selected from the group consisting of H, substituted or unsubstituted $C_1$-$C_8$ alkyl, and hydroxy;
The heteroatoms in the heteroaryl and heterocycloalkyl are selected from the group consisting of N, O, or S, and the number of heteroatoms is selected from the group consisting of 1, 2, or 3;
preferably,
for said $R_9$, the aryl is selected from phenyl or naphthyl;
for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

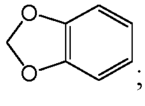
;

the substituent of the alkyl is selected from the group consisting of halogen, hydroxy, cyano, nitro, carboxyl, piperidyl, morpholinyl or -NR$_{13}$R$_{14}$;

$R_{13}$ and $R_{14}$ are each independently selected from the group consisting of H and $C_1$-$C_8$ alkyl.

Further, the compound is as represented by formula II-4:

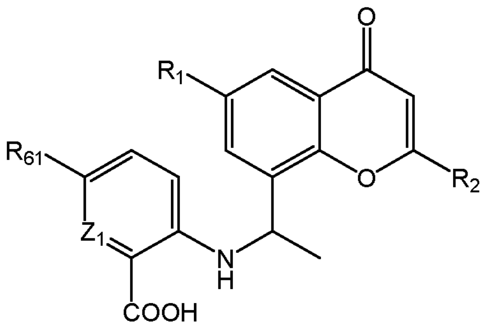

Formula II-4

wherein,

$Z_1$ is selected from CH or N;
$R_1$ is selected from the group consisting of $C_1$-$C_8$ alkyl, halogen, and trifluoromethyl;
$R_{61}$ is selected from the group consisting of H, substituted or unsubstituted $C_1$-$C_8$ alkyl, $C_1$-$C_8$ alkoxy, halogen, cyano, nitro, amino, hydroxy, -C(O)OR$_{81}$, -C(O)R$_{81}$,

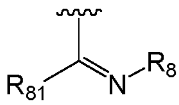

,

cyanomethyl, phosphonyl, sulfonic acid group, sulfonamido, boric acid group, -NHR$_8$ or - C(O)NHR$_8$;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

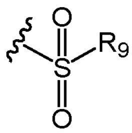

;

$R_9$ is each independently selected from the group consisting of $C_1$-$C_8$ alkyl and (6-10)-membered aryl;
$R_2$ is selected from the group consisting of substituted or unsubstituted $C_1$-$C_8$ alkyl, substituted or unsubstituted (4-10)-membered cycloalkyl, -NR$_{11}$R$_{12}$,

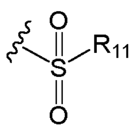

,

and -SR$_{11}$;
$R_{11}$ and $R_{12}$ are each independently selected from the group consisting of H, substituted or unsubstituted $C_1$-$C_8$ alkyl, and amino;
the substituent of the alkyl is selected from the group consisting of deuterium, halogen, hydroxy, cyano, nitro, carboxyl, (4-10)-membered heterocycloalkyl, or -NR$_{13}$R$_{14}$;
$R_{13}$ and $R_{14}$ are each independently selected from the group consisting of H and $C_1$-$C_8$ alkyl;
the substituent of the cycloalkyl, aryl, heteroaryl or heterocycloalkyl is selected from the group consisting of halogen, hydroxy, amino, carboxyl, nitro, cyano, -C(O)NR$_{16}$R$_{17}$, -C(O)OR$_{16}$, substituted or unsubstituted $C_1$-$C_8$ alkyl or $C_1$-$C_8$ alkoxy;
$R_{16}$ and $R_{17}$ are each independently selected from the group consisting of H, substituted or unsubstituted $C_1$-$C_8$ alkyl,

and hydroxy;

The heteroatoms in the heteroaryl and heterocycloalkyl are selected from the group consisting of N, O, or S, and the number of heteroatoms is selected from the group consisting of 1, 2, or 3;

preferably,

for said $R_9$, the aryl is selected from phenyl or naphthyl;

for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

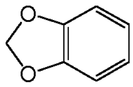

;

the substituent of the alkyl is selected from the group consisting of halogen, hydroxy, cyano, nitro, carboxyl, piperidyl, morpholinyl or $-NR_{13}R_{14}$;

$R_{13}$ and $R_{14}$ are each independently selected from the group consisting of H and $C_1$-$C_8$ alkyl.

Further, the compound is as represented by formula II-5:

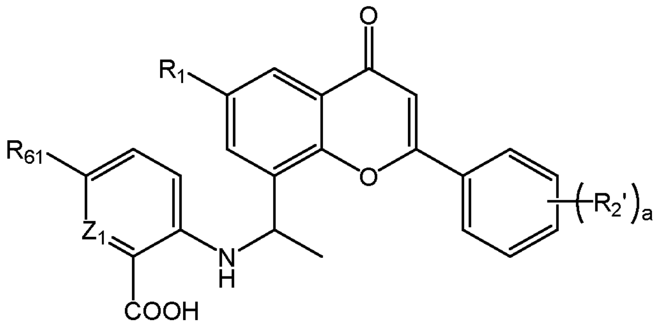

Formula II-5

wherein,

$Z_1$ is selected from CH or N;

$R_1$ is selected from the group consisting of $C_1$-$C_8$ alkyl, halogen, and trifluoromethyl;

$R_{61}$ is selected from the group consisting of H, substituted or unsubstituted $C_1$-$C_8$ alkyl, $C_1$-$C_8$ alkoxy, halogen, cyano, nitro, amino, hydroxy, $-C(O)OR_{81}$, $-C(O)R_{81}$,

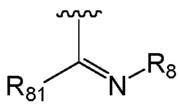

,

cyanomethyl, phosphonyl, sulfonic acid group, sulfonamido, boric acid group, $-NHR_8$ or $-C(O)NHR_8$;

$R_{81}$ is selected from the group consisting of H, substituted or unsubstituted $C_1$-$C_8$ alkyl, and amino;

$R_8$ is selected from the group consisting of substituted or unsubstituted $C_1$-$C_8$ alkyl, hydroxy, and

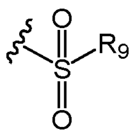

;

$R_9$ is each independently selected from the group consisting of $C_1$-$C_8$ alkyl and phenyl;

$R_2$' is a substituent of benzene ring, and the number of substituents is a; each $R_2$' is independently selected from halogen, hydroxy, amino, carboxyl, nitro, cyano, $-C(O)NR_{16}R_{17}$, $-C(O)OR_{16}$, substituted or unsubstituted $C_1$-$C_8$ alkyl or $C_1$-$C_8$ alkoxy; a is selected from the group consisting of 0, 1, 2, or 3;

$R_{16}$ and $R_{17}$ are each independently selected from the group consisting of H, substituted or unsubstituted $C_1$-$C_8$ alkyl,

and hydroxy;
the substituent of the alkyl is selected from the group consisting of deuterium, halogen, hydroxy, cyano, nitro, carboxyl, piperidyl, morpholinyl or $-NR_{13}R_{14}$;
$R_{13}$ and $R_{14}$ are each independently selected from the group consisting of H and $C_1$-$C_8$ alkyl.

Further, the compound is as represented by formula III-1:

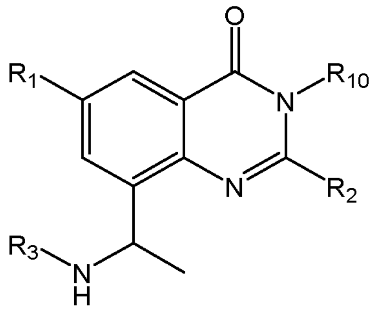

Formula III-1

wherein,

$R_1$ is selected from the group consisting of substituted or unsubstituted $C_1$-$C_8$ alkyl, $C_1$-$C_8$ alkoxy, halogen, cyano, nitro, amino, hydroxy or carboxyl;
$R_3$ is selected from the group consisting of (6-10)-membered aryl substituted with n $R_6$, (5-10)-membered heteroaryl substituted with n $R_6$, (4-10)-membered heterocycloalkyl substituted with n $R_6$ or

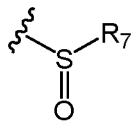

;

each $R_6$ is independently selected from the group consisting of substituted or unsubstituted $C_1$-$C_8$ alkyl, $C_1$-$C_8$ alkoxy, halogen, cyano, nitro, amino, hydroxy, $-C(O)OR_{81}$, $-C(O)R_{81}$,

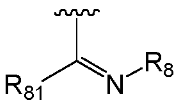

,

cyanomethyl, phosphonyl, sulfonic acid group, sulfonamido, boric acid group, - $NHR_8$ or $-C(O)NHR_8$; alternatively, two $R_6$ linked to the same atom form =O; n is selected from the group consisting of 0, 1, 2, 3, or 4;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

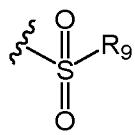

;

$R_7$ and $R_9$ are each independently selected from the group consisting of $C_1$-$C_8$ alkyl and (6-10)-membered aryl;
$R_{10}$ is selected from the group consisting of H as well as substituted or unsubstituted $C_1$-$C_8$ alkyl;
$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, $-NR_{11}R_{12}$,

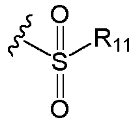

and $-SR_{11}$;

$R_{11}$ and $R_{12}$ are each independently selected from the group consisting of H, substituted or unsubstituted $C_1$-$C_8$ alkyl, and amino;

the substituent of the alkyl is selected from the group consisting of deuterium, halogen, hydroxy, cyano, nitro, carboxyl, (4-10)-membered heterocycloalkyl, or $-NR_{13}R_{14}$;

$R_{13}$ and $R_{14}$ are each independently selected from the group consisting of H and $C_1$-$C_8$ alkyl;

the substituent of the cycloalkyl, aryl, heteroaryl or heterocycloalkyl is selected from the group consisting of halogen, hydroxy, amino, carboxyl, nitro, cyano, $-C(O)NR_{16}R_{17}$, $-C(O)OR_{16}$, substituted or unsubstituted $C_1$-$C_8$ alkyl or $C_1$-$C_8$ alkoxy;

$R_{16}$ and $R_{17}$ are each independently selected from the group consisting of H, substituted or unsubstituted $C_1$-$C_8$ alkyl, and hydroxy;

The heteroatoms in the heteroaryl and heterocycloalkyl are selected from the group consisting of N, O, or S, and the number of heteroatoms is selected from the group consisting of 1, 2, or 3.
preferably,

for said $R_3$, the aryl is phenyl; and the heteroaryl is selected from the group consisting of isoindolinyl, pyridyl, pyrimidyl, pyridazinyl, thienyl, furyl, pyrazolyl, imidazolyl or pyrrolyl; the heterocycloalkyl is piperidyl;

for $R_7$ and $R_9$, the aryl is selected from phenyl or naphthyl;

for $R_2$, the aryl is selected from phenyl, anthranyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

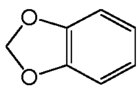
;

the substituent of the alkyl is selected from the group consisting of deuterium, halogen, hydroxy, cyano, nitro, carboxyl, piperidyl, morpholinyl or $-NR_{13}R_{14}$;

$R_{13}$ and $R_{14}$ are each independently selected from the group consisting of H and $C_1$-$C_8$ alkyl.

Further, the compound is as represented by formula III-2:

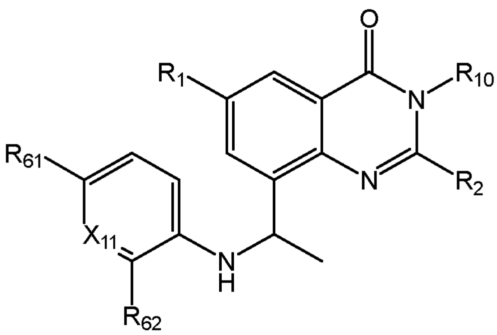

Formula III-2

wherein,

$X_{11}$ is selected from CH or N;

$R_1$ is selected from the group consisting of substituted or unsubstituted $C_1$-$C_8$ alkyl, $C_1$-$C_8$ alkoxy, halogen, cyano, nitro, amino, hydroxy or carboxyl;

$R_{61}$ and $R_{62}$ are each independently selected from the group consisting of H, substituted or unsubstituted $C_1$-$C_8$ alkyl,

$C_1$-$C_8$ alkoxy, halogen, cyano, nitro, amino, hydroxy, -C(O)OR$_{81}$, - C(O)R$_{81}$,

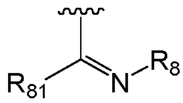

,

cyanomethyl, phosphonyl, sulfonic acid group, sulfonamido, boric acid group, -NHR$_8$ or -C(O)NHR$_8$;
R$_{81}$ is selected from the group consisting of H, substituted or unsubstituted $C_1$-$C_8$ alkyl, and amino;
R$_8$ is selected from the group consisting of substituted or unsubstituted $C_1$-$C_8$ alkyl, hydroxy, and

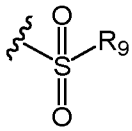

;

R$_9$ is selected from the group consisting of $C_1$-$C_8$ alkyl and (6-10)-membered aryl;
R$_{10}$ is selected from the group consisting of H as well as substituted or unsubstituted $C_1$-$C_8$ alkyl;
R$_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, -NR$_{11}$R$_{12}$,

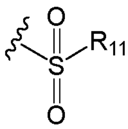

,

and -SR$_{11}$;
R$_{11}$ and R$_{12}$ are each independently selected from the group consisting of H, substituted or unsubstituted $C_1$-$C_8$ alkyl, and amino;
the substituent of the alkyl is selected from the group consisting of deuterium, halogen, hydroxy, cyano, nitro, carboxyl, (4-10)-membered heterocycloalkyl, or -NR$_{13}$R$_{14}$;
R$_{13}$ and R$_{14}$ are each independently selected from the group consisting of H and $C_1$-$C_8$ alkyl;
the substituent of the cycloalkyl, aryl, heteroaryl or heterocycloalkyl is selected from the group consisting of halogen, hydroxy, amino, carboxyl, nitro, cyano, -C(O)NR$_{16}$R$_{17}$, -C(O)OR$_{16}$, substituted or unsubstituted $C_1$-$C_8$ alkyl or $C_1$-$C_8$ alkoxy;
R$_{16}$ and R$_{17}$ are each independently selected from the group consisting of H, substituted or unsubstituted $C_1$-$C_8$ alkyl, and hydroxy;
the heteroatoms in the heteroaryl and heterocycloalkyl are selected from the group consisting of N, O, or S, and the number of heteroatoms is selected from the group consisting of 1, 2, or 3.
preferably,
for R$_9$, the aryl is selected from phenyl or naphthyl;
for R$_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

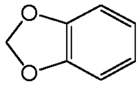

;

the substituent of the alkyl is selected from the group consisting of deuterium, halogen, hydroxy, cyano, nitro, carboxyl, piperidyl, morpholinyl or -NR$_{13}$R$_{14}$;
R$_{13}$ and R$_{14}$ are each independently selected from the group consisting of H and $C_1$-$C_8$ alkyl.

Further, the compound is as represented by formula III-3:

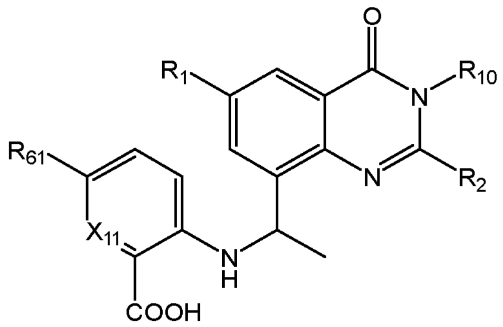

Formula III-3

wherein,

$X_{11}$ is selected from $CR_{71}$ or N;
$R_{71}$ is selected from the group consisting of H, halogen, and $C_1$-$C_8$ alkyl;
$R_1$ is selected from the group consisting of substituted or unsubstituted $C_1$-$C_8$ alkyl, $C_1$-$C_8$ alkoxy, halogen, cyano, nitro, amino, hydroxy or carboxyl;
$R_{61}$ is selected from the group consisting of H, substituted or unsubstituted $C_1$-$C_8$ alkyl, $C_1$-$C_8$ alkoxy, halogen, cyano, nitro, amino, hydroxy, -C(O)O$R_{81}$, -C(O)$R_{81}$,

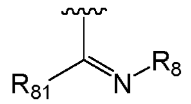

,

cyanomethyl, phosphonyl, sulfonic acid group, sulfonamido, boric acid group, -NH$R_8$ or - C(O)NH$R_8$;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

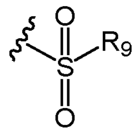

;

$R_9$ is selected from the group consisting of $C_1$-$C_8$ alkyl and (6-10)-membered aryl;
$R_{10}$ is selected from the group consisting of H as well as substituted or unsubstituted $C_1$-$C_8$ alkyl;
$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, -N$R_{11}R_{12}$,

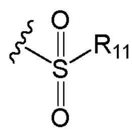

,

and -S$R_{11}$;
$R_{11}$ and $R_{12}$ are each independently selected from the group consisting of H, substituted or unsubstituted $C_1$-$C_8$ alkyl, and amino;
the substituent of the alkyl is selected from the group consisting of deuterium, halogen, hydroxy, cyano, nitro, (4-10)-membered heterocycloalkyl, or -N$R_{13}R_{14}$;
$R_{13}$ and $R_{14}$ are each independently selected from the group consisting of H and $C_1$-$C_8$ alkyl;
the substituent of the cycloalkyl, aryl, heteroaryl or heterocycloalkyl is selected from the group consisting of halogen, hydroxy, amino, carboxyl, nitro, cyano, -C(O)N$R_{16}R_{17}$, -C(O)O$R_{16}$, substituted or unsubstituted $C_1$-$C_8$ alkyl or $C_1$-$C_8$ alkoxy;
$R_{16}$ and $R_{17}$ are each independently selected from the group consisting of H, substituted or unsubstituted $C_1$-$C_8$ alkyl,

and hydroxy;

the heteroatoms in the heteroaryl and heterocycloalkyl are selected from the group consisting of N, O, or S, and the number of heteroatoms is selected from the group consisting of 1, 2, or 3.

preferably,

for $R_9$, the aryl is selected from phenyl or naphthyl;

for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

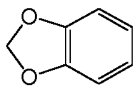

;

the substituent of the alkyl is selected from the group consisting of halogen, hydroxy, cyano, nitro, carboxyl, piperidyl, morpholinyl or $-NR_{13}R_{14}$;

$R_{13}$ and $R_{14}$ are each independently selected from the group consisting of H and $C_1$-$C_8$ alkyl.

Further, the compound is as represented by formula III-4:

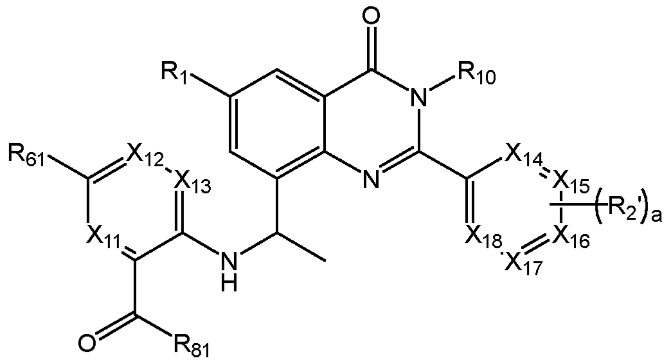

Formula III-4

wherein,

$X_{11}$, $X_{12}$, $X_{13}$, $X_{14}$, $X_{15}$, $X_{16}$, $X_{17}$, and $X_{18}$ are each independently selected from the group consisting of $CR_{71}$ or N;

$R_{61}$ is selected from the group consisting of H, substituted or unsubstituted $C_1$-$C_8$ alkyl, $C_1$-$C_8$ alkoxy, halogen, cyano, nitro, amino, hydroxy, carboxyl, cyanomethyl, phosphonyl, sulfonic acid group, sulfonamido, boric acid group, $-NHR_8$ or $-C(O)NHR_8$;

$R_{71}$ is selected from the group consisting of H, halogen, and $C_1$-$C_8$ alkyl

$R_{81}$ is selected from the group consisting of hydroxy, $C_1$-$C_8$ alkoxy, and amino;

$R_8$ is selected from the group consisting of hydroxy and

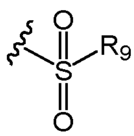

;

$R_9$ is selected from the group consisting of $C_1$-$C_8$ alkyl and phenyl;

$R_1$ is selected from the group consisting of substituted or unsubstituted $C_1$-$C_8$ alkyl, $C_1$-$C_8$ alkoxy, halogen, cyano, nitro, amino, hydroxy or carboxyl;

$R_{10}$ is selected from the group consisting of H as well as substituted or unsubstituted $C_1$-$C_8$ alkyl;

$R_2$' is a ring substituent, and the number of substituents is a; each $R_2$' is independently selected from the group consisting of halogen, hydroxy, amino, carboxyl, nitro, cyano, - $C(O)NR_{16}R_{17}$, $-C(O)OR_{16}$, substituted or unsubstituted $C_1$-$C_8$ alkyl or $C_1$-$C_8$ alkoxy; a is selected from the group consisting of 0, 1, 2, or 3;

$R_{16}$ and $R_{17}$ are each independently selected from the group consisting of H and $C_1$-$C_8$ alkyl;

the substituent of the alkyl is selected from the group consisting of deuterium, halogen, hydroxy, cyano, nitro, carboxyl,

piperidyl, morpholinyl or $-NR_{13}R_{14}$;
$R_{13}$ and $R_{14}$ are each independently selected from the group consisting of H and $C_1$-$C_8$ alkyl.

Further, the compound is as represented by formula III-5:

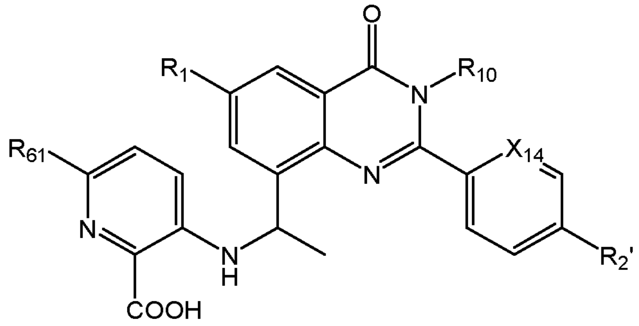

Formula III-5

wherein,

$X_{14}$ is selected from CH or N;
$R_{61}$ is selected from the group consisting of H, substituted or unsubstituted $C_1$-$C_8$ alkyl, $C_1$-$C_8$ alkoxy, halogen, cyano, nitro, amino, hydroxy, carboxyl, cyanomethyl, phosphonyl, sulfonic acid group, sulfonamido, boric acid group, $-NHR_8$ or $-C(O)NHR_8$;
$R_8$ is selected from the group consisting of hydroxy and

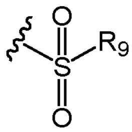

;

$R_9$ is selected from the group consisting of $C_1$-$C_8$ alkyl and phenyl;
$R_1$ is selected from the group consisting of substituted or unsubstituted $C_1$-$C_8$ alkyl, $C_1$-$C_8$ alkoxy, halogen, cyano, nitro, amino, hydroxy or carboxyl;
$R_{10}$ is selected from the group consisting of H as well as substituted or unsubstituted $C_1$-$C_8$ alkyl;
$R_2$' is selected from the group consisting of halogen, hydroxy, amino, carboxyl, nitro, cyano, $C_1$-$C_8$ alkyl or $C_1$-$C_8$ alkoxy; a is selected from the group consisting of 0, 1, 2, or 3;
the substituent of the alkyl is selected from the group consisting of halogen, hydroxy, cyano, nitro, carboxyl, piperidyl, morpholinyl or $-NR_{13}R_{14}$;
$R_{13}$ and $R_{14}$ are each independently selected from the group consisting of H and $C_1$-$C_8$ alkyl.

Further, the compound is as represented by formula III-6:

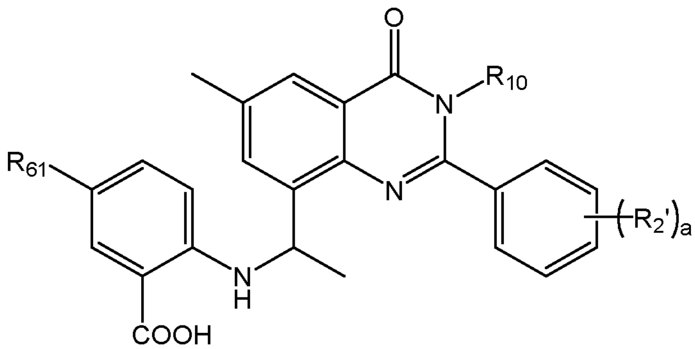

Formula III-6

wherein,

$R_{61}$ is selected from the group consisting of H, substituted or unsubstituted $C_1$-$C_8$ alkyl, $C_1$-$C_8$ alkoxy, halogen, cyano, nitro, amino, hydroxy, carboxyl, cyanomethyl, phosphonyl, sulfonic acid group, sulfonamido, boric acid group, -NHR$_8$ or -C(O)NHR$_8$;

$R_8$ is selected from the group consisting of hydroxy and

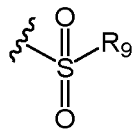

;

$R_9$ is selected from the group consisting of $C_1$-$C_8$ alkyl and phenyl;

$R_{10}$ is selected from the group consisting of H as well as substituted or unsubstituted $C_1$-$C_8$ alkyl;

$R_2$' is a substituent of benzene ring, and the number of substituents is a; each $R_2$' is independently selected from the group consisting of halogen, hydroxy, amino, carboxyl, nitro, cyano, $C_1$-$C_8$ alkyl or $C_1$-$C_8$ alkoxy; a is selected from the group consisting of 0, 1, 2, or 3;

the substituent of the alkyl is selected from the group consisting of halogen, hydroxy, cyano, nitro, carboxyl, piperidyl, morpholinyl or -NR$_{13}$R$_{14}$;

$R_{13}$ and $R_{14}$ are each independently selected from the group consisting of H and $C_1$-$C_8$ alkyl.

Further, the compound is as represented by formula III-7:

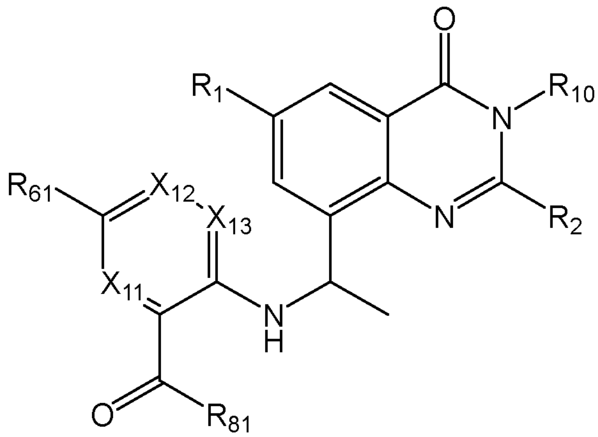

Formula III-7

wherein,

$X_{11}$, $X_{12}$, and $X_{13}$ are each independently selected from the group consisting of CR$_{71}$ or N;

$R_{61}$ is selected from the group consisting of H, substituted or unsubstituted $C_1$-$C_8$ alkyl, $C_1$-$C_8$ alkoxy, halogen, cyano, nitro, amino, hydroxy, carboxyl, cyanomethyl, phosphonyl, sulfonic acid group, sulfonamido, boric acid group, -NHR$_8$ or -C(O)NHR$_8$;

$R_{71}$ is selected from the group consisting of H, halogen, and $C_1$-$C_8$ alkyl

$R_{81}$ is selected from the group consisting of hydroxy, $C_1$-$C_8$ alkoxy, and amino;

$R_8$ is selected from the group consisting of hydroxy and

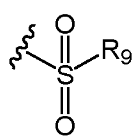

;

$R_9$ is selected from the group consisting of $C_1$-$C_8$ alkyl and phenyl;

$R_1$ is selected from the group consisting of substituted or unsubstituted $C_1$-$C_8$ alkyl, $C_1$-$C_8$ alkoxy, halogen, cyano, nitro, amino, hydroxy or carboxyl;

$R_{10}$ is selected from the group consisting of H as well as substituted or unsubstituted $C_1$-$C_8$ alkyl;
$R_2$ is selected from the group consisting of

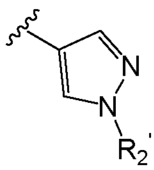, 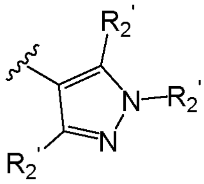, 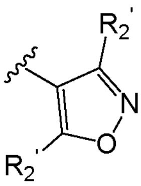,

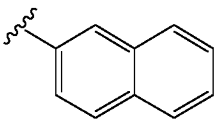, 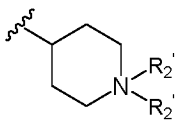, 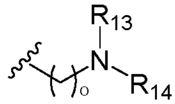, 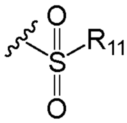, and 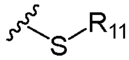;

$R_{11}$ is each independently selected from the group consisting of H as well as substituted or unsubstituted $C_1$-$C_8$ alkyl;
each $R_2$' is independently selected from the group consisting of halogen, hydroxy, amino, carboxyl, nitro, cyano, -C(O)$NR_{15}R_{16}$, substituted or unsubstituted $C_1$-$C_8$ alkyl or $C_1$-$C_8$ alkoxy; a is selected from the group consisting of 0, 1, 2, or 3;
O is an integer selected from 1 to 3;
$R_{15}$ and $R_{16}$ are each independently selected from the group consisting of H and $C_1$-$C_8$ alkyl;
the substituent of the alkyl is selected from the group consisting of deuterium, halogen, hydroxy, cyano, nitro, carboxyl, piperidyl, morpholinyl or -$NR_{13}R_{14}$;
$R_{13}$ and $R_{14}$ are each independently selected from the group consisting of H and $C_1$-$C_8$ alkyl; alternatively, $R_{13}$ and $R_{14}$ are linked to form

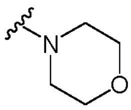.

Further, the compound is as represented by formula III-8:

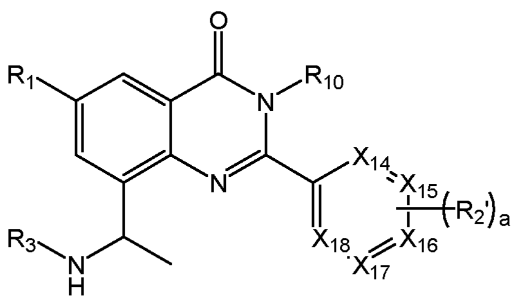

Formula III-8

wherein,

$X_{14}$, $X_{15}$, $X_{16}$, $X_{17}$, and $X_{18}$ are each independently selected from the group consisting of $CR_{71}$ or N;
$R_{71}$ is selected from the group consisting of H, halogen, and $C_1$-$C_8$ alkyl
$R_1$ is selected from the group consisting of substituted or unsubstituted $C_1$-$C_8$ alkyl, $C_1$-$C_8$ alkoxy, halogen, cyano, nitro, amino, hydroxy or carboxyl;
$R_{10}$ is selected from the group consisting of H as well as substituted or unsubstituted $C_1$-$C_8$ alkyl;
$R_2$' is a ring substituent, and the number of substituents is a; each $R_2$' is independently selected from the group consisting of halogen, hydroxy, amino, carboxyl, nitro, cyano, - C(O)$NR_{15}R_{16}$, substituted or unsubstituted $C_1$-$C_8$ alkyl or $C_1$-$C_8$ alkoxy; a is selected from the group consisting of 0, 1, 2, or 3;
$R_{15}$ and $R_{16}$ are each independently selected from the group consisting of H and $C_1$-$C_8$ alkyl;
$R_3$ is selected from the group consisting of

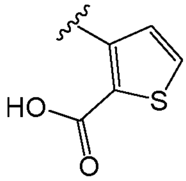, 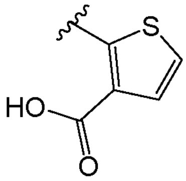, 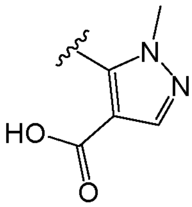,

($n \times R_6$)-substituted phenyl, ($n \times R_6$)-substituted pyridyl, ($n \times R_6$)-substituted pyrimidyl, ($n \times R_6$)-substituted pyridazinyl, ($n \times R_6$)-substituted thienyl, ($n \times R_6$)-substituted furyl, ($n \times R_6$)-substituted pyrazolyl, ($n \times R_6$)-substituted imidazolyl or ($n \times R_6$)-substituted pyrrolyl;

each $R_6$ is independently selected from the group consisting of substituted or unsubstituted $C_1$-$C_8$ alkyl, $C_1$-$C_8$ alkoxy, halogen, cyano, nitro, amino, hydroxy, -C(O)O$R_{81}$, -C(O)$R_{81}$,

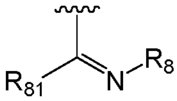,

cyanomethyl, phosphonyl, sulfonic acid group, sulfonamido, boric acid group, - NH$R_8$ or -C(O)NH$R_8$; alternatively, two $R_6$ linked to the same atom form =O; n is selected from the group consisting of 0, 1, 2, 3, or 4;

$R_{81}$ is selected from the group consisting of H, substituted or unsubstituted $C_1$-$C_8$ alkyl, and amino;

$R_8$ is selected from the group consisting of substituted or unsubstituted $C_1$-$C_8$ alkyl, hydroxy, and

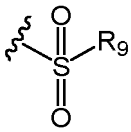;

$R_9$ is selected from the group consisting of $C_1$-$C_8$ alkyl and (6-10)-membered aryl;

the substituent of the alkyl is selected from the group consisting of deuterium, halogen, hydroxy, cyano, nitro, carboxyl, piperidyl, morpholinyl or -N$R_{13}R_{14}$;

$R_{13}$ and $R_{14}$ are each independently selected from the group consisting of H and $C_1$-$C_8$ alkyl.

Further, the compound is as represented by formula IV:

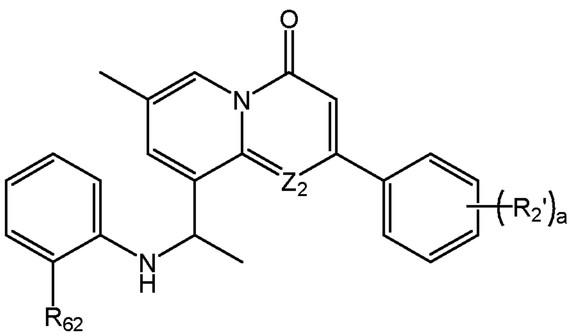

Formula IV

wherein,

$Z_2$ is selected from CH or N;

$R_{62}$ is selected from the group consisting of H, substituted or unsubstituted $C_1$-$C_8$ alkyl, $C_1$-$C_8$ alkoxy, halogen, cyano, nitro, amino, hydroxy, carboxyl, cyanomethyl, phosphonyl, sulfonic acid group, sulfonamido, boric acid group, -NH$R_8$ or -C(O)NH$R_8$;

$R_8$ is selected from the group consisting of hydroxy and

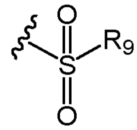

;

$R_9$ is selected from the group consisting of $C_1$-$C_8$ alkyl and phenyl;

$R_2$' is a ring substituent, and the number of substituents is a; each $R_2$' is independently selected from the group consisting of halogen, hydroxy, amino, carboxyl, nitro, cyano, $C_1$-$C_8$ alkyl or $C_1$-$C_8$ alkoxy; a is selected from the group consisting of 0, 1, 2, or 3;

the substituent of the alkyl is selected from the group consisting of halogen, hydroxy, cyano, nitro, carboxyl, piperidyl, morpholinyl or $-NR_{13}R_{14}$;

$R_{13}$ and $R_{14}$ are each independently selected from the group consisting of H and $C_1$-$C_8$ alkyl.

Further, the compound is selected from the group consisting of:

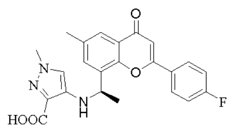 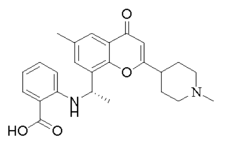 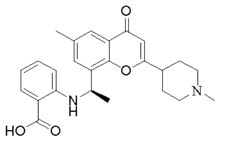 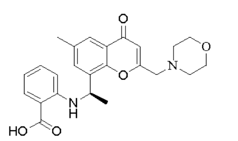 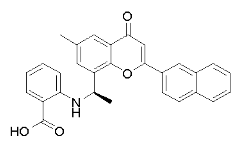

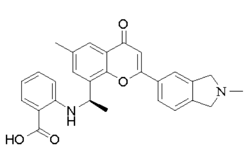 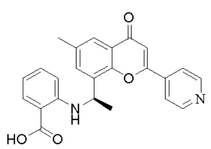 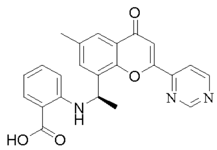 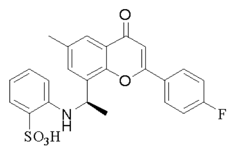 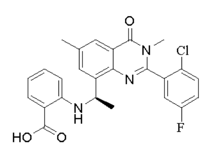

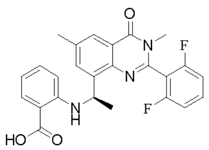 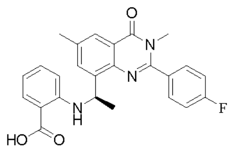 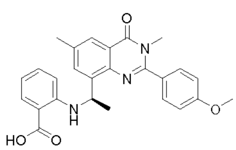 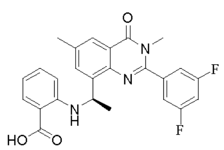 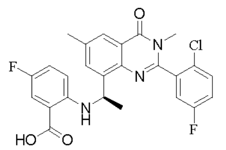

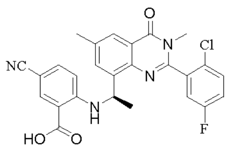 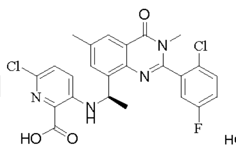 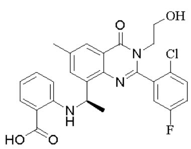 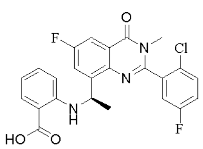 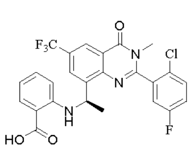 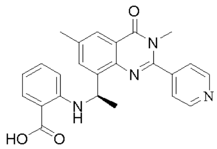

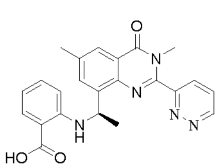 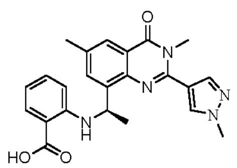 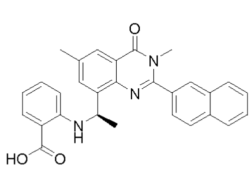 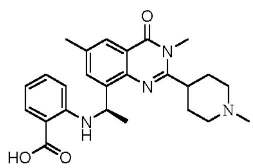 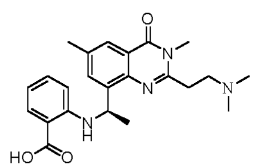

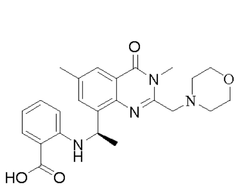 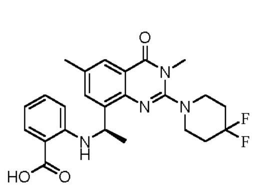 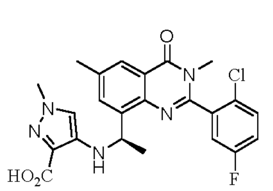 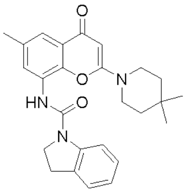 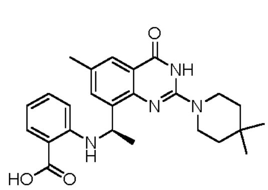

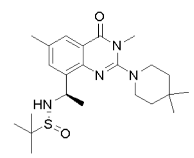 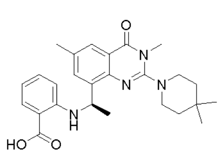 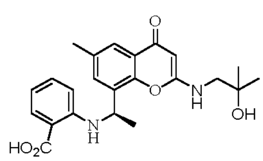 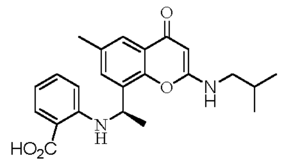 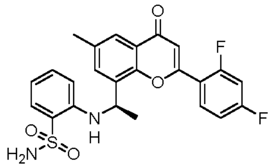

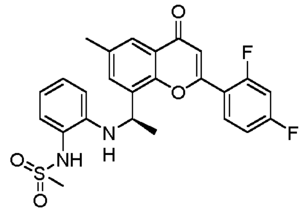 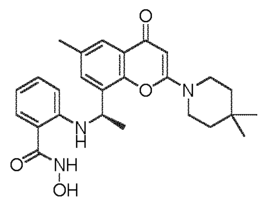 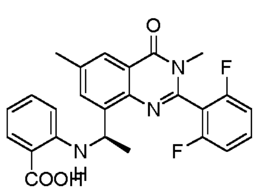 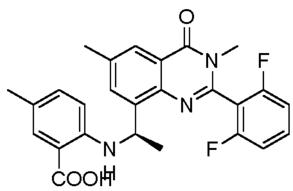

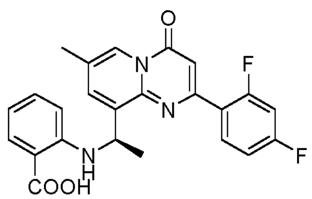

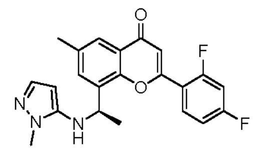 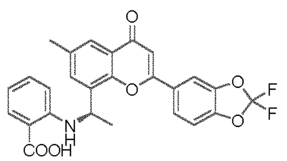 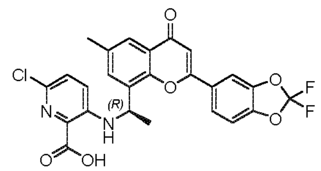 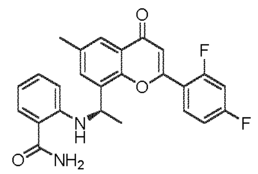

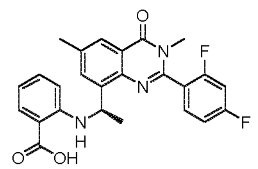 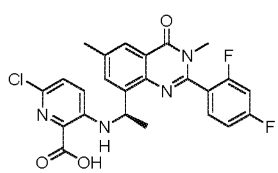 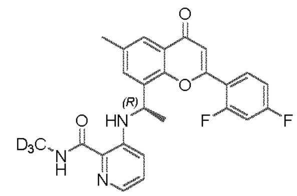 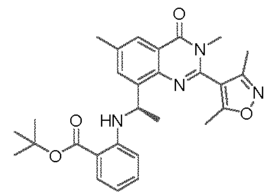

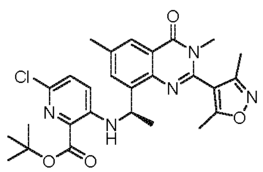 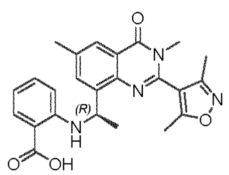 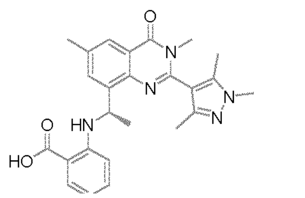 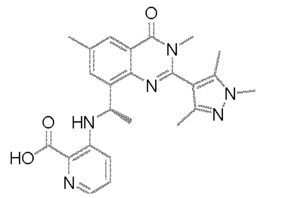

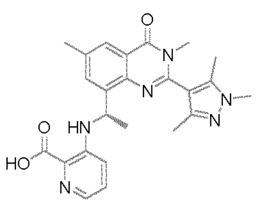 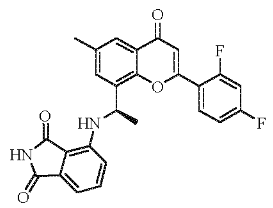 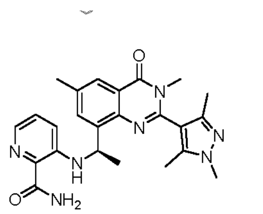 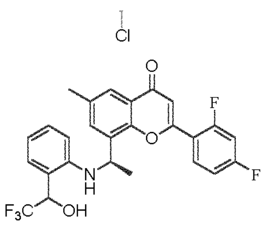

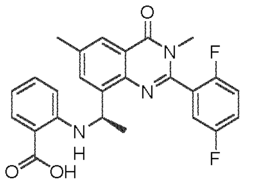
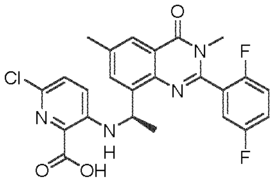
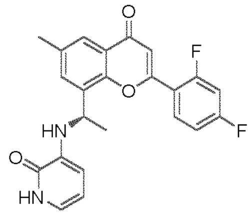
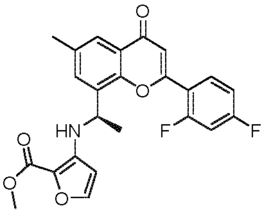

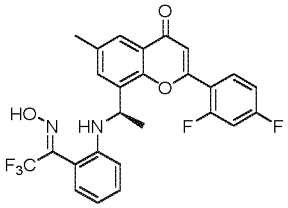
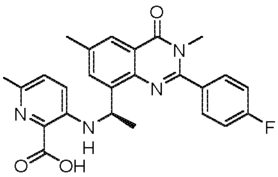
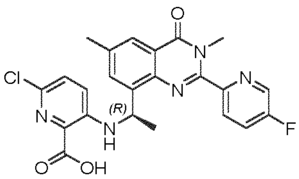
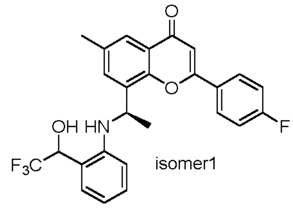
isomer1

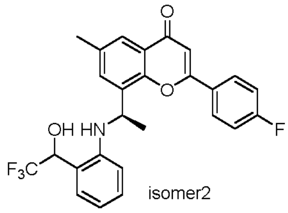
isomer2
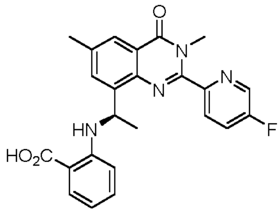
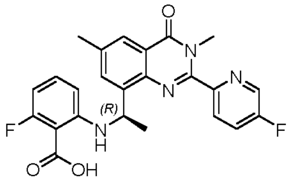
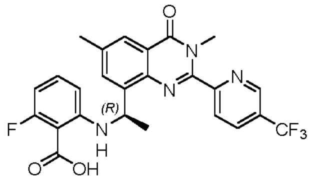

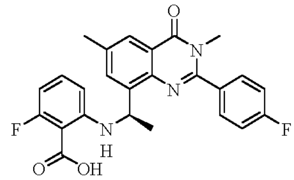
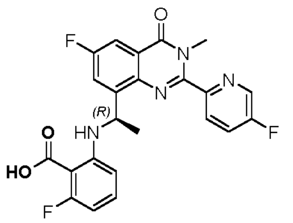
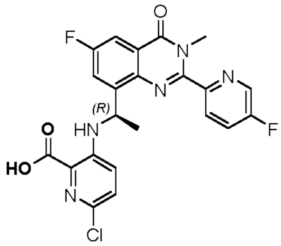
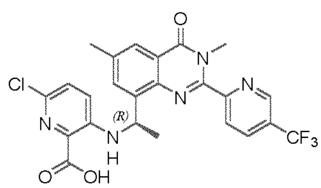

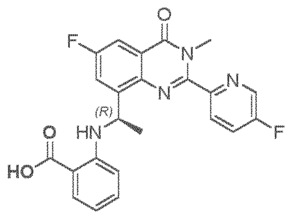
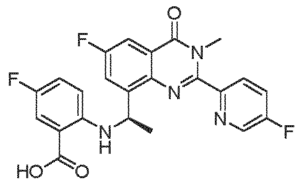
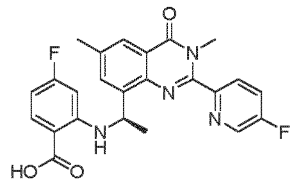
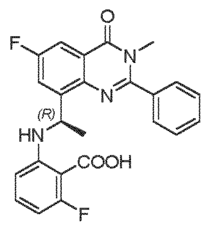

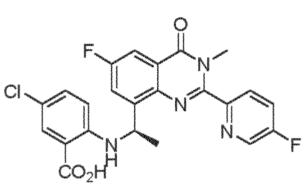
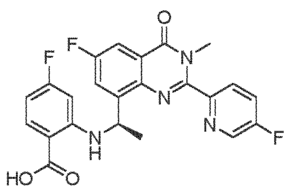
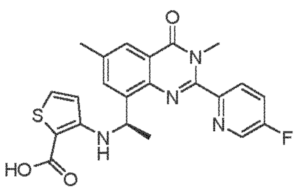
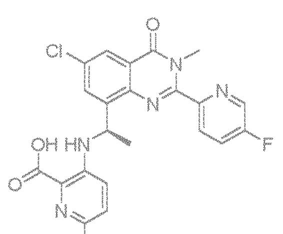

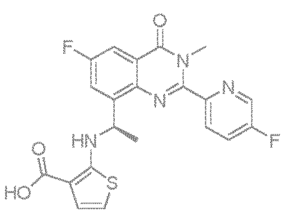
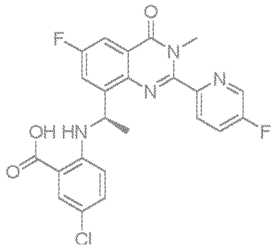
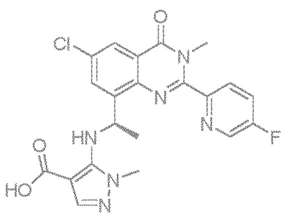
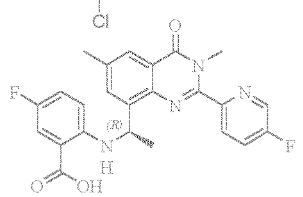

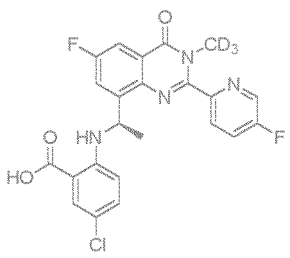
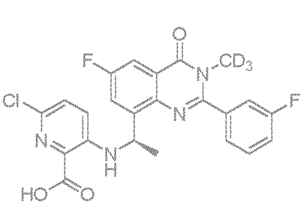
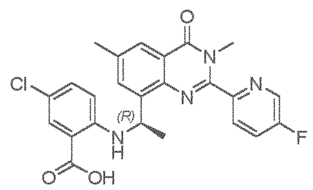
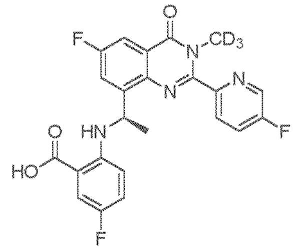
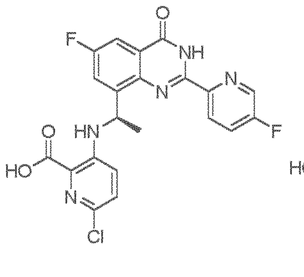
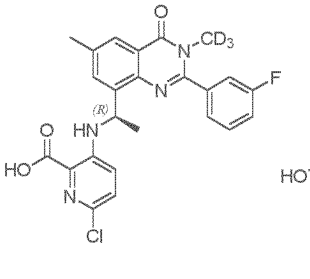
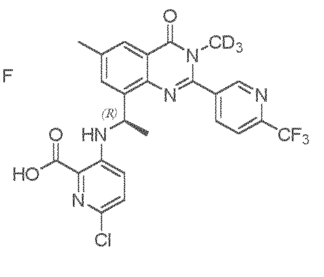
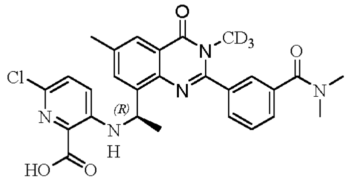
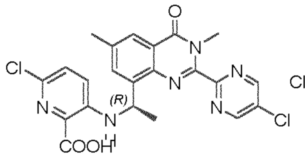
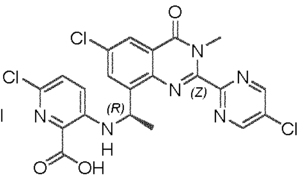
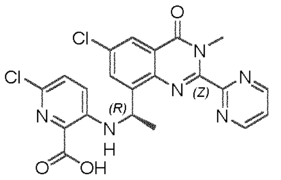
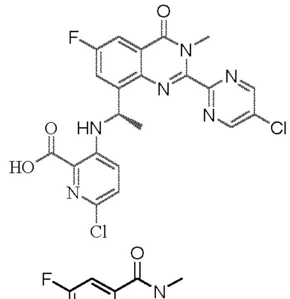
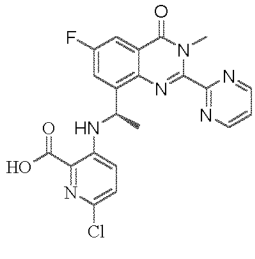
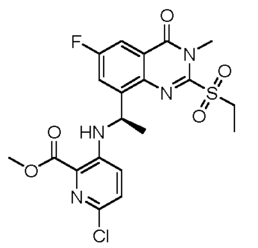
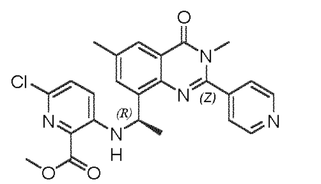
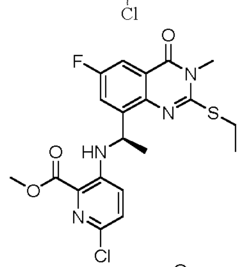
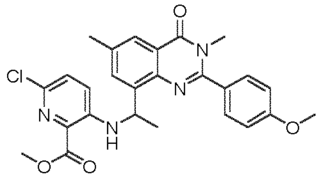
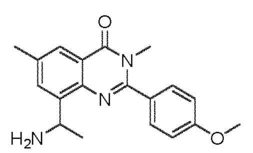
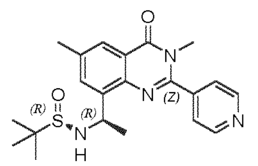
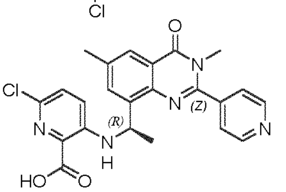
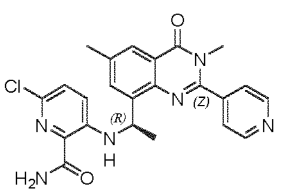
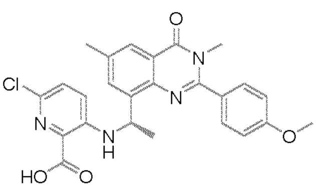
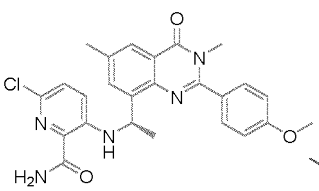
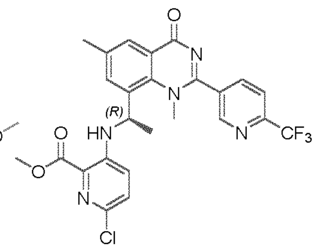
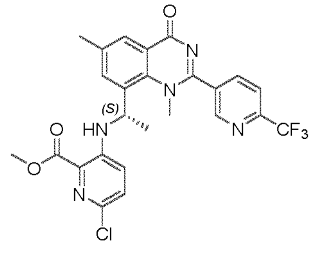

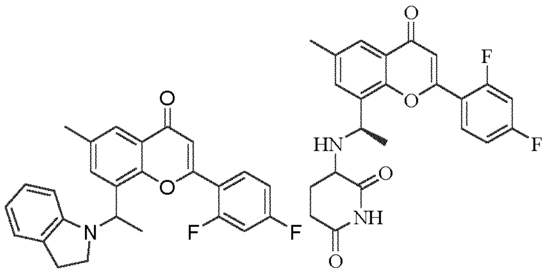

.

The present invention also provides the use of above compounds, or a salt thereof, or a stereoisomer thereof, or a solvate thereof, or a hydrate thereof, or a prodrug thereof, or a deuterated compound thereof in the manufacture of PI3K inhibitors;

preferably, the PI3K inhibitor is a selective PI3K inhibitor.

The present invention also provides the use of above compounds, or a salt thereof, or a stereoisomer thereof, or a solvate thereof, or a hydrate thereof, or a prodrug thereof, or a deuterated compound thereof in the manufacture of medicaments for the prevention and/or treatment of diseases associated with PI3K.

Further, the disease is cancer, inflammation, or cardiovascular diseases related to PI3K;

preferably, the cancers are breast cancer, colorectal cancer, gastric cancer, colon cancer, rectal cancer, ovarian cancer, and prostate cancer.

The present invention also provides a medicament, which is prepared from the above compound, or a salt thereof, or a stereoisomer thereof, or a solvate thereof, or a hydrate thereof, or a prodrug thereof, or a deuterated compound thereof as the active ingredient, in combination with pharmaceutically acceptable excipients or auxiliary ingredients.

The compounds and derivatives provided in the present invention can be named according to IUPAC (International Union of Pure and Applied Chemistry) or CAS (Chemical Abstracting Service, Columbus, OH) naming system.

For the definition of terms used in the present invention: unless defined otherwise, the initial definition provided for the group or term herein applies to the group or term of the whole specification; for the terms that are not specifically defined herein, they should have the same meaning as commonly understood by one of ordinary skill in the art to which the disclosure belongs.

"Substitution" means that the hydrogen in a molecule is substituted with other different atoms or molecules.

The minimum and maximum contents of carbon atoms in hydrocarbon groups are represented by prefixes, for example, the prefix $C_a$-$C_b$ alkyl indicates any alkyl containing "a"-"b" carbon atoms. Therefore, for example, "$C_1$-$C_8$ alkyl" refers to an alkyl containing 1-8 carbon atoms; "$C_1$-$C_8$ alkoxy" refers to an alkoxy containing 1-8 carbon atoms.

"Alkyl" refers to a saturated hydrocarbon chain containing a specified number of carbon atoms. For example, $C_1$-$C_8$ alkyl refers to alkyls having 1-8 carbon atoms, i.e. 1, 2, 3, 4, 5, 6, 7, or 8 carbon atoms. The alkyl group can be linear or branched. Typical branched alkyls have one, two, or three branches. Alkyls include methyl, ethyl, propyl (n-propyl and isopropyl), butyl (n-butyl, isobutyl, and tert-butyl), pentyl (n-pentyl, isopentyl, and neopentyl), hexyl, and the same.

"Halogen" refers to fluorine, chlorine, bromine, or iodine.

In the present invention, a cycloalkyl refers to a saturated or partially saturated non-aromatic cyclic group consisting of carbons, without ring heteroatoms, which has a single ring or polycyclic rings (including fused, bridged, and spiro ring systems). Heterocycloalkyl refers to a saturated or partially saturated non-aromatic cyclic group containing at least one heteroatom; it includes a single ring or polycyclic rings (including fused, bridged, and spiro ring systems); among them, heteroatoms refer to N, O, and S. Examples of heterocyclic groups include, for example, piperidyl, piperazinyl, and morpholinyl.

In the present invention, aryl refers to an unsaturated aromatic group, which does not contain any ring heteroatom and has a single ring or multi-rings (including fused, bridged, and spiro ring system), such as phenyl, anthranyl, or naphthyl. Heteroaryl refers to an aromatic unsaturated ring containing at least one heteroatom; it has a single ring or multi-rings (including fused, bridged, and spiro ring system); among them, heteroatoms refer to N, O, and S. For example, pyridyl, pyrimidyl, pyrazinyl, pyridazinyl, pyrazolyl, imidazolyl, pyrrolyl, furyl, thienyl, oxazolyl, isoindolinyl, etc.

In the present invention, the structure of the ketone group formed by $R_4$ and $R_5$ in $-CR_4R_5-$ and carbon is

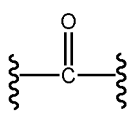

.

In the present invention, the structure of phosphonyl is

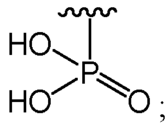

the structure of sulfonic acid group is

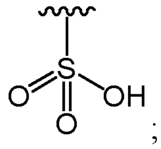

the structure of sulfonamide is

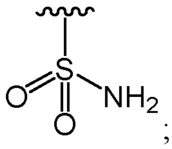

the structure of boric acid group is

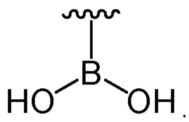

The compounds prepared in the present invention can be used for the preparation of PI3K selective inhibitors, as well as in the manufacture of medicaments for the prevention and/or treatment of PI3K-related diseases, such as medicaments for the prevention and/or treatment of cancer. The present invention provides a new choice for clinical treatment of cancer and has good application prospects.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

## Examples

The starting materials and equipment used in the specific examples of the present invention are all known products obtained by purchasing those commercially available.

### General intermediates: Synthesis of (*R*)-8-(1-aminoethyl)-2-(2,4-difluorophenyl)-6-methyl-4*H*-indolin-4-one (4-7)

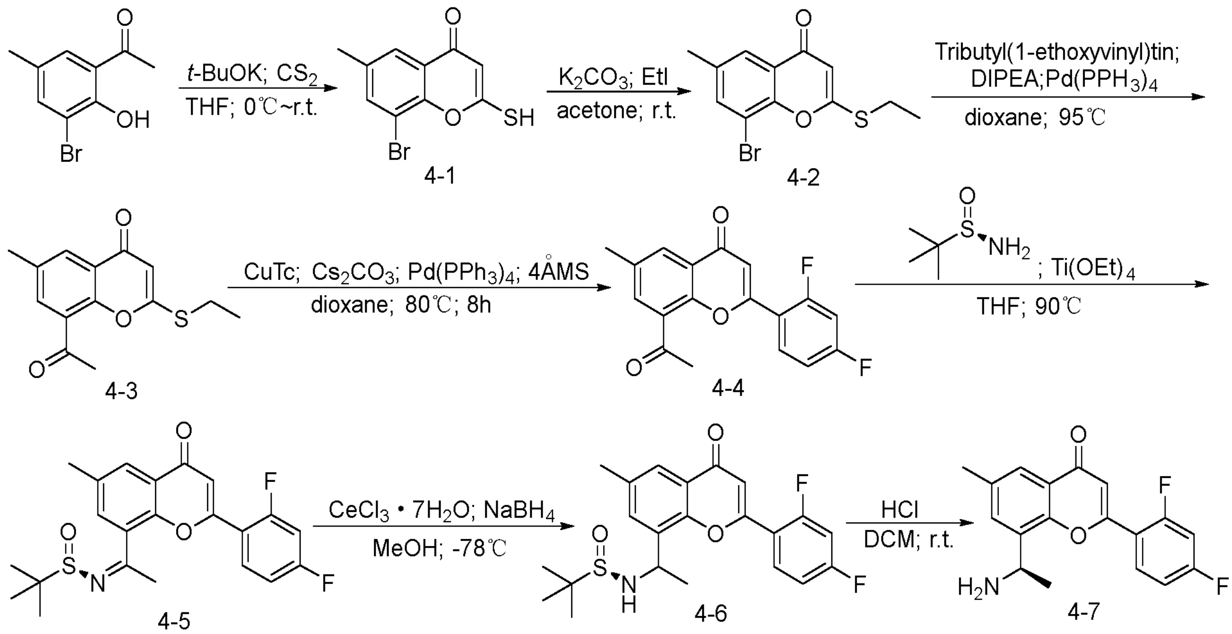


Step 1 intermediate: Synthesis of 8-bromo-2-thiol-6-methyl-4*H*-indolin-4-one **(4-1)**

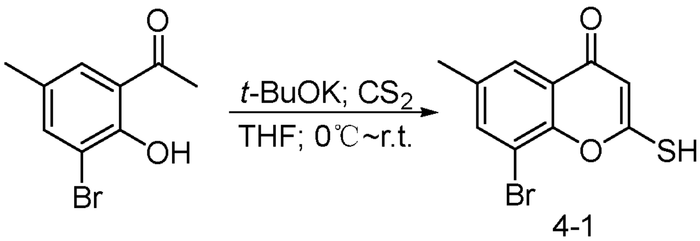


4-1

Procedures: 1-(3-bromo-2-hydroxy-5-methylphenyl)ethanone (1.3g; 5.6 mmol) was added into THF (4 mL), and then the system was purged with nitrogen for three times. The reaction was stirred in an ice-water bath, to which was added t-BuOK (1 M in THF; 17 mL) dropwise. After addition, the reaction was warmed to room temperature and stirred for 30 min. The reaction was cooled and stirred in an ice-water bath, and then the solution of $CS_2$ (1.06 g; 14 mmol) in THF (10 mL) was slowly added. After addition, the reaction was naturally warmed to room temperature and stirred overnight. In an ice-water bath, the reaction solution was adjusted to pH 2 with HCl (1N), and then extracted with EA (20 mL × 3). The organic phase was dried over anhydrous sodium sulfate, concentrated, triturated in hot EA, filtered, and further dried, to obtain compound **4-1** (1.3 g; 4.8 mmol), with a yield of 86%. MS: *m/z* 271 $[M+H]^+$.

Step 2 intermediate: Synthesis of 8-bromo-2-(ethylthio)-6-methyl-4*H*-indolin-4-one **(4-2)**

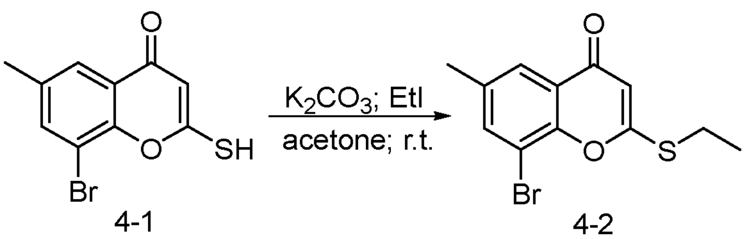


4-1 4-2

Procedures: **4-1** (1.3 g; 4.8 mmol) was added to acetone (6.5 mL), to which were then added potassium carbonate (0.86 g; 6.2 mmol) and iodoethane (1.1 g; 7.2 mmol). The reaction was stirred at room temperature and

monitored by TLC. After completion of the reaction, water (10 mL) and dichloromethane (10 mL) were added. The resultant solution was filtered, and the filter cake was rinsed with dichloromethane (10 mL × 2), followed by extraction. The organic phase was dried and concentrated. The residue was subjected to column chromatography to obtain intermediate **4-2** (950 mg; 3.2 mmol), with a yield of 66%. MS: *m*/*z* 299 [M+H]$^+$.

Step 3 intermediate: Synthesis of 8-acetyl-2-(ethylthio)-6-methyl-4H-indolin-4-one **(4-3)**

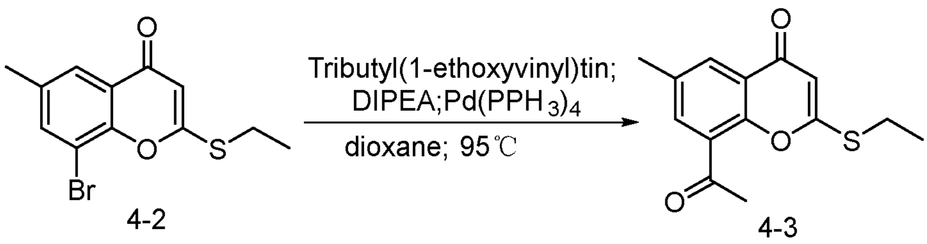


Procedures: **4-2** (299 mg; 1 mmol), Tin reagent (541 mg; 1.5 mmol), DIPEA (258 mg; 2 mmol), and tetrakis(triphenylphosphine)palladium (173 mg; 0.15 mmol) were added into dioxane (6 mL), and then under nitrogen protection, the reaction was stirred overnight at 95 °C; after the reaction solution was cooled to room temperature, concentrated hydrochloric acid (0.5 mL) was added, and then the reaction was stirred for 30 min. Then, saturated KF solution (3 mL) was added, and then the reaction was stirred for 1 h. The reaction solution was filtered, extracted with ethyl acetate, followed by column chromatography, to obtain intermediate **4-3** (200 mg; 0.76 mmol), with a yield of 76%. MS: *m*/*z* 263 [M+H]$^+$.

Step 4 intermediate: Synthesis of 8-acetyl-2-(2,4-difluorophenyl)-6-methyl-4*H*-indolin-4-one **(4-4)**

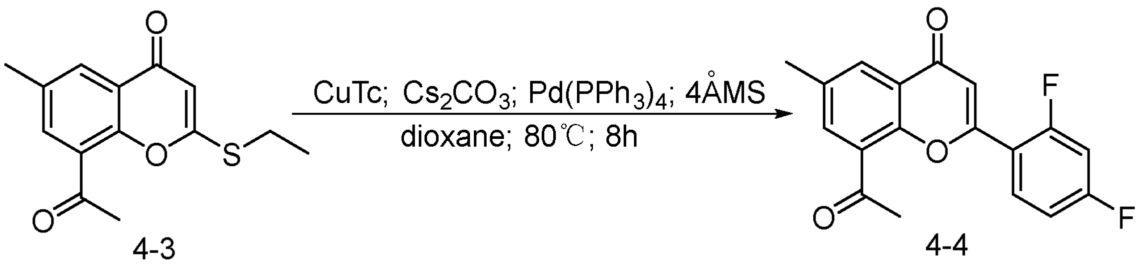


Procedures: **4-3** (200 mg; 0.63 mmol) was dissolved in dioxane (2 mL), and then the system was purged with nitrogen for 3 min, followed by addition of 2,4-difluorophenylboronic acid (1.26 g; 5.06 mmol), CuTc (202 mg; 3.17 mmol), tetrakis(triphenylphosphine)palladium (146 mg; 0.13 mmol), cesium carbonate (412 mg; 1.27 mmol), and molecular sieve (200 mg). Then, the tube was sealed, and the reaction was stirred at 80 °C for 8 h, to which were added water and ethyl acetate. The resultant solution was filtered. The filtrate was extracted with ethyl acetate, followed by column chromatography to obtain intermediate **4-4** (198 mg; 0.63 mmol) with a yield of 63%. MS: *m*/*z* 315 [M+H]$^+$.

Step 5 intermediate: Synthesis of (*R*,*Z*)-N-(1-(2-(2,4-difluorophenyl)-6-methyl-4-oxo-4H-indolin-8-yl)ethylene)-2-isobutyl-2-sulfenamide **(4-5)**

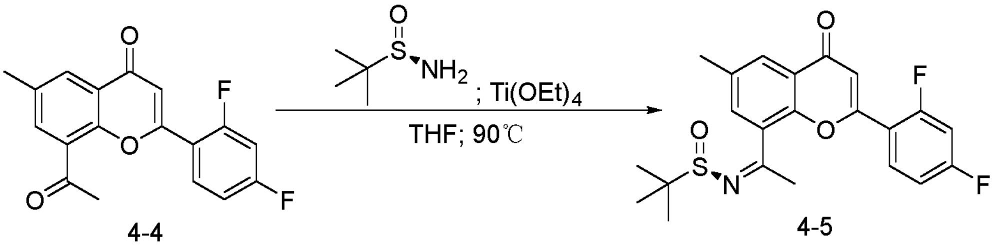


Procedures: **4-4** (198 mg; 0.63 mmol) and (*R*)-(+)-tert-butylsulfenamide (191 mg; 1.58 mmol) were added into tetrahydrofuran (1.1 mL), followed by addition of tetraethyl titanate (718 mg; 3.15 mmol), and then the reaction was stirred overnight at 90 °C, to which were added water and ethyl acetate. The resultant solution was filtered. The filtrate was extracted with ethyl acetate, followed by column chromatography to obtain intermediate **4-5** (217 mg; 0.52 mmol) with a yield of 82%. MS: *m*/*z* 418 $[M+H]^+$.

Step 6 intermediate: Synthesis of (*R*)-N-(1-(2-(2,4-difluorophenyl)-6-methyl-4-oxo-4*H*-indolin-8-yl)ethyl)-2-isobutan-2-sulfenamide (**4-6**)

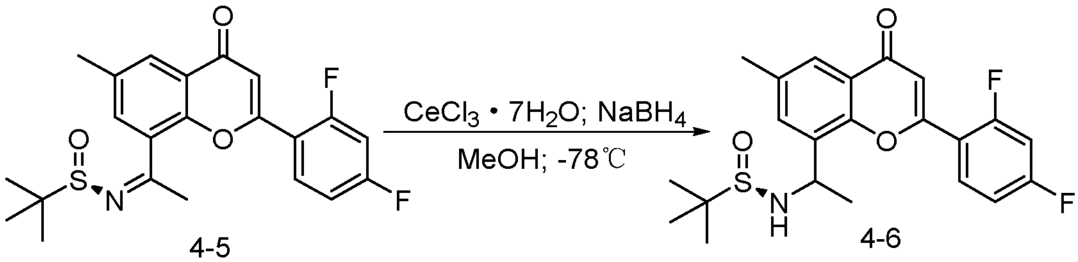


Procedures: **4-5** (217 mg; 0.52 mmol) and cerous chloride heptahydrate (97 mg; 0.26 mmol) were added into methanol (6.6 mL), and then dissolved to become clear under nitrogen protection, followed by cooling to -78 °C; under stirring at low temperature, $NaBH_4$ (49 mg; 1.3 mmol) was added, and then the reaction was stirred overnight. After completion of the reaction, ethyl acetate was added, and the resultant solution was filtered, followed by column chromatography to obtain intermediate **4-6** (170 mg; 0.4 mmol) with a yield of 78%. MS: *m*/*z* 420 $[M+H]^+$.

Step 7 intermediate: Synthesis of (*R*)-8-(1-aminoethyl)-2-(2,4-difluorophenyl)-6-methyl-4H-indolin-4-one **(4-7)**

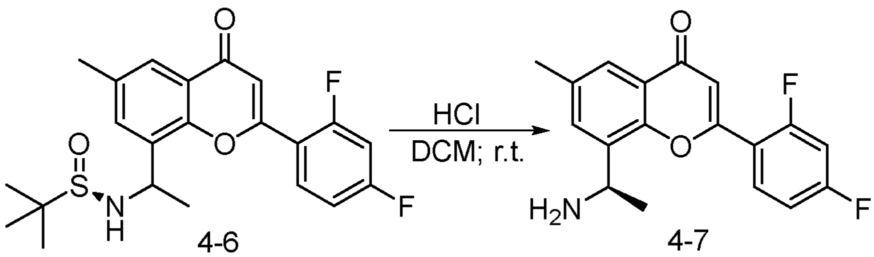


Procedures: **4-6** (170 mg; 0.4 mmol) was dissolved in dichloromethane (3 mL), to which was added the solution of HCl in dioxane (1 mL), and then the reaction was stirred at room temperature. TLC detection indicated that the reaction was completed, and then the reaction solution was rotatory evaporated to dry, followed by addition of water. The resultant solution was extracted with ethyl acetate, and the pH of the water phase was adjusted to be alkaline, and then extracted with ethyl acetate. The organic phase was dried, and rotatory evaporated to dry, to obtain intermediate **4-7** (114 mg; 0.36 mmol), with a yield of 90%. MS: *m*/*z* 316 $[M+H]^+$.

**Example 1: Synthesis of compound N-(2-(4,4-dimethylpiperidin-1-yl)-6-methyl-4-oxo-4H-chromen-8-yl)indolin-1-formamide (compound 1)**

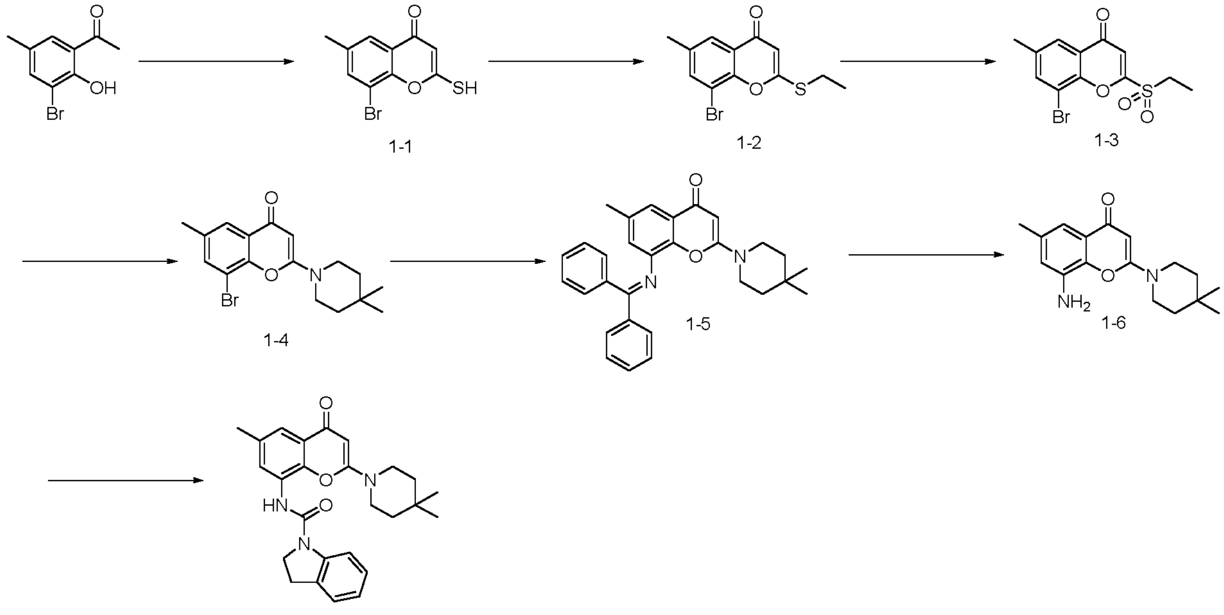

1-1 1-2 1-3 1-4 1-5 1-6

1

Step 1: Synthesis of intermediate 8-bromo-2-thiol-6-methyl-4H-chromen-4-one **(1-1)**

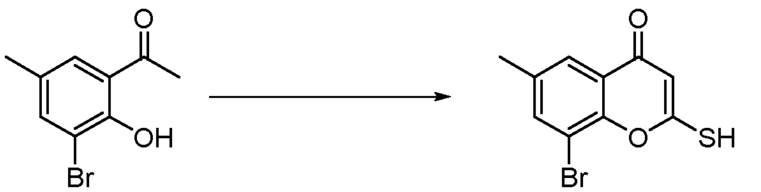

1-1

To a 500 mL reaction flask, were added compound 1-(3-bromo-2-hydroxy-5-methylphenyl)ethane-1-one (8.1 g, 35.4 mmol) and tetrahydrofuran (100 mL), and then the reaction was cooled to -70 °C in a dry ice bath, followed by adding NaHMDS (2.0 M in THF, 53 mL, 106 mmol) dropwise. After addition, the reaction was naturally warmed to 0 °C, and stirred for 1 h. Then, the reaction was cooled to -20 °C, to which was added carbon disulfide (8.1 g, 106 mmol), and then the reaction was naturally warmed and allowed to react overnight. After completion of the reaction, the solution was cooled to 0 °C, and adjusted to be pH = 4-5 with dilute hydrochloric acid (1N). The reaction was stirred for 1 h, and extracted with ethyl acetate (50 mL × 3). The organic layers were combined, dried over anhydrous $Na_2SO_4$, and concentrated. The residue was triturated in ethyl acetate, and filtered, to obtain the intermediate **1-1** (6.6 g), with a yield of 69%. MS: *m/z* 271.1, 273.1 $[M+H]^+$.

Step 2: Synthesis of intermediate 8-bromo-2-(ethylthio)-6-methyl-4H-chromen-4-one **(1-2)**

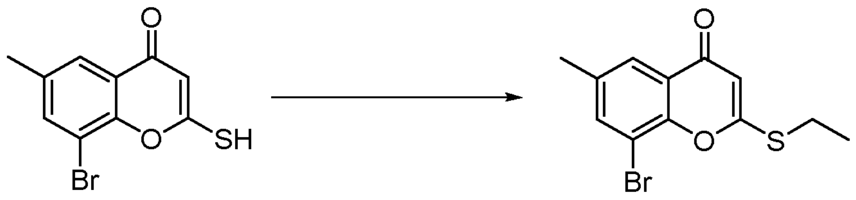

1-1 1-2

To a 250 mL reaction flask, were added compound **1-1** (6.6 g, 24.4 mmol), potassium carbonate (5.1 g, 36.6 mmol) and acetone (70 mL), and then iodoethane (4.5 g, 29 mmol) was added under stirring. The reaction was heated under refluxing. After completion of the reaction, the reaction solution was added with water (30 mL), and extracted with ethyl acetate (30 mL × 3). The organic layers were combined, dried over anhydrous $Na_2SO_4$, and concentrated. The residue was purified by silica gel column chromatography, to obtain compound **1-2** (4.5 g), with a yield of 62%. MS: *m*/*z* 299.1, 301.1 $[M+H]^+$.

Step 3: Synthesis of intermediate 8-bromo-2-(ethylsulfonyl)-6-methyl-4H-chromen-4-one **(1-3)**

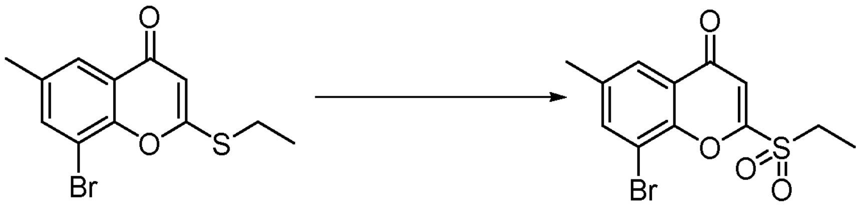

1-2 1-3

To a 100 mL reaction flask, were added compound **1-2** (1 g, 3.3 mmol) and dichloromethane (20 mL), and then m-chloroperoxybenzoic acid (2.3 g, 13.3 mmol) was added under stirring. The reaction was stirred overnight. After completion of the reaction, the solution was filtered, and then the filtrate was washed with sodium thiosulfate aqueous solution. Subsequently, the organic layer was washed with saturated $Na_2CO_3$ aqueous solution, dried, and concentrated, to obtain the crude product compound 1-3 (1.3 g), with a yield of 118%. MS: *m*/*z* 331.1, 333.1 $[M+H]^+$.

Step 4: Synthesis of intermediate 8-bromo-2-(4,4-dimethylpiperidin-1-yl)-6-methyl-4H-chromen-4-one **(1-4)**

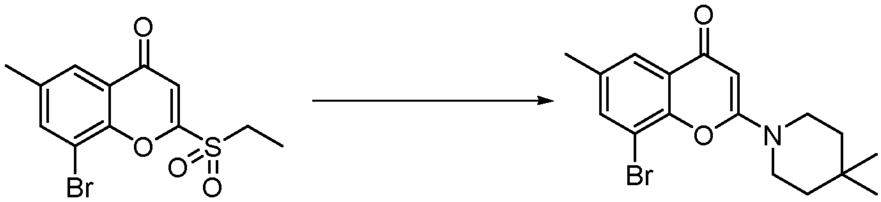

1-3 1-4

To a 100 mL reaction flask, were added compound **1-3** (1.3 g, 3.9 mmol), diisopropylethylamine (1.5 g, 11.7 mmol), 4,4-dimethylpiperidine hydrochloride (0.6 g, 3.9 mmol) and dichloromethane (20 mL), and then the mixture was allowed to react overnight. After completion of the reaction, the reaction solution was added with water (10 mL), and then extracted with dichloromethane (10 mL × 3). The organic layers were combined, and dried over anhydrous $Na_2SO_4$, and concentrated, followed by silica gel column chromatography, to obtain compound **1-4** (1 g), with a yield of 74%. MS: *m*/*z* 350.1, 352.1 $[M+H]^+$.

Step 5. Synthesis of intermediate 2-(4,4-dimethylpiperidin-1-yl)-8-(diphenylmethylene)amino)-6-methyl-4H-chromen-4-one **(1-5)**

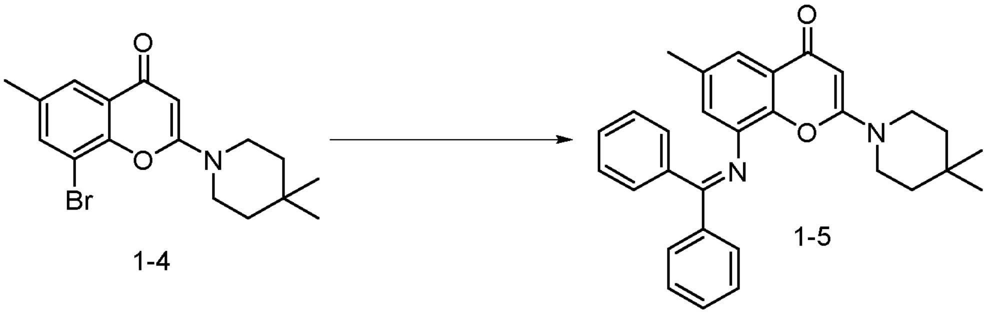

1-4

1-5

To a 100 mL reaction flask, were added compound **1-4** (1 g, 2.9 mmol), diphenylmethylimine (0.68 g, 3.7 mmol), cesium carbonate (1.4 g, 4.3 mmol), and toluene (20 mL), and then the system was purged with nitrogen, followed by addition of 2,2'-bis(diphenylphosphino)-1,1'-binaphthyl (0.36 g, 0.6 mmol) and palladium acetate (0.13 g, 0.6 mmol). The mixture was heated to 100 °C, and allowed to react overnight. After completion of the reaction, the reaction solution was added with water (20 mL), and then extracted with ethyl acetate (20 mL × 3). The organic layers were combined, and dried over anhydrous $Na_2SO_4$, and concentrated to obtain the mixture, which was directly used in the next step.

Step 6: Synthesis of intermediate 8-amino-2-(4,4-dimethylpiperidin-1-yl)-6-methyl-4H-chromen-4-one **(1-6)**

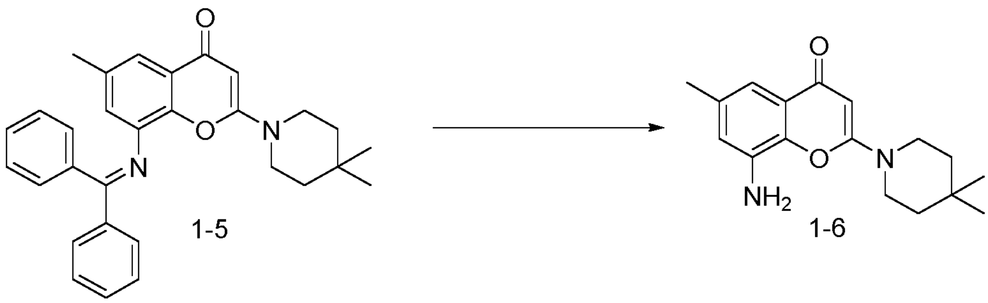

1-5

1-6

To a 100 mL reaction flask, was added the mixture obtained in the previous step, and then tetrahydrofuran (20 mL) was added to dissolve, followed by addition of 2N dilute hydrochloric acid (15 mL). The mixture was stirred at room temperature. After completion of the reaction, the solution was adjusted to be pH = 8-9 with $Na_2CO_3$ solution, and then extracted with ethyl acetate (20 mL × 3). The organic layers were combined, and dried over anhydrous $Na_2SO_4$, and concentrated, followed by silica gel column chromatography, to obtain compound **1-6** (0.7 g), with a yield of 84%. MS: *m/z* 287.2 $[M+H]^+$.

Step 7: Synthesis of compound N-(2-(4,4-dimethylpiperidin-1-yl)-6-methyl-4-oxo-4H-chromen-8-yl)indolin-1-formamide (compound **1)**

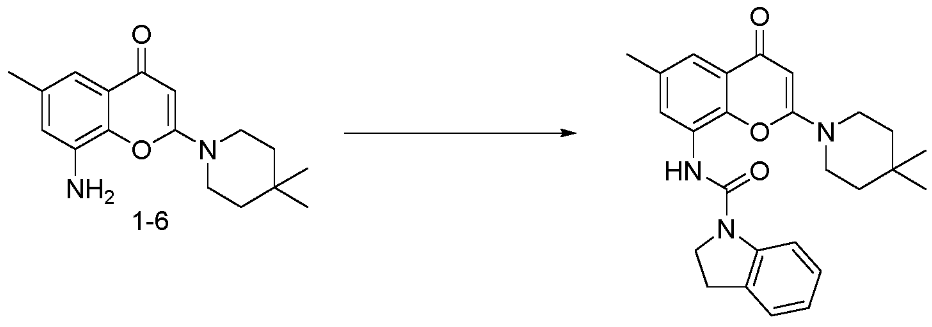

1-6

1

To a 25 mL reaction flask, were added compound **1-6** (50 mg, 0.18 mmol) and dichloromethane (3 mL), followed by addition of triphosgene (27 mg, 0.09 mmol) in an ice bath, and then the reaction was stirred at room temperature for 1 h. Subsequently, triethylamine (71 mg, 0.7 mmol) and indoline (25 mg, 0.21 mmol) were successively added. After completion of the reaction, the reaction solution was added with water (3 mL), and then extracted with ethyl acetate (3 mL × 3). The organic layers were combined, and dried over anhydrous $Na_2SO_4$, and concentrated, followed by purification via prep-TLC, to obtain compound (compound 1, 40 mg), with a yield of 52%. MS: *m/z* 432.2 $[M+H]^+$, $^1H$ NMR (400 MHz, DMSO-$d_6$) $\delta$ 8.52 (s, 1H), 7.84 (d, *J* = 8.0 Hz, 1H), 7.52 (d, *J* = 3.0 Hz, 2H), 7.21 (d, *J* = 7.4 Hz, 1H), 7.11 (t, *J* = 7.8 Hz, 1H), 6.91 (t, *J* = 7.4 Hz, 1H), 5.48 (s, 1H), 4.14 (t, *J* = 8.6 Hz, 2H), 3.45 (dd, *J* = 6.9, 4.7 Hz, 4H), 3.20 (t, *J* = 8.6 Hz, 2H), 2.38 (s, 3H), 1.30 (t, *J* = 5.8 Hz, 4H), 0.91 (s, 6H).

**Example 2: Synthesis of compound (R)-2-((1-(2-((2-hydroxy-2-methylpropyl)amino)-6-methyl-4-oxo-4H-chromen-8-yl)ethyl)amino)benzoic acid (compound** 7)

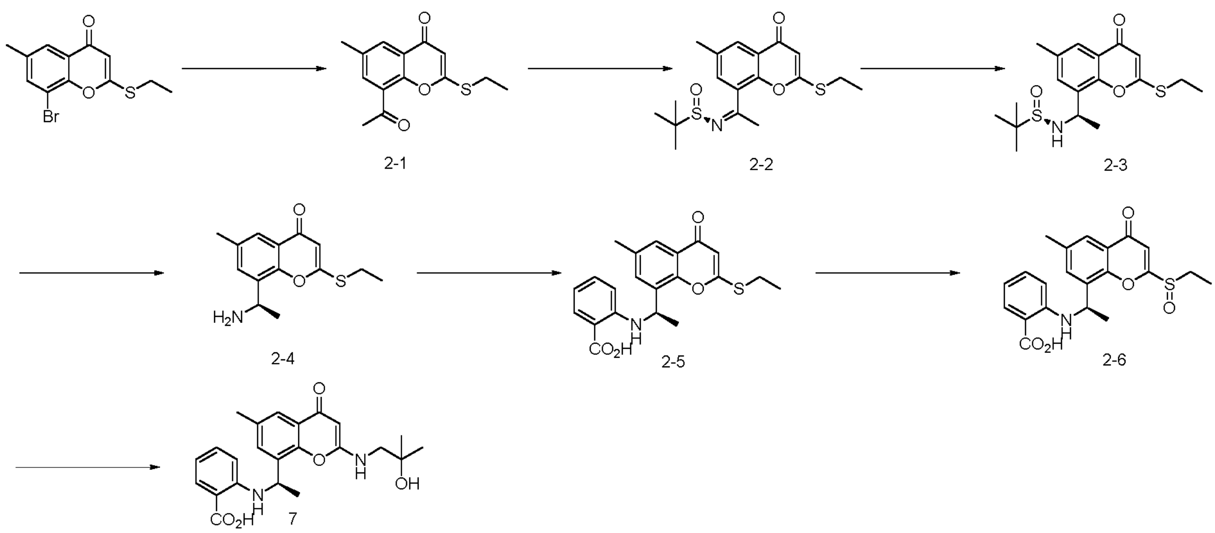

2-1 2-2 2-3

2-4 2-5 2-6

7

Step 1: Synthesis of intermediate 8-acetyl-2-(ethylthio)-6-methyl-4H-chromen-4-one (2-1)

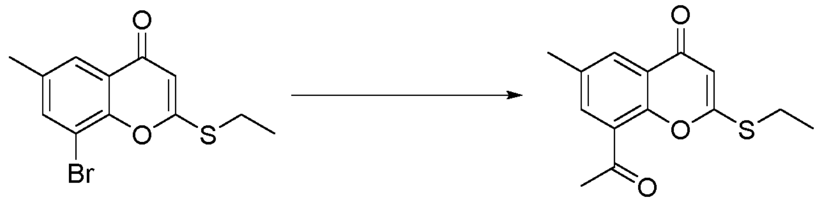

2-1

To a 100 mL reaction flask, were added compound 8-bromo-2-(ethylthio)-6-methyl-4H-chromen-4-one (700 mg, 2.3 mmol), tributyl(1-ethoxyvinyl)tin (1 g, 2.8 mmol), and dioxane (15 mL), and then the system was purged with nitrogen, followed by addition of [1,1'-bis(diphenylphosphino)ferrocene]dichloropalladium(II) (168 mg, 0.23 mmol). The reaction was heated to 95 °C and allowed to react for 3-4 h. After completion of the reaction, the reaction solution was cooled to room temperature, to which was added 6 N dilute hydrochloric acid (5 mL), and then stirred for 1 h, followed by addition of saturated KF aqueous solution (15 mL). The resultant solution was stirred for 1 h and filtered. The filter cake was rinsed three times with ethyl acetate (20 mL), and then the solution was separated. The organic layer was dried over anhydrous $Na_2SO_4$, and concentrated, followed by silica gel column chromatography, to obtain intermediate **2-1** (400 mg), with a yield of 66%. MS: *m/z* 263 $[M+H]^+$.

Step 2: Synthesis of intermediate (R,Z)-N-(1-(2-(ethylthio)-6-methyl-4-oxo-4H-chromen-8-yl)ethylene)-2-methylpropane-2-sulfonamide **(2-2)**

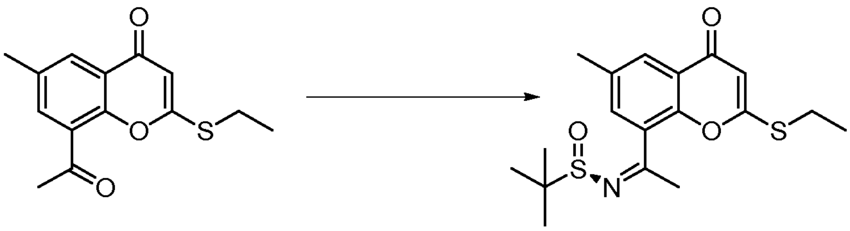

2-1 2-2

To a 100 mL reaction flask, were added compound **2-1** (400 mg, 1.5 mmol), (R)-tert-butylsulfenamide (370 mg, 3 mmol), tetraisopropyl titanate (870 mg, 3.1 mmol), and tetrahydrofuran (10 mL), and then the system was purged with nitrogen and heated overnight under refluxing. After completion of the reaction, the solution was cooled to room temperature, added with saturated saline (10 mL), stirred for 0.5 h, and filtered. The filter cake was rinsed three times with ethyl acetate (10 mL), and then the solution was separated. The organic layer was combined, dried over anhydrous $Na_2SO_4$, and concentrated, followed by silica gel column chromatography, to obtain crude product intermediate **2-2** (700 mg), with a yield of 128%. MS: *m/z* 366 $[M+H]^+$.

Step 3: Synthesis of intermediate (R)-N-((R)-1-(2-(ethylthio)-6-methyl-4-oxo-4H-chromen-8-yl)ethyl)-2-methylpropane-2-sulfonamide **(2-3)**

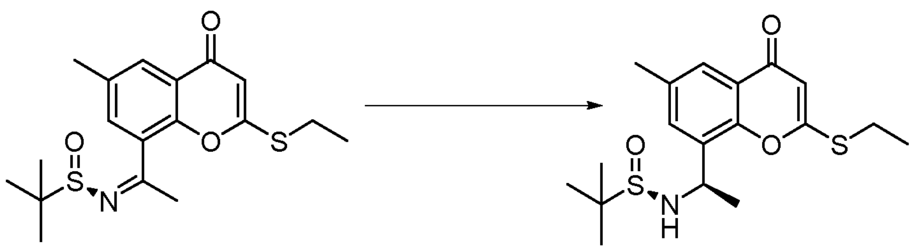

2-2 2-3

To a 50 mL reaction flask, were added compound **2-2** (375 mg, 1 mmol), cerous chloride heptahydrate (187 mg,

0.5 mmol), and methanol (10 mL), and then the reaction was cooled to - 70 °C under stirring. The solution of $NaBH_4$ (114 mg, 3 mmol) in methanol (2 mL) was added dropwise, and after addition, the reaction was naturally warmed and kept overnight. The reaction solution was added with ethyl acetate and filtered. The filtrate was rotatory evaporated, and then ethyl acetate was added. The reaction solution was further filtered, and the filtrate was concentrated, followed by silica gel column chromatography, to obtain intermediate **2-3** (80 mg), with a yield of 22%. MS: *m/z* 368 $[M+H]^+$.

Step 4: Synthesis of intermediate (R)-8-(1-aminoethyl)-2-(ethylthio)-6-methyl-4H-chromen-4-one **(2-4)**

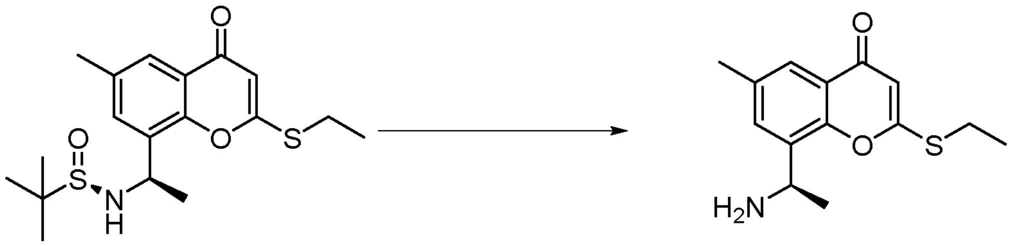

2-3 2-4

To a 25 mL reaction flask, were added compound **2-3** (80 mg, 0.22 mmol) and ethyl acetate (3 mL), to which was added the solution of HCl in dioxane (3 mL), and then the reaction was stirred at room temperature, until the reaction was completed. The solvent was removed by concentration, and then $NaHCO_3$ aqueous solution (5 mL) was added. The resultant solution was extracted with dichloromethane (5 mL × 3). The organic layer was combined, dried over anhydrous $Na_2SO_4$, and concentrated, to obtain intermediate **2-4** (50 mg), with a yield of 86%. MS: *m/z* 264 $[M+H]^+$.

Step 5. Synthesis of intermediate (R)-2-((1-(2-(ethylthio)-6-methyl-4-oxo-4H-chromen-8-yl)ethyl)amino)benzoic acid **(2-5)**

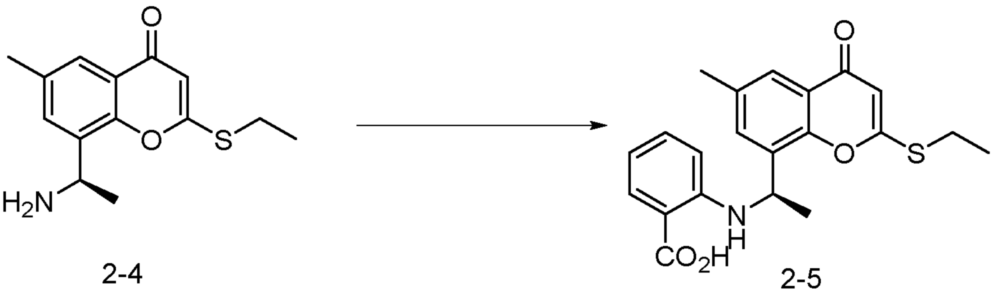

2-4 2-5

To a 25 mL reaction flask, were added compound **2-4** (50 mg, 0.19 mmol), 2-iodobenzoic acid (39 mg, 0.29 mmol), triethylamine (38 mg, 0.38 mml), and N,N-dimethylacetamide (1 mL), and then the system was purged with nitrogen, followed by addition of copper powder (12 mg, 0.19 mmol). The reaction was heated to 110 °C and allowed to react for about 1-2 h. After completion of the reaction, the solution was cooled to room temperature, and adjusted to be pH = 4-5 with 1N HCl aqueous solution. The resultant solution was added with ethyl acetate (5 mL), and then the layers were separated. The organic layers were combined, dried over anhydrous $Na_2SO_4$, and concentrated, followed by prep.-TLC, to obtain intermediate **2-5** (45 mg), with a yield of 62%. MS: *m/z* 384 $[M+H]^+$.

Step 6: Synthesis of intermediate 2-(((1R)-1-(2-(ethylsulfinyl)-6-methyl-4-oxo-4H-chromen-8-yl)ethyl)amino)benzoic acid **(2-6)**

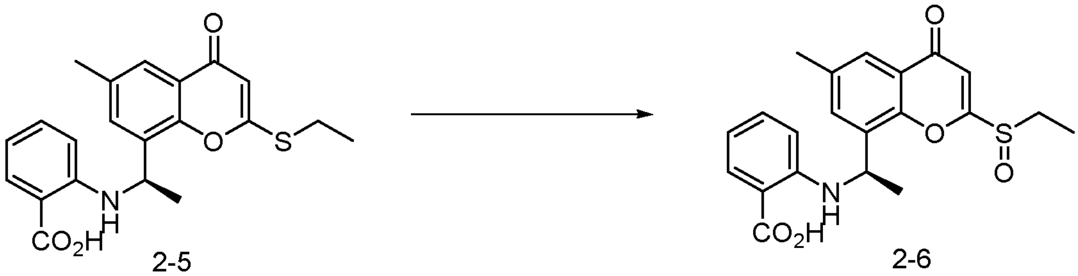

2-5 2-6

To a 25 mL reaction flask, were added compound **2-5** (45 mg, 0.12 mmol) and dichloromethane (3 mL), followed by addition of m-chloroperoxybenzoic acid (31 mg, 0.18 mmol) under stirring, and then the reaction was stirred overnight at room temperature. After completion of the reaction, the solution was filtered, and the filtrate was washed with sodium thiosulfate aqueous solution. The organic layer was dried and concentrated, to obtain crude product **2-6** (60 mg), with a yield of 125%. MS: *m*/*z* 400 $[M+H]^+$.

Step 7: Synthesis of compound (R)-2-((1-(2-((2-hydroxy-2-methylpropyl)amino)-6-methyl-4-oxo-4H-chromen-8-yl) ethyl)amino)benzoic acid (compound **7)**

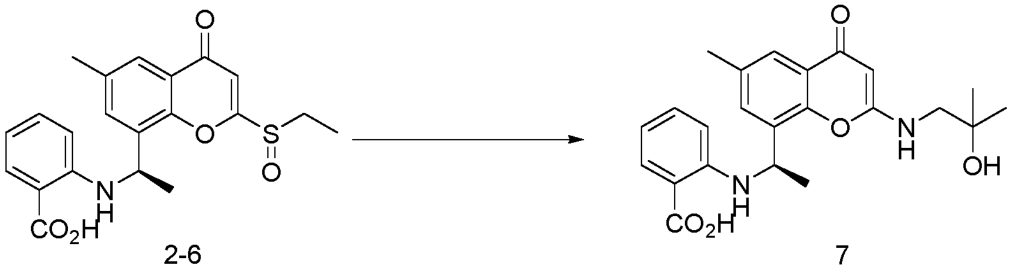

2-6 7

To a 25 mL reaction flask, were added compound **2-6** (30 mg, 0.08 mmol), 1-amino-2-methyl-2-propanol (10 mg, 0.11 mmol), diethylpropylethylamine (39 mg, 0.3 mmol), and dichloromethane, and the reaction was heated overnight at 40 °C. After completion of the reaction, the reaction product was directly purified by prep.-TLC, to obtain compound (compound **7, 7** mg), with a yiled of 21%. MS: *m*/*z* 411 $[M+H]^+$, $^1H$ NMR (400 MHz, DMSO-$d_6$) $\delta$ 12.8 (s,1H), 8.67 (s, 1H), 7.97 (d, *J* = 7.0 Hz, 1H), 7.79 (dd, *J* = 7.9, 1.7 Hz, 1H), 7.56 (d, *J* = 2.2 Hz, 1H), 7.32 (d, *J* = 2.2 Hz, 1H), 7.17 (t, *J* = 7.8 Hz, 1H), 6.50 (t, *J* = 7.5 Hz, 1H), 6.44 (d, *J* = 8.4 Hz, 1H), 5.33 (s, 1H), 5.15 (d, *J* = 7.0 Hz, 1H), 3.39 (s, 1H), 3.17 (d, *J* = 6.2 Hz, 2H), 2.28 (s, 3H), 1.55 (d, *J* = 6.6 Hz, 3H), 1.16 (d, *J* = 7.6 Hz, 6H).

**Example 3: Synthesis of compound (R)-2-(2,4-difluorophenyl)-6-methyl-8-(1-(1-methyl-1H-pyrazol-5-yl)amino) ethyl)-4H-chromen-4-one (compound 42)**

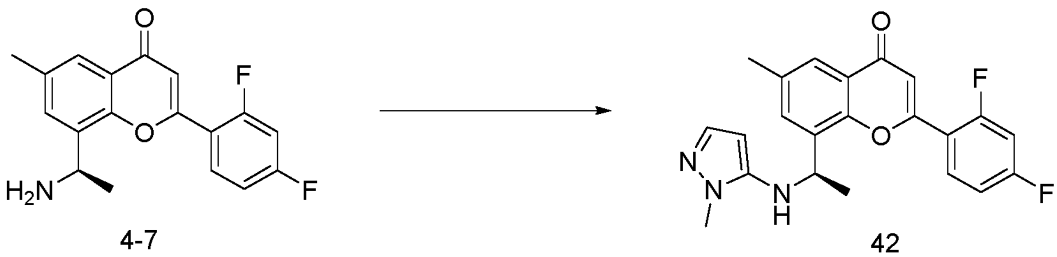

4-7 42

To a 25 mL reaction flask, were added intermediate **4-7** (Its synthesis referred to the synthesis of general intermediates, 20 mg, 0.06 mmol), 5-bromo-1-methyl-1H-pyrazole (21 mg, 0.13 mmol), sodium tert-butoxide (18 mg, 0.19 mmol) and toluene (2 mL), and then the system was purged with nitrogen, followed by addition of tris(dibenzylideneacetone)dipalladium (12 mg, 0.01 mmol) and 2-di-tert-butylphosphino-2',4',6'-triisopropylbiphenyl (6 mg, 0.01 mmol). The reaction was heated to 100 °C, and allowed to react overnight. After completion of the reaction, the reaction solution was

added with water (2 mL), and then extracted with ethyl acetate (3 mL × 3). The organic layers were combined, dried over anhydrous $Na_2SO_4$, and concentrated, followed by purification via prep-TLC, to obtain the target compound (6 mg), with a yield of 25%. MS: *m/z* 396.1 $[M+H]^+$, 1H NMR (400 MHz, DMSO-$d_6$) $\delta$ 7.78 (d, *J* = 2.2 Hz, 1H), 7.70 (d, *J* = 2.2 Hz, 1H), 7.61-5.52 (m, 1H), 7.37 - 7.30 (m, 2H), 6.81 (s, 1H), 6.75 - 6.61 (m, 3H), 5.41 (t, *J* = 6.7 Hz, 1H), 3.25 (s, 3H), 2.37 (s, 3H), 1.71 (d, *J* = 6.7 Hz, 3H).

**Example 4: Synthesis of compound (R)-6-chloro-3-((1-(6-fluoro-2-(5-fluoropyridin-2-yl)-3-methyl-4-oxo-3,4-dihydroquinazolin-8-yl)ethyl)amino)pyridinecarboxylic acid (compound 37)**

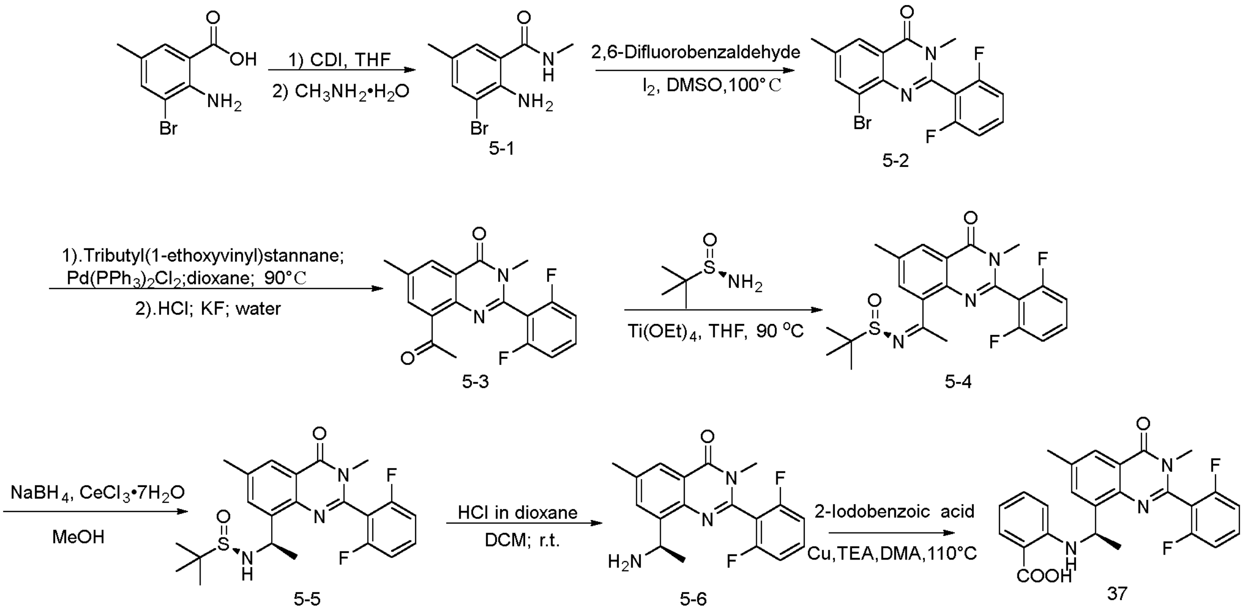


Intermediate: Synthesis of 2-amino-3-bromo-N,5-dimethylbenzamide **(5-1)**

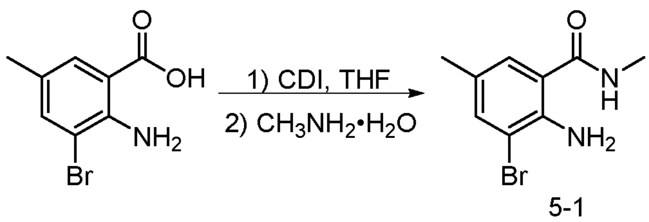


2-amino-3-bromo-5-methylbenzoic acid (200 g, 0.87 mol) was dissolved in THF (2 L), to which was added CDI (156 g, 0.96 mol) in batches, and then the system was gradually bubbling up. After reaction for 2h, TLC detection indicated disappearance of the intermediate. During work-up and concentration, once solids precipitated, the reaction was poured into water (10× the volume of tetrahydrofuran), and then triturated. After filtration, the filter cake was rinsed with water and absolute ethanol, and then rotatory evaporated to dry, to obtain white solids **5-1** (204 g, 0.84 mol), with a yield of 96%. MS: *m/z* 243/245 $[M+H]^+$.

Intermediate: Synthesis of 8-bromo-2-(2,6-difluorophenyl)-3,6-dimethylquinazolin-4(3*H*)-one **(5-2)**

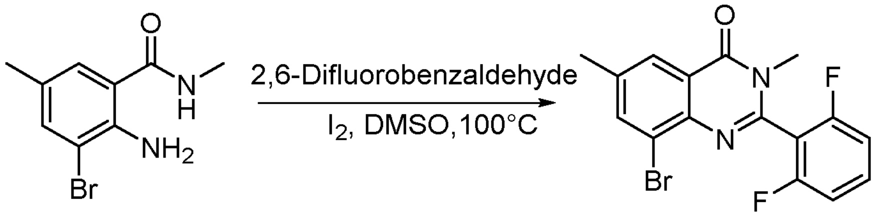


5-1 5-2

**5-1** (2.0 g, 8.26 mmol), 2,6-difluorobenzaldehyde (1.4 g, 9.91 mmol), and iodine (2.51 g, 9.91 mmol) were sequentially added into DMSO (20 mL), and then stirred at 100 °C for 4 h. LCMS indicated disappearance of raw materials. After work-up and cooling, the system was added to ice water containing sodium thiosulfate, and then filtered. The filter cake was rinsed with ethanol, and then rotatory evaporated to dry, to obtain off-white solids **5-2** (2.2 g, 6.04 mmol), with a yield of 73%. MS: *m*/*z* 365/367 $[M+H]^+$.

Intermediate: Synthesis of 8-acetyl-2-(2,6-difluorophenyl)-3,6-dimethylquinazolin-4(3*H*)-one **(5-3)**

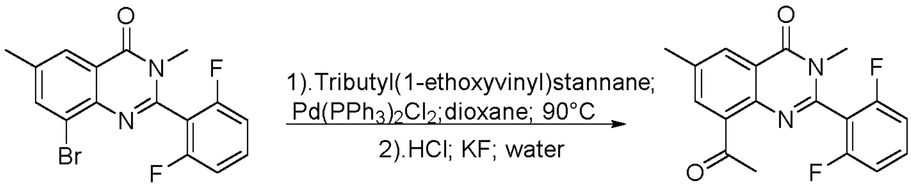


5-2 5-3

**5-2** (2.2 g, 6.04 mmol), tributyl(1-ethoxyvinyl)tin (2.6 g, 7.25 mmol), and bis(triphenylphosphine)palladium(II) chloride (0.48 g, 0.60 mmol) were successively added into anhydrous dioxane (11 mL), and then the system was purged with nitrogen for three times, and allowed to react at 90 °C for 6h. TLC indicated disappearance of raw materials. After work-up and cooling, the reaction solution was added with DCM (20 mL), and adjusted to be pH 2-3 with 2 N HCl, followed by stirring for 1-2 h. TLC detection showed complete reaction of the intermediate, and then KF saturated solution was added. The resultant solution was stirred for 1 h and filtered. The filtrate was extracted with DCM (50 mL × 2). The organic phases were combined, dried over anhydrous $Na_2SO_4$, and concentrated to dry, to obtain crude product, which was triturated in EA and filtered. The filter cake was rotatory evaporated to dry, to obtain light yellow solid **5-3** (1.7 g, 5.18 mmol), with a yield of 87%. MS: *m*/*z* 329 $[M+H]^+$.

Intermediate: Synthesis of (*R*,*Z*)-*N*-(1-(2-(2,6-difluorophenyl)-3,6-dimethyl-4-oxo-3,4-dihydroquinazolin-8-yl)ethylene)-2-methylpropane-2-sulfamide **(5-4)**

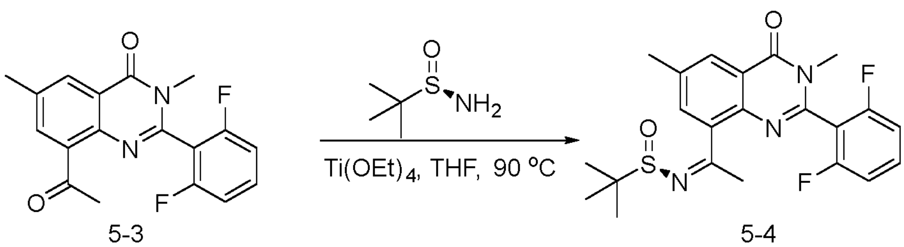


5-3 5-4

**5-3** (1.7 g, 5.18 mmol), (*R*)-(+)-tert-butylsulfenamide (1.3 g, 10.36 mmol), and tetraethyl titanate (4.7 g, 20.72 mmol) were successively added into tetrahydrofuran (8.5 mL), and then stirred for 12 h at 90 °C. TLC indicated disappearance of raw materials. After work-up and cooling, the reaction solution was poured to ice water (85 mL) and filtered. The filter cake was triturated in DCM (50 mL × 4), and the organic phase was collected. The filtrate was extracted with DCM (50 mL × 2). The organic phases were combined, dried over anhydrous $Na_2SO_4$, and concentrated to dry, to obtain crude product **5-4** as yellow oil (1.5 g, 3.48 mmol), which can be directly used in the next step. MS: *m*/*z* 432 $[M+H]^+$.

Intermediate: Synthesis of N-((R)-1-(2-(2,6-difluorophenyl)-3,6-dimethyl-4-oxo-3,4-dihydroquinazolin-8-yl)ethyl)-2-methylpropane-2-sulfonamide **(5-5)**

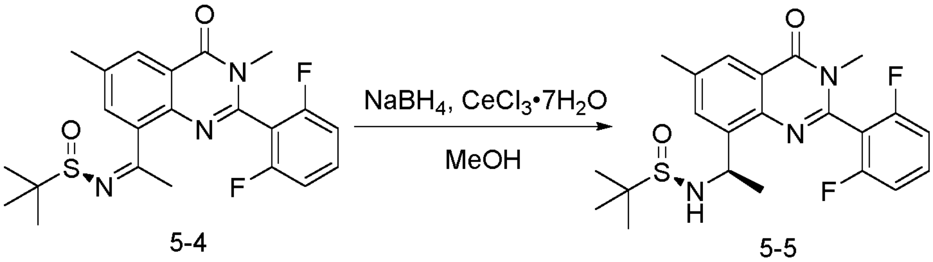


5-4 5-5

Crude product **5-4** (1.5 g, 3.48 mmol) and cerous chloride heptahydrate (0.6 g, 1.74 mmol) were successively added to methanol (22 mL), and then the system was purged with nitrogen for three times, and cooled to (-60 °C)-(-70 °C) under stirring, followed by addition of $NaBH_4$ (0.4 g, 10.44 mmol) in portions. After addition, the temperature was kept for 30 min, and then the reaction was warmed to room temperature. TLC indicated disappearance of raw materials, followed by work-up. The reaction was quenched by adding water, and then DCM (100 mL×3) was added, followed by extraction. The organic phase was combined, dried over anhydrous $Na_2SO_4$, and concentrated, followed by column chromatography, to obtain yellow solid **5-5** (0.8 g, 1.85 mmol), with a yield of 36% for two steps. MS: *m/z* 434 $[M+H]^+$.

Intermediate: Synthesis of (*R*)-8-(1-aminoethyl)-2-(2,6-difluorophenyl)-3,6-dimethylquinazolin-4(3*H*)-one **(5-6)**

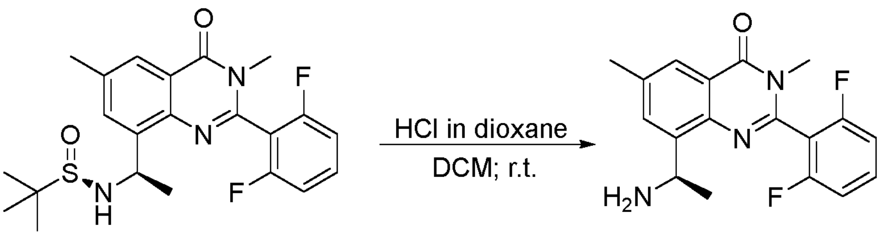


5-5 5-6

**5-5** (0.8 g, 1.85 mmol) was dissolved in dichloromethane (4 mL), to which was added the solution of HCl in dioxane (4 N, 2 mL), and then stirred at room temperature. TLC indicated disappearance of raw materials, followed by work-up. The reaction solution was added with water (50 mL), and then adjusted to pH 7-8 with $NaHCO_3$, followed by extraction with DCM (50 mL×3). The organic phase was combined, dried over anhydrous $Na_2SO_4$, and concentrated, to obtain solid **5-6** (0.58 g, 1.75 mmol), with a yield of 95%. MS: *m/z* 317 $[M+H]^+$.

Compound: Synthesis of (*R*)-2-((1-(2-(2,6-difluorophenyl)-3,6-dimethyl-4-oxo-3,4-dihydroquinazolin-8-yl)ethyl)amino) benzoic acid (compound **37)**

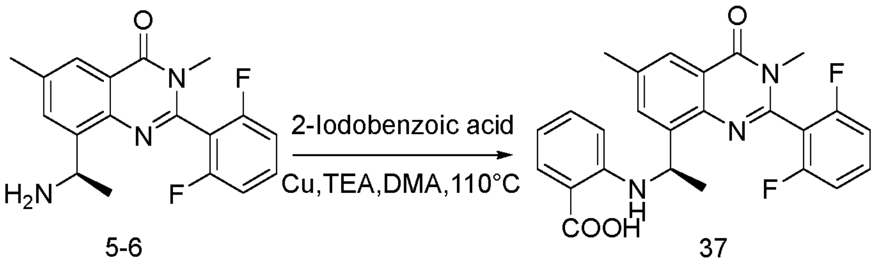


5-6 37

**5-6** (70 mg, 0.21 mmol), 2-iodobenzoic acid (79 mg, 0.32 mmol), copper powder (21 mg, 0.32 mmol), and triethylamine (43 mg, 0.42 mmol) were added into DMA (1 mL), and then stirred at 110 °C for 4 h. TLC detection indicated

disappearance of raw materials, followed by work-up. The reaction solution was added with water (10 mL), and extracted with EA (10 mL×3). The organic phase was dried over anhydrous $Na_2SO_4$ and concentrated to dry, followed by column chromatography, to obtain compound 37 as white solid (35 mg, 0.078 mmol), with a yield of 37%. MS: *m/z* 450 [M+H]+.

$^1$H NMR (400 MHz, Chloroform-*d*) δ 8.04 (d, *J* = 2.0 Hz, 1H), 7.97 (dd, *J* = 8.0, 1.7 Hz, 1H), 7.78 (s, 1H), 7.61 (d, *J* = 2.1 Hz, 1H), 7.57 - 7.45 (m, 1H), 7.18 (t, *J* = 7.3 Hz, 1H), 7.11 (td, *J* = 8.2, 2.5 Hz, 2H), 6.54 (s, 1H), 6.49 (d, *J* = 8.6 Hz, 1H), 5.59 (q, *J* = 6.6 Hz, 1H), 3.50 (s, 3H), 2.44 (s, 3H), 1.60 (d, *J* = 6.6 Hz, 3H).

**Example 5: Synthesis of compound (*R*)-2-((1-(2-(2,4-difluorophenyl)-6-methyl-4-oxo-4*H*-chromen-8-yl)ethyl) amino)benzenesulfonamide (compound 27)**

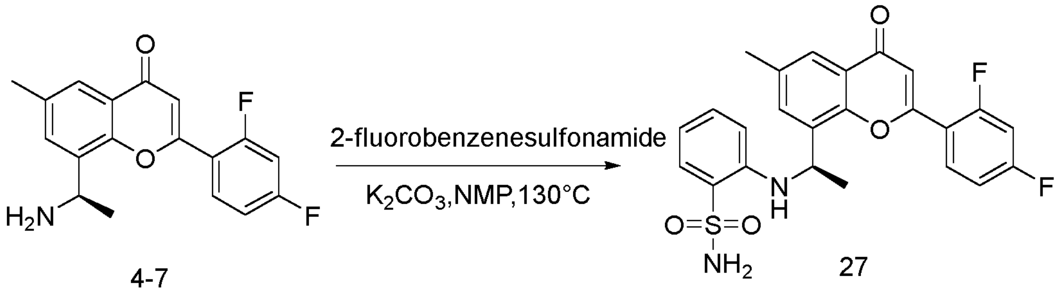


4-7 27

(*R*)-8-(1-aminoethyl)-2-(2,4-difluorophenyl)-6-methyl-4*H*-chromen-4-one (Its synthesis referred to the synthesis of general intermediates, 30 mg, 95.14 μmol), 2-fluorobenzenesulfonamide (25 mg, 142.71 μmol), and potassium carbonate (26 mg, 190.28 μmol) were added into NMP (1 mL), and then stirred at 130 °C for 4 h. TLC detection indicated the complete reaction of raw materials, followed by work-up. The reaction solution was added with water (20 mL), and extracted with EA (10 mL×3). The organic phase was dried over anhydrous $Na_2SO_4$ and concentrated to dry, followed by column chromatography, to obtain compound 27 as white solid (20 mg, 45.51 mmol), with a yield of 45%. MS: *m/z* 471 [M+H]+.

$^1$H NMR (400 MHz, Chloroform-d) δ 8.01 (s, 1H), 8.02-7.92 (m, 1H), 7.74 (dd, *J* = 8.5, 5.2 Hz, 2H), 7.61-7.57 (m, 1H), 7.23 (d, *J* = 8.5 Hz, 2H), 7.19 (s, 1H), 6.45 (d, *J* = 8.6 Hz, 1H), 5.63 (q, *J* = 6.6 Hz, 1H), 3.66 (s, 4H), 2.43 (s, 3H), 1.60 (d, *J* = 6.7 Hz, 3H).

**Example 6: Synthesis of 2-(2,4-difluorophenyl)-8-(1-(indolin-1-yl)ethyl)-6-methyl-4*H*-benzopyran-4-one (compound 116)**

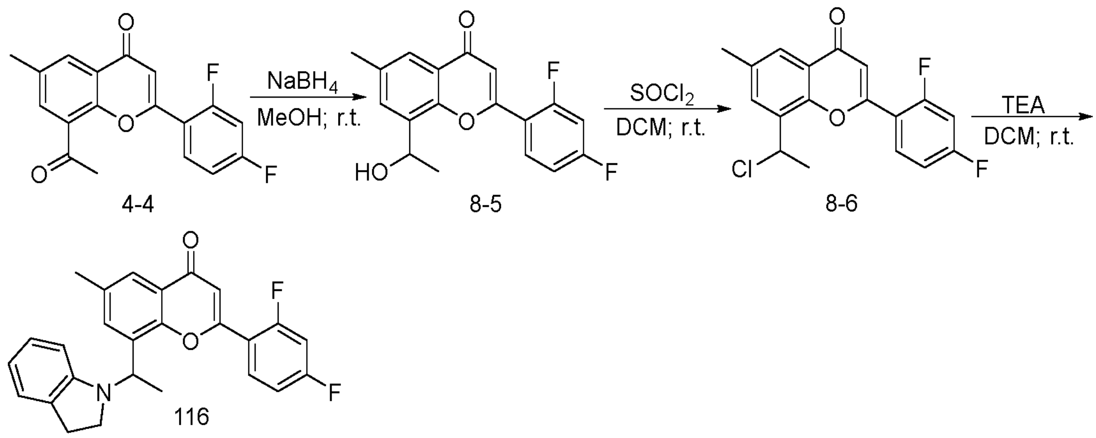


4-4 8-5 8-6 116

Step 1: Synthesis of intermediate 2-(2,4-difluorophenyl)-8-(1-hydroxyethyl)-6-methyl-4H-indolin-4-one **(8-5)**

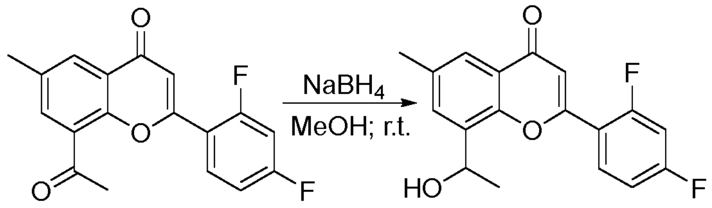


4-4 8-5

Procedures: Compound **4-4** (Its synthesis referred to the synthesis of general intermediates, 314 mg; 1 mmol) was dissolved in methanol (5ml), to which was added $NaBH_4$ (114 mg; 3 mmol) in an ice-water bath, and then the system was naturally warmed to room temperature, and stirred overnight. The reaction was detected by TLC. After completion of the reaction, the solution was poured into saturated $NH_4Cl$ solution, and extracted with dichloromethane (10 mL×3). The organic phase was dried over anhydrous $Na_2SO_4$ and rotatory evaporated to dry, to obtain pure compound **8-5** (273 mg; 0.86 mmol), with a yield of 86%. MS: *m/z* 317 $[M+H]^+$.

Step 2: Synthesis of intermediate 8-(1-chloroethyl)-2-(2,4-difluorophenyl)-6-methyl-4H-indolin-4-one **(8-6):**

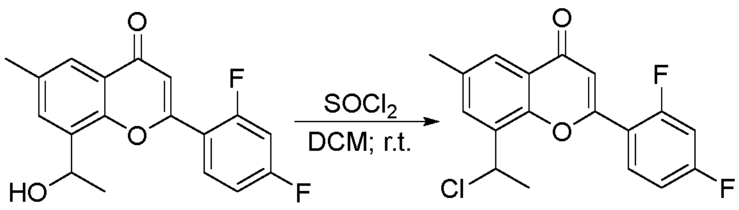


8-5 8-6

Procedures: compound **8-5** (273 mg; 0.86 mmol) was dissolved in DCM (3ml), to which was added thionyl chloride (113 mg; 0.95 mmol) under stirring at room temperature, and then the reaction was stirred overnight. The reaction was detected by TLC. After completion of the reaction, the reaction solution was mixed with silica gel and dried, and then subjected to column chromatography, to obtain pure compound **8-6** (280 mg; 0.86 mmol), with a yield of 84%. MS: *m/z* 335 $[M+H]^+$.

Step 3: Synthesis of target compound 2-(2,4-difluorophenyl)-8-(1-(indolin-1-yl)ethyl)-6-methyl-4H-benzopyran-4-one (compound 116):

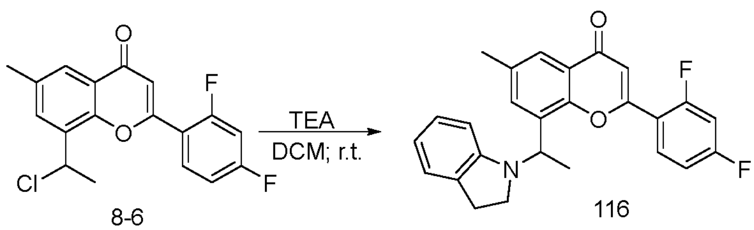


8-6 116

Procedures: compound **8-6** (50 mg; 0.15 mmol) was dissolved in DCM (1 mL), to which were successively added indoline (20 mg; 0.17 mmol) and triethylamine (18 mg; 0.18 mmol) under stirring at room temperature, and then the reaction was stirred overnight. The reaction was detected by TLC. After completion of the reaction, the reaction solution was subjected to column chromatography, to obtain pure compound 116 (30 mg; 0.07 mmol), with a yield of 84%. MS: *m/z* 418 $[M+H]^+$; $^1H$ NMR (400 MHz, DMSO-$d_6$) $\delta$ 8.02 (td, *J* = 8.9, 6.5 Hz, 1H), 7.83-7.77 (m, 1H), 7.74 (d, *J* = 2.2 Hz, 1H), 7.54 (ddd, *J* = 11.9, 9.2, 2.6 Hz, 1H), 7.15 (td, *J* = 8.5, 2.6 Hz, 1H), 7.04-6.96 (m, 1H), 6.93-6.84 (m, 1H), 6.77 (d, *J* = 0.9 Hz, 1H), 6.59-6.47 (m, 1H), 6.39 (d, *J* = 7.8 Hz, 1H), 5.37 (q, *J* = 6.9 Hz, 1H), 3.44 (t, *J* = 9.5 Hz, 2H), 2.91-2.77 (m, 2H), 2.44 (s, 3H), 1.54 (d, *J* = 6.9 Hz, 3H).

**Example 7: Synthesis of compound (R)-3-((1-(2-(2,4-difluorophenyl)-6-methyl-4-oxo-4*H*-chromen-8-yl)ethyl) amino)-*N*-(methyl-*d*$_3$)picolinamide (compound 48)**

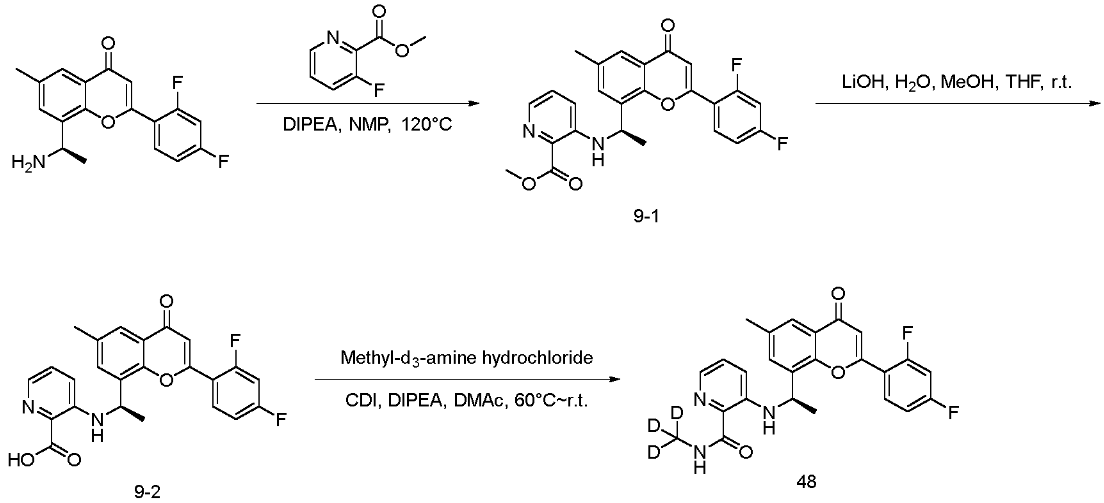


Step 1: Synthesis of intermediate methyl (R)-3-((1-(2-(2,4-difluorophenyl)-6-methyl-4-oxo-4*H*-chromen-8-yl)ethyl)amino)pyridinecarboxylate (**9-1**)

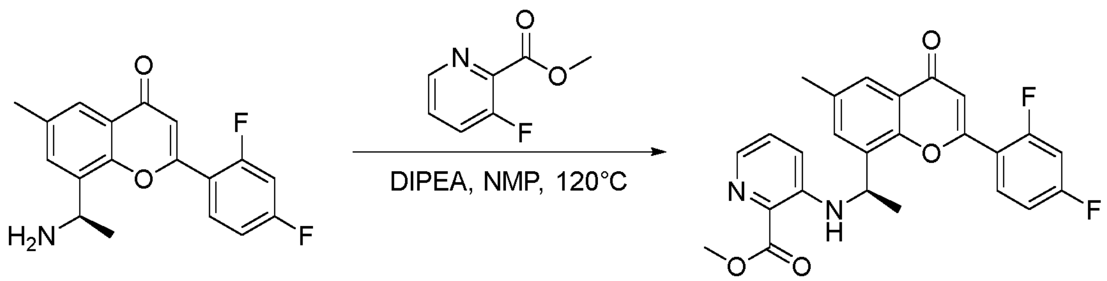


9-1

Procedures: (R)-8-(1-aminoethyl)-2-(2,4-difluorophenyl)-6-methyl-4*H*-chromen-4-one (200 mg, 0.63 mmol), methyl 3-fluoropyridinecarboxylate (197 mg, 1.27 mmol), and *N,N*-diisopropylethylamine (246 mg, 1.90 mmol) were added into *N*-methylpyrrolidone (2 mL), and then heated to 120 °C and allowed to react overnight under stirring. The reaction system was cooled to room temperature, and added with ethyl acetate (10 mL) for dilution. The resultant solution was washed with water (20 mL×2). The organic layer was dried over anhydrous $Na_2SO_4$, concentrated, and purified by column chromatography, to obtain 110 mg of product (Intermediate **9-1),** with a yield of 38.5%. MS: *m*/*z* 451.1 $[M+H]^+$.

Step 2: Synthesis of intermediate (R)-3-((1-(2-(2,4-difluorophenyl)-6-methyl-4-oxo-4H-chromen-8-yl)ethyl)amino)pyridinecarboxylic acid (9-2)

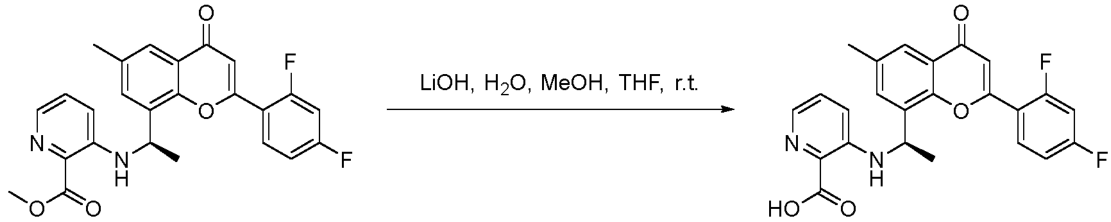


9-1 9-2

Procedures: Intermediate **9-1** (90 mg, 0.2 mmol) was dissolved in tetrahydrofuran (2 mL), to which were added lithium hydroxide monohydrate (84 mg, 2.0 mmol), water (1 mL), and methanol (1 mL), and then the mixture was stirred and reacted at room temperature for 4 h. The reaction solution was adjusted to be about pH 3 with 0.5 N of hydrochloric acid, and then extracted with dichloromethane (15 mL×2). The organic layer was dried over anhydrous $Na_2SO_4$, concentrated, and purified by column chromatography, to obtain 60 mg of product (Intermediate **9-2)**, with a yield of 68.8%. MS: *m/z* 437.2 $[M+H]^+$.

Step 3: Synthesis of compound (R)-3-((1-(2-(2,4-difluorophenyl)-6-methyl-4-oxo-4H-chromen-8-yl)ethyl)amino)-*N*-(methyl-$d_3$)picolinamide (compound **48**)

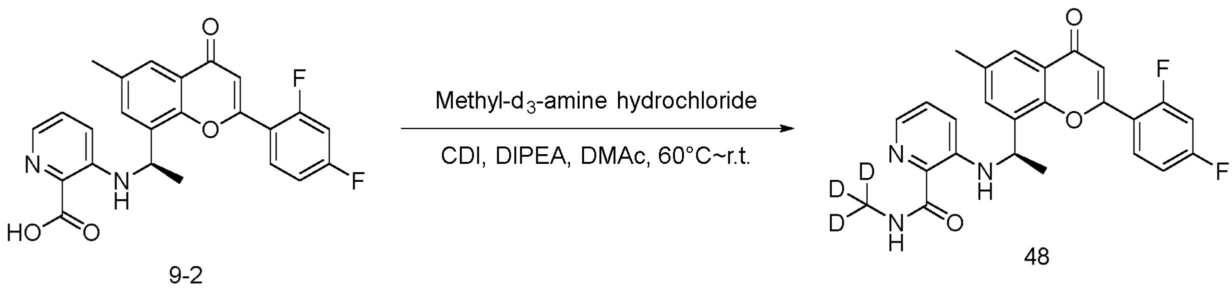


9-2 48

Procedures: Intermediate **9-2** (24 mg, 0.06 mmol) was dissolved in *N,N*-dimethylacetamide (0.5 mL), to which was added N,N'-carbonyldiimidazole (10 mg, 0.06 mmol), and then the mixture was heated to 60 °C and reacted for 1 h, followed by cooling to room temperature. To the reaction system, were added N,N-diisopropylethylamine (14 mg, 0.11 mmol) and methyl-$d_3$-amine hydrochloride (12 mg, 0.17 mmol), and then the reaction was stirred and reacted at room temperature for 2 h. The reaction solution was diluted with ethyl acetate (10 mL), and washed with water (20 mL×2). The organic layer was dried over anhydrous $Na_2SO_4$, concentrated, and purified by column chromatography, to obtain 13 mg of product (compound **48)**, with a yield of 52.2%. MS: *m/z* 453.2 $[M+H]^+$. $^1H$ NMR (400 MHz, DMSO-$d_6$) $\delta$ 9.11 (d, *J* = 6.6 Hz, 1H), 8.72 (s, 1H), 8.14 (td, *J* = 8.8, 6.4 Hz, 1H), 7.79 - 7.73 (m, 2H), 7.61 - 7.53 (m, 2H), 7.36 - 7.30 (m, 1H), 7.18 (dd, *J* = 8.6, 4.3 Hz, 1H), 6.90 (dd, *J* = 8.8, 1.3 Hz, 1H), 6.80 (d, *J= 0.8* Hz, 1H), 5.20 (p, *J* = 6.6 Hz, 1H), 2.35 (s, 3H), 1.62 (d, *J* = 6.6 Hz, 3H).

**Example 8: Synthesis of compound (R)-2-((1-(2-(2,4-difluorophenyl)-7-methyl-4-oxo-4*H*-pyrido[1,2-a]pyrimidin-9-yl)ethyl)amino)benzoic acid (compound 41)**

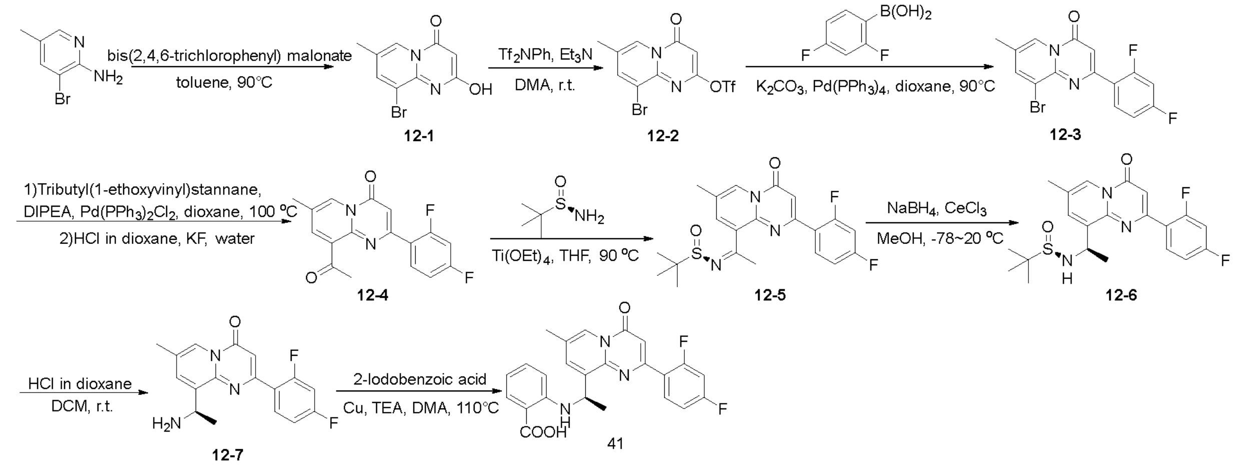


Intermediate: Synthesis of 9-bromo-2-hydroxy-7-methyl-4H-pyrido[1,2-a]pyrimidin-4-one (**12-1**)

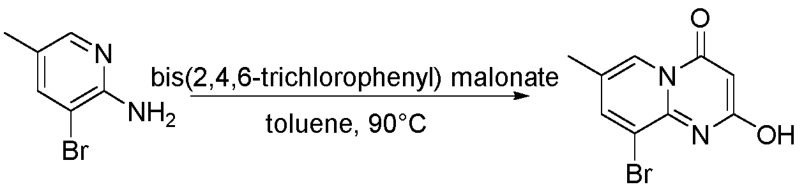


**12-1**

2-amino-3-bromo-5-methylpyridine (1.87 g, 10.0 mmol) and 2,4,6-bis(2,4,6-trichlorophenyl) maleate (5.56 g, 12.0 mmol) were successively added into toluene (19 mL), and then reacted at 90 °C for 4 h. TLC detection indicated the reaction was completed. The reaction solution was cooled to room temperature and filtered. The solid was rinsed with petroleum ether (20 mL), and then rotatory evaporated to dry, to obtain **12-1** (2.00 g, 0.79 mol), with a yield of 79%. MS: *m/z* 255.0 $[M+H]^+$.

Intermediate: Synthesis of 9-bromo-7-methyl-4-oxo-4*H*-pyrido[1,2-a]pyrimidin-2-yltrifluoromethanesulfonate **(12-2)**

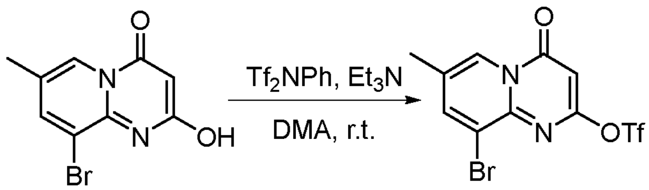


**12-1** **12-2**

**12-1** (2.00 g, 7.9 mmol), N-phenylbis(trifluoromethanesulphonimide) (5.64 g, 15.8 mmol), and triethylamine (1.60 g, 15.8 mmol) were successively added into DMA (20 mL), and then reacted at room temperature for 4 h. TLC detection indicated the reaction was completed. The reaction system was added with water (20 mL), and extracted with ethyl acetate (20 mL). The organic layer was concentrated to dry, and then the residue was purified by column chromatography, to obtain **12-2** (2.2 g, 5.7 mmol), with a yield of 72%. MS: *m/z* 386.9 $[M+H]^+$.

Intermediate: Synthesis of 9-bromo-2-(2,4-difluorophenyl)-7-methyl-4H-pyrido[1,2-a]pyrimidin-4-one **(12-3)**

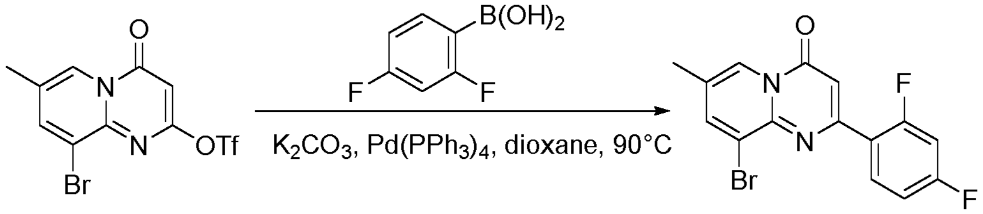


**12-2** **12-3**

**12-2** (2.2 g, 5.7 mmol), 2,4-difluorophenylboronic acid (1.8 g, 11.4 mmol), potassium carbonate (1.6 g, 11.4 mmol), and tetrakis(triphenylphosphine)palladium (0.7 g, 0.6 mmol) were successively added into 1,4-dioxane (22 mL), and then reacted overnight at 90 °C. TLC detection indicated the reaction was completed. The reaction system was cooled to room termperature, added with water (20 mL), and then extracted with ethyl acetate (20 mL). The organic layer was concentrated to dry, and then the residue was purified by column chromatography, to obtain **12-3** (1.5 g, 4.3 mmol), with a yield of 75%. MS: *m*/*z* 351.0 $[M+H]^+$.

Intermediate: Synthesis of 9-acetyl-2-(2,4-difluorophenyl)-7-methyl-4H-pyrido[1,2-a]pyrimidin-4-one **(12-4)**

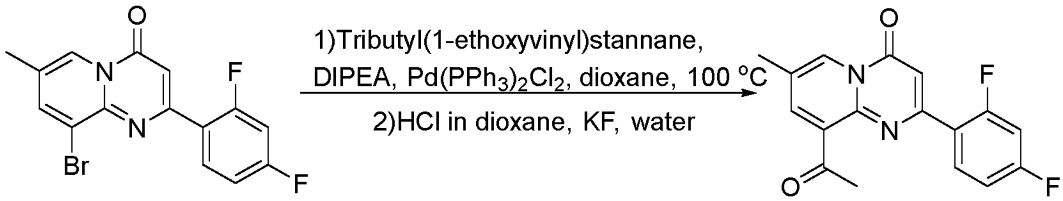


**12-3** **12-4**

**12-3** (1.5 g, 4.3 mmol), tributyl(1-ethoxyvinyl)tin (2.3 g, 6.5 mmol), $Pd(PPh_3)Cl_2$ (0.28 g, 0.4 mmol), and DIPEA(1.1 g, 8.6 mmol) were successively added into anhydrous DMA (15 mL), and reacted at 100 °C for 2h under nitrogen protection. TLC detection indicated the reaction was completed. The reaction system was cooled to room termperature, to which was added 4N HCl/dioxane to adjust pH to be 2-3, followed by addition of water (1 mL). After stirring for 2 h, TLC detection indicated completion of the reaction, and then KF saturated solution was added. The resultant solution was stirred for 2 h and filtered. The filtrate was extracted with ethyl acetate (20 mL×2). The solid was triturated in ethyl acetate (20 mL) and filtered. The organic layer was combined, dried over anhydrous $Na_2SO_4$, and filtered. The organic layer was concentrated to dry. The residue was purified by column chromatography, to obtain **12-4** (1.3 g, 4.1 mmol), with a yield of 95%. MS: *m*/*z* 315.1 $[M+H]^+$.

Intermediate: Synthesis of (R,Z)-N-(1-(2-(2,4-difluorophenyl)-7-methyl-4-oxo-4H-pyrido[1,2-a]pyrimidin-9-yl)ethylene)-2-methylpropane-2-sulfonamide (12-5)

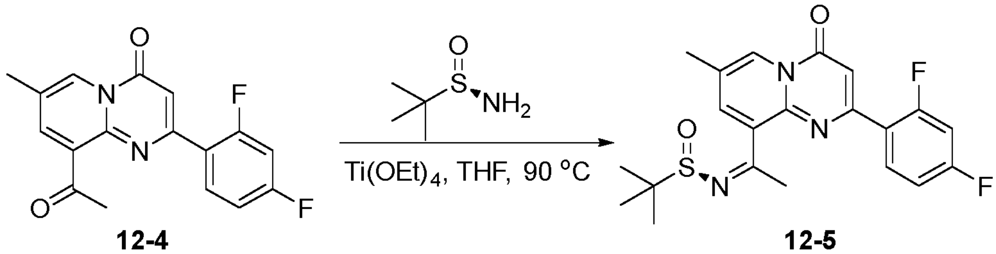


**12-4** **12-5**

**12-4** (1.3 g, 4.1 mmol), (R)-(+)-tert-butylsulfenamide (1.0 g, 8.2 mmol) and tetraethyl titanate (4.7 g, 20.5 mmol) were added into THF (13 mL), and then reacted overnight at 90 °C. TLC detection indicated completion of the reaction. The reaction solution was cooled to room temperature, and diluted with ethyl acetate (20 mL), followed by adding water (10 mL) and filtering. The solution was separated. The water layer was extracted with ethyl acetate (20 mL×2). The organic layer was combined, dried over anhydrous $Na_2SO_4$, and filtered. The organic layer was concentrated to dry. The residue was

purified by column chromatography, to obtain **12-5** (1.6 g, 3.8 mmol), with a yield of 93%. MS: *m/z* 418.1 [M+H]$^+$.

Intermediate: Synthesis of (R)-N-((R)-1-(2-(2,4-difluorophenyl)-7-methyl-4-oxo-4H-pyrido[1,2-a]pyrimidin-9-yl)ethyl)-2-methylpropane-2-sulfonamide **(12-6)**

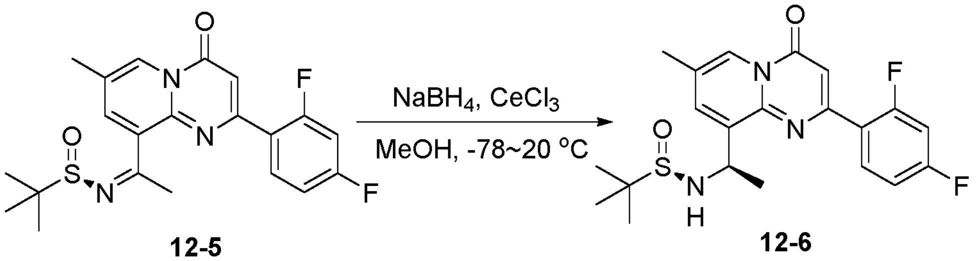


**12-5** **12-6**

**12-5** (1.6 g, 3.8 mmol) and $CeCl_3 \cdot 7H_2O$ (0.6 g, 1.9 mmol) were successively added into MeOH (32 mL), and then the system was cooled to (-60 °C)-(-70 °C) under nitrogen protection, followed by addition of $NaBH_4$ (0.4 g, 11.4 mmol) in portions. After addition, the reaction was naturally warmed to room temperature. TLC indicated disappearance of raw materials. The reaction was added with water (20 mL) and ethyl acetate (40 mL×2) for extraction. The organic layer was combined, dried over anhydrous $Na_2SO_4$, and filtered. The organic layer was concentrated to dry, and purified by column chromatography, to obtain **12-6** (1.0 g, 2.4 mmol), with a yield of 63%. MS: *m/z* 420.1 [M+H]$^+$.

Intermediate: Synthesis of (*R*)-9-(1-aminoethyl)-2-(2,4-difluorophenyl)-7-methyl-4H-pyrido[1,2-a]pyrimidin-4-one **(12-7)**

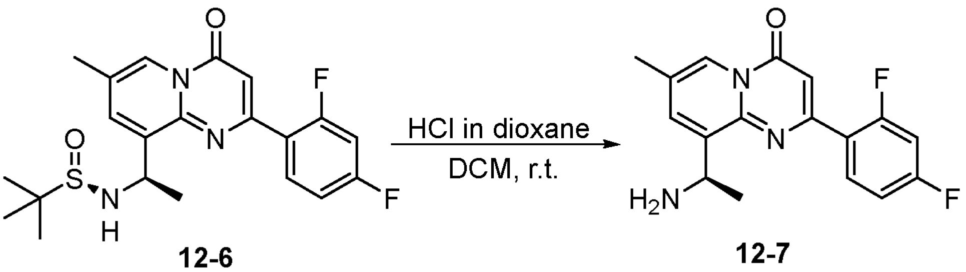


**12-6** **12-7**

**12-6** (1.0 g, 2.4 mmol) was dissolved in DCM (2 mL), to which was added the solution of HCl in dioxane (4N, 2 mL), and then the reaction was stirred at room temperature. TLC detection indicated completion of the reaction. The reaction solution was added with water (10 mL), and separated. The organic layer was removed, and then the water phase was adjusted to be pH 7-8 with saturated $NaHCO_3$ solution. The resultant solution was extracted with DCM (2 mL × 2). The organic layer was combined, dried over anhydrous $Na_2SO_4$, and filtered. The organic layer was concentrated to dry, to obtain **12-7** (0.63 g, 2.0 mmol), with a yield of 83%. MS: *m/z* 316.1 [M+H]$^+$.

Compound: Synthesis of (*R*)-2-((1-(2-(2,4-difluorophenyl)-7-methyl-4-oxo-4H-pyrido[1,2-a]pyrimidin-9-yl)ethyl)amino) benzoic acid (compound **41**)

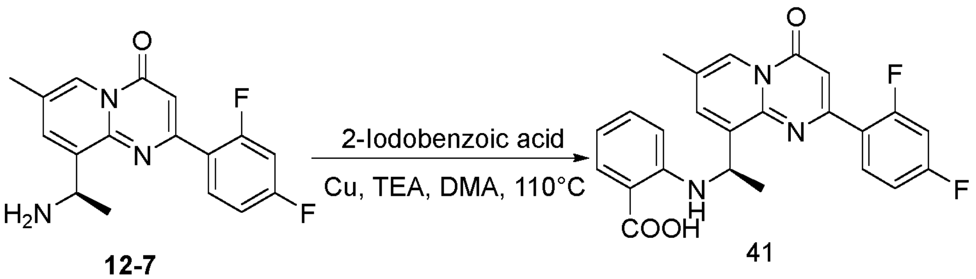


12-7

41

**12-7** (63 mg, 0.2 mmol), 2-iodobenzoic acid (99 mg, 0.4 mmol), copper powder (25 mg, 0.4 mmol), and triethylamine (60 mg, 0.6 mmol) were added into DMA (1 mL), and then stirred for 4 h at 110 °C under nitrogen protection. TLC indicated completion of the reaction. The reaction was added with water (2 mL) and ethyl acetate (1 mL×2) for extraction. The organic layer was combined, and concentrated to dry. The residue was purified by column chromatography, to obtain compound **41** (60 mg, 0.14 mmol), with a yield of 70%. MS: *m/z* 436.1 $[M+H]^+$.

$^1H$ NMR (400 MHz, DMSO-$d_6$) δ 12.78 (s, 1H), 8.76 (s, 1H), 8.54 (s, 1H), 8.34 - 8.26 (m, 1H), 7.84 - 7.78 (m, 2H), 7.51 - 7.43 (m, 1H), 7.34 - 7.27 (m, 1H), 7.18 (t, *J* = 7.8 Hz, 1H), 6.83 (d, *J* = 1.5 Hz, 1H), 6.54 (t, *J* = 7.5 Hz, 1H), 6.38 (d, *J* = 8.5 Hz, 1H), 5.55 - 5.44 (m, 1H), 2.35 (s, 3H), 1.64 (d, *J* = 6.6 Hz, 3H).

**Example 9: Synthesis of compound 3-((1-(2-(2,4-difluorophenyl)-6-methyl-4-oxo-4H-indolin-8-yl)ethyl)amino) piperidine-2,6-dione (compound 117)**

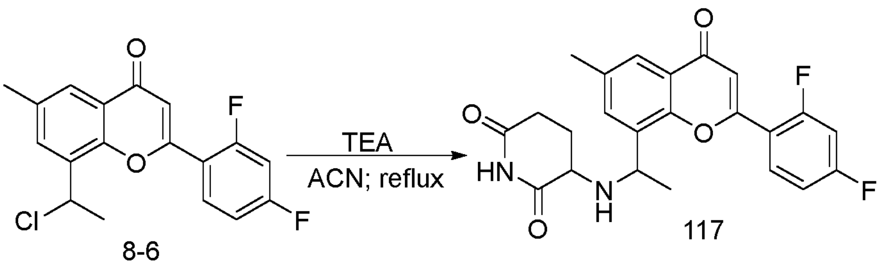


8-6

117

Step 1: Synthesis of target compound 3-((1-(2-(2,4-difluorophenyl)-6-methyl-4-oxo-4H-indolin-8-yl)ethyl)amino)piperidine-2,6-dione (compound **117)**

Procedures: Compound **8-6** (Its synthesis referring to the synthesis of intermediate **8-6** in Example 6, 50 mg; 0.15 mmol) was dissolved in acetonitrile (1 mL), to which were successively added 3-amino-piperidine-2,6-dione (22 mg; 0.17 mmol) and triethylamine (18 mg; 0.18 mmol) under stirring at room temperature, and then the reaction was heated to 90 °C and stirred overnight. The reaction was detected by TLC. After completion of the reaction, the reaction was purified by column chromatography, to obtain pure compound **117** (35 mg; 0.08 mmol), with a yield of 55%. MS: *m/z* 427 $[M+H]^+$; $^1H$ NMR (400 MHz, DMSO-$d_6$) δ 10.67 (s, 1H), 8.08 (tdd, *J* = 9.0, 6.3, 3.1 Hz, 1H), 7.82(s, 1H), 7.74 (s, 1H), 7.57 (ddd, *J* = 11.6, 8.8, 2.5 Hz, 1H), 7.38 (tt, *J* = 8.4, 3.1 Hz, 1H), 6.75 (s, 1H), 4.87-4.38 (m, 1H), 3.21 (m, 1H), 2.47-2.38 (m, 1H), 2.06 (m, 1H), 1.87 (m, 1H), 1.71 (m, 1H), 1.42 (m, 1H), 2.44 (s, 3H), 1.54 (d, *J* = 6.9 Hz, 3H).

**Example 10: Synthesis of 2-(2,4-difluorophenyl)-6-methyl-8-((1R)-1-((2-(2,2,2-trifluoro-1-hydroxyethyl)phenyl) amino)ethyl)-4H-chromen-4-one (compound 57)**

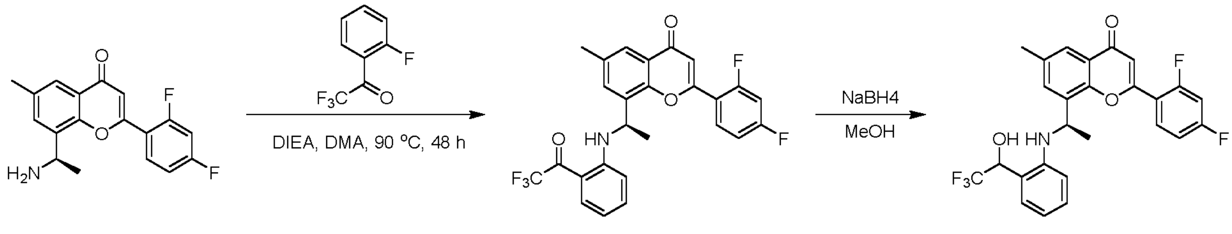


4-7 16-1 57

Step 1: Synthesis of (R)-2-(2,4-difluorophenyl)-6-methyl-8-(1-((2-(2,2,2-trifluoroacetyl) phenyl)amino)ethyl)-4*H*-chromen-4-one **(16-1)**

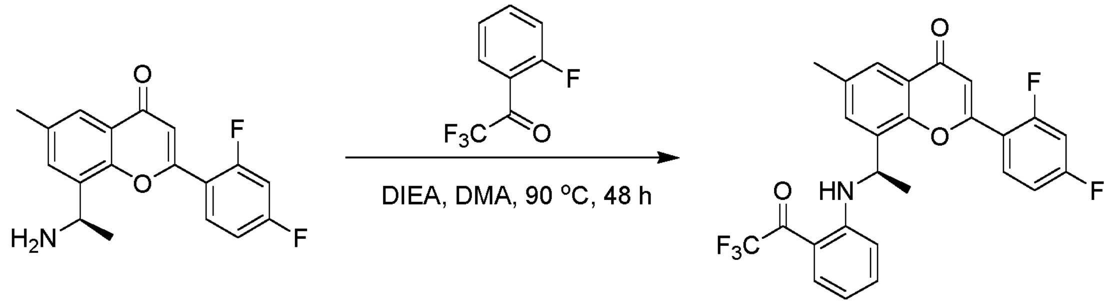


**4-7** **16-1**

Procedures: **4-7** (Its synthesis referred to the synthesis of general intermediates, 0.1 g, 317.14 μmol), 2,2,2-trifluoro-1-(2-fluorophenyl)ethane-1-one (121.85 mg, 634.28 μmol) and N-ethyl-N-isopropylpropan-2-amine (81.98 mg, 634.28 μmol) were dissolved in N,N-dimethylacetamide (5.0 mL), and then the reaction was stirred for 48 h at 95 °C under nitrogen protection. The reaction was detected by TLC. After completion of the reaction, the reaction was added with saline (10.0 mL) and EA (1 mL×3) for extraction. The organic phase was combined, dried over anhydrous $Na_2SO_4$, and rotatory evaporated to dry. The crude product was purified by column chromatography, to obtain compound **16-1** (76.0 mg, 155.92 mmol), with a yield of 49%. MS: *m*/*z* 488.1 $[M+H]^+$.

Step 2: Synthesis of compound 2-(2,4-difluorophenyl)-6-methyl-8-((1R)-1-((2-(2,2,2-trifluoro-1-hydroxyethyl)phenyl) amino)ethyl)-4H-chromen-4-one (compound **57)**

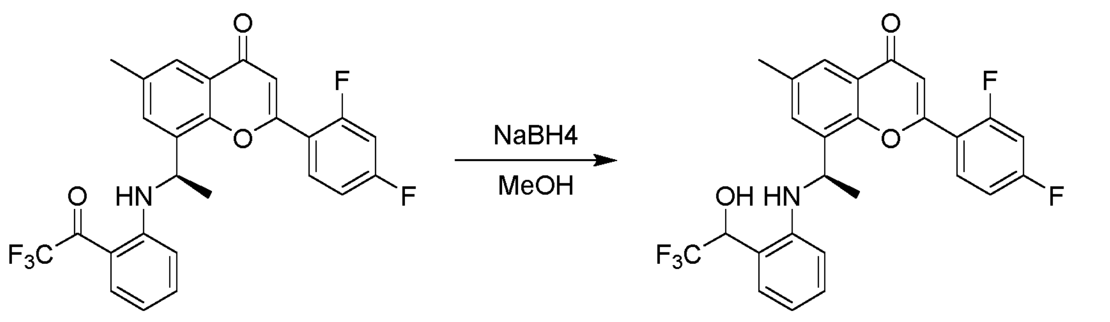


**16-1** 57

Procedures: **16-1** (70.0 mg, 143.61 μmol) was dissolved in methanol (5.0 mL), to which was added $NaBH_4$ (10.87 mg, 287.22 μmol), and then the reaction was stirred at room temperature for 2 h, and monitored by TLC. After completion of the reaction, the reaction solution was directly purified by column chromatography, to obtain compound 57 (30.0 mg; 61.29 mmol), with a yield of 42%. MS: *m*/*z* 490.1 $[M+H]^+$.

$^1H$ NMR (400 MHz, DMSO-$d_6$) δ 8.11 (td, *J* = 8.9, 6.5 Hz, 1H), 7.73 (dd, *J* = 2.3, 1.0 Hz, 1H), 7.62 - 7.54 (m, 2H),

7.38 - 7.23 (m, 2H), 6.97 (q, *J* = 6.5, 5.4 Hz, 2H), 6.81 (s, 1H), 6.56 (t, *J* = 7.4 Hz, 1H), 6.32 (d, *J* = 8.3 Hz, 1H), 6.13 (d, *J* = 7.1 Hz, 1H), 5.55 (d, *J* = *8.9* Hz, 1H), 5.16 (t, *J*= 6.8 Hz, 1H), 2.33 (s, 3H), 1.57 (d, *J* = 6.6 Hz, 3H).

**Example 11: Synthesis of compound (R)-2-((1-(2-(4,4-difluorocyclohexyl)-6-methyl-4-oxo-4H-chromen-8-yl) ethyl)amino)benzoic acid (compound 58)**

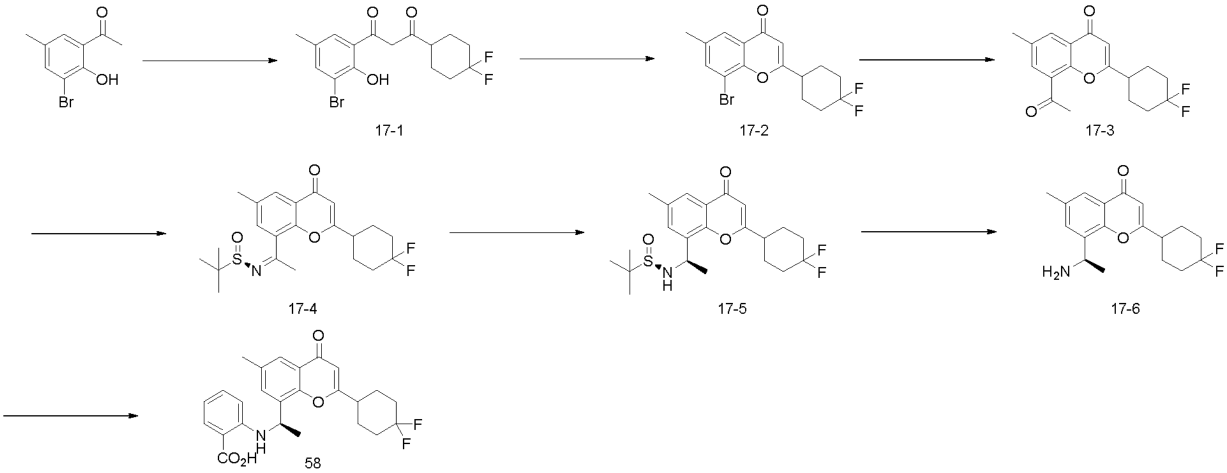

17-1 17-2 17-3 17-4 17-5 17-6 58

Step 1: Synthesis of intermediate 1-(3-bromo-2-hydroxy-5-methylphenyl)-3-(4,4-difluorocyclohexyl)propane-1,3-dione **(17-1)**

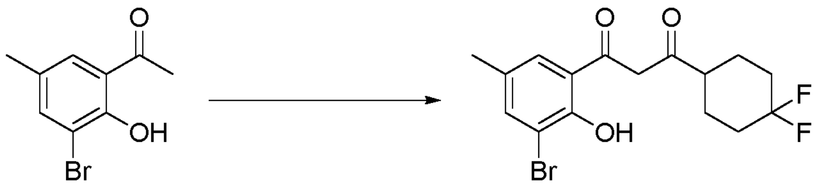

17-1

To a 100 mL reaction flask, were added 1-(3-bromo-2-hydroxy-5-methylphenyl)ethane-1-one (1.3 g, 5.7 mmol) and tetrahydrofuran (15 mL), to which was added Lithium bis(trimethylsilyl)amide (1 M, 34.2 mL, 34.2 mmol) dropwise at -70 °C, and then reacted at this temperature for 1 h. To a 50 mL reaction flask, were added compound 4,4-difluorocyclohexane-1-carboxylic acid (1.1 g, 6.8 mmol), N,N-dimethylformamide (one drop) and dichloromethane (20 mL), to which was added oxalyl chloride (0.73 g, 8.6 mmol) dropwise in an ice bath, and then reacted at room temperature for 1 h. After concentration, tetrahydrofuran (10 mL) was added, and then added dropwise to the reaction solution at -70 °C. The mixture was allowed to react overnight. After completion of the reaction, the solution was adjusted to pH = 5-6 with 1N of hydrochloric acid, and then extracted with ethyl acetate (30 mL × 3). The organic layers were combined, dried over anhydrous $Na_2SO_4$, and concentrated. The residue was purified by silica gel column chromatography, to obtain intermediate **17-1** (0.97 g), with a yield of 45%. MS: *m/z* 375.1, 377.1 $[M+H]^+$.

Step 2: Synthesis of intermediate 8-bromo-2-(4,4-difluorocyclohexyl)-6-methyl-4H-chromen-4-one **(17-2)**

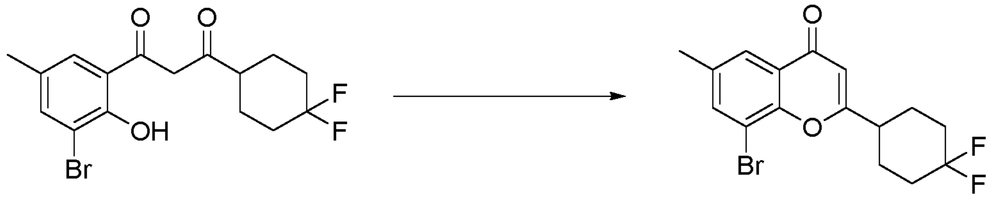

17-1 17-2

To a 50 mL reaction flask, were added compound **17-1** (970 mg, 2.6 mmol), concentrated sulfuric acid (2 mL) and acetic acid (10 mL), and then the reaction solution was heated 100 °C and reacted. After completion of the reaction, the reaction solution was cooled to room temperature, added with water (10 mL), and extracted with ethyl acetate (10 mL x 3). The organic layers were combined, dried over anhydrous $Na_2SO_4$, and concentrated. The residue was purified by silica gel column chromatography, to obtain intermediate **17-2** (870 mg), with a yield of 94%. MS: *m/z* 357.1, 359.1 $[M+H]^+$.

Step 3: Synthesis of intermediate 8-acetyl-2-(4,4-difluorocyclohexyl)-6-methyl-4H-chromen-4-one **(17-3)**

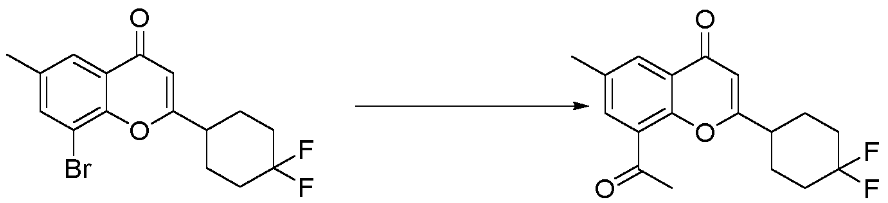

17-2 17-3

To a 100 mL reaction flask, were added compound **17-2** (870 mg, 2.5mmol), tributyl(1-ethoxyvinyl)tin (1.3 g, 3.7 mmol), diethylpropylethylamine (945 mg, 7.3 mmol) and dioxane (10 mL), and then the system was purged with nitrogen, followed by addition of tetrakis(triphenylphosphine)palladium(0) (282 mg, 0.24 mmol). The reaction was heated to 95 °C, and reacted overnight. After completion of the reaction, the solution was cooled to room temperature, to which was added 6N dilute hydrochloric acid (5 mL), and stirred for 1 h, followed by addition of saturated KF aqueous solution (15 mL). The reaction solution was stirred for 1 h, and filtered. The filter cake was rinsed with ethyl acetate (20 mL) for three times, and then the resultant solution was separated. The organic layer was dried over anhydrous $Na_2SO_4$, and concentrated. The residue was purified by silica gel column chromatography, to obtain intermediate **17-3** (670 mg), with a yield of 84%. MS: *m/z* 321.1 $[M+H]^+$.

Step 4: Synthesis of intermediate (R,Z)-N-(1-(2-(4,4-difluorocyclohexyl)-6-methyl-4-oxo-4H-chromen-8-yl)ethylene)-2-methylpropane-2-sulfonamide (17-4)

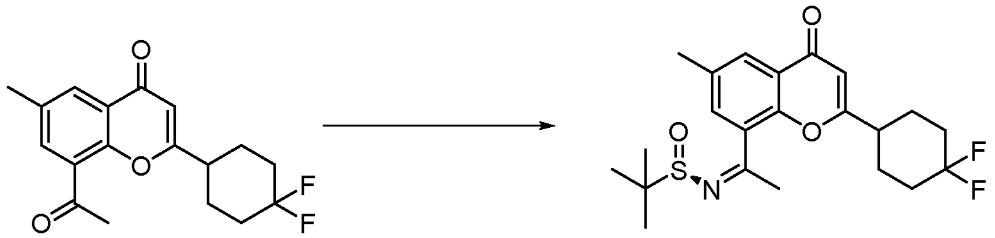

17-3 17-4

To a 50 mL reaction flask, were added compound **17-3** (670 mg, 2.1 mmol), (R)-tert-butylsulfenamide (507 mg, 4.2 mmol), tetraisopropyl titanate (2.4 g, 8.4 mmol) and tetrahydrofuran (15 mL), and then the system was purged with nitrogen, and heated overnight under refluxing. After completion of the reaction, the solution was cooled to room temperature, to which was added saturated saline (10 mL), and stirred for 0.5 h. The reaction solution was filtered. The filter cake was rinsed with ethyl acetate (10 mL) for three times, and then the resultant solution was separated. The organic layers were combined, dried over anhydrous $Na_2SO_4$, and concentrated. The residue was purified by silica gel

column chromatography, to obtain intermediate **17-4** (780 mg), with a yield of 88%. MS: *m/z* 424.1 [M+H]+.

Step 5. Synthesis of intermediate (R)-N-((R)-1-(2-(4,4-difluorocyclohexyl)-6-methyl-4-oxo-4H-chromen-8-yl)ethyl)-2-methylpropane-2-sulfonamide (17-5)

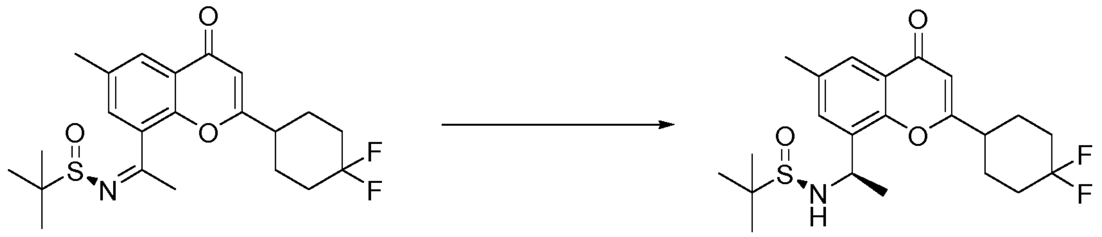

17-4 17-5

To a 50 mL reaction flask, were added compound 17-4 (780 mg, 1.8 mmol), cerous chloride heptahydrate (343 mg, 0.9 mmol) and methanol (10 mL), and then cooled to -70 °C under stirring, followed by adding the solution of $NaBH_4$ (210 mg, 5.5 mmol) in methanol (2 mL). After completion of the reaction, the solution was naturally warmed and kept overnight, to which was added ethyl acetate, and then filtered. The filtrate was rotatory evaporated, to which was then added ethyl acetate, followed by filtration. The filtrate was concentrated, and the residue was purified by silica gel column chromatography, to obtain intermediate 17-5 (500 mg), with a yield of 65%. MS: *m/z* 426.1 [M+H]+.

Step 6: Synthesis of intermediate ((R)-8-(1-aminoethyl)-2-(4,4-difluorocyclohexyl)-6-methyl-4H-chromen-4-one (17-6)

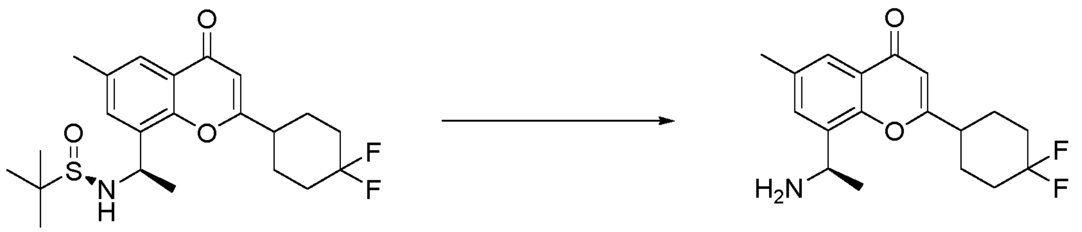

17-5 17-6

To a 25 mL reaction flask, were added compound **17-5** (500 mg, 1.2 mmol) and ethyl acetate (5 mL), to which was added the solution of HCl in dioxane (5 mL) under stirring, and then the reaction was stirred at room temperature until completion. The solvent was removed by concentration, and then $NaHCO_3$ aqueous solution was added. The resultant solution was extracted with dichloromethane (5mL x 3). The organic layers were combined, dried over anhydrous $Na_2SO_4$, and concentrated, to obtain intermediate **17-6** (250 mg), with a yield of 65%. MS: *m/z* 322.1 [M+H]+.

Step 7: Synthesis of compound (R)-2-((1-(2-(4,4-difluorocyclohexyl)-6-methyl-4-oxo-4H-chromen-8-yl)ethyl)amino) benzoic acid (compound **58)**

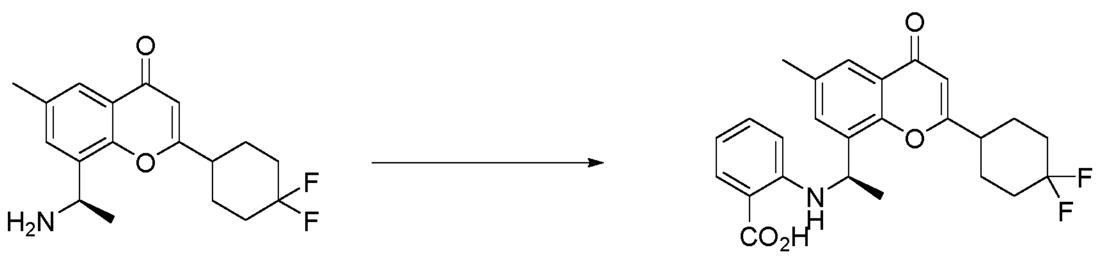

17-6 58

To a 25 mL reaction flask, were added compound **17-6** (60 mg, 0.19 mmol), 2-iodobenzoic acid (72 mg, 0.29 mmol), triethylamine (38 mg, 0.38 mml), and N,N-dimethylacetamide (1 mL), and then the system was purged with nitrogen, followed by addition of copper powder (12 mg, 0.19 mmol). The reaction was heated to 110 °C and allowed to react for about 1-2 h. After completion of the reaction, the solution was cooled to room temperature, and adjusted to be pH = 4-5 with 1N HCl aqueous solution. The resultant solution was added with ethyl acetate (5 mL), and then the layers were separated. The organic layers were combined, dried over anhydrous $Na_2SO_4$, and concentrated, followed by prep.-TLC, to obtain compound (compound 58, 18 mg), with a yield of 21%. MS: *m/z* 442.1 $[M+H]^+$, $^1H$ NMR (400 MHz, DMSO-$d_6$) $\delta$ 12.78 (s, 1H), 8.43 (s, 1H), 7.81 (dd, *J* = 7.9, 1.7 Hz, 1H), 7.69 (d, *J* = 2.1 Hz, 1H), 7.54 (d, *J*= 2.2 Hz, 1H), 7.28 - 7.16 (m, 1H), 6.54 (t, *J* = 7.5 Hz, 1H), 6.46 (d, *J* = 8.4 Hz, 1H), 6.25 (s, 1H), 5.13 (d, *J* = 6.7 Hz, 1H), 2.34 (s, 3H), 2.22 - 1.64 (m, 9H), 1.60 (d, *J* = 6.6 Hz, 3H).

**Example 12: Synthesis of compound methyl (R)-3-((1-(2-(2,4-difluorophenyl)-6-methyl-4-oxo-4H-chromen-8-yl) ethyl)amino)furan-2-carboxylate (compound 63)**

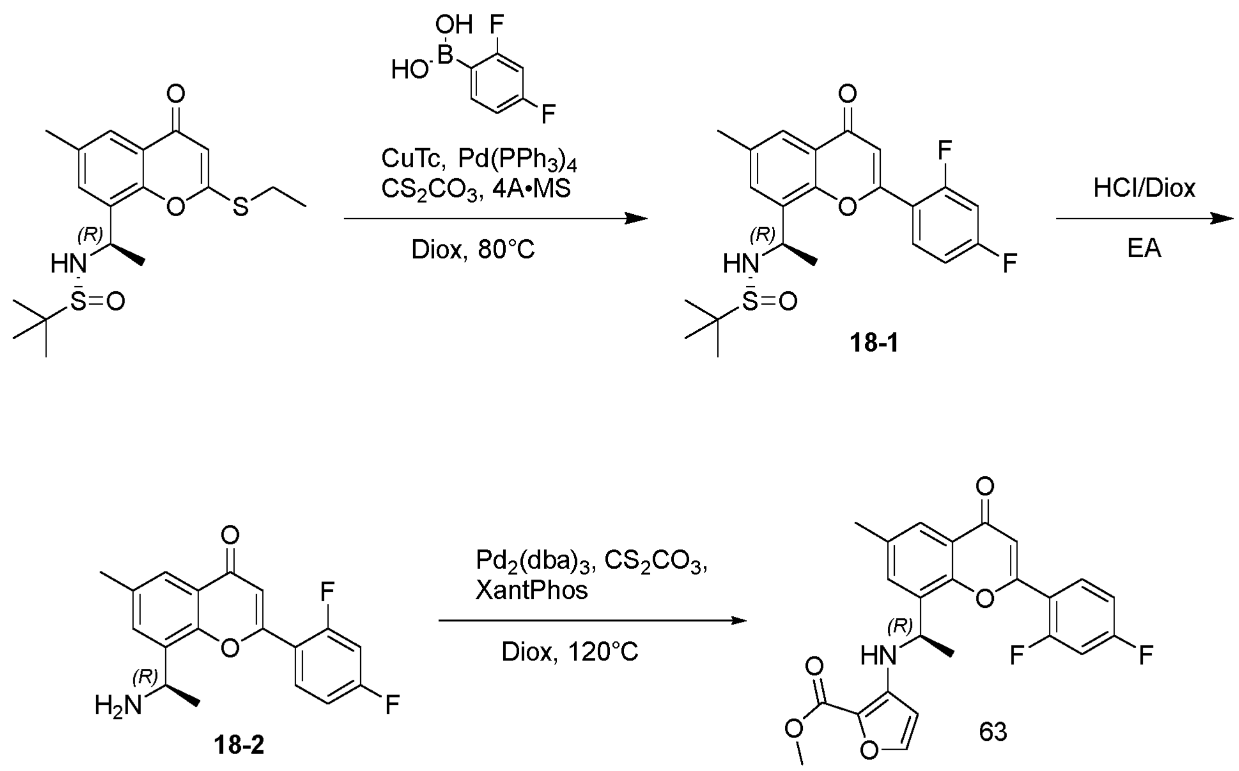

18-1
18-2
63

Step 1: Synthesis of intermediate (R)-N-((R)-1-(2-(2,4-difluorophenyl)-6-methyl-4-oxo-4H-chromen-8-yl)ethyl)-2-methylpropane-2-sulfonamide **(18-1)**

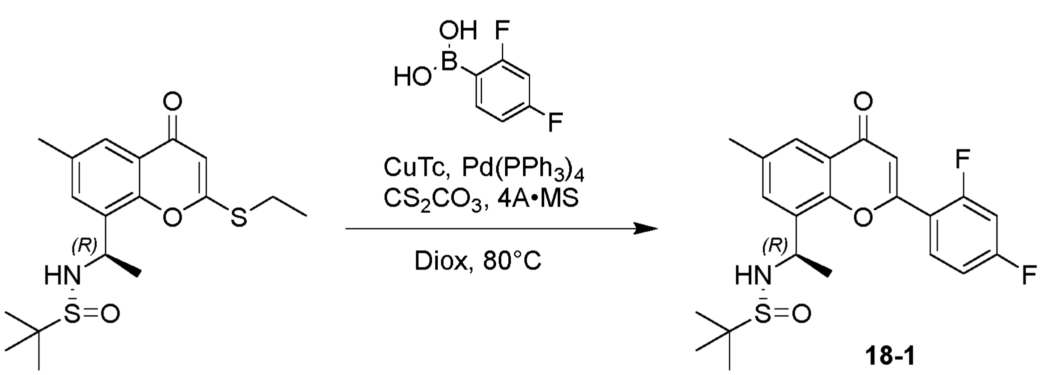

**18-1**

(R)-N-((R)-1-(2-(ethylthio)-6-methyl-4-oxo-4H-chromen-8-yl)ethyl)-2-methylpropane-2-sulfonamide (2.0 g, 5.45 mmol), (2,4-difluorophenyl)boric acid (6.9 g, 43.59 mmol), cesium carbonate (3.6 g, 10.91 mmol), copper(I) thiophene-2-carboxylate (5.2 g, 27.25 mmol), tetrakis(triphenylphosphine)palladium (1.2 g, 1.09 mmol), 4A molecular sieve (200 mg), and 1,4-dioxane (40 mL) were successively added into a sealed tank, and then under nitrogen protection, the mixture was allowed to react in the sealed tank at 100 °C until the reaction was completed. The reaction solution was cooled to room temperature, to which was added silica gel, followed by concentration. The concentrate was purified by silica gel column chromatography (petroleum ether/ethyl acetate = 1/1), to obtain (R) -N-((R)-1-(2-(2,4-difluorophenyl)-6-methyl-4-oxo-4H-chromen-8-yl)ethyl)-2-methylpropane-2-sulfonamide (1.0 g, 2.4 mmol), with a yield of 44%. MS: *m/z* 420 $[M+H]^+$.

Step 2: Synthesis of intermediate ((R) -8-(1-aminoethyl)-2-(2,4-difluorophenyl)-6-methyl-4H-chromen-4-one **(18-2)**

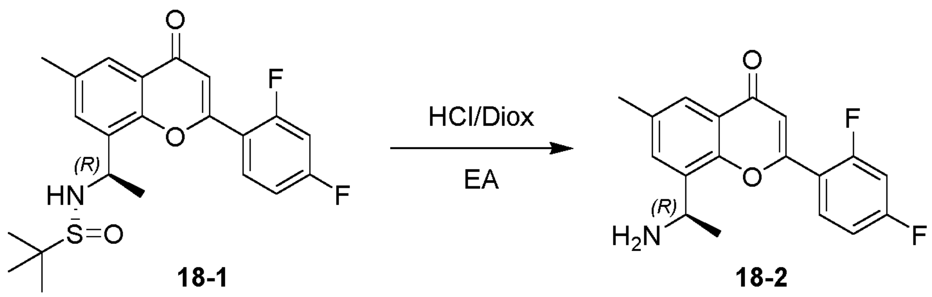


**18-1** **18-2**

**18-1** (1.0 g, 2.38 mmol) was dissolved in ethyl acetate (20 mL), and then cooled in an ice-water bath, to which was added the solution of HCl in dioxane (4N, 5 mL). The mixture was stirred and reacted at room temperature for 2 h. The reaction was monitored by TLC. After completion of the reaction, the solution was added with water (30 mL), and then poured into separatory funnel and separated. The water phase was adjusted to pH 7-8 with $Na_2CO_3$, and then extracted with ethyl acetate (50 mL×3). The organic phase was dried over anhydrous $Na_2SO_4$, and concentrated, to obtain (R)-8-(1-aminoethyl)-2-(2,4-difluorophenyl)-6-methyl-4H-chromen-4-one (700 mg, 2.2 mmol), with a yield of 93%. MS: *m/z* 316 $[M+H]^+$.

Step 3: Synthesis of compound methyl (R)-3-((1-(2-(2,4-difluorophenyl)-6-methyl-4-oxo-4H-chromen-8-yl)ethyl)amino) furan-2-carboxylate (compound 63)

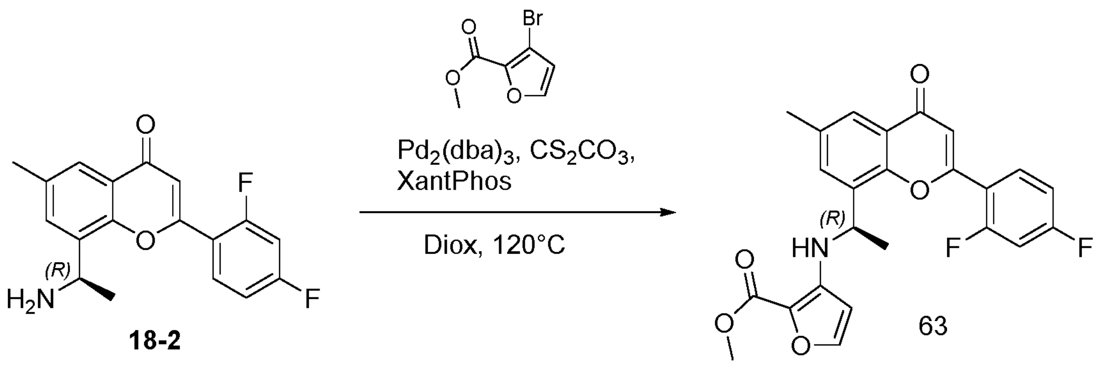


**18-2** 63

**18-2** (100 mg, 0.32 mmol), methyl 3-bromofuran-2-carboxylate (162 mg, 0.79 mmol), cesium carbonate (310 mg, 0.95 mmol), 4,5-bis(diphenylphosphino)-9,9-dimethylxanthene (55 mg, 0.09 mmol), and tris(dibenzylideneacetone) dipalladium (58 mg, 0.09 mmol) were added to 1,4-dioxane (2 mL), and under nitrogen protection, the mixture was reacted at 120 °C. The reaction was monitored by TLC. After completion of the reaction, the solution was cooled to room temperature, to which was directly added silica gel, followed by concentration. The concentrate was purified by silica gel column chromatography (petroleum ether/ethyl acetate = 2/1), to obtain methyl (R)-3-((1-(2-(2,4-difluorophenyl)-6-methyl-4-oxo-4H-chromen-8-yl)ethyl)amino)furan-2-carboxylate (20 mg, 0.05 mmol), with a yield of 17%. MS: *m/z* *440* $[M+H]^+$.

$^1H$ NMR (400 MHz, Chloroform-d) $\delta$ 7.93 (dd, $J$ = 2.4, 0.8 Hz, 1H), 7.84 (d, $J$ = 6.2 Hz, 1H), 7.52 (d, $J$ = 2.4 Hz, 1H), 7.16 (d, $J$ = 2.2 Hz, 1H), 7.11 - 7.07 (m, 1H), 7.02 (s, 1H), 6.84 (s, 1H), 5.89 (d, $J$ = 2.0 Hz, 1H), 5.11 (q, $J$ = 6.6 Hz, 1H), 3.91 (s, 3H), 2.43 (s, 3H), 1.67 (d, $J$ = 6.8 Hz, 3H).

**Example 13: Synthesis of compound (R,E)-2-(2,4-difluorophenyl)-6-methyl-8-(1-((2-(2,2,2-trifluoro-1-(hydroxyimino)ethyl)phenyl)amino)ethyl)-4H-chromen-4-one (compound 64)**

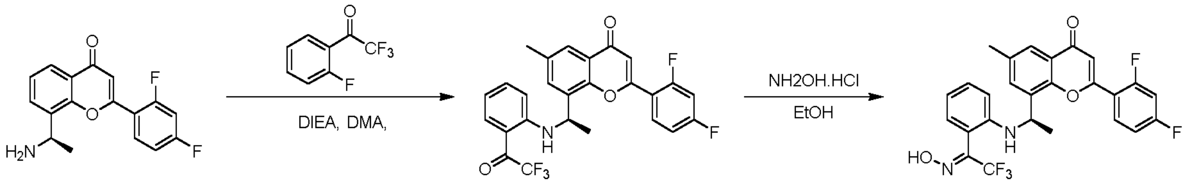


4-7 19-1 64

Step 1: Synthesis of (R)-2-(2,4-difluorophenyl)-6-methyl-8-(1-((2-(2,2,2-trifluoroacetyl) phenyl)amino)ethyl)-4H-chromen-4-one (19-1)

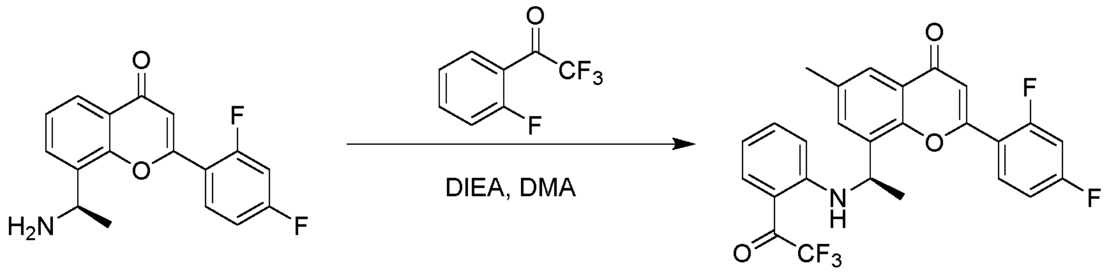


**4-7** **19-1**

Procedures: **4-7** (Its synthesis referred to the synthesis of general intermediates, 0.1 g, 317.14 mmol), 2,2,2-trifluoro-1-(2-fluorophenyl)ethane-1-one (121.85 mg, 634.28 mmol), and N-ethyl-N-isopropylpropan-2-amine (81.98 mg, 634.28 mmol) were dissolved in N,N-dimethylacetamide (2.0 mL), and then reacted and stirred at 80 °C under nitrogen protection. The reaction was monitored by TLC. After completion of the reaction, the solution was added with saline (5.0 mL) and extracted with EA (5 mL×3). The organic phase was combined, dried over anhydrous $Na_2SO_4$, and concentrated. The crude product was purified by column chromatography, to obtain **19-1** (80 mg, 164.13 mmol), with a yield of 51%. MS: *m/z 488.1* $[M+H]^+$.

Step 2: Synthesis of compound (R,E)-2-(2,4-difluorophenyl)-6-methyl-8-(1-((2-(2,2,2-trifluoro-1-(hydroxyimino)ethyl) phenyl)amino)ethyl)-4H-chromen-4-one (compound **64):**

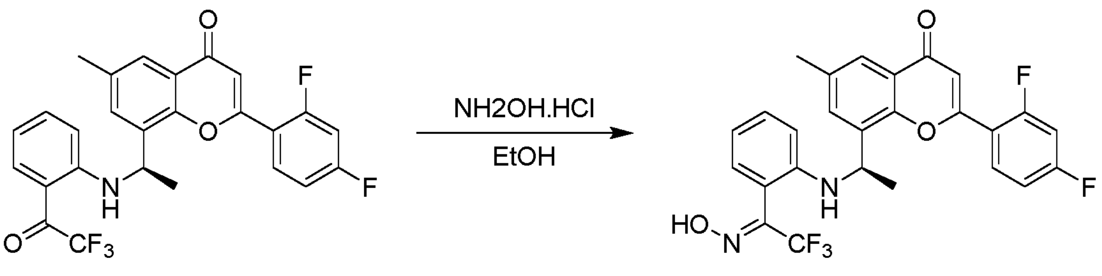


**19-1** 64

Procedures: **19-1** (20.0 mg, 47.19 μmol) was dissolved in ethanol (4.0 mL), to which was added hydroxylamine hydrochloride (29.84 mg, 441.56 mmol). The reaction was stirred at 80 °C for 12 h. The reaction was monitored by TLC. After completion of the reaction, the reaction solution was directly purified by column chromatography, to obtain compound 64 (9.0 mg, 17.91 mmol), with a yield of 43%. MS: *m/z* 503.1 [M+H]$^+$.

$^1$H NMR (400 MHz, DMSO-$d_6$) $\delta$ 11.06 (s, 1H), 9.03 (d, *J* = 6.5 Hz, 1H), 7.96 (td, *J* = 8.9, 6.5 Hz, 1H), 7.70 (d, *J* = 8.4 Hz, 1H), 7.59 (dd, *J* = 2.1, 0.9 Hz, 1H), 7.53-7.41 (m, 2H), 7.29 (d, *J* = 2.2 Hz, 1H), 7.04 (s, 1H), 6.76-6.68 (m, 2H), 5.26 (h, *J* = 6.6, 6.2 Hz, 1H), 2.28 (s, 3H), 1.63 (d, *J* = 6.7 Hz, 3H).

**Example 14: Synthesis of compound (R)-6-chloro-3-((1-(6-fluoro-2-(5-fluoropyridin-2-yl)-3-deuteromethyl-4-oxo-3,4-dihydroquinazolin-8-yl)ethyl)amino)benzoic acid (compound 88)**

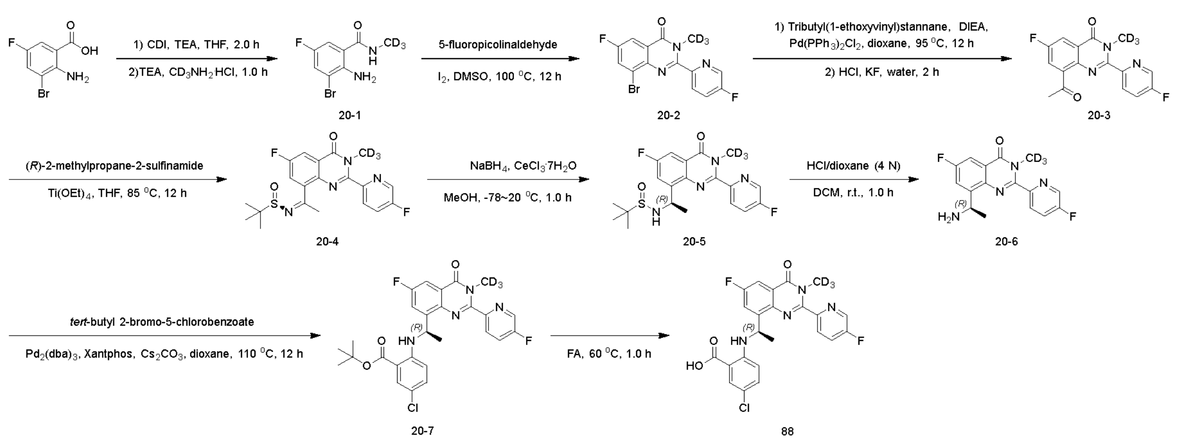


Step 1: Synthesis of intermediate 2-amino-3-bromo-5-fluoro-*N*-deuteromethylbenzene (20-**1)**

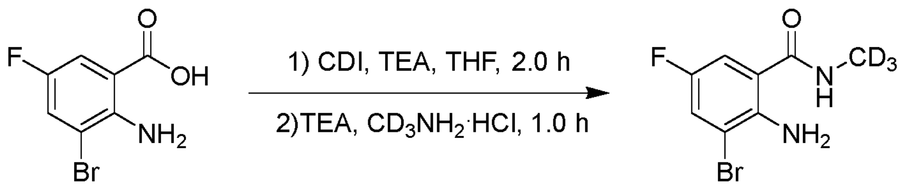


20-1

2-amino-3-bromo-5-fluoro-benzoic acid (4.50 g, 19.2 mmol) was dissolved in anhydrous THF (50 mL), to which was added CDI (4.67 g, 28.8 mmol) in batches, and then the system was gradually bubbling up. After reaction for 2h, the system was cooled to 0-10 °C, to which were added DIEA (7.95 g, 79.2 mmol) and methyl-$d_3$-amine hydrochloride (5.58, 79.2 mmol), and then the mixture was allowed to react for 1.0 h. TLC detection indicated completion of the reaction. The reaction solution was added with water (100 mL) and EA (40 mL×2) for extraction, and then the organic phase was concentrated. The residue was triturated in ethanol and filtered, to obtain **20-1** (3.20 g, 9.14 mmol), with a yield of 66%. MS: *m/z* 250/252 [M+H]$^+$.

Step 2: Synthesis of intermediate 8-bromo-6-fluoro-2-(5-fluoropyridin-2-yl)-3-deuteromethylquinazolin-4(3*H*)-one **(20-2)**

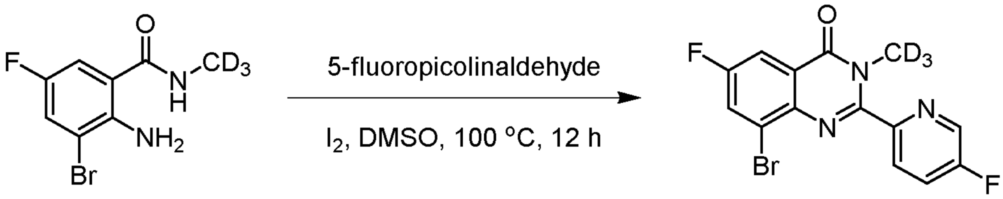


20-1 20-2

**20-1** (3.00 g, 12.0 mmol), 5-fluoropyridin-2-aldehyde (1.80 g, 14.4 mmol) and iodine (3.65 g, 14.4 mol) were successively dissolved in DMSO (45 mL), and then stirred at 100 °C. The reaction was detected with TLC. After completion of the reaction, the system was added with ice-water (100 mL), followed by adding 5.0 mL of saturated sodium thiosulfate solution, and then filtered to obtain the crude product, which was triturated in ethanol and filtered, to obtain **20-2** (4.20 g, 11.8 mmol), with a yield of 98%. MS: *m/z* 355/257 $[M+H]^+$.

Step 3: Synthesis of intermediate 8-acetyl-6-fluoro-2-(5-fluoropyridin-2-yl)-3-deuteromethylquinazolin-4(3H)-one **(20-3)**

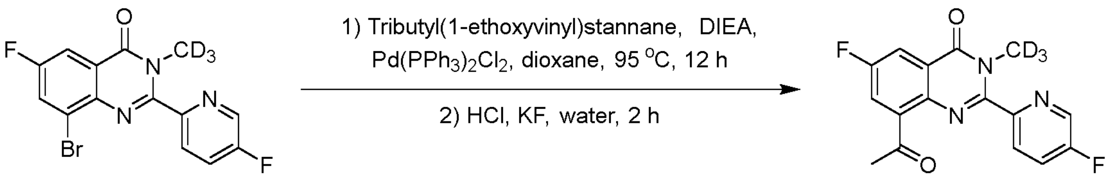


20-2 20-3

**20-2** (4.20 g, 11.8 mmol), Tin reagent (6.37 g, 17.7 mmol), DIEA (4.52 g, 35.2 mol), and $Pd(PPh_3)Cl_2$ (0.82 g, 0.12 mmol) were successively added to dioxane (45 mL), and under nitrogen protection, the reaction was allowed to react at 95 °C for 12 h. TLC detection indicated completion of the reaction. After cooling, the reaction solution was added with DCM (40 mL), and adjusted to pH 2-3 with 6 M HCl, followed by stirring for 1-2 h. The reaction was monitored by TLC, and then added with KF saturated solution, followed by stirring for 1.0 h (pH=7). The reaction solution was filtered, and the filter cake was rinsed with DCM. The water phase was extracted with DCM (30 mL×2). The organic phase was dried over anhydrous $Na_2SO_4$, and concentrated. The crude product was triturated in MTBE, to obtain 20-3 (3.20 g, 10.1 mmol), with a yield of 85%. MS: *m/z* 319 $[M+H]^+$.

Step 4: Synthesis of intermediate (*R*,Z)-N-(1-(6-fluoro-2-(5-fluoropyridin-2-yl)-3-deuteromethyl-4-oxo-3,4-dihydroquinazolin-8-yl)ethylene)-2-methylpropane-2-sulfonamide (**20-4**)

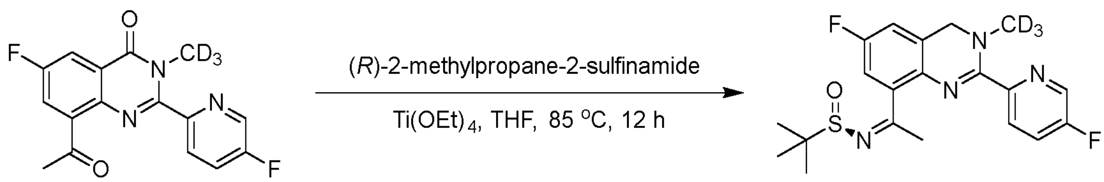


20-3 20-4

**20-3** (3.20 g, 10.1 mmol), (*R*)-(+)-tert-butylsulfenamide (3.06 g, 25.2 mmol) and tetraethyl titanate (11.0 g, 50.5 mmol) were added into anhydrous THF (32 mL), and then stirred at 85 °C for 12 h. TLC detection indicated completion of the reaction. The reaction solution was poured to 100 mL of water, and filtered. The filter cake was rinsed with DCM, and then poured into separatory funnel and separated, followed by extraction with DCM (30 mL×2). The organic phase was dried over anhydrous $Na_2SO_4$, and concentrated to obtain the crude product **20-4** (3.50 g, 7.55 mmol), which was directly used in the next step. MS: *m/z* 422 $[M+H]^+$.

Step 5. Synthesis of intermediate *N*-((*R*)-1-(6-fluoro-2-(5-fluoropyridin-2-yl)-3-deuteromethyl-4-oxo-3,4-dihydroquinazolin-8-yl)ethyl)-2-methylpropane-2-sulfonamide **(20-5)**

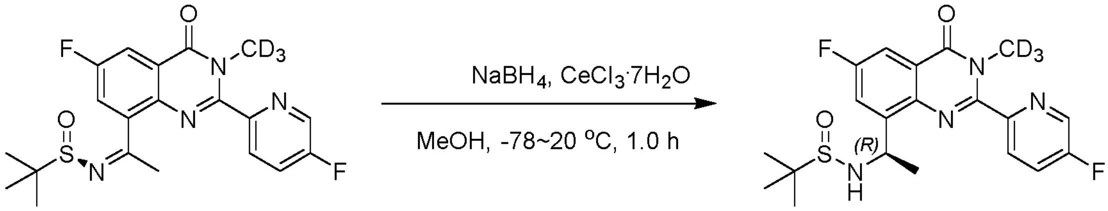


20-4 20-5

The crude product **20-4** (3.50 g, 7.55 mmol) and $CeCl_3 \cdot 7H_2O$ (1.32 g, 3.77 mmol) were successively added to MeOH (70 mL), and then the reaction system was cooled to (-60 °C)-(-70 °C), to which was added $NaBH_4$ (0.63 g, 15.1 mmol) in portions. After addition, the reaction was slowly warmed to room temperature, and then detected with TLC until completion. The reaction solution was added with saturated $NH_4Cl$ aqueous solution (200 mL), and extracted with DCM (80 mL×2). The organic phase was concentrated to obtain the crude product, which was purified by column chromatography, to provide 20-5 (1.90 g, 4.48 mmol), with a yield of 44% for two steps. MS: *m*/*z* 424 $[M+H]^+$. $^1$H-NMR (400 MHz, DMSO-$d_6$) $\delta$ 8.75 (t, *J* = 1.8 Hz, 1H), 8.04 (dd, *J* = 6.7, 1.8 Hz, 2H), 7.87 (dd, *J* = 9.8, 3.1 Hz, 1H), 7.78 (dd, *J* = 8.4, 3.0 Hz, 1H), 6.00 (d, *J* = 8.9 Hz, 1H), 5.31-5.19 (m, 1H), 1.43 (d, *J* = 6.8 Hz, 3H), 1.09 (s, 9H).

Step 6: Synthesis of intermediate (*R*)-8-(1-aminoethyl)-6-fluoro-2-(5-fluoropyridin-2-yl)-3-deuteromethylquinazolin-4(3*H*)-one (**20-6**)

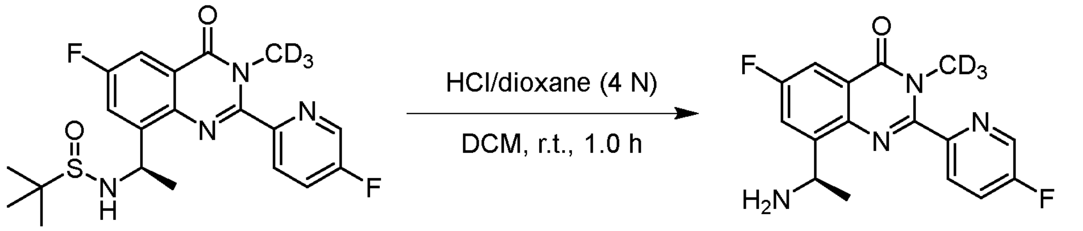


20-5 20-6

**20-5** (1.90 g, 4.48 mmol) was dissolved in DCM/THF (10 mL/20 mL), to which was added the solution of HCl in dioxane, and then stirred at room temperature. The solid precipitated. TLC detection indicated completion of the reaction. After filtration, the solid was dissolved in water, and after the solution became clear, it was adjusted to pH 7-9 with saturated $NaHCO_3$ solution. The solid precipitated, collected by filtration, and dried, to obtain **20-6** (1.00 g, 3.13 mmol), with a yield of 70%. MS: *m*/*z* 320 $[M+H]^+$.

Step 7: Synthesis of intermediate tert-butyl (*R*)-5-chloro-2-((1-(6-fluoro-2-(5-fluoropyridin-2-yl)-3-deuteromethyl-4-oxo-3,4-dihydroquinazolin-8-yl)ethyl)amino)benzoate (**20-7**)

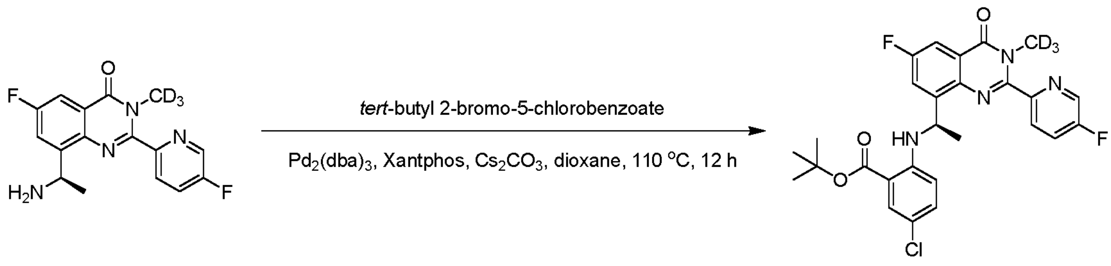


20-6 20-7

**20-6** (150 mg, 0.47 μmol), tert-butyl 2-bromo-5-chlorobenzoate (120 mg, 1.41 μmol), $Pd_2(dba)_3$ (43.0 mg, 0.04 μmol), Xantphos (54.0 mg, 0.09 μmol), and $Cs_2CO_3$ (450 mg, 1.41 μmol) were added into dioxane (2.0 mL), and under nitrogen protection, the reaction was stirred at 110 °C for 12 h. The reaction was monitored by TLC. After completion of the reaction, the solution was added with water (20 mL), and extracted with EA (10 mL×3). The organic phase was concentrated to obtain the crude product, which was purified by column chromatography, to provide **20-7** (170 mg, 0.32 μmol), with a yield of 68%. MS: *m*/*z* 531 $[M+H]^+$.

Step 8: Synthesis of compound (R)-5-chloro-2-((1-(6-fluoro-2-(5-fluoropyridin-2-yl)-3-deuteromethyl-4-oxo-3,4-dihydroquinazolin-8-yl)ethyl)amino)benzoic acid (compound **88**)

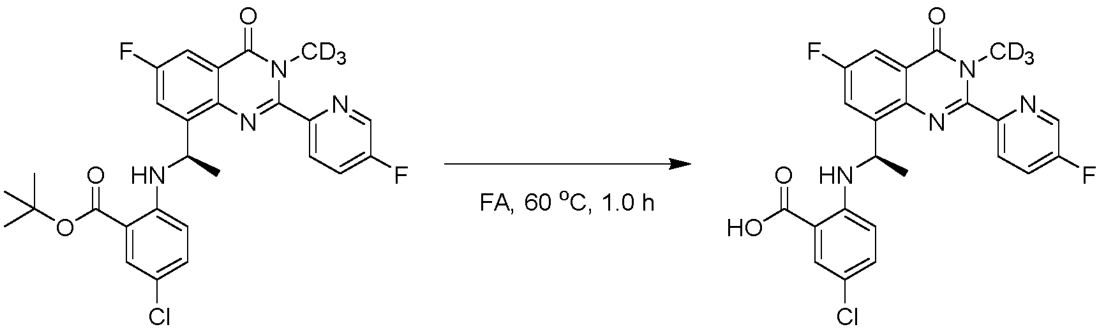


20-7 88

**20**-7 (170 mg, 0.32 μmol) was dissolved in FA (2.0 mL), and then reacted at 60 °C for 1.0 h. The reaction was monitored by TLC. After completion of the reaction, the reaction solution was adjusted to about pH 3 with HCl (1 N), and extracted with EA. The organic phase was concentrated to obtain the crude product, which was purified by Pre-TLC, to provide compound **88** (101 mg, 0.213 μmol), with a yield of 66%. MS: *m*/*z* 474 $[M+H]^+$. $^1$H-NMR (400 MHz, DMSO-$d_6$) $\delta$ 13.16 (s, 1H), 8.76 (d, *J* = 2.8 Hz, 1H), 8.42 (d, *J* = 6.7 Hz, 1H), 8.12 (dd, *J* = 8.8, 4.5 Hz, 1H), 8.03 (td, *J* = 8.7, 2.9 Hz, 1H), 7.77 (dd, *J* = 8.3, 3.0 Hz, 1H), 7.73 (d, *J* = 2.6 Hz, 1H), 7.60 (dd, *J* = 9.4, 3.0 Hz, 1H), 7.21 (dd, *J* = 9.0, 2.7 Hz, 1H), 6.42 (d, *J* = 9.1 Hz, 1H), 5.46 (t, *J* = *6.5* Hz, 1H), 1.59 (d, *J* = 6.7 Hz, 3H).

**Example 15: Synthesis of compound (R)-6-chloro-3-(1-(3,6-dimethyl-4-oxo-2-phenyl-3,4-dihydroquinazolin-8-yl)ethyl)amino)pyridinecarboxylic acid(compound 115):**

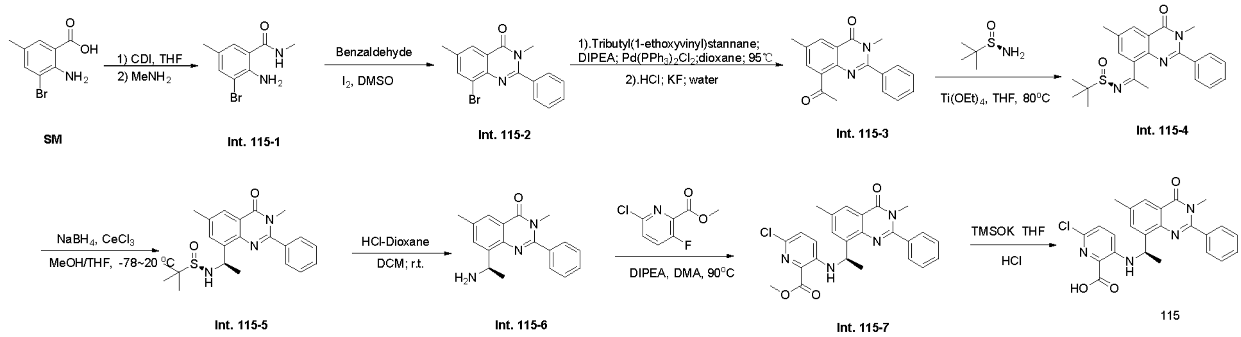


Intermediate: Synthesis of 2-amino-3-bromo-N,5-dimethylbenzamide (Int.C-115-1):

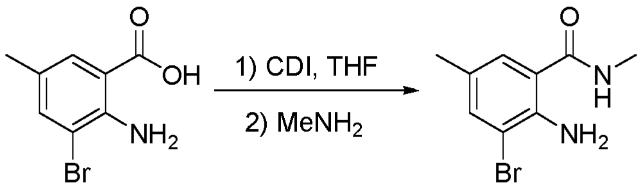


**SM1** **Int.115- 1**

2-amino-3-bromo-5-chloro-N-methylbenzoic acid (SM, 10 g, 43.5 mmol) was dissolved in anhydrous THF (40 mL), to which was added CDI (9.2g, 56.5mol) in portions, and then the system was gradually bubbling up. The mixture was allowed to react for 2 h, and then the system was cooled to 0 °C, to which was added methylamine aqueous solution (30 mL) dropwise. The system was reacted for 1 h. TLC detection indicated completion of the reaction. The reaction solution was added with water (500 mL), stirred at room temperature for 2h, and then subjected to suction filtration. The solid was rinsed with methyl tert-butyl ether (30 mL×2), and dried to provide 7.8 g of **Int.115-1 as** white solids, with a yield of 73.9%. MS: *m*/*z* 243, 245 $[M+H]^+$.

Intermediate: Synthesis of 8-bromo-3,6-dimethyl-2-phenylquinazolin-4(3H)-one **(Int.C-115-1):**

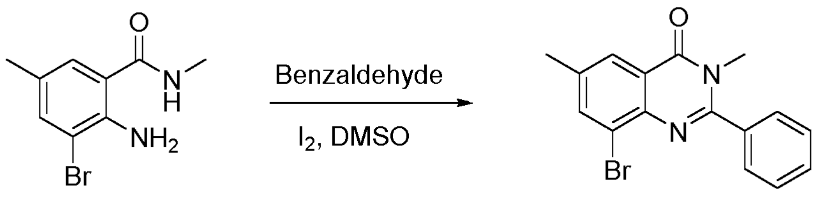


**Int. 115-1** **Int. 115-2**

**Int.115-1** (5 g, 20.6 mmol), benzaldehyde (2.8 g, 26.7 mmol), and iodine (6.8 g, 26.7 mmol) were successively dissolved in DMSO (50mL), and then reacted at 100 °C for 6 h. The reaction was monitored by TLC. After completion of the reaction, the system was added with ice-water, and then saturated sodium thiosulfate solution was slowly added until the solution became colorless. The resultant solution was stirred for 2 h and suction filtered. The solid was rinsed with methyl tert butyl ether (20 mL × 2), and dried to obtain 5.9 g of **Int.115-2** as white solids, with a yield of 87.2%, MS: *m*/*z* 329, 331 $[M+H]^+$.

Intermediate: Synthesis of 8-acetyl-3,6-dimethyl-2-phenylquinazolin-4(3H)-one **(Int.115**-3):

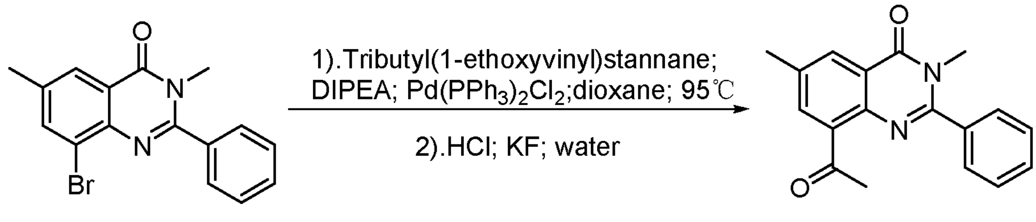


Int. 115-2

Int. 115-3

**Int.115-2** (5.9 g, 17.9 mmoL), Tin reagent (9.8 g, 26.9 mmol), DIPEA (4.7 g, 35.8 mmol) and $Pd(PPh_3)Cl_2$ (1.9 g, 2.7 mmol) were weighed, and successively added into dioxane (60 mL), and then reacted at 95 °C for 8 h under nitrogen protection. The reaction was monitored by TLC. After cooling, 20 mL of 6N HCl was added, and then the reaction was stirred at room temperature for 0.5 h. The reaction was monitored by TLC. The reaction solution was added with 100 mL of water (15.6 g KF), and stirred for 2 h. Then, EA (100 mL) was added, and the resultant solution was suction filtered. The solid was rinsed with EA (50 mL×2),poured into separatory funnel and separated. The water phase was extracted with EA (30 mL). EA layers were combined, washed with saturated saline (30 mL×2),dried over anhydrous $Na_2SO_4$, and concentrated, followed by column chromatography, to obtain 4.3 g of **Int.C115-3** as solid, with a yield of 81.8%, MS: *m/z* 293 $[M+H]^+$.

Intermediate: Synthesis of (R,Z)-N-(1-(3,6-dimethyl-4-oxo-2-phenyl-3,4-dihydroquinazolin-8-yl)ethylene)-2-methylpropane-2-sulfonamide **(Int.115-4):**

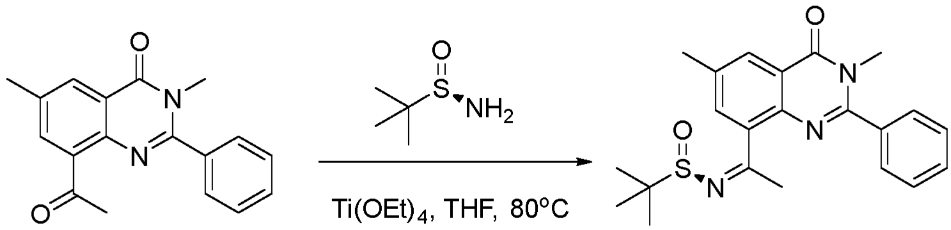


Int. 115-3

Int. 115-4

**Int.115-3** (4.3 g, 14.7 mmol), (R)-(+)-tert-butylsulfenamide (3.6 g, 29.4 mmol), and tetraethyl titanate (10.1 g, 44.1 mmol) were added into anhydrous THF (21 mL), and then stirred at 80 °C for 6 h. The reaction was monitored by TLC. After cooling, the reaction solution was diluted with EA (100 mL), and then poured into water (200 mL) and stirred for 30 min, followed by filtering, separating in a separatory funnel, and concentrating. The residue was purified by column chromatography, to obtain 4.9 g of **Int.115-4** as light yellow solid, with a yield of 84.5%, MS: *m/z* 396.1 $[M+H]^+$.

Intermediate: Syntheis of (R)-N-((R)-1-(3,6-dimethyl-4-oxo-2-phenyl-3,4-dihydroquinazolin-8-yl)ethyl)-2-methylpropane-2-sulfonamide **(Int.115-5)**

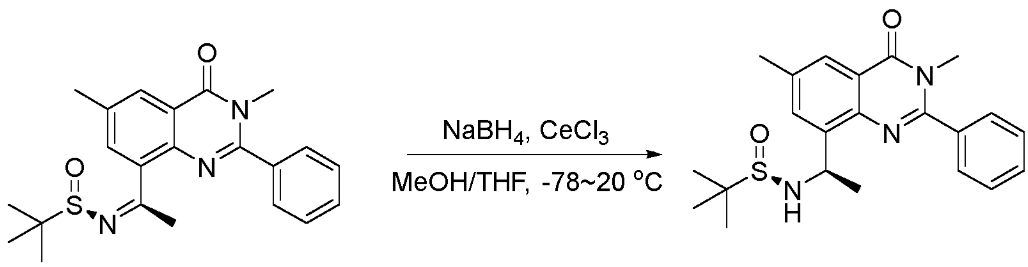


Int. 115-4

Int. 115-5

**Int.C115-4** (4.9 g, 12.4 mmol) and $CeCl_3 \cdot 7H_2O$ (2.3 g, 6.2 mmol) were successively added into MeOH (25 mL) and THF (25 mL), and then dissolved to become clear solution under nitrogen protection, followed by cooling to (-78 °C)-(-20 °C). $NaBH_4$ (706 mg, 18.6 mmol) was dissolved in 5 mL of DMA, and slowly added into the reaction solution dropwise. The reaction was naturally warmed to room temperature (4 H), monitored by TLC, cooled to 0 °C, and then adjusted to pH 3-4 with 1N HCl, followed by addition of DCM (100 mL) and water (150 mL). The resultant solution was poured into a separatory funnel and separated. The water phase was extracted with DCM (50 mL×2). DCM layers were combined, washed once with saturated NaCl solution (50 mL), dried, and concentrated, followed by column chromatography, to obtain 3.6 g of **Int.115-5** as solid, with a yield of 73.1%, MS: *m/z 397.1* $[M+H]^+$, de value: 98.2%.

Intermediate: Synthesis of (R)-8-(1-aminoethyl)-3,6-dimethyl-2-phenylquinazolin-4(3H)-one **(Int.115-6):**

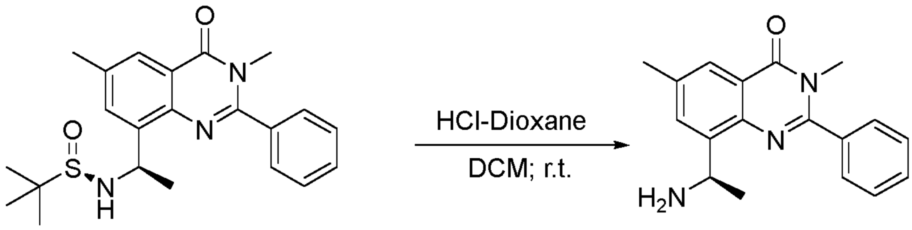


**Int. 115-5** **Int. 115-6**

**Int.115-5** (3.6 g, 9.1 mmol) was dissolved in DCM (20 mL), to which was added HCl-dioxane (4 N, 20 mL), and then allowed to react at room temperature for 3h. The reaction was monitored by TLC. The reaction solution was concentrated to dry, and co-evaporated with DCM for one time, followed by addition of EA (30 mL) and water (30 mL). Once the reaction solution became clear, it was poured into a separatory funnel and separated, and then extracted once with EA (20 mL). The water phase was adjusted to pH 8-9 with $NaHCO_3$, and extracted with DCM (50 mL×4). The organic phase was dried and concentrated, to obtain 2.1 g of **Int.115-6** as white solid, with a yield of 78.9%, MS: *m/z* 294 $[M+H]^+$.

Intermediate: Synthesis of methyl (R)-6-chloro-3-((1-(3,6-dimethyl-4-oxo-2-phenyl-3,4-dihydroquinazolin-8-yl)ethyl) amino)pyridinecarboxylic acid (Int.115-7):

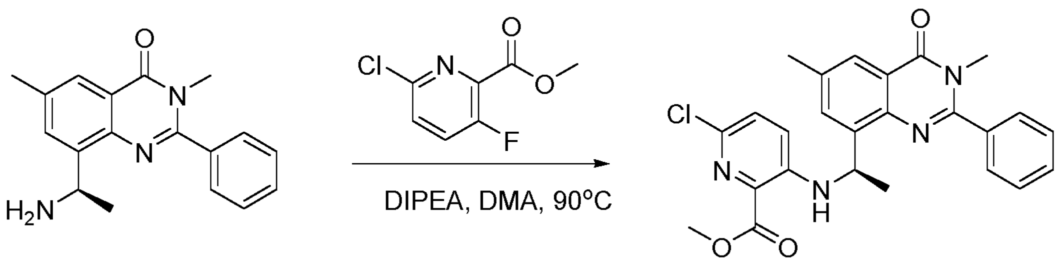


**Int. 115-6** **Int. 115-7**

**Int.115-6** (400 mg, 1.36 mmol), methyl 6-chloro-3-fluoro-pyridine-2-carboxylate (337 mg, 1.77 mmol), and DIPEA (351 mg, 2.72 mmol) were added into DMA (5mL), and then stirred for 6 h at 90 °C. The reaction was monitored by TLC. After completion of the reaction, the solution was added with water (10 mL), and then extracted with EA (10 mL × 3). The EA layers were combined, washed with water (20 mL×3) and saturated saline (20 mL) in sequence, dried over anhydrous $Na_2SO_4$, and concentrated, followed by column chromatography, to obtain 450 mg solid **(Int.115-7),** with a yield of 71.4%, MS: *m/z* 463 $[M+H]^+$.

Synthesis of compound (R)-6-chloro-3-(1-(3,6-dimethyl-4-oxo-2-phenyl-3,4-dihydroquinazolin-8-yl)ethyl)amino)pyridinecarboxylic acid (compound 115)

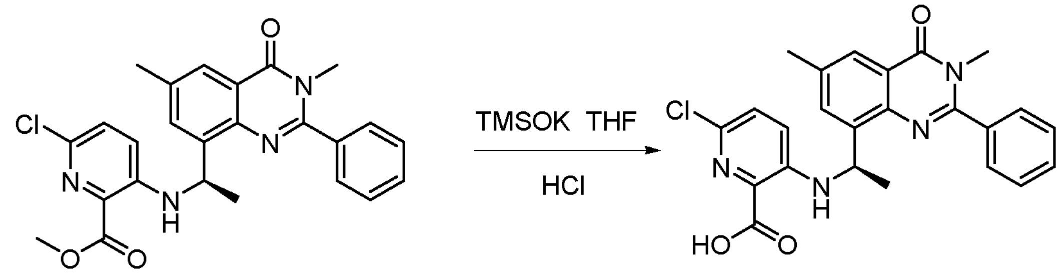


**Int. 115-7** **115**

**Int.115-7** (450 mg, 0.97 mmol) was dissolved in THF (5 mL), to which was added potassium trimethylsilanolate (250 mg, 1.84 mmoL), and allowed to react at room temperature for 15 min. The reaction was monitored by TLC. The reaction solution was added with water (20 mL), and adjusted to pH 2-3 with 1N HCl. The resultant solution was extracted with DCM (20 mL×3). DCM layers were combined, washed with saturated saline (15 mL), dried over anhydrous $Na_2SO_4$, and concentrated, followed by column chromatography, to obtain 360 mg solid (compound 115), with a yield of 82.5%, MS: *m*/*z* 449 $[M+H]^+$.

$^1$H NMR (400 MHz, Chloroform-d) $\delta$ 8.55 (d, *J* = 2.9 Hz, 1H), 8.23 (d, *J* = 2.4 Hz, 1H), 8.07 (dd, *J= 8.7,* 4.4 Hz, 1H), 7.68 - 7.61 (m, 2H), 5.13 (p, *J* = 6.7 Hz, 1H), 4.70 (d, *J* = 6.6 Hz, 1H), 3.66 (s, 3H), 1.60 (d, *J* = 6.8 Hz, 3H), 1.16 (s, 9H).

**Example 16: Synthesis of compound (R)-6-chloro-3-((1-(2-(5-fluoropyridin-2-yl)-3,6-dimethyl-4-oxo-3,4-dihydroquinazolin-8-yl)ethyl)amino)pyridinecarboxylic acid (compound 66)**

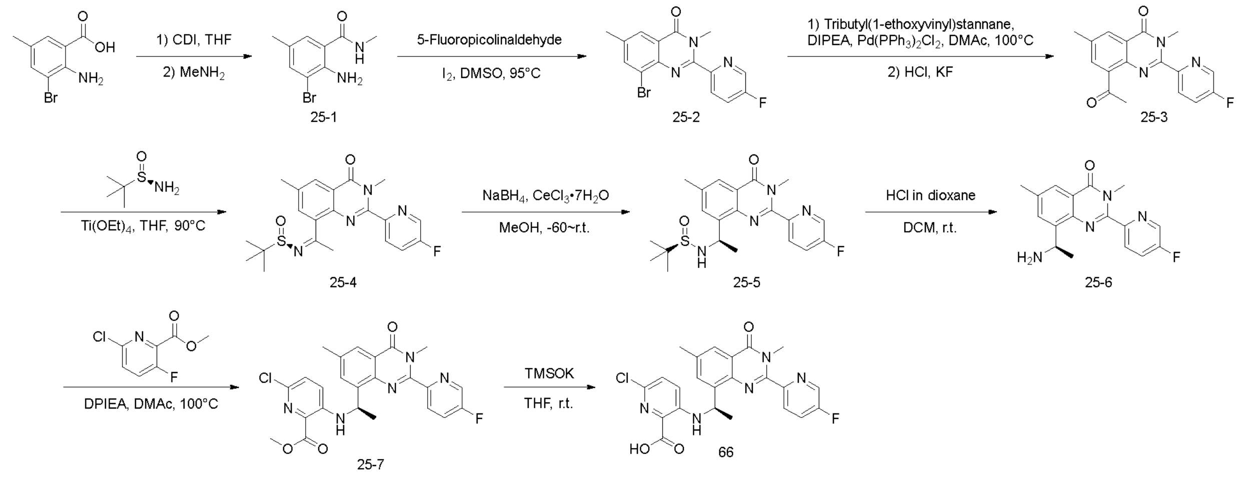


Step 1: Synthesis of intermediate 2-amino-3-bromo-N,5-dimethylbenzamide (25-1)

2-amino-3-bromo-5-methylbenzoic acid (230 g, 1.0 mol) was dissolved in THF (2L), to which was added CDI (178 g, 1.1 mol) in portions. The system was gradually bubbling up, and after 2 hours of reaction, the system was cooled to 10 °C, and then methylamine aqueous solution (920 mL) was added dropwise. The temperature during the dropwise addition process was controlled below 15 °C. After addition, the mixture was allowed to react for 1 h. TLC detection showed completion of the reaction. The reaction solution was concentrated to remove some solvent until solid was precipitated, and then poured into water (2 L) and stirred for 1 h. After filtration, the solid was dried under reduced pressure to constant weight, to obtain 230 g of product (intermediate **25-1**), with a yield of 94.6%. MS: *m*/*z* 243.0/245.0 $[M+H]^+$.

Step 2: Synthesis of intermediate 8-bromo-2-(5-fluoropyridin-2-yl)-3,6-dimethylquinazolin-4(3*H*)-one **(25-2)**

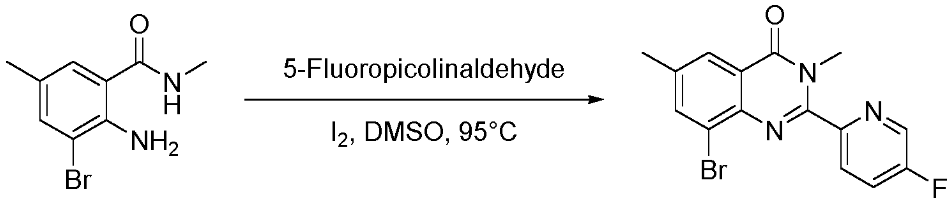


25-1 25-2

Intermediate **25-1** (60.0 g, 0.25 mol), 5-fluoropyridin-2-formaldehyde (37.0 g, 0.30 mol), and iodine (75.2 g, 0.30 mol) were successively added into dimethylsulfoxide (900 mL) at room temperature, and then heated to 95 °C and reacted overnight. The reaction solution was cooled to room temperature, and slowly poured into water (3000 mL), to which was added saturated sodium thiosulfate aqueous solution (300 mL), and then stirred for 2 h. After filtration, the filter cake was sequentially rinsed once with water (100 mL), absolute ethanol (100 mL), and methyl tert-butyl ether (100 mL), respectively, and then dried in vacuum, to obtain 74.7 g of product (intermediate **25-2),** with a yield of 86.9%. MS: *m*/*z* 243.0/245.0 $[M+H]^+$.

Step 3 : Synthesis of intermediate 8-acetyl-2-(5-fluoropyridin-2-yl)-3,6-dimethylquinazolin-4(3H)-one (25-3)

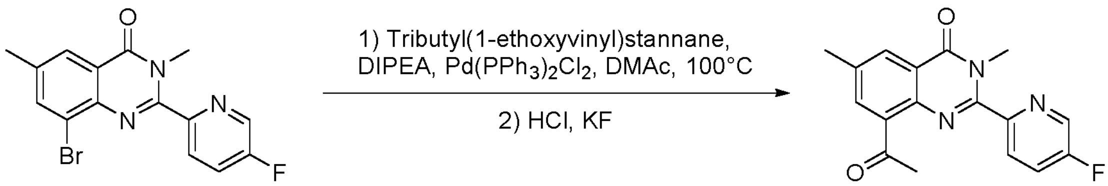


25-2 25-3

Intermediate **25-2** (74.7 g, 0.21 mol), tributyl(1-ethoxyvinyl)tin (116.2 g, 0.32 mol), *N,N*-diisopropylethylamine (83.2 g, 0.64 mol), and $Pd(PPh_3)_2Cl_2$ (15.0 g, 0.02 mol) were successively added into *N,N*-dimethylacetamide (800 mL) at room temperature, and then the system was purged with nitrogen for three times. The reaction solution was heated to 100 °C and reacted overnight, and then cooled to room temperature, to which was slowly added the solution of HCl in dioxane (4 mol/L). The reaction solution was adjusted to pH 1-2, to which was added a small amount of water (20 mL), and then stirred and reacted for 1 h. The reaction solution was diluted with ethyl acetate (2000 mL), and then water (4000 mL) was added. The resultant solution was adjusted to pH>7 with KF aqueous solution, stirred for 2 h, and filtered. The filter cake was triturated in ethyl acetate (500 mL × 3). The filtrate was combined and separated in a separatory funnel. The aqueous layer was extracted with ethyl acetate (500 mL). The organic layers were combined, dried over anhydrous $Na_2SO_4$, and concentrated. The obtained residue was triturated in methanol (500 mL), to obtain 60.8 g of product (intermediate **25-3),** with a yield of 91.0%. MS: *m*/*z* 312.0 $[M+H]^+$.

Step 4: Synthesis of intermediate (R,Z)-N-(1-(2-(5-fluoropyridin-2-yl)-3,6-dimethyl-4-oxo-3,4-dihydroquinazolin-8-yl) ethylene)-2-methylpropane-2-sulfenamide (25-4)

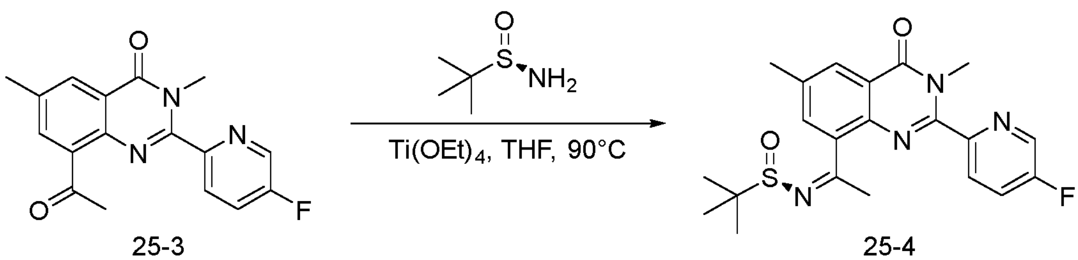


25-3 25-4

Intermediate **25-3** (60.8 g, 0.20 mol), (R)-(+)-tert-butylsulfenamide (47.3 g, 0.39 mol), and tetraethyl titanate (222.7 g, 0.98 mol) were successively added into dry tetrahydrofuran (300 mL) at room temperature, heated to 90 °C, and reacted overnight. The reaction solution was cooled to room termperature, and diluted by adding dichloromethane (2000 mL), to which was added water (4000 mL). The resultant solution was stirred for 0.5 h and filtered. The filter cake was triturated in dichloromethane (500 mL × 3). The filtrate was combined and separated in a separatory funnel. The aqueous layer was extracted with dichloromethane (500 mL). The organic layers were combined, dried over anhydrous $Na_2SO_4$, and concentrated, to obtain 90.0 g of crude product as yellow oil (intermediate 25-4), which was directly used in the next step of reaction. MS: *m/z* 415.1 $[M+H]^+$.

Step 5. Synthesis of intermediate (*R*)-*N*((*R*)-1-(2-(5-fluoropyridin-2-yl)-3,6-dimethyl-4-oxo-3,4-dihydroquinazolin-8-yl) ethyl)-2-methylpropane-2-sulfenamide **(25-5)**

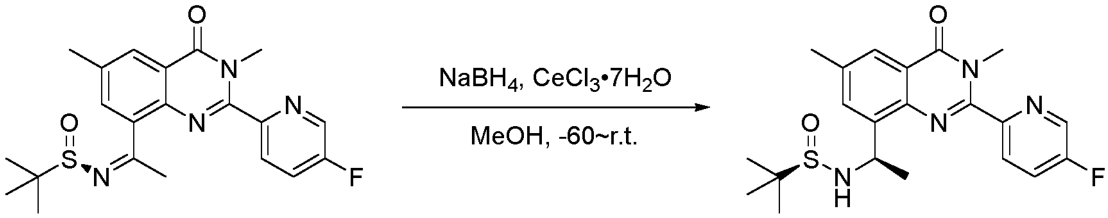


25-4 25-5

Intermediate **25-4** (90.0 g, 0.20 mol) and $CeCl_3 \cdot 7H_2O$ (36.4 g, 0.10 mol) were successively added into MeOH (2000 mL) at room temperature, and then cooled to -65 °C in a dry ice bath. $NaBH_4$ (22.2 g, 0.58 mol) was dissolved in methanol in batches, and added into the reaction system dropwise, during which the temperature was controlled not to exceed -60 °C. This took about 30 min, and then the dry ice bath was removed. The reaction was naturally warmed to room temperature, and diluted by adding dichloromethane (2000 mL), to which was added water (4000 mL). The resultant solution was adjusted to pH 6 with 1N HCl, and then poured into a separatory funnel and separated. The water layer was extracted with dichloromethane (500 mL). The organic layers were combined, washed once with saturated $NaHCO_3$ solution (500 mL), dried over anhydrous $Na_2SO_4$, and concentrated, followed by column chromatography and recrystallization in methanol/water (1:1), to obtain 50.8 g of product (intermediate 25-5), with a yield of 62.4%, MS: *m/z* 417.1 $[M+H]^+$. $^1H$ NMR (400 MHz, Chloroform-d) $\delta$ 8.55 (d, *J* = 2.8 Hz, 1H), 8.06 (dt, *J* = 5.5, 4.4 Hz, 2H), 7.63 (td, *J* = 8.4, 2.9 Hz, 1H), 7.53 (d, *J* = 2.0 Hz, 1H), 5.08 (p, *J* = 6.8 Hz, 1H), 4.91 (d, *J* = 6.8 Hz, 1H), 3.65 (s, 3H), 2.50 (s, 3H), 1.60 (d, *J* = 6.8 Hz, 3H), 1.15 (s, 9H).

Step 6: Synthesis of intermediate (R)-8-(1-aminoethyl)-2-(5-fluoropyridin-2-yl)-3,6-dimethylquinazolin-4(3H)-one (25-6)

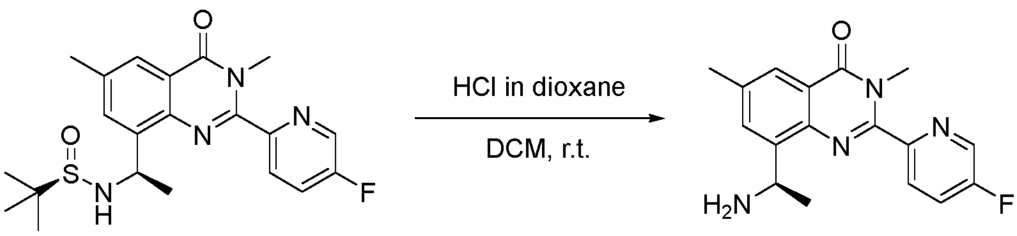


25-5 25-6

Intermediate **25-5** (50.8 g, 0.12 mol) was added to dichloromethane (1000 mL) at room temperature, to which was added the solution of HCl in dioxane (4 mol/L, 500 mL) dropwise, and then reacted for 2 h under stirring. The reaction solution was concentrated, and the residue was dissolved in water (1000 mL), followed by extraction with ethyl acetate (500 mL). The ethyl acetate layer was washed once with 1N HCl (100 ml) and discarded. The acidic water layers were combined, adjusted to pH of greater than 7, and then extracted with dichloromethane (1000 mL × 3). The organic layers were combined, dried over anhydrous $Na_2SO_4$, and concentrated. The residue was triturated in methyl tert-butyl ether (300 ml), to obtain 33.4 g of product (intermediate **25-6),** with a yield of 87.7%, MS: *m/z* 313.1 $[M+H]^+$. $^1H$ NMR (400 MHz, Chloroform-d) $\delta$ 8.55 (d, *J* = 2.8 Hz, 1H), 8.03 (dd, *J* = 2.1, 1.0 Hz, 1H), 7.97 (dd, *J* = 8.7, 4.4 Hz, 1H), 7.66 - 7.57 (m, 2H), 4.85

(q, $J$ = 6.7 Hz, 1H), 3.67 (s, 3H), 2.50 (s, 3H), 2.12 (s, 2H), 1.52 (d, $J$ = 6.7 Hz, 3H).

Step 7: Synthesis of intermediate methyl (R)-6-chloro-3-((1-(2-(5-fluoropyridin-2-yl)-3,6-dimethyl-4-oxo-3,4-dihydroquinazolin-8-yl)ethyl)amino)pyridinecarboxylate (25-7)

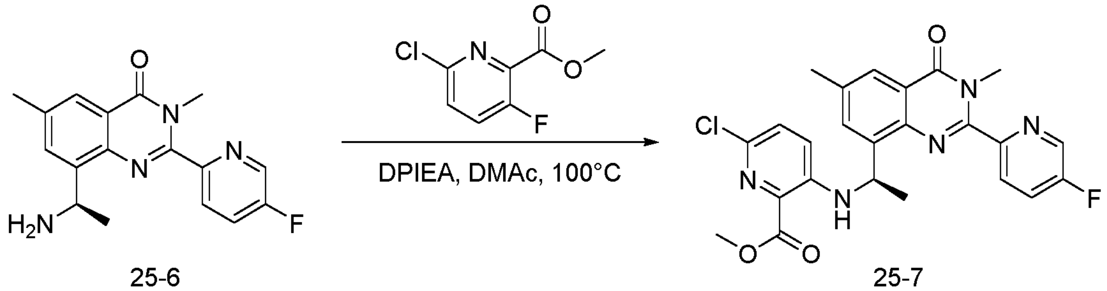


25-6 25-7

Intermediate **25-6** (200 mg, 0.64 mmol), 6-chloro-methyl 3-fluoropyridinecarboxylate (243 mg, 1.28 mmol), and DIPEA (248 mg, 1.92 mmol) were added into *N,N*-dimethylacetamide (4 mL), heated to 100 °C, and reacted overnight under stirring. The reaction system was cooled to room temperature, diluted with ethyl acetate (20 mL), and washed with water (20 mL×2). The organic layer was dried over anhydrous $Na_2SO_4$, concentrated, and purified by column chromatography, to obtain 210 mg of product (intermediate 25-7), with a yield of 68.0%, MS: $m/z$ 482.1 $[M+H]^+$.

Step 8: Synthesis of compound (*R*)-6-chloro-3-((1-(2-(5-fluoropyridin-2-yl)-3,6-dimethyl-4-oxo-3,4-dihydroquinazolin-8-yl)ethyl)amino)pyridinecarboxylic acid (compound **66**)

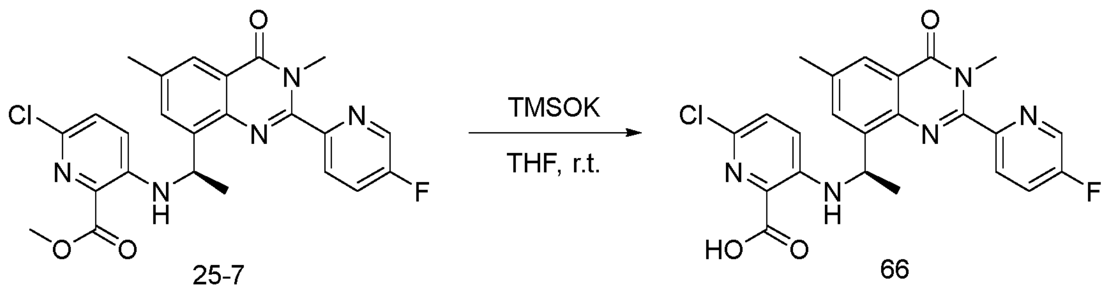


25-7 66

Intermediate 25-7 (210 mg, 0.44 mmol) was dissolved in dry tetrahydrofuran (4 mL), to which was added potassium trimethylsilanolate (168 mg, 1.31 mmol), and then reacted for 20 min under stirring at room temperature. The reaction solution was adjusted to about pH 3 with 0.5 N HCl, and extracted with dichloromethane (15 mL×2). The organic layer was dried over anhydrous $Na_2SO_4$, concentrated, and purified by column chromatography, to obtain 180 mg of product (compound **66),** with a yield of 88.3%, MS: $m/z$ 468.1 $[M+H]^+$. $^1H$ NMR (400 MHz, DMSO-$d_6$) $\delta$ 12.99 (s, 1H), 8.75 (d, $J$ = 2.9 Hz, 1H), 8.43 (d, $J$ = 7.0 Hz, 1H), 8.12 (dd, $J$ = 8.7, 4.5 Hz, 1H), 8.01 (td, $J$ = 8.7, 2.9 Hz, 1H), 7.91 (dd, $J$ = 2.1, 1.0 Hz, 1H), 7.64 (d, $J$ = 2.1 Hz, 1H), 7.29 (d, $J$ = 8.9 Hz, 1H), 7.02 (d, $J$ = 9.1 Hz, 1H), 5.46 (p, $J$ = 6.7 Hz, 1H), 3.49 (s, 3H), 2.41 (s, 3H), 1.59 (d, $J$ = 6.6 Hz, 3H).

**Example 17: Synthesis of compound (*R*)-2-((1-(2-(4,4-dimethylpiperidin-1-yl)-6-dimethyl-4-oxo-3,4-dihydroquinazolin-8-yl)ethyl)amino)benzoic acid (compound 4):**

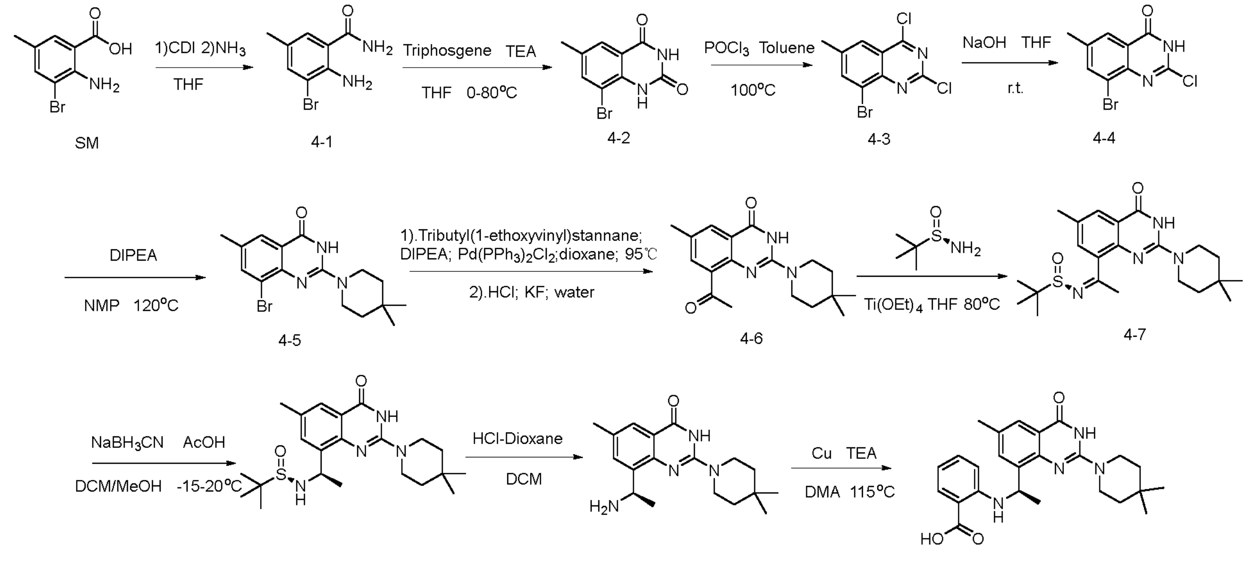


SM 4-1 4-2 4-3 4-4 4-5 4-6 4-7 4-8 4-9 4

Synthesis of intermediate 2-amino-3-bromo-N,5-dimethylbenzamide (4-1)

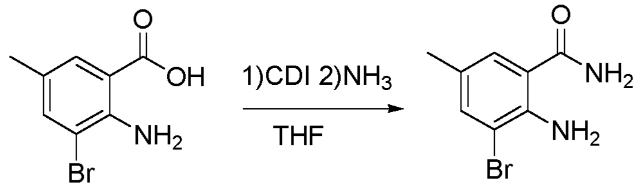


SM 4-1

2-amino-3-bromo-5-methylbenzoic acid (SM, 100 g, 435 mmoL) was dissolved in anhydrous THF (300 mL), to which was added CDI (91.6 g, 565 mmoL) in portions. The system was gradually bubbling up, reacted for 1 h, and cooled to 0 °C, to which was added aqueous ammonia (500 mL). The system was allowed to further react for 2 h, and monitored by TLC. The reaction solution was poured into water (4 L), stirred for 1 h, and filtered. The solid was rinsed with methyl tert-butyl ether (30 mL × 2) and dried, to obtain 87.5 g of white solid (4-1), with a yield of 87.9%, MS: *m/z* 228, 231 $[M+H]^+$.

Synthesis of intermediate 8-bromo-3,6-dimethylquinazolin-2,4(1H,3H)-dione (4-2):

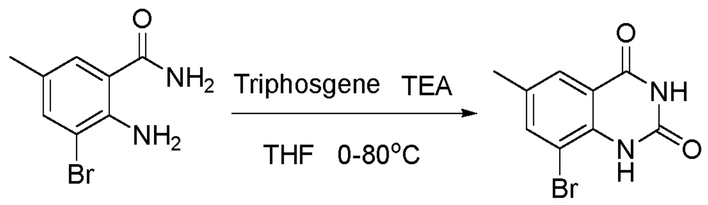


4-1 4-2

**4-1** (31 g, 135 mmoL) and triethylamine (27.4 g, 271. mmoL) were dissolved in anhydrous THF (300 mL), and cooled to 0 °C, to which was slowly added triphosgene (20 g, 68 mmoL) in portions. The ice bath was removed. The reaction was stirred for 30 min, heated to 80 °C, and reacted for 12 h. The reaction was monitored by TLC. Most of THF was removed by concentration. Water (1L) was added, and the resultant solution was stirred for 1 h. After suction filtration, the solid was rinsed with methyl tert-butyl ether (30 mL × 2), and dried, to obtain 23.2 g of white solid **(4-2),** MS: *m/z* 255, 257 $[M+H]^+$.

Synthesis of intermediate 8-bromo-2,4-dichloro-6-methylquinazoline **(4-3):**

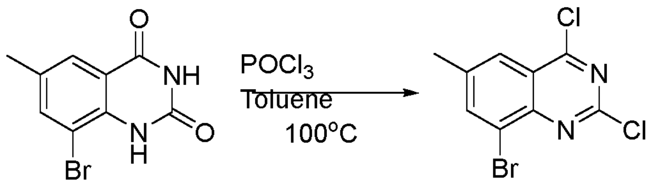


4-2 4-3

**4-2** (23.2 g, 91 mmoL) was dissolved in toluene (116 mL), to which was added phosphorus oxychloride (70 g, 455 mmoL) dropwise, and then reacted at 100 °C for 12 h. The reaction was monitored by TLC. The reaction solution was concentrated to dry, and co-evaporated with DCM for two times, to obtain the crude product **(4-3),** which was directly used in the next step.

Synthesis of intermediate 8-bromo-2-chloro-6-methylquinazolin-4(3H)-one **(4-4):**

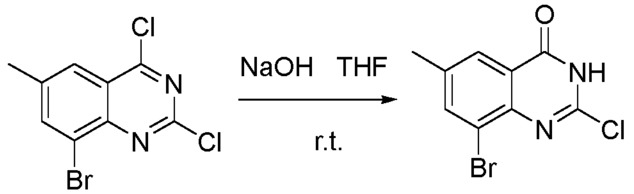


4-3 4-4

**4-3** (crude product) was dissolved in THF (116 mL), to which was added NaOH aqueous solution (2 N, 116 mL), and then the system was gradually dissolved and became clear. The reaction was monitored by TLC. The reaction solution was adjusted to pH 6-7 with HCl (1N), and suction filtered, followed by drying, to obtain 16.5 g of solid **(4-4),** with a yield of 66.3%, MS: *m/z* 273, 275 $[M+H]^+$.

Synthesis of intermediate 8-bromo-2-(4,4-dimethylpiperidin-1-yl)-6-methylquinazolin-4(3H)-one **(4-5):**

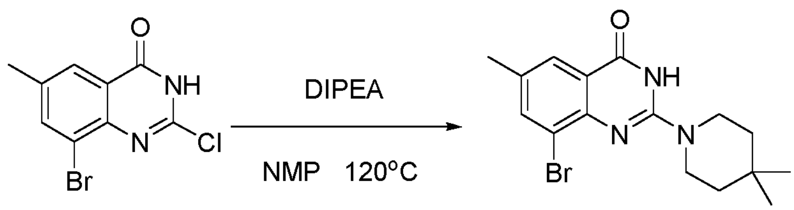


4-4 4-5

**4-4** (16.5 g, 60 mmoL) was dissolved in NMP (90 mL), to which was added 4,4-dimethylpiperidine hydrochloride (10.8 g, 72 mmoL), followed by adding DIPEA (38.7g, 300 mmoL) dropwise, and then the mixture was allowed to react at 120 °C for 3 h. The reaction was monitored by TLC. The reaction solution was cooled, and adjusted to pH 6-7 with dilute HCl (1N). The resultant solution was extracted with DCM (200 mL×3), dried over anhydrous $Na_2SO_4$, concentrated, and purified by column chromatography, to obtain 15 g of yellow solid **(4-5),** with a yield of 71.4%, MS: *m/z* 350/352 $[M+H]^+$.

Synthesis of intermediate 8-acetyl-2-(4,4-dimethylpiperidin-1-yl)-6-dimethylquinazolin-4(3H)-one **(4-6)**

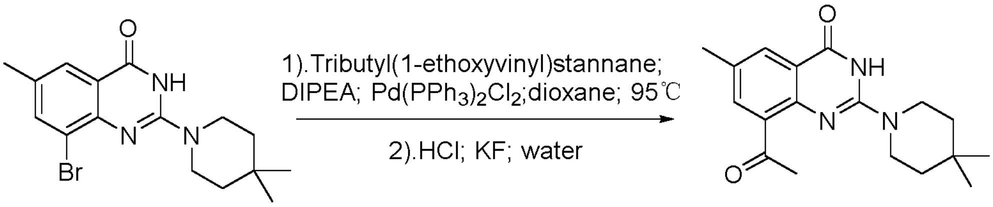


4-5 4-6

**4-5** (2.1 g, 6 mmol), Tin reagent (3.3 g, 9 mmol), DPIEA (1.5 g, 12 mmoL), and $Pd(PPh_3)Cl_2$ (630 mg, 0.9 mmoL) were successively added into dioxane (10 mL), and then reacted at 95 °C for 8 h under nitrogen protection. The reaction was monitored by TLC. The reaction solution was cooled, added with HCl (6N, 10 mL), and allowed to react for 30 min under stirring. The reaction was monitored by TLC. The reaction solution was added with water (30 mL, 5.3 g KF), stirred for 3 h, to which was added EA (30 mL), followed by suction filtration. The filter cake was rinsed with EA (30 mL × 3). EA layers were combined, washed once with saturated NaCl solution (20 mL), dried over anhydrous $Na_2SO_4$, concentrated, and purified by column chromatography, to obtain 1.5 g of solid **(4-6),** with a yield of 79.9%, MS: *m/z* 314 $[M+H]^+$.

Synthesis of intermediate (R,E)-N-(1-2-(4,4-dimethylpiperidin-1-yl)-6-dimethyl-4-oxo-3,4-dihydroquinazolin-8-yl)ethylene)-2-methylpropane-2-sulfamide **(4-7):**

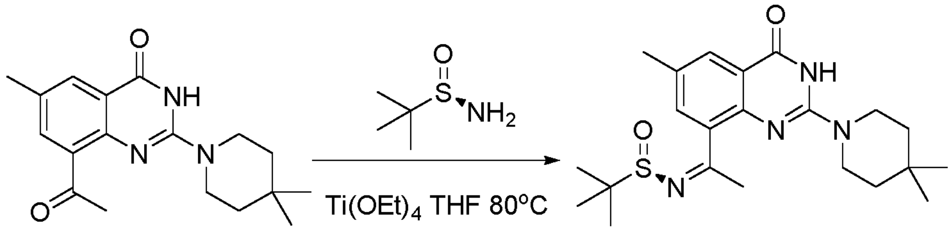


4-6 4-7

**4-6** (1.5 g, 4.8 mmol), (R)-(+)-tert-butylsulfenamide (1.2 g, 9.6 mmol) and tetraethyl titanate (3.3 g, 14.4 mmol) were successively added to THF (5 mL), and stirred at 85 °C for 6 h. The reaction was monitored by TLC. After cooling, the reaction solution was diluted by adding EA (30 mL), to which was added water (30 mL), and then stirred for 30 min, followed by filtration and pouring into a separatory funnel for separation. The aqueous phase was extracted with EA (20 mL×2). EA layers were washed with saturated saline (30 mL) once, dried over anhydrous $Na_2SO_4$, concentrated, and purified by column chromatography, to obtain 1.8 g of light yellow solid **(4-7),** with a yield of 75.4%, MS: *m/z* 417 $[M+H]^+$.

Synthesis of compound (R)-N-((-R-)1-(2-(4,4-dimethylpiperidin-1-yl)-3,6-dimethyl-4-oxo-3,4-dihydroquinazolin-8-yl) ethyl)-2-methylpropane-2-sulfonamide **(4-8):**

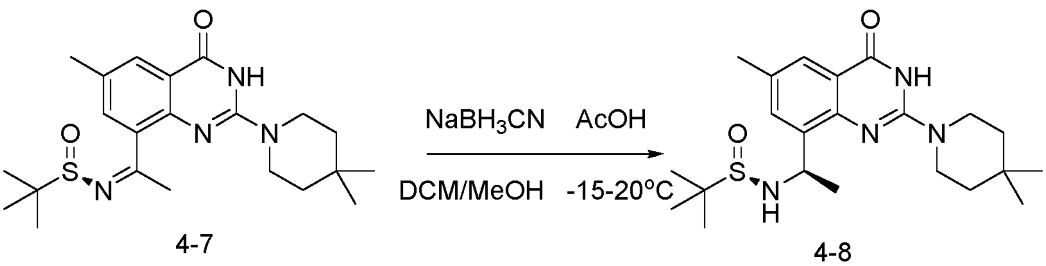


4-7 4-8

**4-7** (1.5 g, 3.6 mmol) was dissolved in DCM/MeOH (7.5 mL/7.5 mL), to which was added glacial acetic acid (1.3 g, 21.6 mmol) dropwise, and then cooled to -10 °C, followed by adding $NaBH_3CN$ (680 mg, 10.8 mmol) in portions. The mixture was naturally warmed and reacted for 12 h. The reaction was monitored by TLC. The reaction solution was adjusted to pH 9-10 with saturated $Na_2CO_3$ solution, and extracted with EA (20 mL×3). EA layers were combined, washed with saturated NaCl solution (10 mL) once, dried, concentrated, and purified by column chromatography, to obtain 1.2 g of

white solid **(4-8)**, with a yield of 79.6%, MS: *m/z 419* [M+H]+, de value: 98%.

Synthesis of intermediate (R)-8-(1-aminoethyl)-2-(4,4-dimethylpiperidin-1-yl)-3,6-dimethylquinazolin-4(3H)-one **(4-9):**

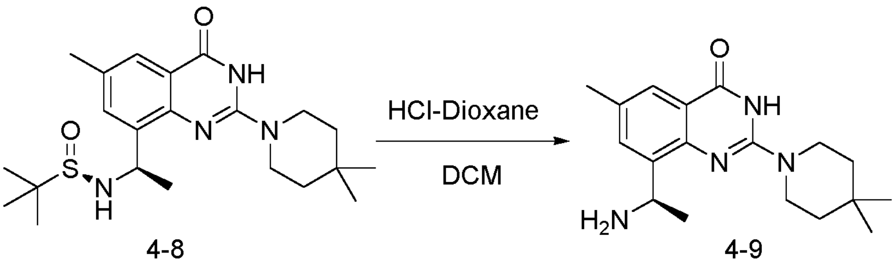


4-8 4-9

**4-8** (1.2 g, 2.9 mmoL) was dissolved in DCM (6 mL), to which was added HCl-dioxane (6 mL) dropwise, and then reacted at room temperature. The reaction was monitored by TLC. The reaction solution was concentrated to dry, and then water (10 mL) and EA (10 mL) were added and dissolved, until the clear solution was obtained. The resultant solution was poured into a separatory funnel and separated. The aqueous phase was extracted once with EA (10 mL), and adjusted to pH 8-9 with $NaHCO_3$, followed by extraction with DCM (15 mL×3). The organic phase was dried and concentrated, to obtain 0.8 g of light yellow solid (4-9), with a yield of 88.8%, MS: *m/z* 315 [M+H]+.

Synthesis of compound (R)-2-((1-(2-(4,4-dimethylpiperidin-1-yl)-6-dimethyl-4-oxo-3,4-dihydroquinazolin-8-yl)ethyl)yl) benzoic acid (compound 4)

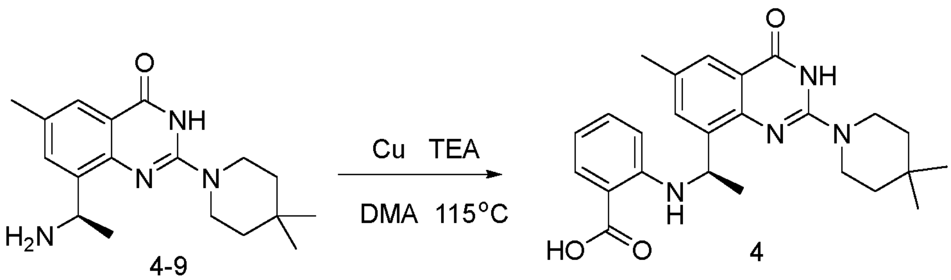


4-9 4

**4-9** (70 mg, 0.22 mmoL), 2-iodobenzoic acid (83 mg, 0.33 mmoL), TEA (45 mg, 0.44 mmoL), and copper nanoparticles (14 mg, 0.22 mmoL) were successively added into DMA (1 mL), and then reacted at 115 °C for 3 h. The reaction was monitored by TLC. The reaction solution was added with water (15 mL), adjusted to pH 3-4 with dilute HCl (1N), and extracted with EA (10 mL×3). EA layers were combined, washed with saturated NaCl solution (10 mL×3), dried over anhydrous $Na_2SO_4$, concentrated, and purified by column chromatography, to obtain 35 mg of solid (compound 4), with a yield of 37.9%, MS: *m/z* 435 [M+H]+.

$^1H$ NMR (400 MHz, $CDCl_3$) $\delta$ 7.99 (d, *J* = 7.9 Hz, 1H), 7.89 (s, 1H), 7.57 (s, 1H), 7.22 (t, *J* = 7.8 Hz, 1H), 6.67 (s, 1H), 6.60 (dd, *J* = 10.6, 8.2 Hz, 1H), 5.56 (d, *J* = 6.8 Hz, 1H), 3.22 (s, 4H), 2.39 (s, 3H), 1.72 (d, *J* = 6.5 Hz, 3H), 1.26 (s, 4H), 1.03 (s, 6H).

**Example 18: Synthesis of compound (R)-2-((1-(2-(4,4-dimethylpiperidin-1-yl)-3,6-dimethyl-4-oxo-3,4-dihydroquinazolin-8-yl)ethyl)amino)benzoic acid (compound 6):**

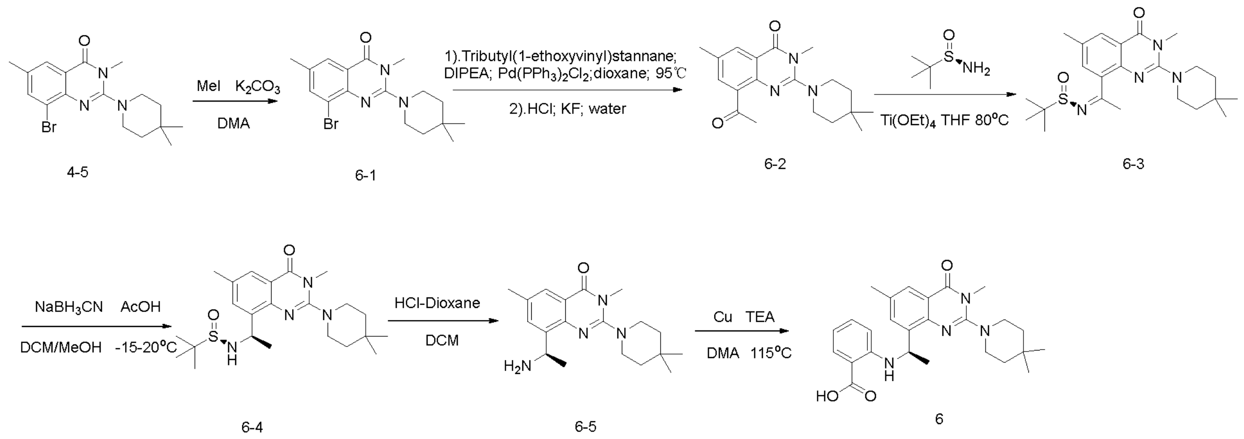


4-5 6-1 6-2 6-3

6-4 6-5 6

Synthesis of intermediate 8-bromo-2-(4,4-dimethylpiperidin-1-yl)-3,6-dimethylquinazolin-4(3H)-one **(6-1):**

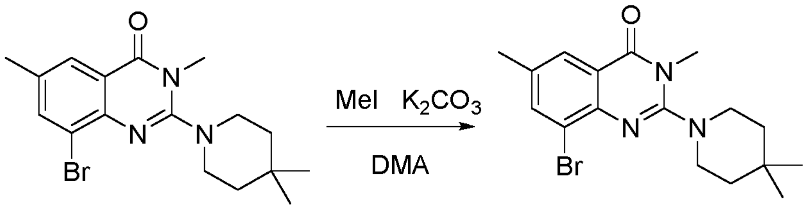


4-5 6-1

**4-5** (1.5 g, 0.43 mmol, its synthesis referring to the synthesis of intermediate **4-5** in Example 17) and $K_2CO_3$ (0.6 g, 8.6 mmoL) were successively added to DMA (15mL), to which was added MeI (1.9 g,12.9 mmoL) dropwise, and then reacted at room temperature. The reaction was monitored by TLC. The reaction solution was added with water (30 mL) and EA (30 mL×3) for extraction. EA layers were combined, washed with water (20 mL×3), dried over anhydrous $Na_2SO_4$, concentrated, and purified by column chromatography, to obtain 1.1 g of solid **(6-1),** with a yield of 70.5%, MS: *m*/*z* 364, 366 $[M+H]^+$.

Synthesis of intermediate 8-acetyl-2-(4,4-dimethylpiperidin-1-yl)-3,6-dimethylquinazolin-4(3H)-one **(6-2)**

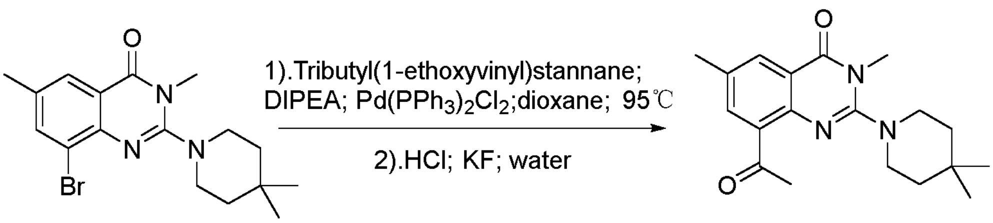


6-1 6-2

**6-1** (1.1 g, 3 mmol), Tin reagent (1.7 g, 4.5 mmoL), DIPEA (0.8 g, 6 mmoL), and $Pd(PPh_3)Cl_2$ (0.3 g, 0.45 mmol) were successively added to dioxane (10 mL), and reacted at 95 °C for 8 h under nitrogen protection. The reaction was monitored by TLC. After cooling, the reaction solution was added with HCl (4 N, 5 mL), and reacted for 30 min under stirring. The reaction was monitored by TLC. The reaction solution was added with water (30 mL, 2.7 g KF), stirred for 3 h, and suction filtered. The filter cake was washed with EA (20 mL×3). EA layers were combined, washed with saturated saline (20 mL) once, dried over anhydrous $Na_2SO_4$, concentrated, and purified by column chromatography, to obtain 0.7 g of solid **(6-2),** with a yield of 70.7%, MS: *m*/*z* 328 $[M+H]^+$.

Synthesis of intermediate (R,E)-N-(1-2-(4,4-dimethylpiperidin-1-yl)-3,6-dimethyl-4-oxo-3,4-dihydroquinazolin-8-yl) ethylene)-2-methylpropane-2-sulfamide **(6-3)**

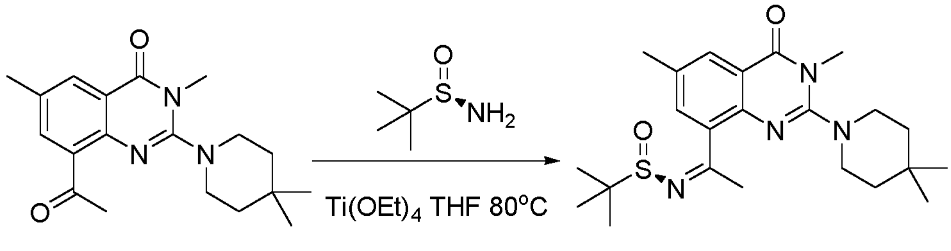


6-2 6-3

**6-2** (0.7 g, 2.1 mmol), (R)-(+)-tert-butylsulfenamide (0.5 g, 4.3 mmol), and tetraethyl titanate (1.5 g, 0.64 mmol) were successively added into anhydrous THF (5 mL), and reacted at 85 °C for 6 h. The reaction was monitored by TLC. After cooling, the reaction solution was diluted by adding EA (30 mL), and then poured to water (30 mL) and stirred for 30 min. After filtration, the solution was poured into a separatory funnel and separated. The aqueous phase was extracted with EA (20 mL×2),washed with saturated NaCl solution (30 mL) once, dried over anhydrous $Na_2SO_4$, concentrated, and purified by column chromatography, to obtain 0.7 g of light yellow solid **(6-3),** with a yield of 76%, MS: *m/z* 431 $[M+H]^+$.

Synthesis of compound (R)-N-((-R-)1-(2-(4,4-dimethylpiperidin-1-yl)-3,6-dimethyl-4-oxo-3,4-dihydroquinazolin-8-yl) ethyl)-2-methylpropane-2-sulfonamide **(6-4):**

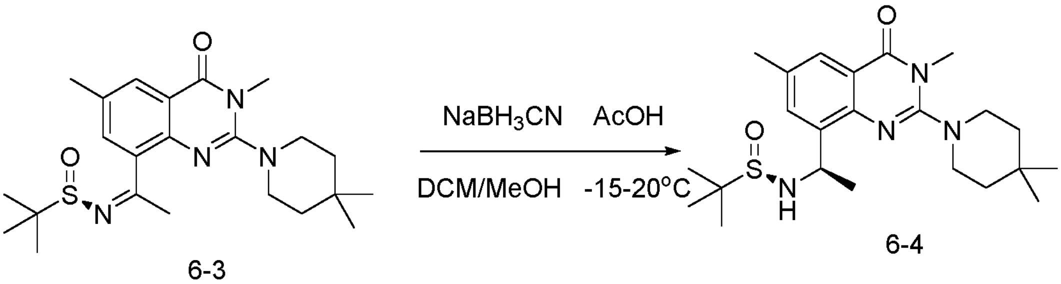


6-4

6-3

**6-3** (0.7 g, 1.6 mmol) was dissolvd in DCM/MeOH (3.5 mL/3.5 mL), to which was added glacial acetic acid (0.6 g, 10 mmol) dropwise, and then cooled to -10 °C, followed by adding $NaBH_3CN$ (0.3 g, 4.9 mmol) in portions. The mixture was naturally warmed and reacted for 12 h. The reaction was monitored by TLC. The reaction solution was adjusted to pH 9-10 with saturated $Na_2CO_3$ solution, and extracted with EA (20 mL×3). EA layers were combined, washed with saturated NaCl solution (10 mL) once, dried, concentrated, and purified by column chromatography, to obtain 0.6 g of white solid (6-4), with a yield of 85.3%, MS: *m/z 433* $[M+H]^+$, de value: 98%.

Synthesis of intermediate (R)-8-(1-aminoethyl)-2-(4,4-dimethylpiperidin-1-yl)-3,6-dimethylquinazolin-4(3H)-one **(6-5):**

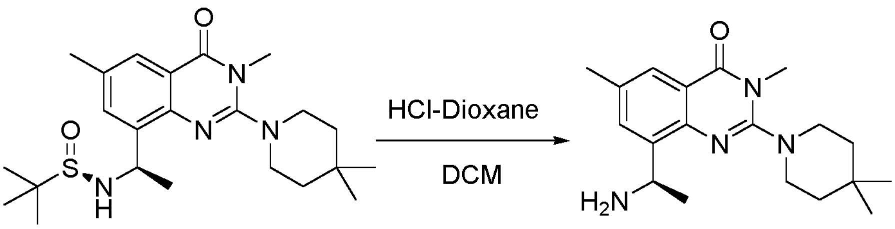


6-4 6-5

**6-4** (0.6 g, 1.4 mmoL) was dissolved in DCM (3 mL), to which was added HCl-dioxane (3 mL) dropwise, and then reacted at room temperature. The reaction was monitored by TLC. The reaction solution was concentrated to dry, and then water (10 mL) and EA (10 mL) were added and dissolved, until the clear solution was obtained. The resultant solution was poured into a separatory funnel and separated. The aqueous phase was extracted once with EA (10 mL), and adjusted to pH 8-9 with $NaHCO_3$, followed by extraction with DCM (15 mL×3). The organic phase was dried and concentrated, to obtain 0.4 g of light yellow solid **(6-5),** with a yield of 87.8%, MS: *m/z* 329 $[M+H]^+$.

Synthesis of compound (R)-2-((1-(2-(4,4-dimethylpiperidin-1-yl)-3,6-dimethyl-4-oxo-3,4-dihydroquinazolin-8-yl)ethyl) yl) benzoic acid (compound **6)**

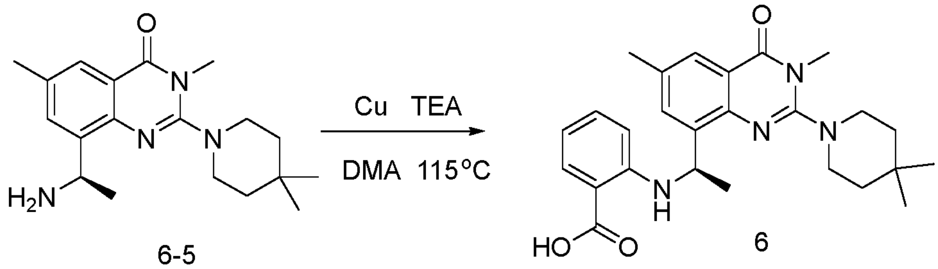


6-5 6

**6-5** (70 mg, 0.22 mmoL), 2-iodobenzoic acid (83 mg, 0.33 mmoL), TEA (43 mg, 0.43 mmoL), and copper nanoparticles (14 mg, 0.22 mmoL) were successively added into DMA (1 mL), and then reacted at 115 °C for 3 h. The reaction was monitored by TLC. The reaction solution was added with water (15 mL), adjusted to pH 3-4 with dilute HCl (1N), and extracted with EA (10 mL×3). EA layers were combined, washed with saturated NaCl solution (10 mL×3), dried over anhydrous $Na_2SO_4$, concentrated, and purified by column chromatography, to obtain 35 mg of solid (compound 6), with a yield of 36.6%, MS: *m/z* 489 $[M+H]^+$.

$^1H$ NMR (400 MHz, Chloroform-d) $\delta$ 7.99 (d, *J* = 7.9 Hz, 1H), 7.89 (s, 1H), 7.57 (s, 1H), 7.22 (t, *J* = 7.8 Hz, 1H), 6.67 (s, 1H), 6.60 (dd, *J* = 10.6, 8.2 Hz, 1H), 5.56 (d, *J* = 6.8 Hz, 1H), 3.57 (s, 3H), 3.22 (s, 4H), 2.39 (s, 3H), 1.72 (d, *J* = 6.5 Hz, 3H), 1.26 (s, 4H), 1.03 (s, 6H).

**Example 19 Synthesis of compound (R)-6-chloro-3-((1-(3,6-dimethyl-4-oxo-2-(pyridin-4-yl)-3,4-dihydroquinazolin-8-yl)ethyl)amino)pyridinecarboxylic acid(compound 109)**

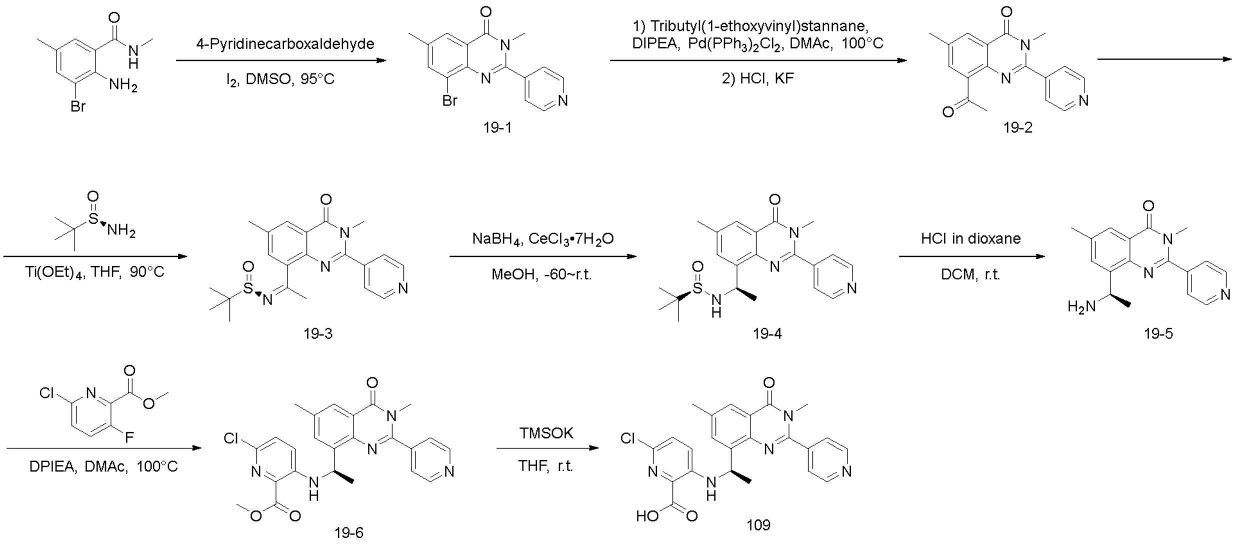


Step 1: Synthesis of intermediate 8-bromo-3,6-dimethyl-2-(pyridin-4-yl)quinazolin-4(3H)-one (**19-1**)

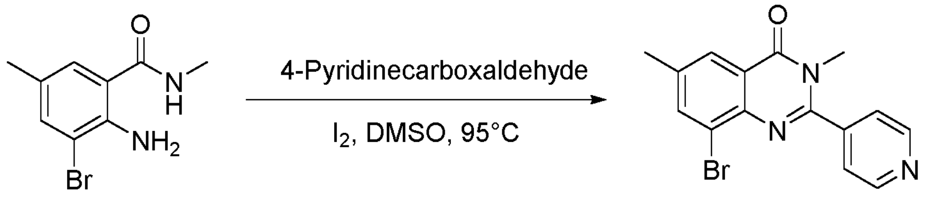


19-1

2-amino-3-bromo-N,5-dimethylbenzamide (2.4 g, 10.0 mmol), 4-pyridinformaldehyde (1.3 g, 12.0 mmol), and iodine (3.0 g, 12.0 mmol) were successively added into dimethylsulfoxide (25 mL) at room temperature, and then heated to 95 °C and reacted overnight. The reaction solution was cooled to room temperature, and slowly poured into water (50 mL), to which was added saturated sodium thiosulfate aqueous solution (10 mL), and then stirred for 2 h. After filtration, the filter cake was sequentially rinsed once with water (20 mL), absolute ethanol (20 mL), and methyl tert-butyl ether (20 mL), respectively, and then dried in vacuum, to obtain 2.9 g of product (intermediate **19-1),** with a yield of 87.9%. MS: *m/z* 330.0 $[M+H]^+$.

Step 2: Synthesis of intermediate 8-acetyl-3,6-dimethyl-2-(pyridin-4-yl)quinazolin-4(3H)-one **(19-2)**

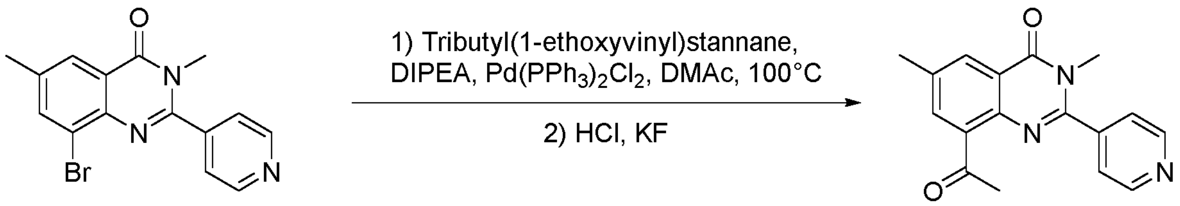


19-1 19-2

Intermediate **19-1** (2.9 g, 8.8 mmol), tributyl(1-ethoxyvinyl)tin (4.8 g, 13.2 mmol), *N,N*-diisopropylethylamine (3.4 g, 26.4 mmol), and $Pd(PPh_3)_2Cl_2$ (0.6 g, 0.9 mmol) were successively added into N,N-dimethylacetamide (40 mL) at room temperature, and then the system was purged with nitrogen for three times. The reaction solution was heated to 100 °C and reacted overnight, and then cooled to room temperature, to which was slowly added the solution of HCl in dioxane (4 mol/L). The reaction solution was adjusted to pH 1-2, to which was added a small amount of water (1 mL), and then stirred and reacted for 1 h. The reaction solution was diluted with ethyl acetate (20 mL), and then water (20 mL) was added. The resultant solution was adjusted to pH>7 with KF aqueous solution, stirred for 2 h, and filtered. The filter cake was triturated in ethyl acetate (20 mL × 3). The filtrate was combined and separated in a separatory funnel. The aqueous layer was extracted with ethyl acetate (20 mL). The organic layers were combined, dried over anhydrous $Na_2SO_4$, and concentrated. The obtained residue was triturated in methanol (20 mL), to obtain 2.5 g of product (intermediate **19-2),** with a yield of 96.6%. MS: *m/z* 294.1 $[M+H]^+$.

Step 3: Synthesis of intermediate (R,Z)-N-(1-(3,6-dimethyl-4-oxo-2-(pyridin-4-yl)-3,4-dihydroquinazolin-8-yl)ethylene)-2-methylpropane-2-sulfonamide(**19-3**)

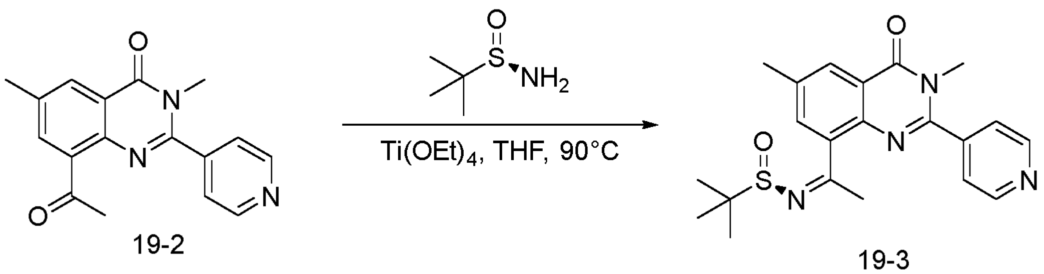


19-2 19-3

Intermediate **19-2** (2.5 g, 8.5 mmol), (R)-(+)-tert-butylsulfenamide (2.1 g, 17.0 mmol), and tetraethyl titanate (9.7 g, 42.5 mmol) were successively added into dry tetrahydrofuran (25 mL) at room temperature, heated to 90 °C, and reacted

overnight. The reaction solution was cooled to room termperature, and diluted by adding dichloromethane (25 mL), to which was added water (50 mL). The resultant solution was stirred for 0.5 h and filtered. The filter cake was triturated in dichloromethane (20 mL × 3). The filtrate was combined and separated in a separatory funnel. The aqueous layer was extracted with dichloromethane (20 mL). The organic layers were combined, dried over anhydrous $Na_2SO_4$, and concentrated, to obtain 3.0 g of crude product (intermediate **19-3),** with a yield of 88.6%. MS: *m*/*z* 397.2 $[M+H]^+$.

Step 4: Synthesis of intermediate (*R*)-*N*-((*R*)-1-(3,6-dimethyl-4-oxo-2-(pyridin-4-yl)-3,4-dihydroquinazolin-8-yl)ethylene)-2-methylpropane-2-sulfonamide **(19-4)**

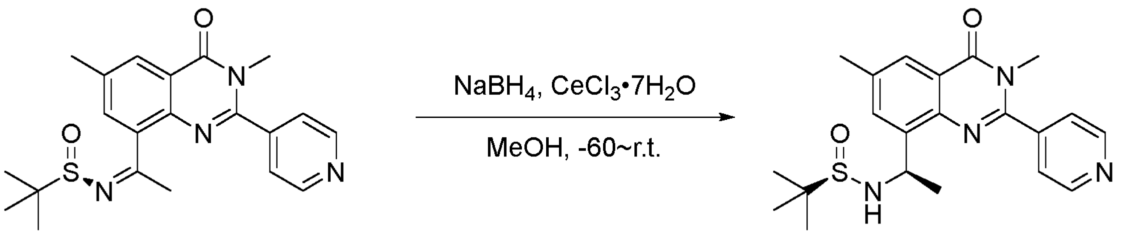


19-3 19-4

Intermediate **19-3** (3.0 g, 7.5 mmol) and $CeCl_3{\cdot}7H_2O$ (1.4 g, 3.8 mmol) were successively added into MeOH (30 mL) at room temperature, and then cooled to -65 °C in a dry ice bath. $NaBH_4$ (0.6 g, 15.0 mmol) was dissolved in methanol in batches, and added into the reaction system dropwise, during which the temperature was controlled not to exceed -60 °C. This took about 30 min, and then the dry ice bath was removed. The reaction was naturally warmed to room temperature, and diluted by adding dichloromethane (30 mL), to which was added water (60 mL), and then poured into a separatory funnel and separated. The water layer was extracted with dichloromethane (50 mL). The organic layers were combined, dried over anhydrous $Na_2SO_4$, and concentrated, followed by column chromatography and recrystallization in methanol/water (1:1), to obtain 2.0 g of product (intermediate **19-4),** with a yield of 66.7%, MS: *m*/*z* 399.2 $[M+H]^+$.

Step 5. Synthesis of intermediate (*R*)-8-(1-aminoethyl)-3,6-dimethyl-2-(pyridin-4-yl)quinazolin-4(3*H*)-one **(19-5)**

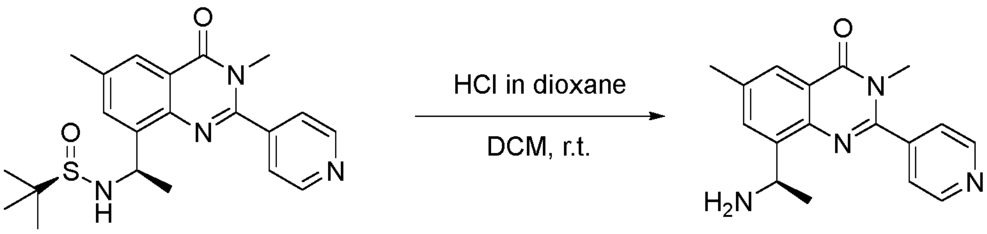


19-4 19-5

Intermediate **19-4** (2.0 g, 5.0 mmol) was added to dichloromethane (20 mL) at room temperature, to which was added the solution of HCl in dioxane (4 mol/L, 10 mL) dropwise, and then reacted for 2 h under stirring. The reaction solution was concentrated, and the residue was dissolved in water (10 mL), followed by extraction with ethyl acetate (5 mL). The ethyl acetate layer was washed once with 1N HCl (10 ml) and discarded. The acidic water layers were combined, adjusted to pH of greater than 7, and then extracted with dichloromethane (10 mL × 3). The organic layers were combined, dried over anhydrous $Na_2SO_4$, and concentrated, to obtain 1.2 g of product (intermediate 19-5), with a yield of 81.3%, MS: *m*/*z* 295.1 $[M+H]^+$.

Step 6: Synthesis of intermediate methyl (*R*)-6-chloro-3-((1-(3,6-dimethyl-4-oxo-2-(pyridin-4-yl)-3,4-dihydroquinazolin-8-yl)ethyl)amino)pyridinecarboxylate **(19-6)**

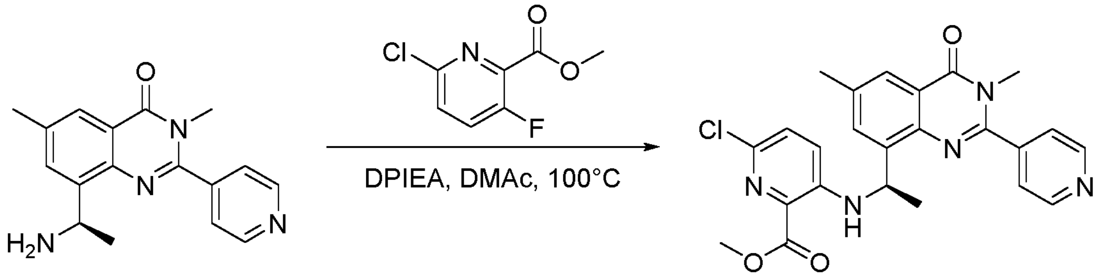


19-5 19-6

Intermediate **19-5** (59 mg, 0.2 mmol), 6-chloro-methyl 3-fluoropyridinecarboxylate (76 mg, 0.4 mmol), and DIPEA (77 mg, 0.6 mmol) were added into N,N-dimethylacetamide (1 mL), heated to 100 °C, and reacted overnight under stirring. The reaction system was cooled to room temperature, diluted with ethyl acetate (2 mL), and washed with water (2 mL×2). The organic layer was dried over anhydrous $Na_2SO_4$, concentrated, and purified by column chromatography, to obtain 60 mg of product (intermediate **19-6),** with a yield of 64.7%, MS: *m/z* 464.1 $[M+H]^+$.

Step 7: Synthesis of compound *(R)*-6-chloro-3-((1-(3,6-dimethyl-4-oxo-2-(pyridin-4-yl)-3,4-dihydroquinazolin-8-yl) ethyl)amino)pyridinecarboxylic acid (compound **109)**

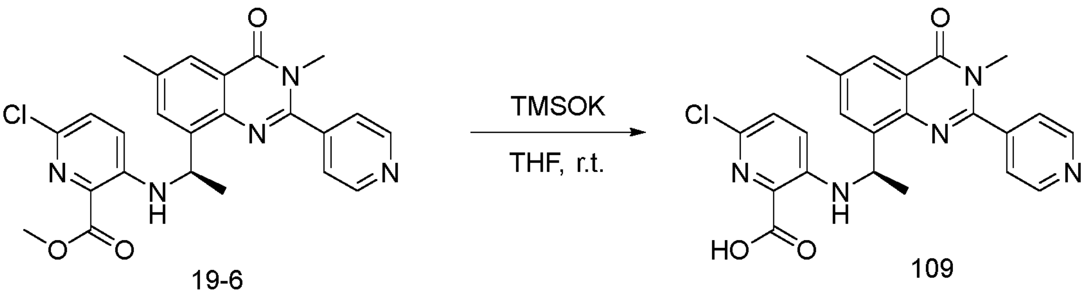


19-6 109

Intermediate **19-6** (46 mg, 0.1 mmol) was dissolved in dry tetrahydrofuran (1 mL), to which was added potassium trimethylsilanolate (26 mg, 0.2 mmol), and then reacted for 20 min under stirring at room temperature. The reaction solution was adjusted to about pH 2 with 0.5 N HCl, and extracted with dichloromethane (1 mL×2). The organic layer was dried over anhydrous $Na_2SO_4$, concentrated, and purified by column chromatography, to obtain 30 mg of product (compound **109),** with a yield of 66.7%, MS: *m/z* 450.1 $[M+H]^+$. $^1$H NMR (400 MHz, DMSO-$d_6$) $\delta$ 12.99 (s, 1H), 8.88 - 8.73 (m, 2H), 8.31 (d, *J* = 7.4 Hz, 1H), 7.92 (d, *J* = 1.9 Hz, 1H), 7.84 - 7.74 (m, 2H), 7.68 (d, *J* = 2.1 Hz, 1H), 7.32 (d, *J* = 9.0 Hz, 1H), 7.11 (d, *J* = 9.1 Hz, 1H), 5.41 (p, *J* = 6.7 Hz, 1H), 3.82 (s, 3H), 2.42 (s, 3H), 1.59 (d, *J* = 6.6 Hz, 3H).

Using a method similar to the above examples, the following compounds could be synthesized using appropriate reagents under suitable reaction conditions.

Table 1. Compounds of the present invention.

| Compound No. | Structure | HNMR |
|---|---|---|
| **1** | 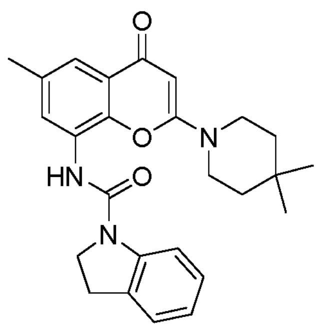 | $^1$H NMR (400 MHz, DMSO-d$_6$) $\delta$ 8.52 (s, 1H), 7.84 (d, *J* = 8.0 Hz, 1H), 7.52 (d, *J* = 3.0 Hz, 2H), 7.21 (d, *J* = 7.4 Hz, 1H), 7.11 (t, *J* = 7.8 Hz, 1H), 6.91 (t, *J* = 7.4 Hz, 1H), 5.48 (s, 1H), 4.14 (t, *J* = 8.6 Hz, 2H), 3.45 (dd, *J* = 6.9, 4.7 Hz, 4H), 3.20 (t, *J* = 8.6 Hz, 2H), 2.38 (s, 3H), 1.30 (t, J = 5.8 Hz, 4H), 0.91 (s, 6H).<br><br>LCMS (M+1): 432.2 |

(continued)

| Compound No. | Structure | HNMR |
|---|---|---|
| **4** | 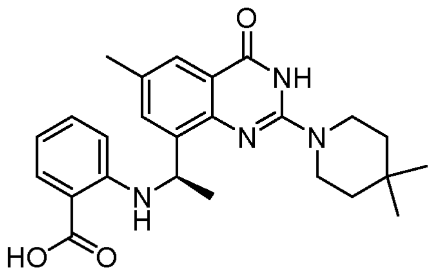 | $^{1}$H NMR (400 MHz, Chloroform-d) $\delta$ 7.99 *(d, J* = 7.9 Hz, 1H), 7.89 (s, 1H), 7.57 (s, 1H), 7.22 (t, *J* = 7.8 Hz, 1H), 6.67 (s, 1H), 6.60 (dd, *J*= 10.6, 8.2 Hz, 1H), 5.56 (d, *J* = 6.8 Hz, 1H), 3.22 (s, 4H), 2.39 (s, 3H), 1.72 (d, *J* = 6.5 Hz, 3H), 1.26 (s, 4H), 1.03 (s, 6H). LCMS (M+1): 435 |
| **5** | 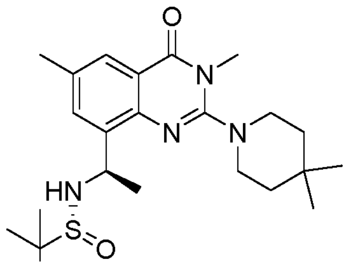 | $^{1}$HNMR (400 MHz, Chloroform-d) $\delta$ 7.89 (dd, *J*= 2.1, 1.0 Hz, 1H), 7.38 (d, *J* = 2.1 Hz, 1H), 4.82 (p, *J*= 7.0 Hz, 1H), 3.54 (s, 3H), 3.15 (dd, *J* = 7.3, 4.2 Hz, 4H), 2.41 (s, 3H), 1.61 (s, 3H), 1.55 (dd, *J* = 7.2, 4.2 Hz, 4H), 1.23 (s, 9H), 1.03 (s, 6H) LCMS (M+1): 433 |
| **6** | 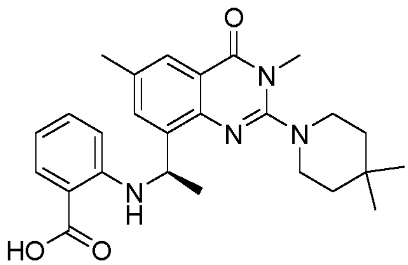 | $^{1}$H NMR (400 MHz, Chloroform-d) $\delta$ 7.99 (d, J = 7.9 Hz, 1H), 7.89 (s, 1H), 7.57 (s, 1H), 7.22 (t, J = 7.8 Hz, 1H), 6.67 (s, 1H), 6.60 (dd, J = 10.6, 8.2 Hz, 1H), 5.56 (d, J = 6.8 Hz, 1H), 3.57 (s, 3H), 3.22 (s, 4H), 2.39 (s, 3H), 1.72 (d, J = 6.5 Hz, 3H), 1.26 (s, 4H), 1.03 (s, 6H). LCMS (M+1): 449 |
| **7** | 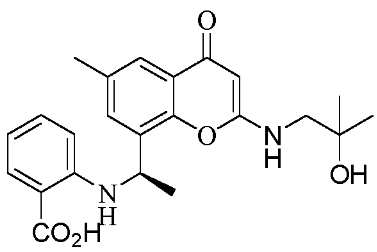 | $^{1}$H NMR (400 MHz, DMSO-d6) $\delta$ 12.8(s,1H),8.67 (s, 1H), 7.97 (d, J = 7.0 Hz, 1H), 7.79 (dd, J = 7.9, 1.7 Hz, 1H), 7.56 (d, J = 2.2 Hz, 1H), 7.32 (d, J = 2.2 Hz, 1H), 7.17 (t, J = 7.8 Hz, 1H), 6.50 (t, J = 7.5 Hz, 1H), 6.44 (d, J = 8.4 Hz, 1H), 5.33 (s, 1H), 5.15 (d, J = 7.0 Hz, 1H), 3.39 (s, 1H), 3.17 (d, J = 6.2 Hz, 2H), 2.28 (s, 3H), 1.55 (d, J = 6.6 Hz, 3H), 1.16 (d, J = 7.6 Hz, 6H). LCMS (M+1): 411 |
| **8** | 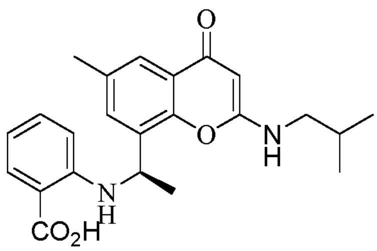 | $^{1}$H NMR (400 MHz, DMSO-$d_6$) $\delta$ 12.80 (s, 1H), 8.51 (s, 1H), 8.04 (s, 1H), 7.80 (dd, J = 8.0, 1.7 Hz, 1H), 7.57 (d, J = 2.2 Hz, 1H), 7.31 (d, J = 2.3 Hz, 1H), 7.20 (t, J = 7.8 Hz, 1H), 6.52 (t, J = 7.5 Hz, 1H), 6.42 (d, J = 8.5 Hz, 1H), 5.22 (s, 1H), 5.11 (s, 1H), 3.05 (s, 2H), 2.28 (s, 3H), 1.88 (dt, J = 13.4, 6.7 Hz, 1H), 1.56 (d, J = 6.6 Hz, 3H), 0.94 (dd, J = 6.6, 1.8 Hz, 6H). LCMS (M+1): 395 |

(continued)

| Compound No. | Structure | HNMR |
|---|---|---|
| **27** | 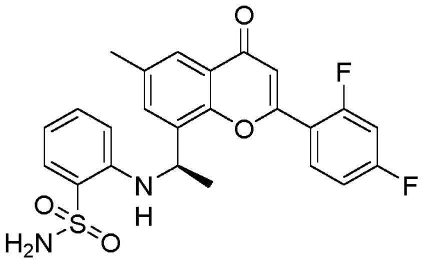 | $^{1}$H NMR (400 MHz, Chloroform-d) $\delta$ *8.01* (s, 1H), 8.02- 7.92 (m, 1H), 7.74 (dd, J = 8.5, 5.2 Hz, 2H), 7.61 - 7.57 (m, 1H), 7.23 (d, J = 8.5 Hz, 2H), 7.19 (s, 1H), 6.45 (d, J = 8.6 Hz, 1H), 5.63 (q, J = 6.6 Hz, 1H), 3.66 (s, 4H), 2.43 (s, 3H), 1.60 (d, J = 6.7 Hz, 3H).<br>LCMS (M+1): 471 |
| **28** | 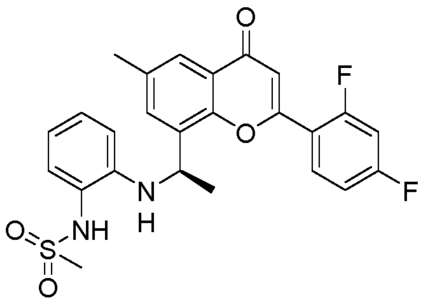 | $^{1}$H NMR (400 MHz, DMSO-d6) $\delta$ 8.96 (s, 1H), 8.11 (td, J = 8.8, 6.5 Hz, 1H), 8.01 (s, 1H) 7.73 (d, J = 2.2 Hz, 1H), 7.67 (d, J = 2.2 Hz, 1H), 7.57 (ddd, J = 11.9, 9.2, 2.5 Hz, 1H), 7.34 (td, J = 8.5, 2.5 Hz, 1H), 7.13 (dd, J = 7.8, 1.6 Hz, 1H), 6.81 (s, 1H), 6.56 (t, J = 7.5 Hz, 1H), 6.36 (d, J = 8.0 Hz, 1H), 5.69 (d, J = 6.6 Hz, 1H), 5.24 - 5.07 (m, 1H), 3.00 (s, 3H), 2.34 (s, 3H), 1.58 (d, J = 6.6 Hz, 3H).<br>LCMS (M+1): 485 |
| **32** | 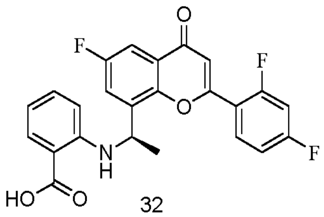 32 | LCMS (M+1): 440 |
| **35** | 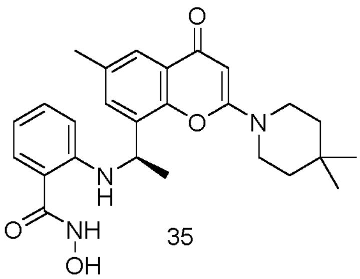 35 | $^{1}$H NMR (400 MHz, DMSO-$d_6$) $\delta$ *11.13* (s, 1H), 9.02 (s, 1H), 7.99 (d, J = 6.2 Hz, 1H), 7.62 - 7.55 (m, 1H), 7.42 - 7.32 (m, 2H), 7.18 - 7.08 (m, 1H), 6.54 (q, J = 8.3, 7.5 Hz, 1H), 6.38 (d, J = 8.4 Hz, 1H), 5.52 (s, 1H), 5.08 - 4.95 (m, 1H), 3.54 (t, J = 5.9 Hz, 4H), 2.29 (d, J = 4.1 Hz, 3H), 1.53 (d, J = 6.6 Hz, 3H), 1.42 (t, J = 5.9 Hz, 4H), 0.98 (s, 6H).<br>LCMS (M+1): 450.2 |
| **37** | 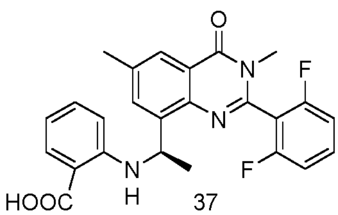 37 | $^{1}$H NMR (400 MHz, Chloroform-d) $\delta$ 8.04 (d, J = 2.0 Hz, 1H), 7.97 (dd, J = 8.0, 1.7 Hz, 1H), 7.78 (s, 1H), 7.61 (d, J = 2.1 Hz, 1H), 7.57 - 7.45 (m, 1H), 7.18 (t, J = 7.3 Hz, 1H), 7.11 (td, J = 8.2, 2.5 Hz, 2H), 6.54 (s, 1H), 6.49 (d, J = 8.6 Hz, 1H), 5.59 (q, J = 6.6 Hz, 1H), 3.50 (s, 3H), 2.44 (s, 3H), 1.60 (d, J = 6.6 Hz, 3H),<br>LCMS (M+1): 450 |
| **38** | 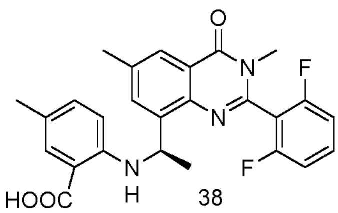 38 | $^{1}$H NMR (400 MHz, Chloroform-d) $\delta$ 8.03 (s, 1H), 7.78 (s, 1H), 7.60 (d, J = 2.1 Hz, 1H), 7.51 (ddd, J = 14.8, 8.5, 6.3 Hz, 1H), 7.11 (td, J = 8.2, 4.2 Hz, 2H), 7.01 (dd, J = 8.7, 2.3 Hz, 1H), 6.42 (d, J = 8.7 Hz, 1H), 5.56 (q, J = 6.7 Hz, 1H), 3.50 (s, 3H), 2.43 (s, 3H), 2.17 (s, 3H), 1.59 (d, J = 6.7 Hz, 3H).<br>LCMS (M+1): 464 |

(continued)

| Compound No. | Structure | HNMR |
|---|---|---|
| **41** | 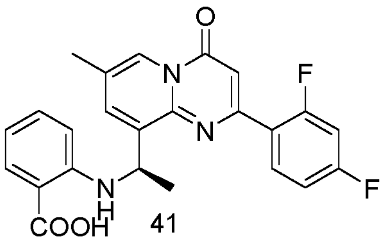 41 | $^{1}$H NMR (400 MHz, DMSO-$d_6$) $\delta$ 12.78 (s, 1H), 8.76 (s, 1H), 8.54 (s, 1H), 8.34 - 8.26 (m, 1H), 7.84 - 7.78 (m, 2H), 7.51 - 7.43 (m, 1H), 7.34 - 7.27 (m, 1H), 7.18 (t, J = 7.8 Hz, 1H), 6.83 (d, J = 1.5 Hz, 1H), 6.54 (t, J = 7.5 Hz, 1H), 6.38 (d, J = 8.5 Hz, 1H), 5.55 - 5.44 (m, 1H), 2.35 (s, 3H), 1.64 (d, J = 6.6 Hz, 3H). LCMS (M+1): 436 |
| **42** | 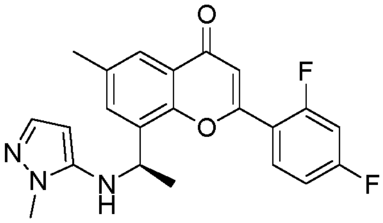 | $^{1}$H NMR (400 MHz, DMSO-$d_6$) $\delta$ 7.78 (d, J = 2.2 Hz, 1H), 7.70 (d, J = 2.2 Hz, 1H), 7.61-5.52 (m, 1H), 7.37 - 7.30 (m, 2H), 6.81 (s, 1H), 6.75 - 6.61 (m, 3H), 5.41 (t, J = 6.7 Hz, 1H), 3.25 (s, 3H), 2.37 (s, 3H), 1.71 (d, J = 6.7 Hz, 3H). LCMS (M+1): 396.1 |
| **43** | 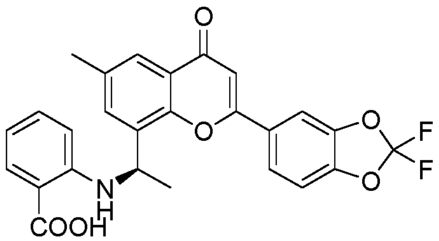 | $^{1}$H NMR (400 MHz, DMSO-$d_6$) $\delta$ 12.77 (brs, 1H), 8.47(s, 1H), 8.20 (d, J = 1.8 Hz, 1H), 8.05 (dd, J = 8.5, 1.9 Hz, 1H), 7.81 (dd, J = 7.9, 1.7 Hz, 1H), 7.74 (d, J = 2.2 Hz, 1H), 7.62 (d, J = 8.6 Hz, 1H), 7.55 (d, J = 2.3 Hz, 1H), 7.26 - 7.17 (m, 1H), 7.13 (s, 1H), 6.55 (t, J = 7.5 Hz, 1H), 6.51 (d, J = 8.5 Hz, 1H), 5.37 (t, J = 6.3 Hz, 1H), 2.36 (s, 3H), 1.65 (d, J = 6.6 Hz, 3H). LCMS (M+1): 480 |
| **44** | 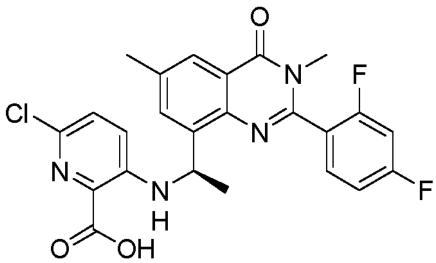 | $^{1}$H NMR (400 MHz, Chloroform-d) $\delta$ 8.25 (s, 1H), 8.07 - 8.02 (m, 1H), 7.58 - 7.50 (m, 2H), 7.13 - 7.02 (m, 2H), 7.06 - 6.97 (m, 1H), 6.93 (d, J = 9.0 Hz, 1H), 5.54 (s, 1H), 3.49 (d, J = 1.7 Hz, 3H), 2.44 (s, 3H), 1.63 (d, J = 6.7 Hz, 3H). LCMS (M+1): 485 |
| **45** | 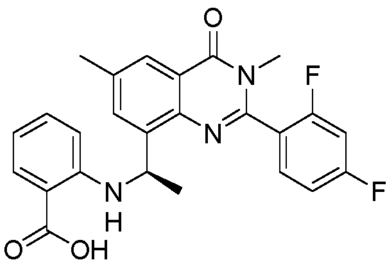 | $^{1}$H NMR (400 MHz, Chloroform-d) $\delta$ 8.02 (s, 1H), 8.00 - 7.91 (m, 1H), 7.75 (dd, J = 8.5, 5.2 Hz, 2H), 7.61 - 7.57 (m, 1H), 7.23 (d, J = 8.5 Hz, 2H), 7.17 (s, 1H), 6.46 (d, J = 8.6 Hz, 1H), 5.63 (q, J = 6.6 Hz, 1H), 3.66 (m, 4H), 2.45 (s, 3H), 1.61 (d, J = 6.7 Hz, 3H). LCMS (M+1): 450 |
| **46** | 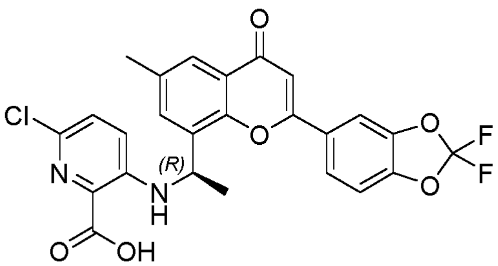 | $^{1}$H NMR (400 MHz, DMSO-$d_6$) $\delta$ 9.55 (s, 1H), 8.21 (s, 1H), 8.05 (d, 1H), 7.74 (d, 1H), 7.60 (d, J = 8.1 Hz, 1H), 7.49 (d, 1H), 7.18 (s, 2H), 7.11 (s, 1H), 5.67 - 5.20 (m, 1H), 2.34 (s, 3H), 1.65 (d, 3H). LCMS (M+1): 515.1 |

(continued)

| Compound No. | Structure | HNMR |
|---|---|---|
| **47** | 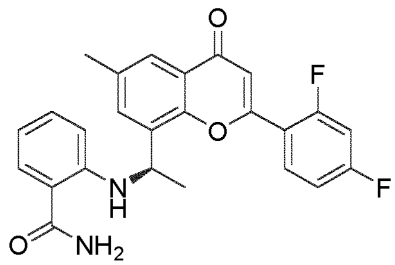 | $^1$H NMR (400 MHz, DMSO-$d_6$) $\delta$ 8.02 (td, J = 8.9, 6.5 Hz, 1H), 7.91 (s, 2H), 7.83-7.77 (m, 1H), 7.74 (s, 2H), 7.54 (ddd, J = 11.9, 9.2, 2.6 Hz, 1H), 7.15 (td, J = 8.5, 2.6 Hz, 1H), 7.04-6.96 (m, 1H), 6.93-6.84 (m, 1H), 6.77 (d, J = 0.9 Hz, 1H), 6.59-6.47 (m, 1H), 6.39 (d, J = 7.8 Hz, 1H), 5.37 (q, J = 6.9 Hz, 1H), 2.44 (s, 3H), 1.54 (d, J = 6.9 Hz, 3H). LCMS (M+1): 435 |
| **48** | 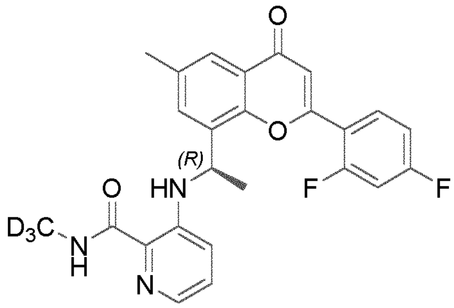 | $^1$H NMR (400 MHz, DMSO-$d_6$) $\delta$ 9.11 (d, J = 6.6 Hz, 1H), 8.72 (s, 1H), 8.14 (td, J = 8.8, 6.4 Hz, 1H), 7.79 - 7.73 (m, 2H), 7.61 - 7.53 (m, 2H), 7.36 - 7.30 (m, 1H), 7.18 (dd, J = 8.6, 4.3 Hz, 1H), 6.90 (dd, J = 8.8, 1.3 Hz, 1H), 6.80 (d, J = 0.8 Hz, 1H), 5.20 (p, J = 6.6 Hz, 1H), 2.35 (s, 3H), 1.62 (d, J = 6.6 Hz, 3H). LCMS (M+1): 453.2 |
| **49** | 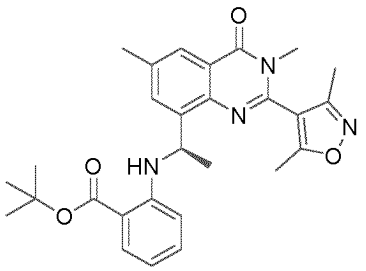 | LCMS (M+1): 489 |
| **50** | 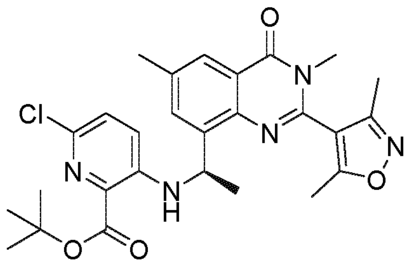 | LCMS (M+1): 524 |
| **51** | 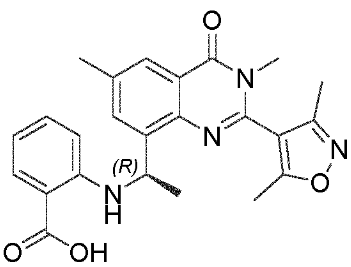 | LCMS (M+1): 433 |

(continued)

| Compound No. | Structure | HNMR |
|---|---|---|
| **52** | 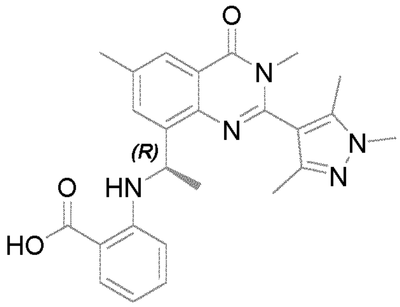 | LCMS (M+1): 446 |
| **53** | 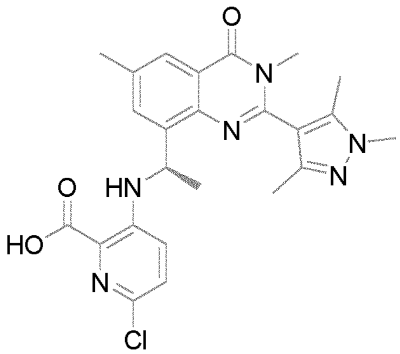 | LCMS (M+1): 481 |
| **54** | 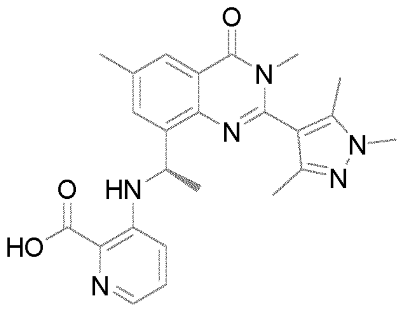 | LCMS (M+1): 447 |
| **55** | 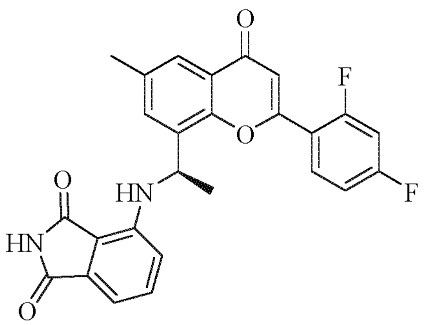 | LCMS (M+1): 461 |
| **56** | 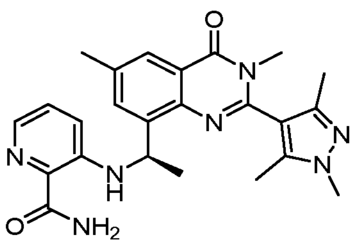 | LCMS (M+1): 446 |

(continued)

| Compound No. | Structure | HNMR |
|---|---|---|
| **57** | 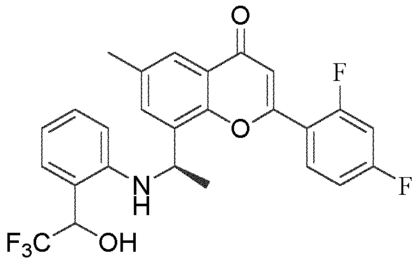 | $^1$H NMR (400 MHz, DMSO-$d_6$) δ *8.11* (td, J = 8.9, 6.5 Hz, 1H), 7.73 (dd, J = 2.3, 1.0 Hz, 1H), 7.62 - 7.54 (m, 2H), 7.38 - 7.23 (m, 2H), 6.97 (q, J = 6.5, 5.4 Hz, 2H), 6.81 (s, 1H), 6.56 (t, J = 7.4 Hz, 1H), 6.32 (d, J = 8.3 Hz, 1H), 6.13 (d, J = 7.1 Hz, 1H), 5.55 (d, J = 8.9 Hz, 1H), 5.16 (t, J = 6.8 Hz, 1H), 2.33 (s, 3H), 1.57 (d, J = 6.6 Hz, 3H). LCMS (M+1): 490.1 |
| **58** | 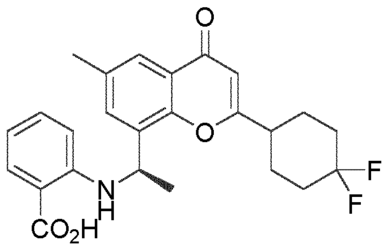 | $^1$H NMR (400 MHz, DMSO-$d_6$) δ 12.78 (s, 1H), 8.43 (s, 1H), 7.81 (dd, J = 7.9, 1.7 Hz, 1H), 7.69 (d, J = 2.1 Hz, 1H), 7.54 (d, J = 2.2 Hz, 1H), 7.28 - 7.16 (m, 1H), 6.54 (t, J = 7.5 Hz, 1H), 6.46 (d, J = 8.4 Hz, 1H), 6.25 (s, 1H), 5.13 (d, J = 6.7 Hz, 1H), 2.34 (s, 3H), 2.22 - 1.64 (m, 9H), 1.60 (d, J = 6.6 Hz, 3H). LCMS (M+1): 442.1 |
| **59** | 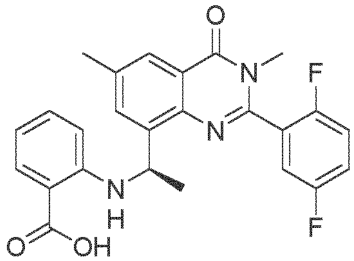 | $^1$H NMR (400 MHz, Chloroform-d) δ 8.03 (t, J = 1.4 Hz, 1H), 7.98 (dd, J = 8.1, 1.7 Hz, 1H), 7.61 (d, J = 2.1 Hz, 1H), 7.31 (ddd, J = 7.8, 4.6, 2.7 Hz, 1H), 7.25 - 7.16 (m, 3H), 6.57 (t, J = 7.5 Hz, 1H), 6.46 (d, J = 8.6 Hz, 1H), 5.57 (d, J = 6.8 Hz, 1H), 3.52 (d, J = 1.8 Hz, 3H), 2.44 (s, 3H), 1.63 (d, J = 6.7 Hz, 3H). LCMS (M+1): 450 |
| **60** | 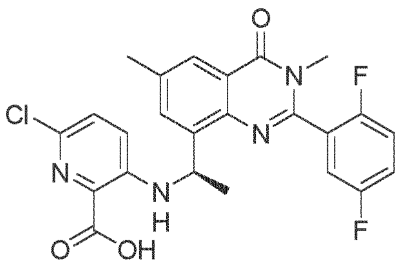 | $^1$H NMR (400 MHz, Chloroform-d) δ 8.02 (s, 1H), 8.00 - 7.91 (m, 1H), 7.22 (s, 4H), 6.93 (d, J = 9.0 Hz, 1H),6.79 (s, 1H), 5.45 (s, 1H), 3.49 (d, J = 1.7 Hz, 3H), 2.44 (s, 3H), 1.63 (d, J = 6.7 Hz, 3H). LCMS (M+1): 485 |
| **61** | 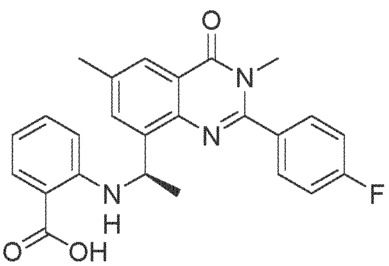 | $^1$H NMR (400 MHz, Chloroform-d) δ 8.02 (s, 1H), 8.00 - 7.95 (m, 1H), 7.65 (dd, J = 8.5, 5.2 Hz, 2H), 7.61 - 7.57 (m, 1H), 7.23 (d, J = 8.5 Hz, 2H), 7.17 (s, 1H), 6.57 (t, J = 7.6 Hz, 1H), 6.46 (d, J = 8.6 Hz, 1H), 5.60 (q, J = 6.6 Hz, 1H), 3.56 (s, 4H), 2.43 (s, 3H), 1.63 (d, J = 6.7 Hz, 3H). LCMS (M+1): 432 |

(continued)

| Compound No. | Structure | HNMR |
|---|---|---|
| **62** | 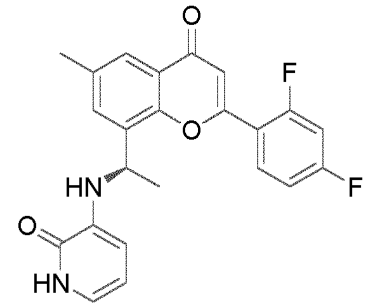 | $^1$H NMR (400 MHz, DMSO-$d_6$) $\delta$ *11.44* (s, 1H), 8.13 (td, J = 8.8, 6.4 Hz, 1H), 7.73 (d, J = 2.3 Hz, 1H), 7.58 (dh, J = 6.0, 2.5 Hz, 2H), 7.36 (td, J = 8.5, 2.6 Hz, 1H), 6.80 (s, 1H), 6.58 (s, 1H), 6.00 - 5.86 (m, 2H), 5.80 (d, J = 7.1 Hz, 1H), 5.03 (q, J = 6.8 Hz, 1H), 2.37 (s, 3H), 1.58 (d, J = 6.7 Hz, 3H). LCMS (M+1): 409 |
| **63** | 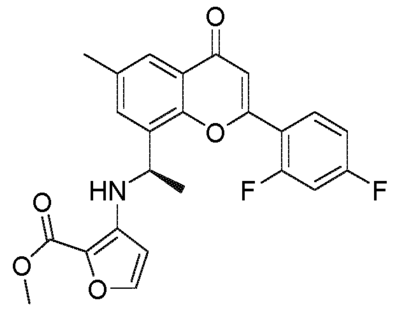 | $^1$H NMR (400 MHz, Chloroform-d) $\delta$ 7.93 (dd, J = 2.4, 0.8 Hz, 1H), 7.84 (d, J = 6.2 Hz, 1H), 7.52 (d, J = 2.4 Hz, 1H), 7.16 (d, J = 2.2 Hz, 1H), 7.11 - 7.07 (m, 1H), 7.02 (s, 1H), 6.84 (s, 1H), 5.89 (d, J = 2.0 Hz, 1H), 5.11 (q, J = 6.6 Hz, 1H), 3.91 (s, 3H), 2.43 (s, 3H), 1.67 (d, J = 6.8 Hz, 3H). LCMS (M+1): 440 |
| **64** | 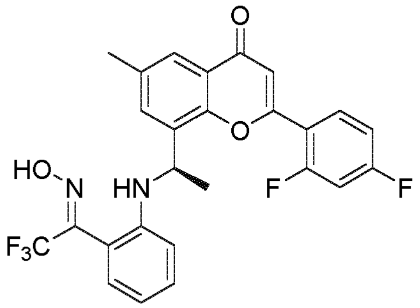 | $^1$H NMR (400 MHz, DMSO-$d_6$) $\delta$ *11.06* (s, 1H), 9.03 (d, J = 6.5 Hz, 1H), 7.96 (td, J = 8.9, 6.5 Hz, 1H), 7.70 (d, J = 8.4 Hz, 1H), 7.59 (dd, J = 2.1, 0.9 Hz, 1H), 7.53 - 7.41 (m, 2H), 7.29 (d, J = 2.2 Hz, 1H), 7.04 (s, 1H), 6.76 - 6.68 (m, 2H), 5.26 (h, J = 6.6, 6.2 Hz, 1H), 2.28 (s, 3H), 1.63 (d, J = 6.7 Hz, 3H). LCMS (M+1): 503.1 |
| **65** | 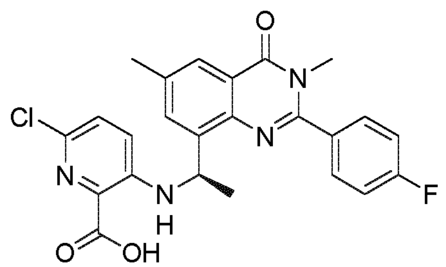 | $^1$H NMR (400 MHz, Chloroform-d) $\delta$ *8.01* (s, 1H), 8.00 - 7.93 (m, 1H), 7.24 (m, 4H), 7.61 -7.57 (m, 1H),6.92 (d, J = 9.0 Hz, 1H),6.75 (s, 1H), 5.43 (s, 1H), 3.40 (d, J = 1.7 Hz, 3H), 2.49 (s, 3H), 1.68 (d, J = 6.7 Hz, 3H). LCMS (M+1): 467 |
| **66** | 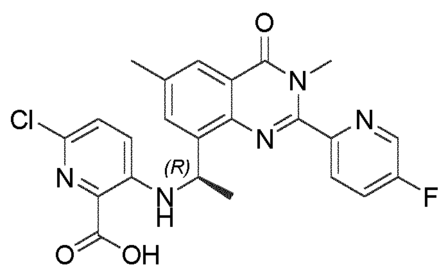 | $^1$H NMR (400 MHz, DMSO-$d_6$) $\delta$ 12.99 (s, 1H), 8.75 (d, J = 2.9 Hz, 1H), 8.43 (d, J = 7.0 Hz, 1H), 8.12 (dd, J = 8.7, 4.5 Hz, 1H), 8.01 (td, J = 8.7, 2.9 Hz, 1H), 7.91 (dd, J = 2.1, 1.0 Hz, 1H), 7.64 (d, J = 2.1 Hz, 1H), 7.29 (d, J = 8.9 Hz, 1H), 7.02 (d, J = 9.1 Hz, 1H), 5.46 (p, J = 6.7 Hz, 1H), 3.49 (s, 3H), 2.41 (s, 3H), 1.59 (d, J = 6.6 Hz, 3H). LCMS (M+1): 468.1 |

(continued)

| Compound No. | Structure | HNMR |
|---|---|---|
| **67 Isomer 1** | 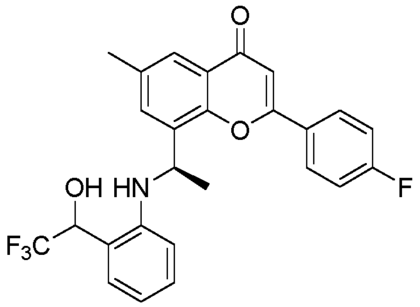 | $^1H$ NMR (400 MHz, DMSO-$d_6$) $\delta$ 8.26 - 8.14 (m, 2H), 7.73 (d, J = 2.3 Hz, 1H), 7.61 (d, J = 2.3 Hz, 1H), 7.42 (t, J = 8.8 Hz, 2H), 7.28 (d, J = 7.5 Hz, 1H), 7.08 (s, 1H), 6.98 (qd, J = 5.9, 2.8 Hz, 2H), 6.57 (t, J = 7.4 Hz, 1H), 6.35 (d, J = 8.3 Hz, 1H), 6.07 (d, J = 5.9 Hz, 1H), 5.59 (q, J = 7.2 Hz, 1H), 5.26 (t, J = 6.5 Hz, 1H), 2.35 (s, 3H), 1.61 (d, J = 6.6 Hz, 3H).<br>LCMS (M+1): 472.1 |
| **68 Isomer 2** | 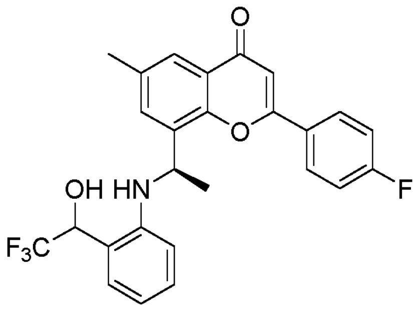 | $^1H$ NMR (400 MHz, DMSO-$d_6$) $\delta$ 8.19 (dd, J = 8.7, 5.3 Hz, 2H), 7.72 (d, J = 2.2 Hz, 1H), 7.54 (d, J = 2.3 Hz, 1H), 7.42 (t, J = 8.6 Hz, 2H), 7.28 (d, J = 7.6 Hz, 1H), 7.08 (s, 1H), 7.02 - 6.93 (m, 2H), 6.56 (t, J = 7.4 Hz, 1H), 6.35 (d, J = 8.3 Hz, 1H), 6.15 (d, J = 7.0 Hz, 1H), 5.56 (t, J = 6.8 Hz, 1H), 5.25 (t, J = 6.8 Hz, 1H), 2.33 (s, 3H), 1.62 (d, J = 6.6 Hz, 3H).<br>LCMS (M+1): 472.1 |
| **69** | 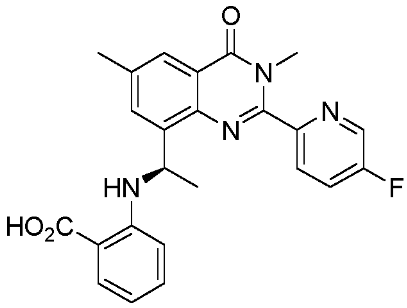 | $^1H$ NMR (400 MHz, DMSO-$d_6$) $\delta$ 12.69 (s, 1H), 8.75 (d, J = 2.9 Hz, 1H), 8.42 (d, J = 5.7 Hz, 1H), 8.11 (dd, J = 8.7, 4.6 Hz, 1H), 8.02 (td, J = 8.8, 2.9 Hz, 1H), 7.90 (t, J = 1.4 Hz, 1H), 7.78 (dd, J = 8.0, 1.7 Hz, 1H), 7.60 (d, J = 2.1 Hz, 1H), 7.16 (ddd, J = 8.6, 7.1, 1.7 Hz, 1H), 6.53 - 6.45 (m, 1H), 6.42 (d, J = 8.5 Hz, 1H), 5.48 (s, 1H), 3.50 (s, 3H), 2.40 (s, 3H), 1.55 (d, J = 6.7 Hz, 3H).<br>LCMS (M+1): 433 |
| **70** | 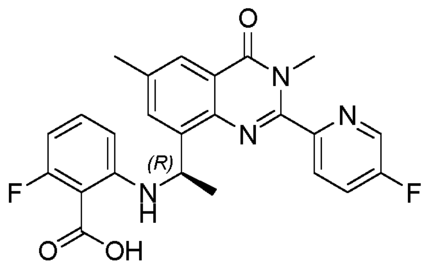 | $^1H$ NMR (400 MHz, DMSO-$d_6$) $\delta$ 8.75 (d, J = 2.9 Hz, 1H), 8.21 (s, 1H), 8.12 (dd, J = 8.7, 4.6 Hz, 1H), 8.02 (td, J = 8.7, 2.9 Hz, 1H), 7.90 (d, J = 2.1 Hz, 1H), 7.61 (d, J = 2.2 Hz, 1H), 7.07 (td, J = 8.3, 6.1 Hz, 1H), 6.27 (dd, J = 11.3, 8.1 Hz, 1H), 6.20 (d, J = 8.5 Hz, 1H), 5.42 (p, J = 6.7 Hz, 1H), 3.50 (s, 3H), 2.41 (s, 3H), 1.53 (d, J = 6.7 Hz, 3H).<br>LCMS (M+1): 451.1 |
| **71** | 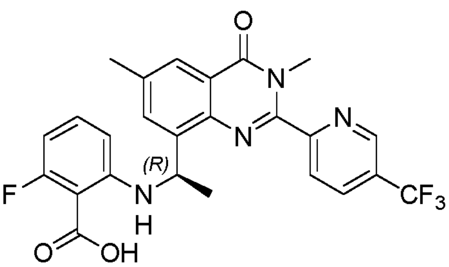 | $^1H$ NMR (400 MHz, Chloroform-d) $\delta$ 8.99 (s, 1H), 8.30 (s, 1H), 8.19 (d, J = 8.2 Hz, 1H), 8.13 - 7.99 (m, 1H),8.00 (s, 1H), 7.51 (s, 1H), 7.07 (s, 1H), 6.87 (s, 1H), 5.47 (s, 1H), 3.65 (s, 3H), 2.43 (s, 3H), 1.62 (s, 3H).<br>LCMS (M+1): 501 |

(continued)

| Compound No. | Structure | HNMR |
|---|---|---|
| **72** | 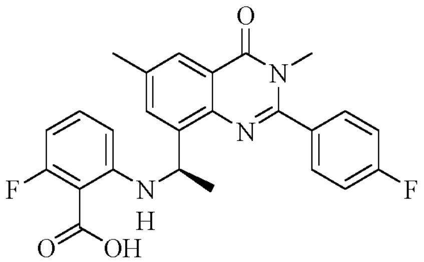 | $^1$H NMR (400 MHz, Chloroform-d) $\delta$ *8.00* (s, 1H), 7.63 (dd, J = 8.4, 5.2 Hz, 2H), 7.54 (s, 1H), 7.23 (t, J = 8.5 Hz, 2H), 7.01 (s, 1H), 6.24 (s, 2H), 5.55 (s, 1H), 3.55 (s, 3H), 2.41 (s, 3H), 1.59 (s, 3H).<br>LCMS (M+1): 450 |
| **73** | 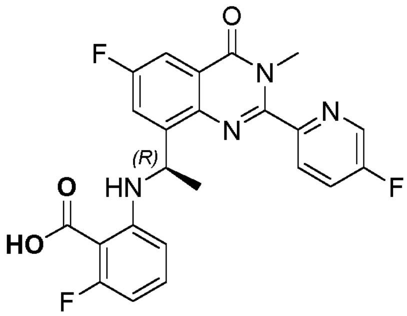 | $^1$H NMR (400 MHz, DMSO-$d_6$) $\delta$ 8.76 (d, J = 2.8 Hz, 1H), 8.45 (s, 1H), 8.16 (dd, J = 8.8, 4.5 Hz, 1H), 8.04 (td, J = 8.8, 2.9 Hz, 1H), 7.73 (dd, J = 8.4, 3.0 Hz, 1H), 7.58 (dd, J = 9.5, 3.1 Hz, 1H), 6.81 (q, J = 7.6 Hz, 1H), 6.19 - 6.13 (m, 1H), 5.96 (d, J = 8.3 Hz, 1H), 5.37 (s, 1H), 3.51 (s, 3H), 1.49 (d, J = 6.6 Hz, 3H).<br>LCMS (M+1): 455 |
| **74** | 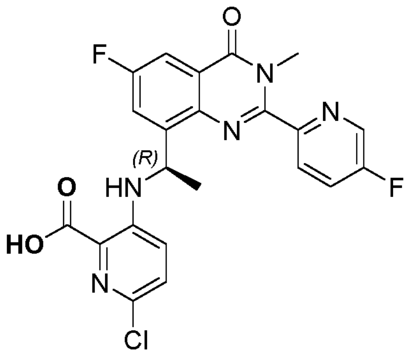 | $^1$H NMR (400 MHz, DMSO-$d_6$) $\delta$ 12.98 (s, 1H), 8.76 (d, J = 2.8 Hz, 1H), 8.13 (d, J = 10.6 Hz, 1H), 8.03 (td, J = 8.7, 2.9 Hz, 1H), 7.90 - 7.56 (m, 2H), 7.44 - 7.15 (m, 1H), 7.03 (dd, J = 25.5, 8.9 Hz, 1H), 5.46 (t, J = 6.9 Hz, 1H), 3.50 (s, 3H), 1.62 (d, J = 6.5 Hz, 3H).<br>LCMS (M+1): 472 |
| **75** | 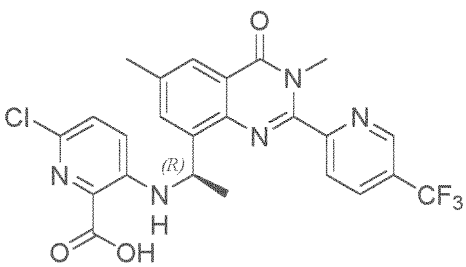 | $^1$H NMR (400 MHz, Chloroform-d) $\delta$ 8.99 (s, 1H), 8.31 (s, 1H), 8.19 (d, J = 8.2 Hz, 1H), 8.13 - 7.99 (m, 1H), 7.53 (s, 1H), 7.07 (s, 1H), 6.84 (s, 1H), 5.47 (s, 1H), 3.68 (s, 3H), 2.43 (s, 3H), 1.65 (s, 3H).<br>LCMS (M+1): 518 |
| **76** | 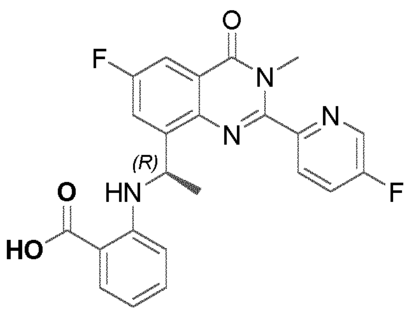 | $^1$H NMR (400 MHz, DMSO-$d_6$) $\delta$ 8.77 (d, J = 2.9 Hz, 1H), 8.55 (s, 1H), 8.13 (dd, J = 8.7, 4.6 Hz, 1H), 8.03 (td, J = 8.8, 2.9 Hz, 1H), 7.78 (ddd, J = 11.4, 8.1, 2.4 Hz, 2H), 7.58 (dd, J = 9.5, 3.0 Hz, 1H), 7.15 (ddd, J = 8.7, 7.1, 1.7 Hz, 1H), 6.55 - 6.47 (m, 1H), 6.36 (d, J = 8.4 Hz, 1H), 5.47 (s, 1H), 3.51 (s, 3H), 1.58 (d, J = 6.7 Hz, 3H).<br>LCMS (M+1): 437 |

(continued)

| Compound No. | Structure | HNMR |
|---|---|---|
| **77** | 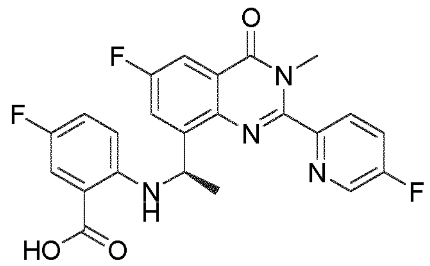 | $^1$H NMR (400 MHz, Chloroform-d) $\delta$ 8.61 (d, J = 2.7 Hz, 1H), 7.99 (dd, J = 8.7, 4.3 Hz, 1H), 7.89 (dd, J = 8.1, 2.9 Hz, 1H), 7.67 (td, J = 9.2, 8.5, 2.9 Hz, 2H), 7.58 - 7.50 (m, 1H), 7.01 - 6.90 (m, 1H), 6.41 (s, 1H), 5.57 (d, J = 6.7 Hz, 1H), 3.71 (s, 3H), 1.69 (d, J = 6.6 Hz, 3H).<br>LCMS (M+1): 455.1 |
| **78** | 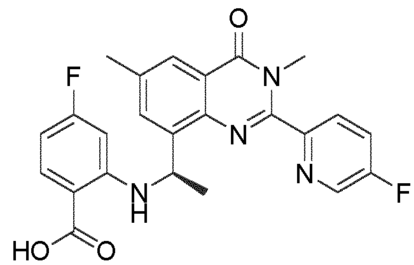 | $^1$H NMR (400 MHz, Chloroform-d) $\delta$ 8.58 (d, J = 2.8 Hz, 1H), 8.07 - 8.05 (m, 1H), 8.02 (dd, J = 8.7, 4.4 Hz, 1H), 7.98 (dd, J = 8.9, 6.7 Hz, 1H), 7.64 (td, J = 8.4, 2.9 Hz, 1H), 7.57 (d, J = 2.2 Hz, 1H), 6.29 - 6.20 (m, 2H), 5.54 (q, J = 6.7 Hz, 1H), 3.70 (s, 3H), 2.46 (s, 3H), 1.63 (d, J = 6.7 Hz, 3H).<br>LCMS (M+1): 451.2 |
| **79** | 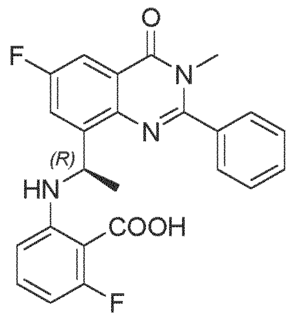 | LCMS (M+1): 436 |
| **80** | 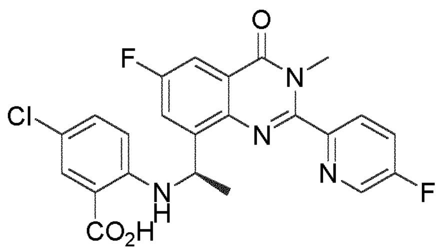 | $^1$H NMR (400 MHz, DMSO-d$_6$) $\delta$ *13.16* (s, 1H), 8.76 (d, J = 2.9 Hz, 1H), 8.47 (s, 1H), 8.12 (dd, J = 8.7, 4.6 Hz, 1H), 8.03 (td, J = 8.8, 2.9 Hz, 1H), 7.77 (dd, J = 8.3, 3.0 Hz, 1H), 7.73 (d, J = 2.7 Hz, 1H), 7.60 (dd, J = 9.4, 3.0 Hz, 1H), 7.21 (dd, J = 9.0, 2.7 Hz, 1H), 6.42 (d, J = 9.1 Hz, 1H), 5.45 (s, 1H), 3.32 (s, 3H), 1.59 (d, J = 6.6 Hz, 3H).<br>LCMS (M+1): 471 |
| **81** | 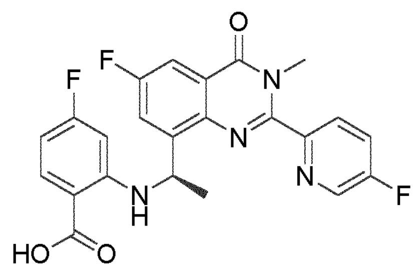 | $^1$H NMR (400 MHz, Chloroform-d) $\delta$ 8.60 (d, J = 2.8 Hz, 1H), 8.07 - 7.95 (m, 2H), 7.89 (dd, J = 8.2, 3.0 Hz, 1H), 7.66 (td, J = 8.3, 2.9 Hz, 1H), 7.50 (dd, J = 9.0, 3.0 Hz, 1H), 6.30 (ddd, J = 8.9, 7.9, 2.4 Hz, 1H), 6.13 (dd, J = 12.2, 2.4 Hz, 1H), 5.54 (q, J = 6.7 Hz, 1H), 3.72 (s, 3H), 1.66 (d, J = 6.7 Hz, 3H).<br>LCMS (M+1): 455.1 |

(continued)

| Compound No. | Structure | HNMR |
|---|---|---|
| **82** | 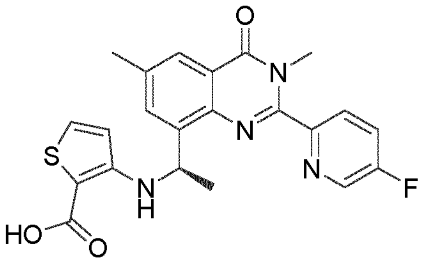 | $^1$H NMR (400 MHz, Chloroform-d) $\delta$ 8.64 - 8.52 (m, 1H), 8.09 - 7.96 (m, 2H), 7.68 (dt, J = 8.4, 4.2 Hz, 1H), 7.55 (d, J = 2.1 Hz, 1H), 7.36 (s, 1H), 6.43 (d, J = 5.5 Hz, 1H), 5.46 (s, 1H), 3.68 (s, 3H), 2.47 (s, 3H), 1.65 (d, J = 6.8 Hz, 3H).<br><br>LCMS (M+1): 439.1 |
| **83** | 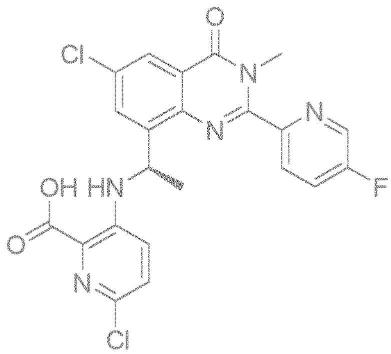 | $^1$HNMR (400 MHz, Chloroform-d) $\delta$ 8.58 (s, 1H), 8.22 (s, 1H), 8.01 (dd, J = 8.7, 4.4 Hz, 1H) 7.70 - 7.56 (m, 2H)7.11 (d, J = 9.0 Hz, 1H), 6.81 (d, J = 8.9 Hz, 1H), 5.65 (q, J = 6.7 Hz, 1H) 3.69 (s, 3H), 1.26 (s, 3H)<br><br>LCMS (M+1): 488.06 |
| **84** | 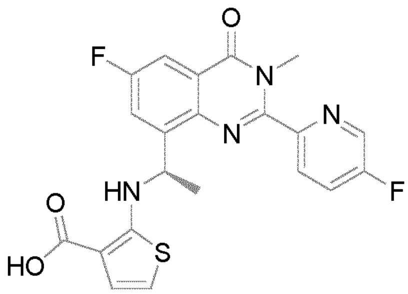 | $^1$HNMR (400 MHz, Chloroform-d) $\delta$ 8.58 (d, J = 2.8 Hz, 1H), 8.03 (dd, J = 8.6, 4.4 Hz, 1H), 7.87 (dd, J = 8.2, 3.0 Hz, 1H), 7.68 (td, J = 8.3, 2.9 Hz, 1H), 7.49 (dd, J = 8.9, 3.0 Hz, 1H), 7.27 (s, 1H), 6.35 (d, J = 5.5 Hz, 1H), 5.48 (s, 1H), 3.69 (s, 3H), 1.66 (s, 3H)<br><br>LCMS (M+1): 433.26 |
| **85** | 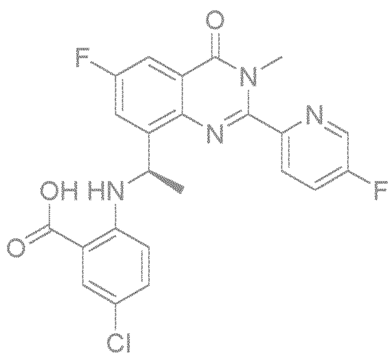 | $^1$H NMR (400 MHz, DMSO-d6) $\delta$ *13.16* (s, 1H), 8.76 (d, J = 2.8 Hz, 1H), 8.43 (d, J = 6.7 Hz, 1H), 8.12 (dd, J = 8.7, 4.6 Hz, 1H), 8.02 (td, J = 8.7, 2.9 Hz, 1H), 7.77 (dd, J = 8.3, 3.0 Hz, 1H), 7.73 (d, J = 2.8 Hz, 1H), 7.60 (dd, J = 9.4, 3.0 Hz, 1H), 7.21 (dd, J = 9.0, 2.7 Hz, 1H), 6.42 (d, J = 9.1 Hz, 1H), 5.46 (t, J = 6.4 Hz, 1H), 3.51 (s, 3H), 1.59 (d, J = 6.7 Hz, 3H).<br><br>LCMS (M+1): 471 |
| **86** | 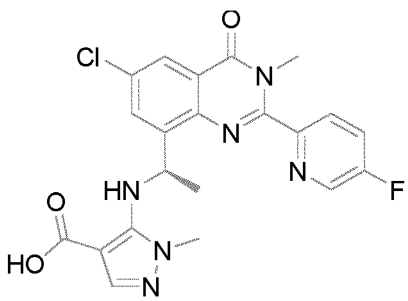 | LCMS (M+1): 457 |

(continued)

| Compound No. | Structure | HNMR |
|---|---|---|
| **87** | 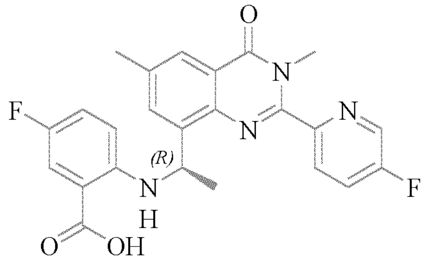 | ¹H NMR (400 MHz, DMSO-$d_6$) $\delta$ 13.01 (s, 1H), 8.75 (d, J = 2.8 Hz, 1H), 8.25 (s, 1H), 8.11 (dd, J = 8.7, 4.6 Hz, 1H), 8.02 (td, J = 8.8, 2.9 Hz, 1H), 7.90 (d, J = 2.0 Hz, 1H), 7.59 (d, J = 2.0 Hz, 1H), 7.49 (dd, J = 9.8, 3.2 Hz, 1H), 7.10 (td, J = 8.7, 3.2 Hz, 1H), 6.41 (dd, J = 9.3, 4.5 Hz, 1H), 5.45 (d, J = 7.0 Hz, 1H), 3.50 (s, 3H), 2.40 (s, 3H), 1.55 (d, J = 6.6 Hz, 3H). LCMS (M+1): 451 |
| **88** | 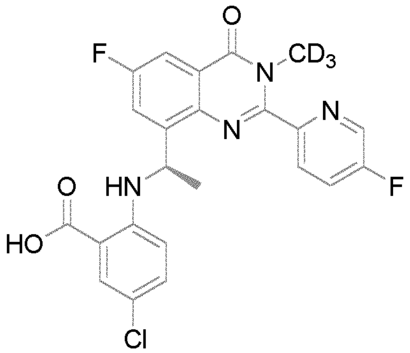 | ¹H NMR (400 MHz, DMSO-$d_6$) $\delta$ 13.16 (s, 1H), 8.76 (d, J = 2.8 Hz, 1H), 8.42 (d, J = 6.7 Hz, 1H), 8.12 (dd, J = 8.8, 4.5 Hz, 1H), 8.03 (td, J = 8.7, 2.9 Hz, 1H), 7.77 (dd, J = 8.3, 3.0 Hz, 1H), 7.73 (d, J = 2.6 Hz, 1H), 7.60 (dd, J = 9.4, 3.0 Hz, 1H), 7.21 (dd, J = 9.0, 2.7 Hz, 1H), 6.42 (d, J = 9.1 Hz, 1H), 5.46 (t, J = 6.5 Hz, 1H), 1.59 (d, J = 6.7 Hz, 3H). LCMS (M+1): 474 |
| **89** | 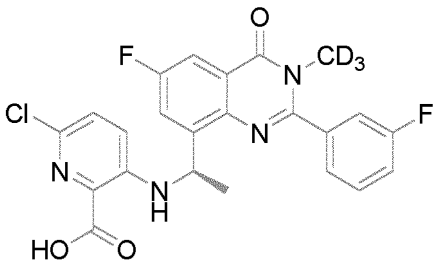 | ¹HNMR (400 MHz, Chloroform-d) $\delta$ 8.25 (s, 1H), 7.86 (s, 1H), 7.74 (d, J = 2.2 Hz, 1H), 7.54 d, J = 2.0 Hz, 1H), 7.45 - 7.30(m,3H), 7.28 (d, J = 9.0 Hz, 1H), 5.35 (q, J = 6.7 Hz, 1H), 1.26 (s, 3H) LCMS (M+1): 474 |
| **90** | 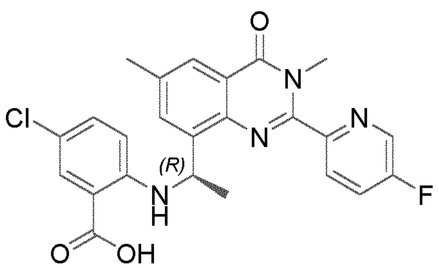 | ¹H NMR (400 MHz, DMSO-$d_6$) $\delta$ 13.12 (s, 1H), 8.75 (d, J = 2.9 Hz, 1H), 8.43 (d, J = 6.8 Hz, 1H), 8.11 (dd, J = 8.7, 4.6 Hz, 1H), 8.01 (td, J = 8.7, 2.9 Hz, 1H), 7.90 (dd, J = 2.1, 1.0 Hz, 1H), 7.71 (d, J = 2.7 Hz, 1H), 7.59 (d, J = 2.1 Hz, 1H), 7.21 (dd, J = 9.0, 2.7 Hz, 1H), 6.45 (d, J = 9.1 Hz, 1H), 5.45 (p, J = 6.7 Hz, 1H), 3.50 (s, 3H), 2.40 (s, 3H), 1.56 (d, J = 6.6 Hz, 3H). LCMS (M+1): 467 |
| **91** | 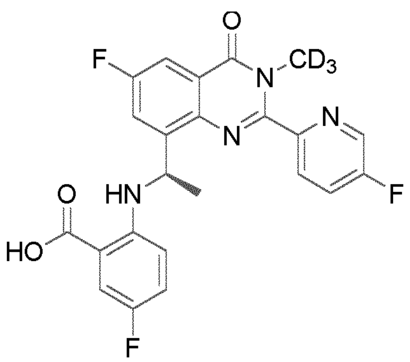 | ¹H NMR (400 MHz, DMSO-$d_6$) $\delta$ 8.76 (d, J = 2.8 Hz, 1H), 8.68 (s, 1H), 8.14 (dd, J = 8.7, 4.6 Hz, 1H), 8.03 (td, J = 8.8, 2.9 Hz, 1H), 7.75 (dd, J = 8.3, 3.0 Hz, 1H), 7.54 (ddd, J = 18.9, 9.6, 3.1 Hz, 2H), 6.99 (td, J = 8.6, 3.2 Hz, 1H), 6.31 (dd, J = 9.2, 4.5 Hz, 1H), 5.43 (q, J = 6.7 Hz, 1H), 1.55 (d, J = 6.6 Hz, 3H). LCMS (M+1): 458 |

(continued)

| Compound No. | Structure | HNMR |
|---|---|---|
| **92** | 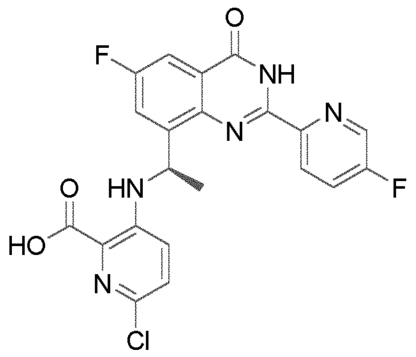 | $^1$H NMR (400 MHz, DMSO-$d_6$) $\delta$ 13.01 (s, 1H), 12.27 (s, 1H), 8.78 (d, J = 2.8 Hz, 1H), 8.67 (dd, J = 8.9, 4.6 Hz, 1H), 8.56 (d, J = 7.2 Hz, 1H), 8.04 (td, J = 8.7, 2.9 Hz, 1H), 7.73 (ddd, J = 21.1, 8.8, 3.1 Hz, 2H), 7.31 (d, J = 8.9 Hz, 1H), 7.09 (d, J = 9.0 Hz, 1H), 5.65 (t, J = 6.9 Hz, 1H), 1.69 (d, J = 6.7 Hz, 3H).<br>LCMS (M+1): 441 |
| **93** | 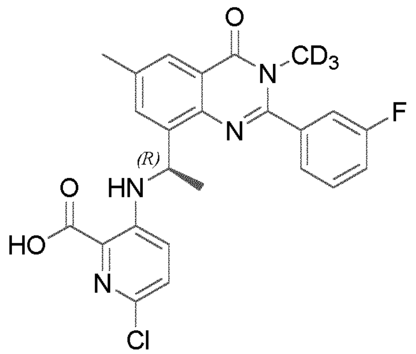 | $^1$H NMR (400 MHz, DMSO-$d_6$) $\delta$ 9.48 (d, J = 58.8 Hz, 1H), 7.86 (s, 1H), 7.67 (d, J = 9.8 Hz, 1H), 7.61 (d, J = 3.8 Hz, 2H), 7.51 (s, 1H), 7.43 (d, J = 8.8 Hz, 1H), 7.18 (s, 1H), 6.87 (s, 1H), 5.41 (s, 1H), 2.36 (d, J = 16.4 Hz, 3H), 1.48 (d, J = 54.4 Hz, 3H).<br>LCMS (M+1): 470 |
| **94** | 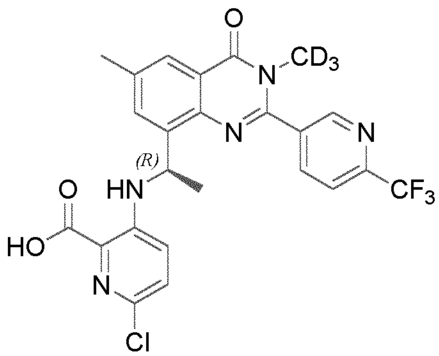 | $^1$H NMR (400 MHz, Chloroform-d) $\delta$ 9.04 (d, J = 2.2 Hz, 1H), 8.31 (d, J = 6.8 Hz, 1H), 8.20 (dd, J = 8.2, 2.2 Hz, 1H), 8.06 (dd, J = 2.2, 1.0 Hz, 1H), 7.92 (d, J = 8.2 Hz, 1H), 7.56 (d, J = 2.0 Hz, 1H), 7.09 (d, J = 8.8 Hz, 1H), 6.86 (d, J = 8.8 Hz, 1H), 5.49 (t, J = 6.6 Hz, 1H), 2.46 (s, 3H), 1.64 (d, J = 6.8 Hz, 3H).<br>LCMS (M+1): 521 |
| **95** | 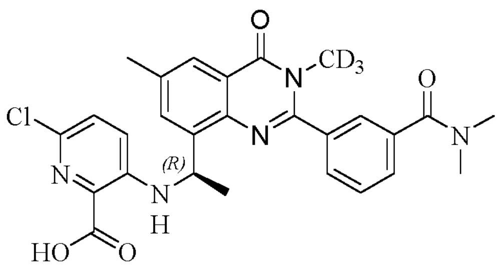 | $^1$H NMR (400 MHz, DMSO-$d_6$) $\delta$ 9.56 (d, J = 7.1 Hz, 1H), 7.86 (d, J = 26.6 Hz, 3H), 7.61 (td, J = 15.3, 7.7 Hz, 3H), 7.36 (d, J = 9.0 Hz, 1H), 7.06 (d, J = 8.9 Hz, 1H), 5.47 (s, 1H), 2.99 (d, J = 9.6 Hz, 6H), 2.39 (s, 3H), 1.56 (d, J = 6.5 Hz, 3H).<br>LCMS (M+1): 524.2 |
| **96** | 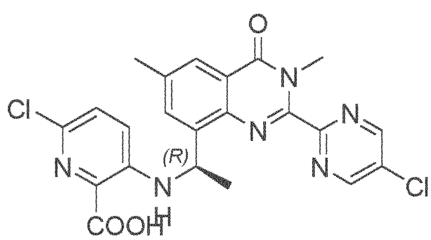 | $^1$H NMR (400 MHz, DMSO-$d_6$) $\delta$ 13.00 (s, 1H), 9.54 (s, 1H), 9.23 (s, 3H), 7.91 (s, 1H), 7.59 (s, 1H), 7.24 (d, J = 48.7 Hz, 1H), 6.87 (d, J = 41.9 Hz, 1H), 5.40 (s, 1H), 3.68 (s, 3H), 2.40 (s, 3H), 1.23 (s, 3H).<br>LCMS (M+1): 485 |

(continued)

| Compound No. | Structure | HNMR |
|---|---|---|
| **97** | 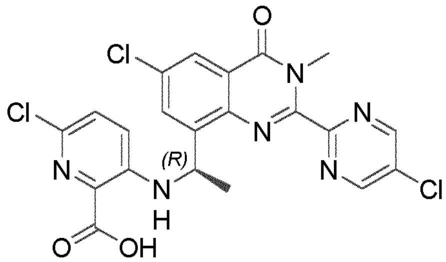 | $^1$H NMR (400 MHz, DMSO-$d_6$) $\delta$ 9.50 (d, J = 58.8 Hz, 1H), 7.86 (s, 1H), 7.67 (d, J = 9.8 Hz, 1H), 7.61 (d, J = 3.8 Hz, 2H), 7.51 (s, 1H), 6.87 (s, 1H), 5.41 (s, 1H), 2.36 (d, J = 16.4 Hz, 3H), 1.48 (d, J = 54.4 Hz, 3H).<br><br>LCMS (M+1): 505 |
| **98** | 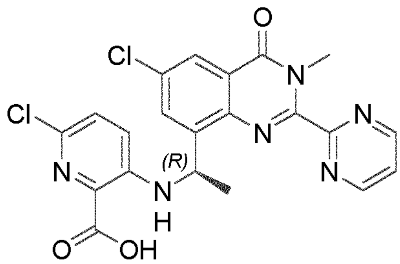 | $^1$H NMR (400 MHz, DMSO-$d_6$) $\delta$ 9.50 (d, J = 58.8 Hz, 1H), 7.86 (s, 1H), 7.69 (d, J = 9.8 Hz, 1H), 7.63 (d, J = 3.8 Hz, 2H), 7.54 (s, 2H), 6.87 (s, 1H), 5.41 (s, 1H), 2.36 (d, J = 16.4 Hz, 3H), 1.48 (d, J = 54.4 Hz, 3H).<br><br>LCMS (M+1): 471 |
| **99** | 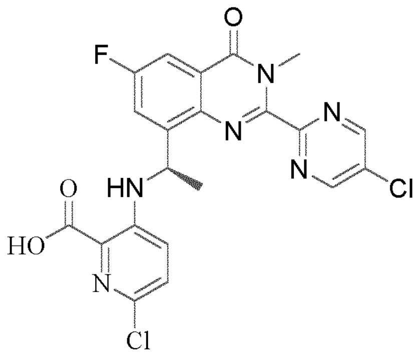 | $^1$H NMR (400 MHz, DMSO-$d_6$) $\delta$ 9.51 (d, J = 58.8 Hz, 1H), 7.79 (s, 1H), 7.78 (d, J = 9.8 Hz, 1H), 7.59 (d, J = 3.8 Hz, 2H), 7.51 (s, 1H), 6.87 (s, 1H), 5.41 (s, 1H), 2.36 (d, J = 16.4 Hz, 3H), 1.48 (d, J = 54.4 Hz, 3H).<br><br>LCMS (M+1): 489 |
| **100** | 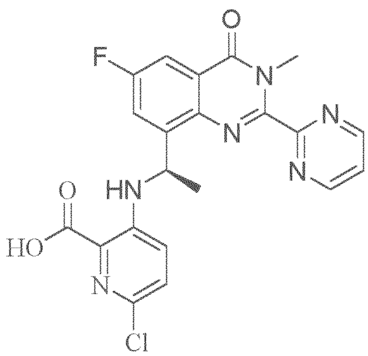 | $^1$H NMR (400 MHz, DMSO-$d_6$) $\delta$ 9.51 (d, J = 58.8 Hz, 1H), 7.78 (s, 1H), 7.76 (d, J = 9.8 Hz, 1H), 7.59 (d, J = 3.8 Hz, 2H), 7.51 (s,2H), 6.86 (s, 1H), 5.41 (s, 1H), 2.36 (d, J = 16.4 Hz, 3H), 1.48 (d, J = 54.4 Hz, 3H).<br><br>LCMS (M+1): 455 |
| | | |
| **102** | 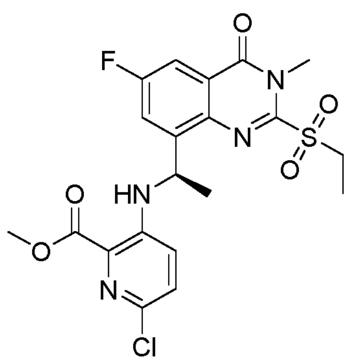 | $^1$H NMR (400 MHz, Chloroform-d) $\delta$ 8.3(brs, 1H), 7.88 (dd, J = 8.1, 2.9 Hz, 1H), 7.44 (dd, J = 8.9, 2.9 Hz, 1H), 7.10 (d, J = 8.9 Hz, 1H), 6.81 (d, J = 8.9 Hz, 1H), 5.51 (p, J = 6.7 Hz, 1H), 3.87 (s, 3H), 3.69 (s, 3H), 3.45(m, 2H), 1.69 (d, J = 6.7 Hz, 3H), 1.22 (m, 3H)<br><br>LCMS (M+1): 482 |

(continued)

| Compound No. | Structure | HNMR |
|---|---|---|
| **103** | 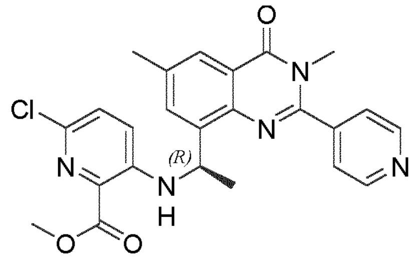 | $^{1}$H NMR (400 MHz, DMSO-$d_6$) $\delta$ 8.81 (d, 2H), 8.31 (d, J = 7.4 Hz, 1H), 7.92 (s, 1H), 7.78 (d, 2H), 7.68 (s, 1H), 7.32 (d, J = 9.0 Hz, 1H), 7.11 (d, J = 9.1 Hz, 1H), 5.48 - 5.37 (m, 1H), 3.82 (s, 3H), 3.39 (s, 3H), 2.42 (s, 3H), 1.59 (d, J = 6.6 Hz, 3H).<br>LCMS (M+1): 464.1 |
| | | |
| **105** | 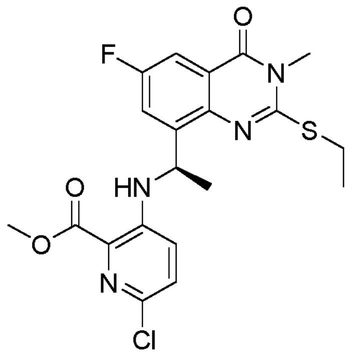 | $^{1}$H NMR (400 MHz, Chloroform-d) $\delta$ 8.3(brs, 1H), 7.88 (dd, J = 8.1, 2.9 Hz, 1H), 7.44 (dd, J = 8.9, 2.9 Hz, 1H), 7.10 (d, J = 8.9 Hz, 1H), 6.81 (d, J = 8.9 Hz, 1H), 5.51 (p, J = 6.7 Hz, 1H), 3.87 (s, 3H), 3.69 (s, 3H), 3.15(m, 2H), 1.69 (d, J = 6.7 Hz, 3H), 1.31 (m, 3H)<br>LCMS (M+1): 451 |
| **106** | 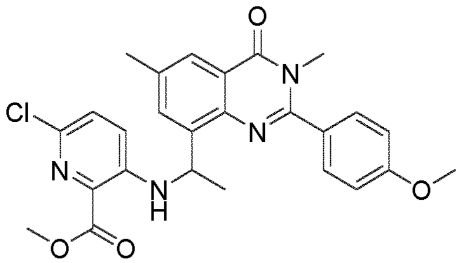 | $^{1}$HNMR (400 MHz, Chloroform-d) $\delta$ 8.02 (dd, J = 2.1, 1.0 Hz, 1H), 7.59 (d, J = 2.0 Hz, 1H), 7.58 (d, J = 2.1 Hz, 1H), 7.50 (d, J = 2.0 Hz, 1H), 7.08 - 7.00 (m, 3H), 6.91 (d, J = 9.0 Hz, 1H), 5.65 (q, J = 6.7 Hz, 1H), 3.98 (s, 3H), 3.91 (s, 3H), 3.58 (s, 3H), 2.42 (s, 3H), 1.64 (d, J = 6.6 Hz, 3H).<br>LCMS (M+1): 493.16 |
| **107** | 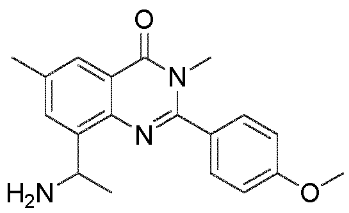 | $^{1}$HNMR (400 MHz, Chloroform-d) $\delta$ 8.02 (dd, J = 2.0, 1.0 Hz, 1H), 7.58 - 7.50 (m, 3H), 7.04 (d, J = 2.1 Hz, 1H), 7.02 (d, J = 2.1 Hz, 1H), 4.81 (q, J = 6.8 Hz, 1H), 3.89 (s, 3H), 3.55 (s, 3H), 2.48 (s, 3H), 1.60 (d, J = 6.8 Hz, 3H).<br>LCMS (M+1): 324.16 |
| **108** | 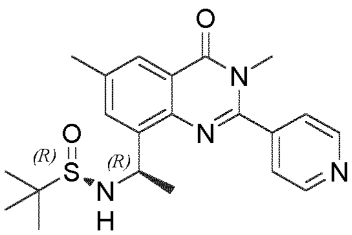 | $^{1}$H NMR (400 MHz, DMSO-$d_6$) $\delta$ 8.80 (d, 2H), 7.91 (s, 1H), 7.79 (s, 1H), 7.73 (d, 2H), 5.92 (d, J = 8.5 Hz, 1H), 5.21 - 5.08 (m, 1H), 3.38 (s, 3H), 2.48 (s, 3H), 1.41 (d, J = 6.8 Hz, 3H), 1.05 (s, 9H).<br>LCMS (M+1): 399.2 |

(continued)

| Compound No. | Structure | HNMR |
|---|---|---|
| **109** | 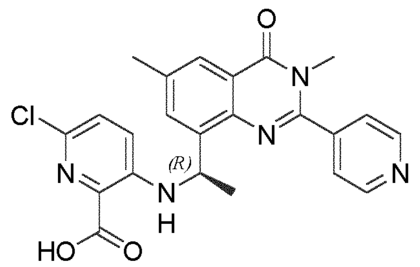 | $^1$H NMR (400 MHz, DMSO-$d_6$) $\delta$ 13.02 (s, 1H), 9.07 (d, J = 7.1 Hz, 1H), 8.81 (d, J = 5.1 Hz, 2H), 7.91 (s, 1H), 7.88 (s, 1H), 7.79 (d, J = 5.0 Hz, 2H), 7.24 (d, J = 8.9 Hz, 1H), 6.93 (d, J = 9.0 Hz, 1H), 5.49 - 5.36 (m, 1H), 3.40 (s, 3H), 2.41 (s, 3H), 1.54 (d, J = 6.6 Hz, 3H). LCMS (M+1): 450.1 |
| **110** | 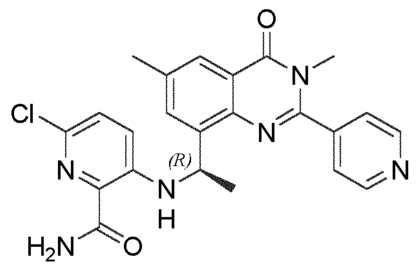 | $^1$H NMR (400 MHz, DMSO-$d_6$) $\delta$ 9.07 (d, J = 7.1 Hz, 1H), 8.81 (d, J = 5.1 Hz, 2H), 7.91 (s, 1H), 7.88 (s, 1H), 7.79 (d, J = 5.0 Hz, 2H), 7.60 (s, 2H), 7.24 (d, J = 8.9 Hz, 1H), 6.93 (d, J = 9.0 Hz, 1H), 5.49 - 5.36 (m, 1H), 3.40 (s, 3H), 2.41 (s, 3H), 1.54 (d, J = 6.6 Hz, 3H). LCMS (M+1): 449.1 |
| **111** | 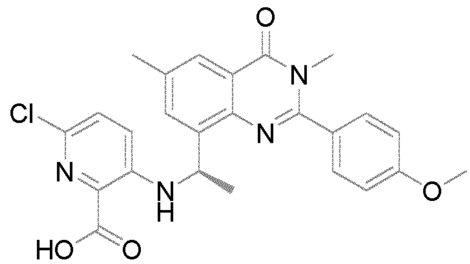 | $^1$HNMR (400 MHz, Chloroform-d) $\delta$ 8.23 (s, 1H), 8.02 (dd, J = 2.1, 1.0 Hz, 1H), 7.59 (d, J = 2.0 Hz, 1H), 7.57 (d, J = 2.1 Hz, 1H), 7.51 (d, J = 2.0 Hz, 1H), 7.09 - 7.03 (m, 3H), 6.96 (d, J = 9.0 Hz, 1H), 5.65 (q, J = 6.7 Hz, 1H), 3.91 (s, 3H), 3.58 (s, 3H), 2.43 (s, 3H), 1.64 (d, J = 6.7 Hz, 3H). LCMS (M+1): 479.14 |
| **112** | 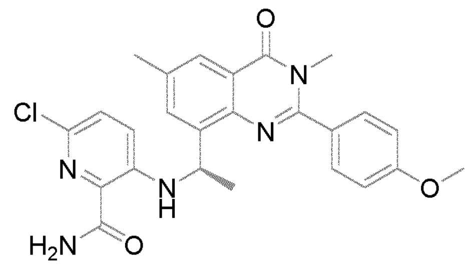 | $^1$H NMR (400 MHz, Chloroform-d) $\delta$ 8.81 (s, 1H), 8.00 (s, 1H), 7.78 - 7.67 (m, 1H), 7.59 (d, J = 8.2 Hz, 2H), 7.54 (d, J = 1.9 Hz, 1H), 7.05 (d, J = 7.9 Hz, 2H), 6.96 (d, J = 8.7 Hz, 1H), 6.81 (d, J = 8.9 Hz, 1H), 5.57 (s, 1H), 5.42 (s, 1H), 3.90 (s, 3H), 3.58 (s, 3H), 2.42 (s, 3H), 1.25 (s, 3H). LCMS (M+1): 478.16 |
| **113** | 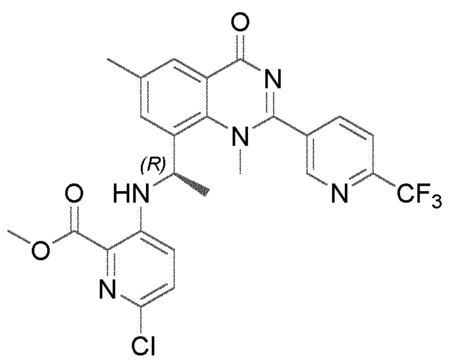 | LCMS (M+1): 532 |

(continued)

| Compound No. | Structure | HNMR |
|---|---|---|
| **114** | 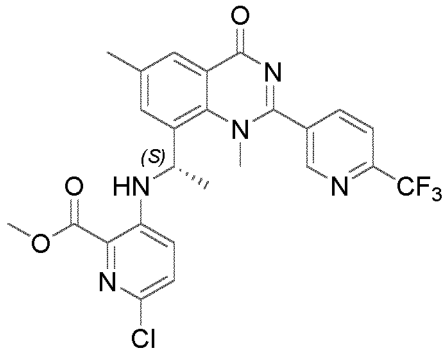 | LCMS (M+1): 532 |
| **115** | 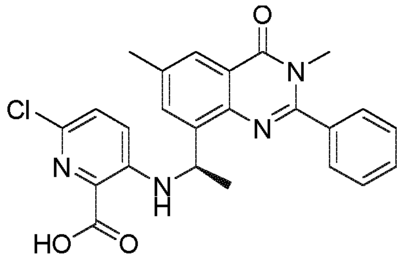 | $^1$H NMR (400 MHz, Chloroform-d) $\delta$ 8.55 (d, J = 2.9 Hz, 1H), 8.23 (d, J = 2.4 Hz, 1H), 8.07 (dd, J = 8.7, 4.4 Hz, 1H), 7.68 - 7.61 (m, 2H), 5.13 (p, J = 6.7 Hz, 1H), 4.70 (d, J = 6.6 Hz, 1H), 3.66 (s, 3H), 1.60 (d, J = 6.8 Hz, 3H), 1.16 (s, 9H).<br>LCMS (M+1): 449 |
| **116** | 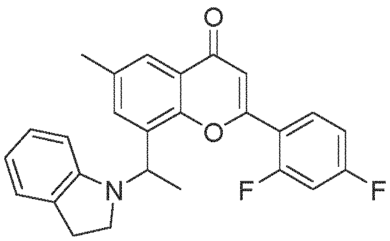 | $^1$H NMR (400 MHz, DMSO-$d_6$) $\delta$ 8.02 (td, J = 8.9, 6.5 Hz, 1H), 7.83-7.77 (m, 1H), 7.74 (d, J = 2.2 Hz, 1H), 7.54 (ddd, J = 11.9, 9.2, 2.6 Hz, 1H), 7.15 (td, J = 8.5, 2.6 Hz, 1H), 7.04-6.96 (m, 1H), 6.93-6.84 (m, 1H), 6.77 (d, J = 0.9 Hz, 1H), 6.59-6.47 (m, 1H), 6.39 (d, J = 7.8 Hz, 1H), 5.37 (q, J = 6.9 Hz, 1H), 3.44 (t, J = 9.5 Hz, 2H), 2.91-2.77 (m, 2H), 2.44 (s, 3H), 1.54 (d, J = 6.9 Hz, 3H).<br>LCMS (M+1): 418 |
| **117** | 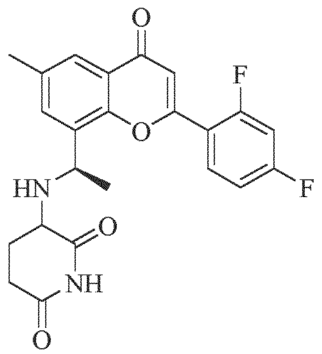 | $^1$H NMR (400 MHz, DMSO-$d_6$) $\delta$ 10.67 (s, 1H), 8.08 (tdd, J = 9.0, 6.3, 3.1 Hz, 1H), 7.82(s, 1H), 7.74 (s, 1H), 7.57 (ddd, J = 11.6, 8.8, 2.5 Hz, 1H), 7.38 (tt, J = 8.4, 3.1 Hz, 1H), 6.75 (s, 1H), 4.87 - 4.38 (m, 1H), 3.21 (m, 1H), 2.47 - 2.38 (m, 1H), 2.06 (m, 1H), 1.87 (m, 1H), 1.71 (m, 1H), 1.42 (m, 1H), 2.44 (s, 3H), 1.54 (d, J = 6.9 Hz, 3H). |

In the following, the beneficial effects of the present invention were demonstrated by specific experimental examples.

**Experimental example 1: PI3Kα*[WT]*/ *[E542K]*/*[E545K]*/*[H1047R]*/PI3Kβ/γ/δ enzyme activity assay**

PI3Kα*[WT]*, PI3Kα*[E542K],* PI3Kα*[E545K],* PI3Kα*[H1047R],* PI3Kβ, PI3Kγ and PI3Kδ kinase reaction was performed in 384-well plate (PerkinElmer, #6008280), with a reaction system of 4 μL. PI3Kα*[WT]* enzyme solution (Promega, # V1721) or PI3Kα*[E542K]* enzyme solution (CarnaBio, # 11-413-20N) or PI3Kα*[E545K]* enzyme solution (CarnaBio, # 11-414-20N) or PI3Kα[H1047R] enzyme solution (Promega, # V1741) or PI3Kβ enzyme solution (Promega, #V1751) or PI3Kγ enzyme solution (ThermoFisher, #PV4786) or PI3Kδ enzyme solution (ThermoFisher, #PV6451) were

added to 384-well plate, and then gradiently diluted compounds were added (starting at a final concentration of 10 μM, 3-fold dilution, 10 doses), followed by adding PIP2: 3PS (Promega, # V1701) and ATP solution (Promega, # V915B) to the plate. After incubation at 25 °C for 1 h, ADP-Glo reagent buffer (Promega, # V9102) was added to each well. The plate was sealed and incubated at 25 °C for 40 min, and then 10 μL of ADP-Glo detection buffer (Promega, #V9102) was added to each well. After incubating at 25 °C for 40 min, the chemiluminescence value was read using an instrument.

The experimental results are shown in the ADP Glo IC50 column in Table 2.

**Experimental example 2: Experiment of inhibitory effects on the proliferation of MCF-10A cells**

MCF-10A cells (Cobioer Bio. #CBP60419MCF) were diluted with complete medium (Cobioer Bio. #CBP60419), seeded in 96-well plate (Corning #3599) at 500 cells/well, and then cultured for 24 h. Then, gradiently diluted compounds were added (starting at a final concentration of 10 μM, 3-fold dilution, 9 dose concentrations), and the final volume was adjusted to 100 μL/well. The plate was incubated at 37 °C under 5% $CO_2$ for 6 days. After the cultivation was completed, CCK8 reagent (Signalway Antibody Bio. #CP002) was added at 10 μL/well, and then the plate was further incubated at 37 °C under 5% $CO_2$ for 2-3 hours. The $OD_{450nm}$ value was obtained by a microplate reader.

The results are shown in the MCF-10A $IC_{50}$ column in Table 2.

**Experimental example 3: Experiment of inhibitory effects on the proliferation of T47D cells**

T47D cells (ATCC. #HTB-133) were diluted with RPMI 1640 (Gibco #22400-089) containing 10% FBS (Hyclone #SH30084.03), seeded in 96-well plate (Biosharp # BS-MP-96W) at 1000 cells/well, and then cultured for 24 h. Then, gradiently diluted compounds were added (starting at a final concentration of 10 μM, 3-fold dilution, 9 dose concentrations), and the final volume was adjusted to 150 μL/well. The plate was incubated at 37 °C under 5% $CO_2$ for 7 days. After the cultivation was completed, CellTiter-Glo reagent (Promega #G7573) was added at 75 μL/well, and then the plate was further incubated at 25 °C for 10 min. After that, the chemiluminescence value was read using an instrument.

The results are shown in the T47D $IC_{50}$ column in Table 2.

Table 2. The activities of compounds according to the present invention.

| Compound No. | ADP-Glo $IC_{50}$ | MCF-10A $IC_{50}$ | T47D $IC_{50}$ |
|---|---|---|---|
| **1** | | | |
| **4** | XX | | |
| **5** | XX | | |
| **6** | X | | X |
| **42** | XX | | |
| **37** | X | | |
| **38** | X | | |
| **27** | XXX | XXX | XXX |
| **43** | X | XXX | X |
| **44** | X | XXX | X |
| **45** | X | XXX | X |
| **46** | X | XXX | X |
| **47** | | XXX | X |
| **28** | XX | XXX | X |
| **48** | XX | XXX | XX |
| **49** | | XXX | XX |
| **50** | | XXX | XX |
| **41** | X | XXX | XX |
| **51** | | XXX | XXX |
| **53** | X | XX | X |

(continued)

| Compound No. | ADP-Glo $IC_{50}$ | MCF-10A $IC_{50}$ | T47D $IC_{50}$ |
|---|---|---|---|
| **55** | XX | XXX | XXX |
| **56** | | XXX | XXX |
| **57** | X | XXX | XXX |
| **58** | X | XXX | X |
| **59** | X | XXX | X |
| **60** | X | XXX | X |
| **61** | X | XXX | X |
| **62** | X | XXX | XX |
| **63** | | XXX | XX |
| **64** | | XXX | XX |
| **65** | | XXX | X |
| **66** | X | XXX | X |
| **67** | | XXX | XX |
| **68** | | XXX | XX |
| **69** | X | XXX | X |
| **70** | X | XXX | XX |
| **71** | XX | XXX | XXX |
| **72** | X | XXX | XX |
| **73** | X | XXX | XX |
| **74** | X | XXX | X |
| **75** | | XXX | XX |
| **76** | | XXX | XX |
| **77** | X | XXX | X |
| **78** | | XXX | XX |
| **79** | | XXX | XX |
| **80** | X | XXX | X |
| **81** | | XXX | XXX |
| **82** | X | XXX | XXX |
| **83** | | XXX | X |
| **84** | | XXX | XXX |
| **85** | | XXX | X |
| **86** | | XXX | XXX |
| **87** | | XXX | X |
| **88** | | XXX | XXX |
| **89** | | XXX | XX |
| **90** | | XXX | X |
| **91** | | XXX | X |
| **92** | | XXX | X |
| **93** | | XXX | X |

(continued)

| Compound No. | ADP-Glo $IC_{50}$ | MCF-10A $IC_{50}$ | T47D $IC_{50}$ |
|---|---|---|---|
| **94** | | XXX | X |
| **96** | X | | |
| **97** | | XX | X |
| **98** | | XXX | X |
| **99** | X | XXX | X |
| **100** | X | XXX | X |
| **102** | XX | XX | X |
| **103** | X | XXX | X |
| **105** | | XXX | XX |
| **106** | | XXX | X |
| **107** | | XXX | X |
| **108** | | XXX | XXX |
| **109** | | XXX | X |
| **110** | | XXX | X |
| **111** | | XXX | |
| **112** | | XX | |
| **113** | | XXX | |
| **114** | | XXX | |
| **115** | | XXX | XXX |
| **116** | | XXX | XXX |
| **117** | XXX | XXX | XXX |

Wherein, XXX represents $IC_{50}$>5 μM, XX represents 1 μM<$IC_{50}$<5 μM, and X represents $IC_{50}$<1 μM.

**Experimental example 4: The pharmacokinetics of the compound according to the present invention in rats**

**1. Experimental methods**

9 healthy adult male SD rats (three for each administration route of each compound) were fasted overnight (>12 h) before gavage, with free acces to water; 4 hours after administration, rats could take food, and were administered via tail vein injection and gastric lavage, respectively. At different time points, intravenous blood sample was collected and anti-coagulated, wherein the anticoagulant was EDTA-K2 (the sample was placed in an ice bath after blood collection). The plasma was separated by centrifugation at 4 °C and 6000 g for 5 min, and stored at -70 °C for testing. The plasma concentration of compounds was determined by LC-MS/MS method.

The plasma concentration of compound at all time points was used to calculate the main pharmacokinetic parameters with Winnolin 8.3 non-compartmental model.

The area under the concentration-time curve $AUC_{all}$ value: calculated using the trapezoidal method; $AUC_{inf}$=$AUC_{all}$+$C_t$/ke, $C_t$ is the plasma concentration at the last measurable time point, and ke is the elimination rate constant;

The elimination half life $t_{1/2}$=0.693/ke;

The clearance rate CL=D/$AUC_{inf}$(D is the administration dosage);

The volume of distribution at steady state $V_{SS}$=CL x MRT, The mean retention time MRT =AUMC/AUC;

$$\text{The absolute bioavailability } F=(AUCi.g. \times Di.v.)/(AUCi.v. \times Di.g.)\times 100\%$$

## 2. Experimental results

Table 3. The pharmacokinetic results of the compound according to the present invention in rats.

| | | Compound **65** | Compound **66** | Compound **74** | Compound **115** |
|---|---|---|---|---|---|
| iv (1 mpk) | $AUC_{all}$ (ng*h/mL) | 1003 | 1394 | 1006 | 978 |
| | $t_{1/2}$ (h) | 2.95 | 3.44 | 2.73 | 3.79 |
| | CL (mL/min/kg) | 16.3 | 10.9 | 16.6 | 17.5 |
| | Vss (L/kg) | 1.71 | 2.20 | 1.33 | 2.14 |
| ig (3 mpk) | $AUC_{all}$ (ng*h/mL) | 3429 | 4045 | 1329 | 2356 |
| | $t_{1/2}$ (h) | 1.34 | 18 | 2.40 | 3.83 |
| | F (%) | 79.6 | 89.0 | 44.8 | 80.3 |

Note: "iv (1 mpk)" in the table refers to tail vein injection, at a dosage of 1 mg/kg; ig (3 mpk) refers to intragastric administration, at a dosage of 3 mg/kg; ig (50 mpk) refers to intragastric administration, at a dosage of 50 mg/kg.

The above experimental results indicated that the compound of the present invention had good pharmacokinetics.

## Experimental example 5: The pharmacokinetics of the compound according to the present invention in beagle dogs

## 1. Experimental methods

9 healthy adult male beagle dogs (three for each administration route of each compound) were fasted overnight (>12 h) before gavage, with free acces to water; 4 hours after administration, rats could take food, and were administered via tail vein injection and gastric lavage, respectively. At different time points, forelimb venous blood sample was collected and anti-coagulated, wherein the anticoagulant was EDTA-K2 (the sample was placed in an ice bath after blood collection). The plasma was separated by centrifugation at 3200 g for 10 min, and stored at -70 °C for testing. The plasma concentration of compounds was determined by LC-MS/MS method.

The plasma concentration of compound at all time points was used to calculate the main pharmacokinetic parameters with Winnolin 8.3 non-compartmental model.

The area under the concentration-time curve $AUC_{all}$ value: calculated using the trapezoidal method; $AUC_{inf}=AUC_{all}+C_t/ke$, $C_t$ is the plasma concentration at the last measurable time point, and ke is the elimination rate constant;

The elimination half life $t_{1/2}=0.693/ke$;

The clearance rate $CL=D/AUC_{inf}$ (D is the administration dosage);

The volume of distribution at steady state $V_{SS}=CL \times MRT$. The mean retention time MRT = AUMC/AUC;

$$\text{The absolute bioavailability } F=(AUCi.g. \times Di.v.)/(AUCi.v. \times Di.g.)\times 100\%$$

## 2. Experimental results

Table 4. The pharmacokinetic results of the compound according to the present invention in beagle dogs.

| | | Compound **65** | Compound **66** | Compound **74** |
|---|---|---|---|---|
| iv (1 mpk) | $AUC_{all}$ (ng*h/mL) | 954 | 2213 | 2389 |
| | $t_{1/2}$ (h) | 2.10 | 3.34 | 5.56 |
| | CL (mL/min/kg) | 17.4 | 7.77 | 6.90 |
| | Vss (L/kg) | 0.828 | 0.879 | 0.663 |

(continued)

| | | Compound **65** | Compound **66** | Compound **74** |
|---|---|---|---|---|
| ig (3 mpk) | $AUC_{all}$ (ng*h/mL) | 760 | 4911 | 3713 |
| | $t_{1/2}$ (h) | 2.13 | 3.81 | 4.11 |
| | F (%) | 27.1 | 74.5 | 52.4 |
| ig (10 mpk) | $AUC_{all}$ (ng*h/mL) | 2372 | 11019 | 13662 |
| | $t_{1/2}$ (h) | 4.19 | 7.70 | 12.7 |
| | F (%) | 24.9 | 49.1 | 62.8 |

Note: "iv (1 mpk)" in the table refers to tail vein injection, at a dosage of 1 mg/kg; ig (3 mpk) refers to intragastric administration, at a dosage of 3 mg/kg; ig (10 mpk) refers to intragastric administration, at a dosage of 10 mg/kg.
The above experimental results indicated that the compound of the present invention had good pharmacokinetics.

**Experimental example 6: Pharmacodynamic evaluation of the compound according to the present invention in NOD SCID mouse model with subcutaneous xenograft tumor of MDA-MB-453 cells:**

**1. Experimental methods**

(1) Cell culture and inoculation:

MDA-MB-453 cell lines were cultured in Leibovitz's L-15 medium + 10% fetal bovine serum + 1% bispecific antibody at 37 ° C under 5% $CO_2$, and passaged once a week. When the cell saturation was between 80% and 90%, and the required quantity was reached, the cells were collected, counted, and inoculated.
When the number of cells in the logarithmic growth phase reached the required amount for the experiment, the cells were collected, and centrifuged at 1000 rpm for 5 minutes to remove the supernatant. The cells were resuspended in the medium, and counted using a cell counter. Based on the counting results, the original solution was diluted to obtain cell suspension at a concentration of $1*10^8$ cells/mL. The cell viability was 94.55%, P14 generation. The diluted cell suspension and matrix gel were diluted in a 1:1 ratio, and after mixing, placed on ice. a 1 mL sterile syringe was used to transfer the suspension. 0.2 mL of cell suspension was subcutaneously inoculated into the right axilla of each mouse. Each mouse was inoculated with $1*10^7$ MDA-MB-453 cells.

(2) Grouping method:

After inoculation, the tumor growth state was observed daily. When the average volume reached about 157.14 $mm^3$, mice were randomly divided into groups based on tumor size and body weight for administration. The same dose of solvent was given to the control group. The day on which tumor cells were inoculated was defined as Day 0.

(3) Experimental observation and data measurement:

The health and mortality status of animals was daily monitored, including tumor growth, activity ability, diet, weight, eyes, hair, and other abnormal behaviors. Body weight was measured daily, while tumor volume was measured twice a week. The tumor volume was measured using a vernier caliper, and the formula is $TV = 0.5\, a \times b^2$, wherein a is the long diameter of the tumor, and b is the short diameter of the tumor. After reaching the experimental endpoint, plasma samples were collected from each group at pre-determined time points for determining the plasma concentration. After euthanizing mice, tumors were collected, photographed, weighed, and frozen.

(4) Anti-tumor pharmacodynamic indexes

Relative tumor proliferation rate, T/C (%), refers to the percentage ratio of tumor volume or weight between the treatment group and the control group at a certain time point. The calculation formula is: $T/C\% = TRTV/CRTV \times 100\%$ wherein, TRTV: the average relative tumor volume (RTV) of the treatment group; CRTV: the average relative tumor volume of the control group; (RTV): $RTV = V_t/V_0$, $V_0$ is the tumor volume of the animal at the time of grouping, and $V_t$ is the tumor volume of the animal after treatment.

Alternatively, T/C%=TTW/CTW× 100%; wherein, TTW: the average tumor weight of the treatment group at the end of experiment; CTV: the average tumor weight of the control group at the end of experiment.
Relative tumor inhibition rate, TGI (%), the calculation formula is: [TGI%=(1-T/C) × 100%; wherein, T and C are the relative tumor volume (RTV) or tumor weight (TW) of the treatment group and the control group at a specific time point, respectively.

**2. Experimental results**

Table 5. Pharmacodynamic evaluation results of the compound according to the present invention in NOD SCID mouse model with subcutaneous xenograft tumor of MDA-MB-453 cells.

| | Compound **66** | | Compound **74** | |
|---|---|---|---|---|
| Route of administration | Oral | | Oral | |
| Dose and frequency | 25 mg/kg, Twice a day | 75 mg/kg, Twice a day | 25 mg/kg, Twice a day | 75 mg/kg, Twice a day |
| TGI% after administrating for 21 days | 71% | 87% | 74% | 85% |

The above experimental results indicated that the compound of the present invention had a good inhibitory effect on breast cancer.
In summary, the compounds prepared in the present invention could be used for the preparation of PI3K selective inhibitors, as well as in the manufacture of medicaments for the prevention and/or treatment of PI3K-related diseases, such as medicaments for the prevention and/or treatment of cancer. The present invention provided a new choice for clinical treatment of cancer and had good application prospects.

## Claims

1. A compound as represented by formula I, or a salt thereof, or a stereoisomer thereof, or a solvate thereof, or a hydrate thereof, or a prodrug thereof, or a deuterated compound thereof:

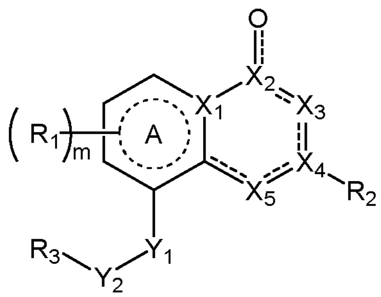

Formula I

wherein,

$R_1$ is a substituent in ring A, which has m substituents; each $R_1$ is independently selected from the group consisting of substituted or unsubstituted $C_1$-$C_8$ alkyl, $C_1$-$C_8$ alkoxy, halogen, cyano, nitro, hydroxy, carboxyl, -$NHR_{15}$, -$NHR_8$, -C(O)$NHR_8$, -C(O)$NHR_{15}$, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl; m is selected from the group consisting of 0, 1, 2, or 3;
$R_{15}$ is selected from the group consisting of H, substituted or unsubstituted $C_1$-$C_8$ alkyl, $C_1$-$C_8$ alkoxy, halogen, cyano, nitro, hydroxy, carboxyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl;

$Y_1$ and $Y_2$ are each independently selected from the group consisting of absence, $CR_4R_5$, and $NR_4$;
$R_4$ and $R_5$ are each independently selected from the group consisting of H and $C_1$-$C_8$ alkyl; alternatively, $R_4$ and $R_5$, together with a carbon, form a ketone group;
$R_3$ is selected from the group consisting of H, (6-10)-membered aryl substituted with n $R_6$, (5-10)-membered heteroaryl substituted with n $R_6$, (4-10)-membered heterocycloalkyl substituted with n $R_6$ or

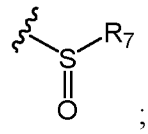
;

each $R_6$ is independently selected from the group consisting of substituted or unsubstituted $C_1$-$C_8$ alkyl, $C_1$-$C_8$ alkoxy, halogen, cyano, nitro, amino, hydroxy, -C(O)O$R_{81}$, -C(O)$R_{81}$,

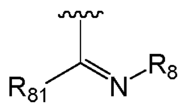
,

cyanomethyl, phosphonyl, sulfonic acid group, sulfonamido, boric acid group, - $NHR_8$ or -C(O)$NHR_8$; alternatively, two $R_6$ linked to the same atom form =O; n is selected from the group consisting of 0, 1, 2, 3, or 4;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

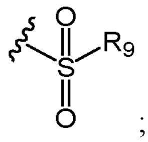
;

$R_7$ and $R_9$ are each independently selected from the group consisting of $C_1$-$C_8$ alkyl and (6-10)-membered aryl;
$X_1$ is selected from C or N; provided that $X_1$ is C, ring A is benzene ring; provided that $X_1$ is N, ring A is dihydropyridine;
$X_2$ is selected from C or N; provided that $X_2$ is C, the bond linking $X_2$ to O is a double bond, and the bond linking $X_2$ to $X_3$ is a single bond; provided that $X_2$ is N, the bond linking $X_2$ to O is a single bond, and the bond linking $X_2$ to $X_3$ is a double bond, with N carrying a positive charge and O carrying a negative charge;
$X_3$ is selected from the group consisting of N, $CR_{10}$ or $NR_{10}$;
$X_4$ is C;
$X_5$ is selected from the group consisting of O, N, $NR_{10}$, or $CR_{10}$;
$R_{10}$ is selected from the group consisting of H as well as substituted or unsubstituted $C_1$-$C_8$ alkyl;
provided that $X_3$ is selected from $CR_{10}$ and $X_5$ is O, the bond linking $X_3$ to $X_4$ is a double bond, the bond linking $X_4$ to $X_5$ is a single bond, and the bond linking $X_5$ to C is a single bond;
provided that $X_3$ is selected from $NR_{10}$ and $X_5$ is N, the bond linking $X_3$ to $X_4$ is a single bond, the bond linking $X_4$ to $X_5$ is a double bond, and the bond linking $X_5$ to C is a single bond;
provided that $X_3$ is selected from $CR_{10}$ and $X_5$ is selected from $CR_{10}$, the bond linking $X_3$ to $X_4$ is a single bond, the bond linking $X_4$ to $X_5$ is a double bond, and the bond linking $X_5$ to C is a single bond; alternatively, the bond linking $X_3$ to $X_4$ is a double bond, the bond linking $X_4$ to $X_5$ is a single bond, and the bond linking $X_5$ to C is a double bond;
provided that $X_3$ is selected from $CR_{10}$ and $X_5$ is N, the bond linking $X_3$ to $X_4$ is a double bond, the bond linking $X_4$ to $X_5$ is a single bond, and the bond linking $X_5$ to C is a double bond;
provided that $X_3$ is N and $X_5$ is selected from $NR_{10}$, the bond linking $X_3$ to $X_4$ is a double bond, the bond linking $X_4$ to $X_5$ is a single bond, and the bond linking $X_5$ to C is a single bond;
$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, -$NR_{11}R_{12}$,

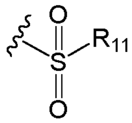

,

and $-SR_{11}$;

$R_{11}$ and $R_{12}$ are each independently selected from the group consisting of H, substituted or unsubstituted $C_1$-$C_8$ alkyl, and amino;

the substituent of the alkyl is selected from the group consisting of deuterium, halogen, hydroxy, cyano, nitro, carboxyl, (4-10)-membered heterocycloalkyl, or $-NR_{13}R_{14}$;

$R_{13}$ and $R_{14}$ are each independently selected from the group consisting of H and $C_1$-$C_8$ alkyl;

the substituent of the cycloalkyl, aryl, heteroaryl or heterocycloalkyl is selected from the group consisting of halogen, hydroxy, amino, carboxyl, nitro, cyano, $-C(O)NR_{16}R_{17}$, $-C(O)OR_{16}$, substituted or unsubstituted $C_1$-$C_8$ alkyl or $C_1$-$C_8$ alkoxy;

$R_{16}$ and $R_{17}$ are each independently selected from the group consisting of H, substituted or unsubstituted $C_1$-$C_8$ alkyl, and hydroxy;

The heteroatoms in the heteroaryl and heterocycloalkyl are selected from the group consisting of N, O, or S, and the number of heteroatoms is selected from the group consisting of 1, 2, or 3.

**2.** The compound according to claim 1, or a salt thereof, or a stereoisomer thereof, or a solvate thereof, or a hydrate thereof, or a prodrug thereof, or a deuterated compound thereof, **characterized in that**:

for said $R_1$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;

for said $R_{15}$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;

for said $R_3$, the aryl is phenyl; and the heteroaryl is selected from the group consisting of isoindolinyl, pyridyl, pyrimidyl, pyridazinyl, thienyl, furyl, pyrazolyl, imidazolyl or pyrrolyl; he heterocycloalkyl is piperidyl;

for $R_7$ and $R_9$, the aryl is selected from phenyl or naphthyl;

for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

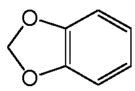

;

the substituent of the alkyl is selected from the group consisting of deuterium, halogen, hydroxy, cyano, nitro, carboxyl, piperidyl, morpholinyl or $-NR_{13}R_{14}$;

$R_{13}$ and $R_{14}$ are each independently selected from the group consisting of H and $C_1$-$C_8$ alkyl.

**3.** The compound according to claim 1, or a salt thereof, or a stereoisomer thereof, or a solvate thereof, or a hydrate thereof, or a prodrug thereof, or a deuterated compound thereof, **characterized in that** the compound is as represented by formula II:

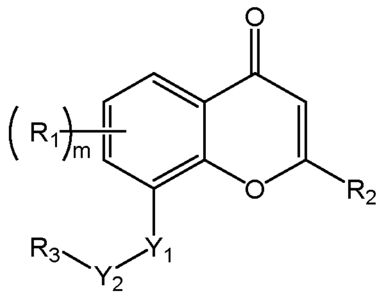

Formula II

wherein,

$R_1$ is a substituent in benzene ring, which has m substituents; each $R_1$ is independently selected from the group consisting of substituted or unsubstituted $C_1$-$C_8$ alkyl, $C_1$-$C_8$ alkoxy, halogen, cyano, nitro, hydroxy, carboxyl, $-NHR_{15}$, $-NHR_8$, $-C(O)NHR_8$, $-C(O)NHR_{15}$, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl; m is selected from the group consisting of 0, 1, 2, or 3;
$R_{15}$ is selected from the group consisting of H, substituted or unsubstituted $C_1$-$C_8$ alkyl, $C_1$-$C_8$ alkoxy, halogen, cyano, nitro, hydroxy, carboxyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl;
$Y_1$ and $Y_2$ are each independently selected from the group consisting of absence, $CR_4R_5$, and $NR_4$;
$R_4$ and $R_5$ are each independently selected from the group consisting of H and $C_1$-$C_8$ alkyl; alternatively, $R_4$ and $R_5$, together with a carbon, form a ketone group;
$R_3$ is selected from the group consisting of (6-10)-membered aryl substituted with n $R_6$, (5-10)-membered heteroaryl substituted with n $R_6$, (4-10)-membered heterocycloalkyl substituted with n $R_6$ or

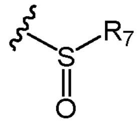

;

each $R_6$ is independently selected from the group consisting of substituted or unsubstituted $C_1$-$C_8$ alkyl, $C_1$-$C_8$ alkoxy, halogen, cyano, nitro, amino, hydroxy, $-C(O)OR_{81}$, $-C(O)R_{81}$,

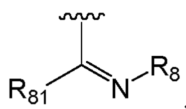

,

cyanomethyl, phosphonyl, sulfonic acid group, sulfonamido, boric acid group, - $NHR_8$ or $-C(O)NHR_8$; alternatively, two $R_6$ linked to the same atom form =O; n is selected from the group consisting of 0, 1, 2, 3, or 4;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

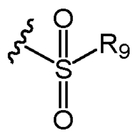

;

$R_7$ and $R_9$ are each independently selected from the group consisting of $C_1$-$C_8$ alkyl and (6-10)-membered aryl;
$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, $-NR_{11}R_{12}$,

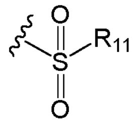

,

and $-SR_{11}$;
provided that $R_6$ is carboxyl, $R_2$ is not selected from the group consisting of substituted or unsubstituted (3-8)-membered cycloalkyl, substituted or unsubstituted (6-10)-membered aryl, and substituted or unsubstituted (5-10)-membered heteroaryl;
$R_{11}$ and $R_{12}$ are each independently selected from the group consisting of H, substituted or unsubstituted $C_1$-$C_8$ alkyl, and amino;
the substituent of the alkyl is selected from the group consisting of deuterium, halogen, hydroxy, cyano, nitro, carboxyl, (4-10)-membered heterocycloalkyl, or $-NR_{13}R_{14}$;
$R_{13}$ and $R_{14}$ are each independently selected from the group consisting of H and $C_1$-$C_8$ alkyl;
the substituent of the cycloalkyl, aryl, heteroaryl or heterocycloalkyl is selected from the group consisting of halogen, hydroxy, amino, carboxyl, nitro, cyano, $-C(O)NR_{16}R_{17}$, $-C(O)OR_{16}$, substituted or unsubstituted $C_1$-$C_8$ alkyl or $C_1$-$C_8$ alkoxy;
$R_{16}$ and $R_{17}$ are each independently selected from the group consisting of H, substituted or unsubstituted $C_1$-$C_8$ alkyl, and hydroxy;
The heteroatoms in the heteroaryl and heterocycloalkyl are selected from the group consisting of N, O, or S, and the number of heteroatoms is selected from the group consisting of 1, 2, or 3;
preferably,
for said $R_1$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;
for said $R_{15}$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;
for said $R_3$, the aryl is phenyl; and the heteroaryl is selected from the group consisting of isoindolinyl, pyridyl, pyrimidyl, pyridazinyl, thienyl, furyl, pyrazolyl, imidazolyl or pyrrolyl; the heterocycloalkyl is piperidyl;
for $R_7$ and $R_9$, the aryl is selected from phenyl or naphthyl;
for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

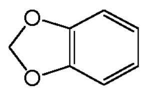

;

the substituent of the alkyl is selected from the group consisting of deuterium, halogen, hydroxy, cyano, nitro, carboxyl, piperidyl, morpholinyl or $-NR_{13}R_{14}$;
$R_{13}$ and $R_{14}$ are each independently selected from the group consisting of H and $C_1$-$C_8$ alkyl.

4. The compound according to claim 1, or a salt thereof, or a stereoisomer thereof, or a solvate thereof, or a hydrate thereof, or a prodrug thereof, or a deuterated compound thereof, **characterized in that** the compound is as represented by formula II-1:

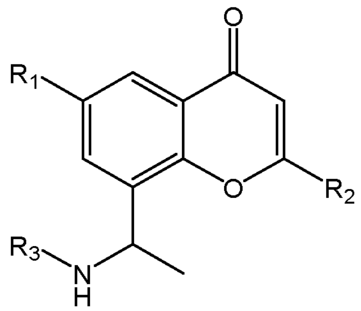

Formula II-1

wherein,

$R_1$ is selected from the group consisting of H, substituted or unsubstituted $C_1$-$C_8$ alkyl, $C_1$-$C_8$ alkoxy, halogen, cyano, nitro, hydroxy, carboxyl, $-NHR_{15}$, $-NHR_8$, $-C(O)NHR_8$, $-C(O)NHR_{15}$, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl;

$R_{15}$ is selected from the group consisting of H, substituted or unsubstituted $C_1$-$C_8$ alkyl, $C_1$-$C_8$ alkoxy, halogen, cyano, nitro, hydroxy, carboxyl, substituted or unsubstituted (3-8)-membered cycloalkyl, substituted or unsubstituted (6-10)-membered aryl, substituted or unsubstituted (4-10)-membered heterocycloalkyl, and substituted or unsubstituted (5-10)-membered heteroaryl;

$R_3$ is selected from the group consisting of (6-10)-membered aryl substituted with n $R_6$, (5-10)-membered heteroaryl substituted with n $R_6$, (4-10)-membered heterocycloalkyl substituted with n $R_6$ or

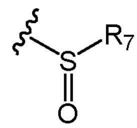
;

each $R_6$ is independently selected from the group consisting of substituted or unsubstituted $C_1$-$C_8$ alkyl, $C_1$-$C_8$ alkoxy, halogen, cyano, nitro, amino, hydroxy, $-C(O)OR_{81}$, $-C(O)R_{81}$,

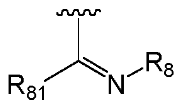
,

cyanomethyl, phosphonyl, sulfonic acid group, sulfonamido, boric acid group, - $NHR_8$ or $-C(O)NHR_8$; alternatively, two $R_6$ linked to the same atom form =O; n is selected from the group consisting of 0, 1, 2, 3, or 4;

$R_{81}$ is selected from the group consisting of H, substituted or unsubstituted $C_1$-$C_8$ alkyl, and amino;

$R_8$ is selected from the group consisting of substituted or unsubstituted $C_1$-$C_8$ alkyl, hydroxy, and

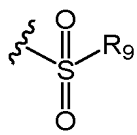
;

$R_7$ and $R_9$ are each independently selected from the group consisting of $C_1$-$C_8$ alkyl and (6-10)-membered aryl;

$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, $-NR_{11}R_{12}$,

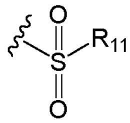,

and $-SR_{11}$;

provided that $R_6$ is carboxyl, $R_2$ is not selected from the group consisting of substituted or unsubstituted (3-8)-membered cycloalkyl, substituted or unsubstituted (6-10)-membered aryl, and substituted or unsubstituted (5-10)-membered heteroaryl;

$R_{11}$ and $R_{12}$ are each independently selected from the group consisting of H, substituted or unsubstituted $C_1$-$C_8$ alkyl, and amino;

the substituent of the alkyl is selected from the group consisting of deuterium, halogen, hydroxy, cyano, nitro, carboxyl, (4-10)-membered heterocycloalkyl, or $-NR_{13}R_{14}$;

$R_{13}$ and $R_{14}$ are each independently selected from the group consisting of H and $C_1$-$C_8$ alkyl;

the substituent of the cycloalkyl, aryl, heteroaryl or heterocycloalkyl is selected from the group consisting of halogen, hydroxy, amino, carboxyl, nitro, cyano, $-C(O)NR_{16}R_{17}$, $-C(O)OR_{16}$, substituted or unsubstituted $C_1$-$C_8$ alkyl or $C_1$-$C_8$ alkoxy;

$R_{16}$ and $R_{17}$ are each independently selected from the group consisting of H, substituted or unsubstituted $C_1$-$C_8$ alkyl, and hydroxy;

The heteroatoms in the heteroaryl and heterocycloalkyl are selected from the group consisting of N, O, or S, and the number of heteroatoms is selected from the group consisting of 1, 2, or 3;

preferably,

for said $R_1$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;

for said $R_{15}$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;

for said $R_3$, the aryl is phenyl; and the heteroaryl is selected from the group consisting of isoindolinyl, pyridyl, pyrimidyl, pyridazinyl, thienyl, furyl, pyrazolyl, imidazolyl or pyrrolyl; he heterocycloalkyl is piperidyl;

for $R_7$ and $R_9$, the aryl is selected from phenyl or naphthyl;

for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

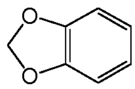;

the substituent of the alkyl is selected from the group consisting of deuterium, halogen, hydroxy, cyano, nitro, carboxyl, piperidyl, morpholinyl or $-NR_{13}R_{14}$;

$R_{13}$ and $R_{14}$ are each independently selected from the group consisting of H and $C_1$-$C_8$ alkyl.

**5.** The compound according to claim 4, or a salt thereof, or a stereoisomer thereof, or a solvate thereof, or a hydrate thereof, or a prodrug thereof, or a deuterated compound thereof, **characterized in that** the compound is as represented by formula II-2:

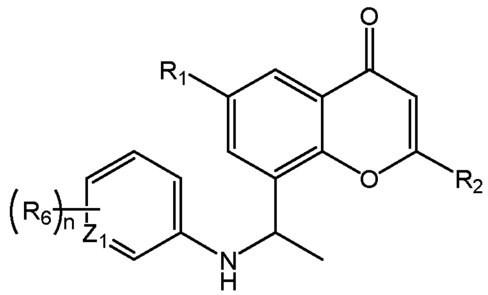

Formula II-2

wherein,

$Z_1$ is selected from CH or N;

$R_1$ is selected from the group consisting of H, substituted or unsubstituted $C_1$-$C_8$ alkyl, $C_1$-$C_8$ alkoxy, halogen, cyano, nitro, hydroxy, carboxyl, $-NHR_{15}$, $-NHR_8$, $-C(O)NHR_8$, $-C(O)NHR_{15}$, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl;

$R_{15}$ is selected from the group consisting of H, substituted or unsubstituted $C_1$-$C_8$ alkyl, $C_1$-$C_8$ alkoxy, halogen, cyano, nitro, hydroxy, carboxyl, substituted or unsubstituted (3-8)-membered cycloalkyl, substituted or unsubstituted (6-10)-membered aryl, substituted or unsubstituted (4-10)-membered heterocycloalkyl, and substituted or unsubstituted (5-10)-membered heteroaryl;

n is selected from the group consisting of 0, 1, 2, 3 or 4;

each $R_6$ is independently selected from the group consisting of substituted or unsubstituted $C_1$-$C_8$ alkyl, $C_1$-$C_8$ alkoxy, halogen, cyano, nitro, amino, hydroxy, $-C(O)OR_{81}$, $-C(O)R_{81}$,

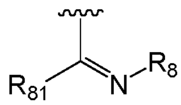

,

cyanomethyl, phosphonyl, sulfonic acid group, sulfonamido, boric acid group, $-NHR_8$ or $-C(O)NHR_8$; alternatively, two $R_6$ linked to the same atom form =O;

$R_{81}$ is selected from the group consisting of H, substituted or unsubstituted $C_1$-$C_8$ alkyl, and amino;

$R_8$ is selected from the group consisting of substituted or unsubstituted $C_1$-$C_8$ alkyl, hydroxy, and

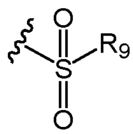

;

$R_9$ is each independently selected from the group consisting of $C_1$-$C_8$ alkyl and (6-10)-membered aryl;

$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, $-NR_{11}R_{12}$,

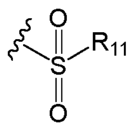

,

and $-SR_{11}$;

provided that $R_6$ is carboxyl, $R_2$ is not selected from the group consisting of substituted or unsubstituted (3-8)-membered cycloalkyl, substituted or unsubstituted (6-10)-membered aryl, and substituted or unsubstituted (5-10)-membered heteroaryl;

$R_{11}$ and $R_{12}$ are each independently selected from the group consisting of H, substituted or unsubstituted $C_1$-$C_8$

alkyl, and amino;

the substituent of the alkyl is selected from the group consisting of deuterium, halogen, hydroxy, cyano, nitro, carboxyl, (4-10)-membered heterocycloalkyl, or $-NR_{13}R_{14}$;

$R_{13}$ and $R_{14}$ are each independently selected from the group consisting of H and $C_1$-$C_8$ alkyl;

the substituent of the cycloalkyl, aryl, heteroaryl or heterocycloalkyl is selected from the group consisting of halogen, hydroxy, amino, carboxyl, nitro, cyano, $-C(O)NR_{16}R_{17}$, $-C(O)OR_{16}$, substituted or unsubstituted $C_1$-$C_8$ alkyl or $C_1$-$C_8$ alkoxy;

$R_{16}$ and $R_{17}$ are each independently selected from the group consisting of H, substituted or unsubstituted $C_1$-$C_8$ alkyl, and hydroxy;

The heteroatoms in the heteroaryl and heterocycloalkyl are selected from the group consisting of N, O, or S, and the number of heteroatoms is selected from the group consisting of 1, 2, or 3;

preferably,

for said $R_1$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;

for said $R_{15}$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl or pyrrolyl;

for $R_9$, the aryl is selected from phenyl or naphthyl;

for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

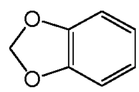
;

the substituent of the alkyl is selected from the group consisting of deuterium, halogen, hydroxy, cyano, nitro, carboxyl, piperidyl, morpholinyl or $-NR_{13}R_{14}$;

$R_{13}$ and $R_{14}$ are each independently selected from the group consisting of H and $C_1$-$C_8$ alkyl.

**6.** The compound according to claim 5, or a salt thereof, or a stereoisomer thereof, or a solvate thereof, or a hydrate thereof, or a prodrug thereof, or a deuterated compound thereof, **characterized in that** the compound is as represented by formula II-3:

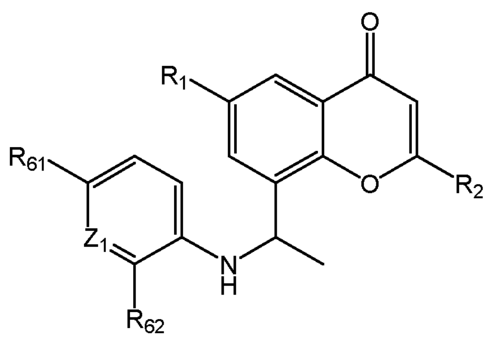

Formula II-3

wherein,

$Z_1$ is selected from CH or N;

$R_1$ is selected from the group consisting of substituted or unsubstituted $C_1$-$C_8$ alkyl and halogen;

$R_{61}$ and $R_{62}$ are each independently selected from the group consisting of H, substituted or unsubstituted $C_1$-$C_8$ alkyl, $C_1$-$C_8$ alkoxy, halogen, cyano, nitro, amino, hydroxy, $-C(O)OR_{81}$, $-C(O)R_{81}$,

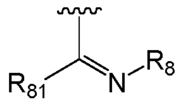

cyanomethyl, phosphonyl, sulfonic acid group, sulfonamido, boric acid group, $-NHR_8$ or $-C(O)NHR_8$;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

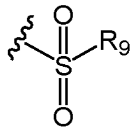

;

$R_9$ is each independently selected from the group consisting of $C_1$-$C_8$ alkyl and (6-10)-membered aryl;
$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, $-NR_{11}R_{12}$,

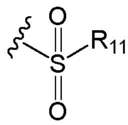

,

and $-SR_{11}$;
provided that $R_6$ is carboxyl, $R_2$ is not selected from the group consisting of substituted or unsubstituted (3-8)-membered cycloalkyl, substituted or unsubstituted (6-10)-membered aryl, and substituted or unsubstituted (5-10)-membered heteroaryl;
$R_{11}$ and $R_{12}$ are each independently selected from the group consisting of H, substituted or unsubstituted $C_1$-$C_8$ alkyl, and amino;
the substituent of the alkyl is selected from the group consisting of deuterium, halogen, hydroxy, cyano, nitro, carboxyl, (4-10)-membered heterocycloalkyl, or $-NR_{13}R_{14}$;
$R_{13}$ and $R_{14}$ are each independently selected from the group consisting of H and $C_1$-$C_8$ alkyl;
the substituent of the cycloalkyl, aryl, heteroaryl or heterocycloalkyl is selected from the group consisting of halogen, hydroxy, amino, carboxyl, nitro, cyano, $-C(O)NR_{16}R_{17}$, $-C(O)OR_{16}$, substituted or unsubstituted $C_1$-$C_8$ alkyl or $C_1$-$C_8$ alkoxy;
$R_{16}$ and $R_{17}$ are each independently selected from the group consisting of H, substituted or unsubstituted $C_1$-$C_8$ alkyl, and hydroxy;
The heteroatoms in the heteroaryl and heterocycloalkyl are selected from the group consisting of N, O, or S, and the number of heteroatoms is selected from the group consisting of 1, 2, or 3;
preferably,
for said $R_9$, the aryl is selected from phenyl or naphthyl;
for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

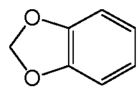

;

the substituent of the alkyl is selected from the group consisting of halogen, hydroxy, cyano, nitro, carboxyl, piperidyl, morpholinyl or $-NR_{13}R_{14}$;
$R_{13}$ and $R_{14}$ are each independently selected from the group consisting of H and $C_1$-$C_8$ alkyl.

7. The compound according to claim 6, or a salt thereof, or a stereoisomer thereof, or a solvate thereof, or a hydrate thereof, or a prodrug thereof, or a deuterated compound thereof, **characterized in that** the compound is as represented by formula II-4:

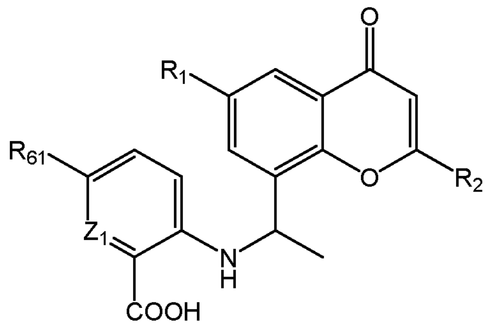

Formula II-4

wherein,

$Z_1$ is selected from CH or N;
$R_1$ is selected from the group consisting of $C_1$-$C_8$ alkyl, halogen, and trifluoromethyl;
$R_{61}$ is selected from the group consisting of H, substituted or unsubstituted $C_1$-$C_8$ alkyl, $C_1$-$C_8$ alkoxy, halogen, cyano, nitro, amino, hydroxy, -C(O)O$R_{81}$, -C(O)$R_{81}$,

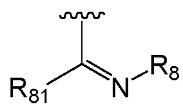

,

cyanomethyl, phosphonyl, sulfonic acid group, sulfonamido, boric acid group, -NH$R_8$ or - C(O)NH$R_8$;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

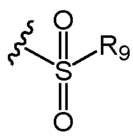

;

$R_9$ is each independently selected from the group consisting of $C_1$-$C_8$ alkyl and (6-10)-membered aryl;
$R_2$ is selected from the group consisting of substituted or unsubstituted $C_1$-$C_8$ alkyl, substituted or unsubstituted (4-10)-membered cycloalkyl, -N$R_{11}R_{12}$,

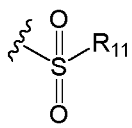

,

and -S$R_{11}$;
$R_{11}$ and $R_{12}$ are each independently selected from the group consisting of H, substituted or unsubstituted $C_1$-$C_8$ alkyl, and amino;
the substituent of the alkyl is selected from the group consisting of deuterium, halogen, hydroxy, cyano, nitro, carboxyl, (4-10)-membered heterocycloalkyl, or -N$R_{13}R_{14}$;
$R_{13}$ and $R_{14}$ are each independently selected from the group consisting of H and $C_1$-$C_8$ alkyl;
the substituent of the cycloalkyl, aryl, heteroaryl or heterocycloalkyl is selected from the group consisting of halogen, hydroxy, amino, carboxyl, nitro, cyano, -C(O)N$R_{16}R_{17}$, -C(O)O$R_{16}$, substituted or unsubstituted $C_1$-$C_8$ alkyl or $C_1$-$C_8$ alkoxy;
$R_{16}$ and $R_{17}$ are each independently selected from the group consisting of H, substituted or unsubstituted $C_1$-$C_8$ alkyl, and hydroxy;
The heteroatoms in the heteroaryl and heterocycloalkyl are selected from the group consisting of N, O, or S, and the number of heteroatoms is selected from the group consisting of 1, 2, or 3;
preferably,

for said $R_9$, the aryl is selected from phenyl or naphthyl;
for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

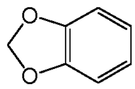;

the substituent of the alkyl is selected from the group consisting of halogen, hydroxy, cyano, nitro, carboxyl, piperidyl, morpholinyl or $-NR_{13}R_{14}$;
$R_{13}$ and $R_{14}$ are each independently selected from the group consisting of H and $C_1$-$C_8$ alkyl.

**8.** The compound according to claim 7, or a salt thereof, or a stereoisomer thereof, or a solvate thereof, or a hydrate thereof, or a prodrug thereof, or a deuterated compound thereof, **characterized in that** the compound is as represented by formula II-5:

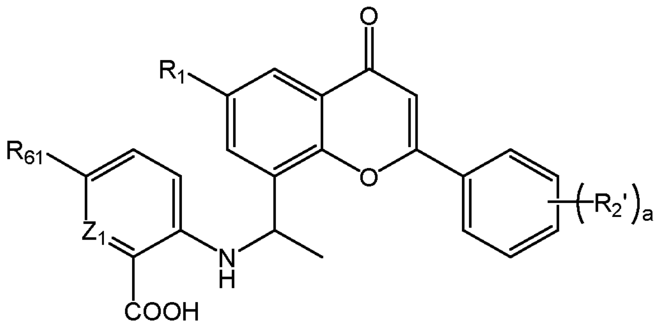

Formula II-5

wherein,

$Z_1$ is selected from CH or N;
$R_1$ is selected from the group consisting of $C_1$-$C_8$ alkyl, halogen, and trifluoromethyl;
$R_{61}$ is selected from the group consisting of H, substituted or unsubstituted $C_1$-$C_8$ alkyl, $C_1$-$C_8$ alkoxy, halogen, cyano, nitro, amino, hydroxy, $-C(O)OR_{81}$, $-C(O)R_{81}$,

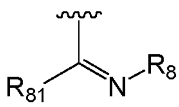,

cyanomethyl, phosphonyl, sulfonic acid group, sulfonamido, boric acid group, $-NHR_8$ or - $C(O)NHR_8$;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

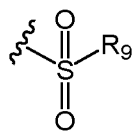;

$R_9$ is each independently selected from the group consisting of $C_1$-$C_8$ alkyl and phenyl;
$R_2$' is a substituent of benzene ring, and the number of substituents is a; each $R_2$' is independently selected from halogen, hydroxy, amino, carboxyl, nitro, cyano, $-C(O)NR_{16}R_{17}$, - $C(O)OR_{16}$, substituted or unsubstituted $C_1$-$C_8$ alkyl or $C_1$-$C_8$ alkoxy; a is selected from the group consisting of 0, 1, 2, or 3;
$R_{16}$ and $R_{17}$ are each independently selected from the group consisting of H, substituted or unsubstituted $C_1$-$C_8$ alkyl, and hydroxy;

the substituent of the alkyl is selected from the group consisting of deuterium, halogen, hydroxy, cyano, nitro, carboxyl, piperidyl, morpholinyl or $-NR_{13}R_{14}$;
$R_{13}$ and $R_{14}$ are each independently selected from the group consisting of H and $C_1$-$C_8$ alkyl.

**9.** The compound according to claim 1, or a salt thereof, or a stereoisomer thereof, or a solvate thereof, or a hydrate thereof, or a prodrug thereof, or a deuterated compound thereof, **characterized in that** the compound is as represented by formula III-1:

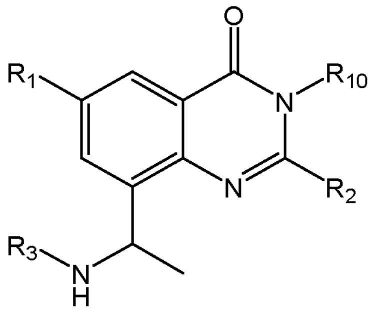

Formula III-1

wherein,

$R_1$ is selected from the group consisting of substituted or unsubstituted $C_1$-$C_8$ alkyl, $C_1$-$C_8$ alkoxy, halogen, cyano, nitro, amino, hydroxy or carboxyl;
$R_3$ is selected from the group consisting of (6-10)-membered aryl substituted with n $R_6$, (5-10)-membered heteroaryl substituted with n $R_6$, (4-10)-membered heterocycloalkyl substituted with n $R_6$ or

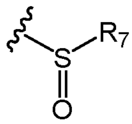

;

each $R_6$ is independently selected from the group consisting of substituted or unsubstituted $C_1$-$C_8$ alkyl, $C_1$-$C_8$ alkoxy, halogen, cyano, nitro, amino, hydroxy, $-C(O)OR_{81}$, $-C(O)R_{81}$,

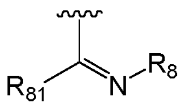

,

cyanomethyl, phosphonyl, sulfonic acid group, sulfonamido, boric acid group, - $NHR_8$ or $-C(O)NHR_8$; alternatively, two $R_6$ linked to the same atom form =O; n is selected from the group consisting of 0, 1, 2, 3, or 4;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

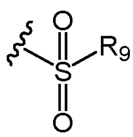

;

$R_7$ and $R_9$ are each independently selected from the group consisting of $C_1$-$C_8$ alkyl and (6-10)-membered aryl;
$R_{10}$ is selected from the group consisting of H as well as substituted or unsubstituted $C_1$-$C_8$ alkyl;
$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, $-NR_{11}R_{12}$,

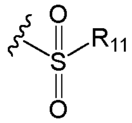

,

and $-SR_{11}$;

$R_{11}$ and $R_{12}$ are each independently selected from the group consisting of H, substituted or unsubstituted $C_1$-$C_8$ alkyl, and amino;

the substituent of the alkyl is selected from the group consisting of deuterium, halogen, hydroxy, cyano, nitro, carboxyl, (4-10)-membered heterocycloalkyl, or $-NR_{13}R_{14}$;

$R_{13}$ and $R_{14}$ are each independently selected from the group consisting of H and $C_1$-$C_8$ alkyl;

the substituent of the cycloalkyl, aryl, heteroaryl or heterocycloalkyl is selected from the group consisting of halogen, hydroxy, amino, carboxyl, nitro, cyano, $-C(O)NR_{16}R_{17}$, $-C(O)OR_{16}$, substituted or unsubstituted $C_1$-$C_8$ alkyl or $C_1$-$C_8$ alkoxy;

$R_{16}$ and $R_{17}$ are each independently selected from the group consisting of H, substituted or unsubstituted $C_1$-$C_8$ alkyl, and hydroxy;

The heteroatoms in the heteroaryl and heterocycloalkyl are selected from the group consisting of N, O, or S, and the number of heteroatoms is selected from the group consisting of 1, 2, or 3.

preferably,

for said $R_3$, the aryl is phenyl; and the heteroaryl is selected from the group consisting of isoindolinyl, pyridyl, pyrimidyl, pyridazinyl, thienyl, furyl, pyrazolyl, imidazolyl or pyrrolyl; the heterocycloalkyl is piperidyl;

for $R_7$ and $R_9$, the aryl is selected from phenyl or naphthyl;

for $R_2$, the aryl is selected from phenyl, anthranyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

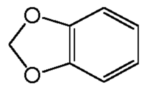

;

the substituent of the alkyl is selected from the group consisting of deuterium, halogen, hydroxy, cyano, nitro, carboxyl, piperidyl, morpholinyl or $-NR_{13}R_{14}$;

$R_{13}$ and $R_{14}$ are each independently selected from the group consisting of H and $C_1$-$C_8$ alkyl.

**10.** The compound according to claim 9, or a salt thereof, or a stereoisomer thereof, or a solvate thereof, or a hydrate thereof, or a prodrug thereof, or a deuterated compound thereof, **characterized in that** the compound is as represented by formula III-2:

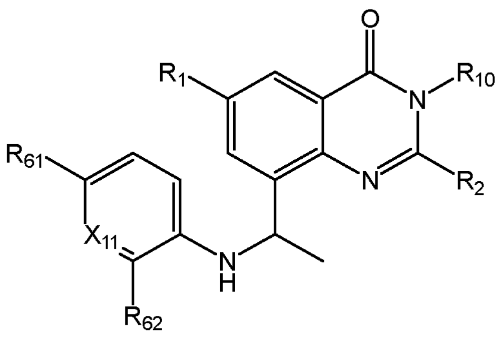

Formula III-2

wherein,

$X_{11}$ is selected from CH or N;

$R_1$ is selected from the group consisting of substituted or unsubstituted $C_1$-$C_8$ alkyl, $C_1$-$C_8$ alkoxy, halogen, cyano, nitro, amino, hydroxy or carboxyl;

$R_{61}$ and $R_{62}$ are each independently selected from the group consisting of H, substituted or unsubstituted $C_1$-$C_8$

alkyl, $C_1$-$C_8$ alkoxy, halogen, cyano, nitro, amino, hydroxy, -C(O)O$R_{81}$, - C(O)$R_{81}$,

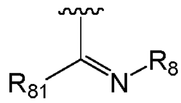

,

cyanomethyl, phosphonyl, sulfonic acid group, sulfonamido, boric acid group, -NH$R_8$ or -C(O)NH$R_8$;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

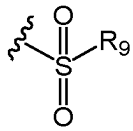

;

$R_9$ is selected from the group consisting of $C_1$-$C_8$ alkyl and (6-10)-membered aryl;
$R_{10}$ is selected from the group consisting of H as well as substituted or unsubstituted $C_1$-$C_8$ alkyl;
$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, -N$R_{11}R_{12}$,

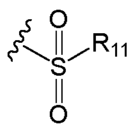

,

and -S$R_{11}$;
$R_{11}$ and $R_{12}$ are each independently selected from the group consisting of H, substituted or unsubstituted $C_1$-$C_8$ alkyl, and amino;
the substituent of the alkyl is selected from the group consisting of deuterium, halogen, hydroxy, cyano, nitro, carboxyl, (4-10)-membered heterocycloalkyl, or -N$R_{13}R_{14}$;
$R_{13}$ and $R_{14}$ are each independently selected from the group consisting of H and $C_1$-$C_8$ alkyl;
the substituent of the cycloalkyl, aryl, heteroaryl or heterocycloalkyl is selected from the group consisting of halogen, hydroxy, amino, carboxyl, nitro, cyano, -C(O)N$R_{16}R_{17}$, -C(O)O$R_{16}$, substituted or unsubstituted $C_1$-$C_8$ alkyl or $C_1$-$C_8$ alkoxy;
$R_{16}$ and $R_{17}$ are each independently selected from the group consisting of H, substituted or unsubstituted $C_1$-$C_8$ alkyl, and hydroxy;
the heteroatoms in the heteroaryl and heterocycloalkyl are selected from the group consisting of N, O, or S, and the number of heteroatoms is selected from the group consisting of 1, 2, or 3.
preferably,
for $R_9$, the aryl is selected from phenyl or naphthyl;
for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

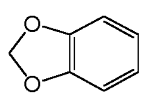

;

the substituent of the alkyl is selected from the group consisting of deuterium, halogen, hydroxy, cyano, nitro, carboxyl, piperidyl, morpholinyl or -N$R_{13}R_{14}$;
$R_{13}$ and $R_{14}$ are each independently selected from the group consisting of H and $C_1$-$C_8$ alkyl.

11. The compound according to claim 9, or a salt thereof, or a stereoisomer thereof, or a solvate thereof, or a hydrate thereof, or a prodrug thereof, or a deuterated compound thereof, **characterized in that** the compound is as represented by formula III-3:

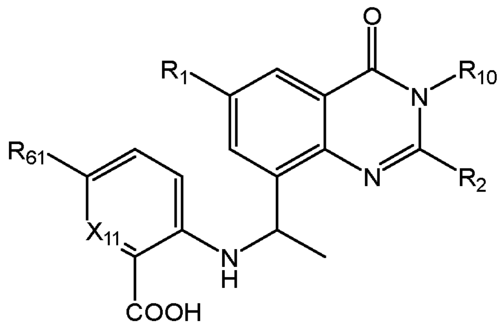

Formula III-3

wherein,

$X_{11}$ is selected from $CR_{71}$ or N;

$R_{71}$ is selected from the group consisting of H, halogen, and $C_1$-$C_8$ alkyl;

$R_1$ is selected from the group consisting of substituted or unsubstituted $C_1$-$C_8$ alkyl, $C_1$-$C_8$ alkoxy, halogen, cyano, nitro, amino, hydroxy or carboxyl;

$R_{61}$ is selected from the group consisting of H, substituted or unsubstituted $C_1$-$C_8$ alkyl, $C_1$-$C_8$ alkoxy, halogen, cyano, nitro, amino, hydroxy, -C(O)O$R_{81}$, -C(O)$R_{81}$,

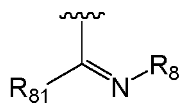

,

cyanomethyl, phosphonyl, sulfonic acid group, sulfonamido, boric acid group, -NH$R_8$ or - C(O)NH$R_8$;

$R_{81}$ is selected from the group consisting of H, substituted or unsubstituted $C_1$-$C_8$ alkyl, and amino;

$R_8$ is selected from the group consisting of substituted or unsubstituted $C_1$-$C_8$ alkyl, hydroxy, and

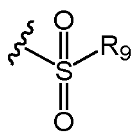

;

$R_9$ is selected from the group consisting of $C_1$-$C_8$ alkyl and (6-10)-membered aryl;

$R_{10}$ is selected from the group consisting of H as well as substituted or unsubstituted $C_1$-$C_8$ alkyl;

$R_2$ is selected from the group consisting of substituted or unsubstituted $C_1$-$C_8$ alkyl, substituted or unsubstituted (3-8)-membered cycloalkyl, substituted or unsubstituted (6-10)-membered aryl, substituted or unsubstituted (4-10)-membered heterocycloalkyl, substituted or unsubstituted (5-10)-membered heteroaryl, -N$R_{11}R_{12}$,

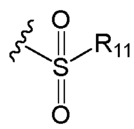

,

and -S$R_{11}$;

$R_{11}$ and $R_{12}$ are each independently selected from the group consisting of H, substituted or unsubstituted $C_1$-$C_8$ alkyl, and amino;

the substituent of the alkyl is selected from the group consisting of deuterium, halogen, hydroxy, cyano, nitro, (4-10)-membered heterocycloalkyl, or -N$R_{13}R_{14}$;

$R_{13}$ and $R_{14}$ are each independently selected from the group consisting of H and $C_1$-$C_8$ alkyl;

the substituent of the cycloalkyl, aryl, heteroaryl or heterocycloalkyl is selected from the group consisting of halogen, hydroxy, amino, carboxyl, nitro, cyano, -C(O)N$R_{16}R_{17}$, -C(O)O$R_{16}$, substituted or unsubstituted $C_1$-$C_8$ alkyl or $C_1$-$C_8$ alkoxy;

$R_{16}$ and $R_{17}$ are each independently selected from the group consisting of H, substituted or unsubstituted $C_1$-$C_8$

alkyl, and hydroxy;
the heteroatoms in the heteroaryl and heterocycloalkyl are selected from the group consisting of N, O, or S, and the number of heteroatoms is selected from the group consisting of 1, 2, or 3.
preferably,
for $R_9$, the aryl is selected from phenyl or naphthyl;
for $R_2$, the aryl is selected from phenyl or naphthyl; the heterocycloalkyl is selected from piperidyl or morpholinyl; and the heteroaryl is selected from the group consisting of isoindolinyl, pyridyl, pyrimidyl, pyridazinyl, thienyl, furyl, pyrazolyl, imidazolyl, pyrrolyl, isoxazolyl or

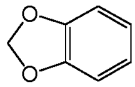;

the substituent of the alkyl is selected from the group consisting of halogen, hydroxy, cyano, nitro, carboxyl, piperidyl, morpholinyl or $-NR_{13}R_{14}$;
$R_{13}$ and $R_{14}$ are each independently selected from the group consisting of H and $C_1$-$C_8$ alkyl.

**12.** The compound according to claim 9, or a salt thereof, or a stereoisomer thereof, or a solvate thereof, or a hydrate thereof, or a prodrug thereof, or a deuterated compound thereof, **characterized in that** the compound is as represented by formula III-4:

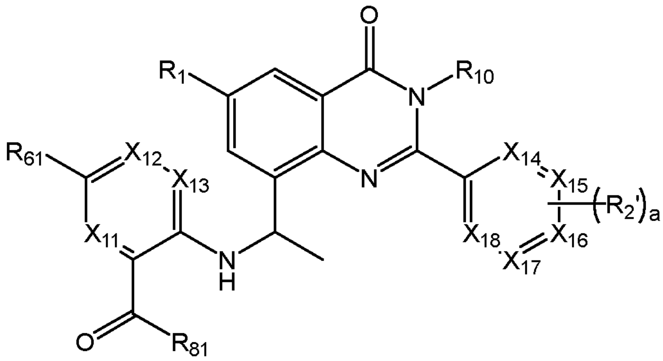

Formula III-4

wherein,

$X_{11}$, $X_{12}$, $X_{13}$, $X_{14}$, $X_{15}$, $X_{16}$, $X_{17}$, and $X_{18}$ are each independently selected from the group consisting of $CR_{71}$ or N;
$R_{61}$ is selected from the group consisting of H, substituted or unsubstituted $C_1$-$C_8$ alkyl, $C_1$-$C_8$ alkoxy, halogen, cyano, nitro, amino, hydroxy, carboxyl, cyanomethyl, phosphonyl, sulfonic acid group, sulfonamido, boric acid group, $-NHR_8$ or $-C(O)NHR_8$;
$R_{71}$ is selected from the group consisting of H, halogen, and $C_1$-$C_8$ alkyl
$R_{81}$ is selected from the group consisting of hydroxy, $C_1$-$C_8$ alkoxy, and amino;
$R_8$ is selected from the group consisting of hydroxy and

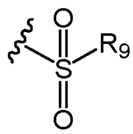;

$R_9$ is selected from the group consisting of $C_1$-$C_8$ alkyl and phenyl;
$R_1$ is selected from the group consisting of substituted or unsubstituted $C_1$-$C_8$ alkyl, $C_1$-$C_8$ alkoxy, halogen, cyano, nitro, amino, hydroxy or carboxyl;
$R_{10}$ is selected from the group consisting of H as well as substituted or unsubstituted $C_1$-$C_8$ alkyl;
$R_2$' is a ring substituent, and the number of substituents is a; each $R_2$' is independently selected from the group consisting of halogen, hydroxy, amino, carboxyl, nitro, cyano, - $C(O)NR_{16}R_{17}$, $-C(O)OR_{16}$, substituted or unsubstituted $C_1$-$C_8$ alkyl or $C_1$-$C_8$ alkoxy; a is selected from the group consisting of 0, 1, 2, or 3;

$R_{16}$ and $R_{17}$ are each independently selected from the group consisting of H and $C_1$-$C_8$ alkyl;
the substituent of the alkyl is selected from the group consisting of deuterium, halogen, hydroxy, cyano, nitro, carboxyl, piperidyl, morpholinyl or -$NR_{13}R_{14}$;
$R_{13}$ and $R_{14}$ are each independently selected from the group consisting of H and $C_1$-$C_8$ alkyl.

**13.** The compound according to claim 12, or a salt thereof, or a stereoisomer thereof, or a solvate thereof, or a hydrate thereof, or a prodrug thereof, or a deuterated compound thereof, **characterized in that** the compound is as represented by formula III-5:

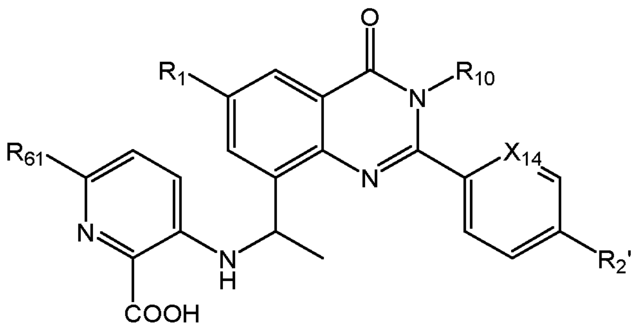

Formula III-5

wherein,

$X_{14}$ is selected from CH or N;
$R_{61}$ is selected from the group consisting of H, substituted or unsubstituted $C_1$-$C_8$ alkyl, $C_1$-$C_8$ alkoxy, halogen, cyano, nitro, amino, hydroxy, carboxyl, cyanomethyl, phosphonyl, sulfonic acid group, sulfonamido, boric acid group, -$NHR_8$ or -C(O)$NHR_8$;
$R_8$ is selected from the group consisting of hydroxy and

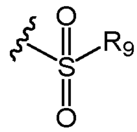

;

$R_9$ is selected from the group consisting of $C_1$-$C_8$ alkyl and phenyl;
$R_1$ is selected from the group consisting of substituted or unsubstituted $C_1$-$C_8$ alkyl, $C_1$-$C_8$ alkoxy, halogen, cyano, nitro, amino, hydroxy or carboxyl;
$R_{10}$ is selected from the group consisting of H as well as substituted or unsubstituted $C_1$-$C_8$ alkyl;
$R_2$' is selected from the group consisting of halogen, hydroxy, amino, carboxyl, nitro, cyano, $C_1$-$C_8$ alkyl or $C_1$-$C_8$ alkoxy; a is selected from the group consisting of 0, 1, 2, or 3;
the substituent of the alkyl is selected from the group consisting of halogen, hydroxy, cyano, nitro, carboxyl, piperidyl, morpholinyl or -$NR_{13}R_{14}$;
$R_{13}$ and $R_{14}$ are each independently selected from the group consisting of H and $C_1$-$C_8$ alkyl.

**14.** The compound according to claim 9, or a salt thereof, or a stereoisomer thereof, or a solvate thereof, or a hydrate thereof, or a prodrug thereof, or a deuterated compound thereof, **characterized in that** the compound is as represented by formula III-6:

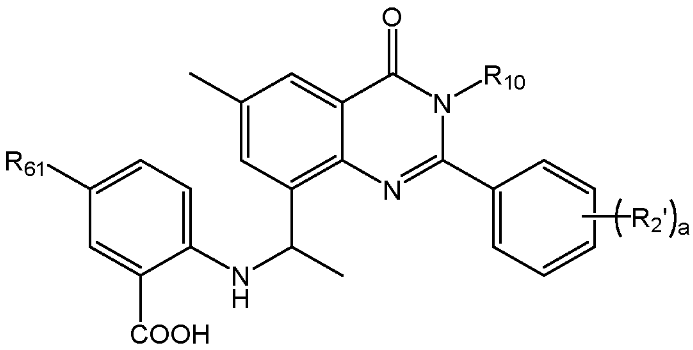

Formula III-6

wherein,

$R_{61}$ is selected from the group consisting of H, substituted or unsubstituted $C_1$-$C_8$ alkyl, $C_1$-$C_8$ alkoxy, halogen, cyano, nitro, amino, hydroxy, carboxyl, cyanomethyl, phosphonyl, sulfonic acid group, sulfonamido, boric acid group, -$NHR_8$ or -C(O)$NHR_8$;
$R_8$ is selected from the group consisting of hydroxy and

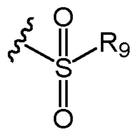
;

$R_9$ is selected from the group consisting of $C_1$-$C_8$ alkyl and phenyl;
$R_{10}$ is selected from the group consisting of H as well as substituted or unsubstituted $C_1$-$C_8$ alkyl;
$R_2$' is a substituent of benzene ring, and the number of substituents is a; each $R_2$' is independently selected from the group consisting of halogen, hydroxy, amino, carboxyl, nitro, cyano, $C_1$-$C_8$ alkyl or $C_1$-$C_8$ alkoxy; a is selected from the group consisting of 0, 1, 2, or 3;
the substituent of the alkyl is selected from the group consisting of halogen, hydroxy, cyano, nitro, carboxyl, piperidyl, morpholinyl or -$NR_{13}R_{14}$;
$R_{13}$ and $R_{14}$ are each independently selected from the group consisting of H and $C_1$-$C_8$ alkyl.

**15.** The compound according to claim 9, or a salt thereof, or a stereoisomer thereof, or a solvate thereof, or a hydrate thereof, or a prodrug thereof, or a deuterated compound thereof, **characterized in that** the compound is as represented by formula III-7:

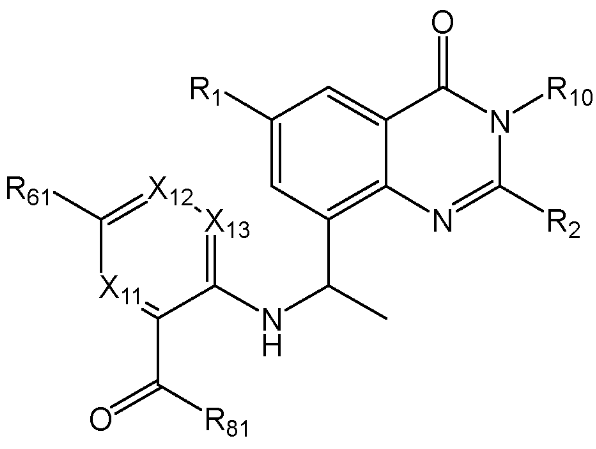

Formula III-7

wherein,

$X_{11}$, $X_{12}$, and $X_{13}$ are each independently selected from the group consisting of $CR_{71}$ or N;
$R_{61}$ is selected from the group consisting of H, substituted or unsubstituted $C_1$-$C_8$ alkyl, $C_1$-$C_8$ alkoxy, halogen, cyano, nitro, amino, hydroxy, carboxyl, cyanomethyl, phosphonyl, sulfonic acid group, sulfonamido, boric acid group, -$NHR_8$ or -C(O)$NHR_8$;
$R_{71}$ is selected from the group consisting of H, halogen, and $C_1$-$C_8$ alkyl
$R_{81}$ is selected from the group consisting of hydroxy, $C_1$-$C_8$ alkoxy, and amino;
$R_8$ is selected from the group consisting of hydroxy and

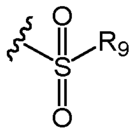
;

$R_9$ is selected from the group consisting of $C_1$-$C_8$ alkyl and phenyl;
$R_1$ is selected from the group consisting of substituted or unsubstituted $C_1$-$C_8$ alkyl, $C_1$-$C_8$ alkoxy, halogen, cyano, nitro, amino, hydroxy or carboxyl;
$R_{10}$ is selected from the group consisting of H as well as substituted or unsubstituted $C_1$-$C_8$ alkyl;
$R_2$ is selected from the group consisting of

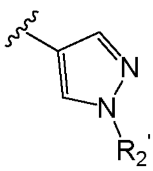
,
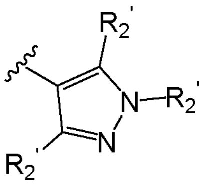
,
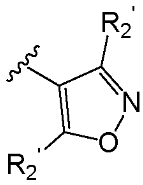
,

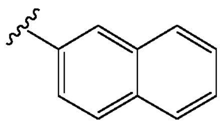
,
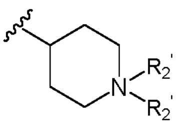
,
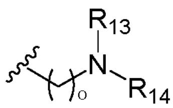
,
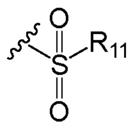
, and
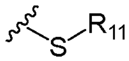
;

$R_{11}$ is each independently selected from the group consisting of H as well as substituted or unsubstituted $C_1$-$C_8$ alkyl;
each $R_2$' is independently selected from the group consisting of halogen, hydroxy, amino, carboxyl, nitro, cyano, -C(O)$NR_{15}R_{16}$, substituted or unsubstituted $C_1$-$C_8$ alkyl or $C_1$-$C_8$ alkoxy; a is selected from the group consisting of 0, 1, 2, or 3;
O is an integer selected from 1 to 3;
$R_{15}$ and $R_{16}$ are each independently selected from the group consisting of H and $C_1$-$C_8$ alkyl;
the substituent of the alkyl is selected from the group consisting of deuterium, halogen, hydroxy, cyano, nitro, carboxyl, piperidyl, morpholinyl or -$NR_{13}R_{14}$;
$R_{13}$ and $R_{14}$ are each independently selected from the group consisting of H and $C_1$-$C_8$ alkyl; alternatively, $R_{13}$ and $R_{14}$ are linked to form

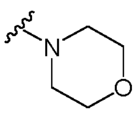
.

**16.** The compound according to claim 9, or a salt thereof, or a stereoisomer thereof, or a solvate thereof, or a hydrate thereof, or a prodrug thereof, or a deuterated compound thereof, **characterized in that** the compound is as represented by formula III-8:

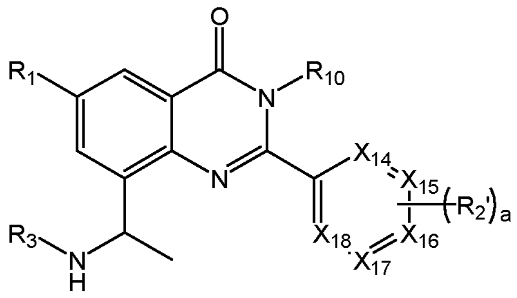

Formula III-8

wherein,

$X_{14}$, $X_{15}$, $X_{16}$, $X_{17}$, and $X_{18}$ are each independently selected from the group consisting of $CR_{71}$ or N;
$R_{71}$ is selected from the group consisting of H, halogen, and $C_1$-$C_8$ alkyl
$R_1$ is selected from the group consisting of substituted or unsubstituted $C_1$-$C_8$ alkyl, $C_1$-$C_8$ alkoxy, halogen, cyano, nitro, amino, hydroxy or carboxyl;
$R_{10}$ is selected from the group consisting of H as well as substituted or unsubstituted $C_1$-$C_8$ alkyl;
$R_2$' is a ring substituent, and the number of substituents is a; each $R_2$' is independently selected from the group consisting of halogen, hydroxy, amino, carboxyl, nitro, cyano, - $C(O)NR_{15}R_{16}$, substituted or unsubstituted $C_1$-$C_8$ alkyl or $C_1$-$C_8$ alkoxy; a is selected from the group consisting of 0, 1, 2, or 3;
$R_{15}$ and $R_{16}$ are each independently selected from the group consisting of H and $C_1$-$C_8$ alkyl;
$R_3$ is selected from the group consisting of

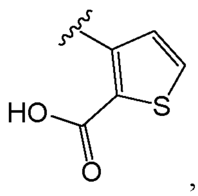 , 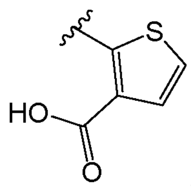 , 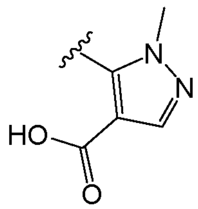 ,

(n × $R_6$)-substituted phenyl, (n × $R_6$)-substituted pyridyl, (n × $R_6$)-substituted pyrimidyl, (n × $R_6$)-substituted pyridazinyl, (n × $R_6$)-substituted thienyl, (n × $R_6$)-substituted furyl, (n × $R_6$)-substituted pyrazolyl, (n × $R_6$)-substituted imidazolyl or (n × $R_6$)-substituted pyrrolyl;
each $R_6$ is independently selected from the group consisting of substituted or unsubstituted $C_1$-$C_8$ alkyl, $C_1$-$C_8$ alkoxy, halogen, cyano, nitro, amino, hydroxy, $-C(O)OR_{81}$, $-C(O)R_{81}$,

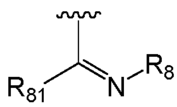 ,

cyanomethyl, phosphonyl, sulfonic acid group, sulfonamido, boric acid group, - $NHR_8$ or $-C(O)NHR_8$; alternatively, two $R_6$ linked to the same atom form =O; n is selected from the group consisting of 0, 1, 2, 3, or 4;
$R_{81}$ is selected from the group consisting of H, substituted or unsubstituted $C_1$-$C_8$ alkyl, and amino;
$R_8$ is selected from the group consisting of substituted or unsubstituted $C_1$-$C_8$ alkyl, hydroxy, and

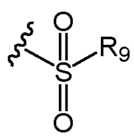 ;

$R_9$ is selected from the group consisting of $C_1$-$C_8$ alkyl and (6-10)-membered aryl;
the substituent of the alkyl is selected from the group consisting of deuterium, halogen, hydroxy, cyano, nitro, carboxyl, piperidyl, morpholinyl or $-NR_{13}R_{14}$;

$R_{13}$ and $R_{14}$ are each independently selected from the group consisting of H and $C_1$-$C_8$ alkyl.

**17.** The compound according to claim 1, or a salt thereof, or a stereoisomer thereof, or a solvate thereof, or a hydrate thereof, or a prodrug thereof, or a deuterated compound thereof, **characterized in that** the compound is as represented by formula IV:

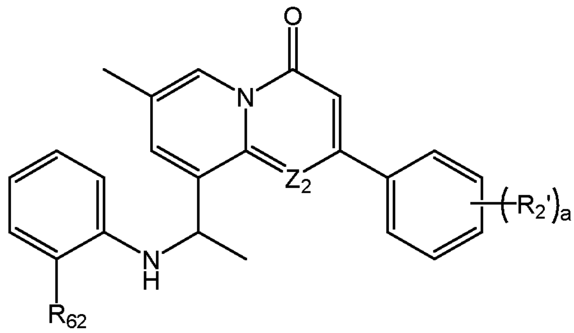

Formula IV

wherein,

$Z_2$ is selected from CH or N;
$R_{62}$ is selected from the group consisting of H, substituted or unsubstituted $C_1$-$C_8$ alkyl, $C_1$-$C_8$ alkoxy, halogen, cyano, nitro, amino, hydroxy, carboxyl, cyanomethyl, phosphonyl, sulfonic acid group, sulfonamido, boric acid group, $-NHR_8$ or $-C(O)NHR_8$;
$R_8$ is selected from the group consisting of hydroxy and

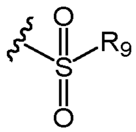
;

$R_9$ is selected from the group consisting of $C_1$-$C_8$ alkyl and phenyl;
$R_2$' is a ring substituent, and the number of substituents is a; each $R_2$' is independently selected from the group consisting of halogen, hydroxy, amino, carboxyl, nitro, cyano, $C_1$-$C_8$ alkyl or $C_1$-$C_8$ alkoxy; a is selected from the group consisting of 0, 1, 2, or 3;
the substituent of the alkyl is selected from the group consisting of halogen, hydroxy, cyano, nitro, carboxyl, piperidyl, morpholinyl or $-NR_{13}R_{14}$;
$R_{13}$ and $R_{14}$ are each independently selected from the group consisting of H and $C_1$-$C_8$ alkyl.

**18.** The compound according to any one of claims 1 to 17, or a salt thereof, or a stereoisomer thereof, or a solvate thereof, or a hydrate thereof, or a prodrug thereof, or a deuterated compound thereof, **characterized in that** the compound is selected from the group consisting of:

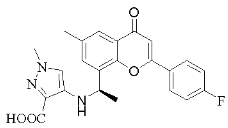
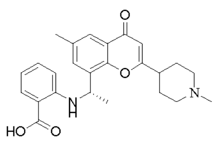
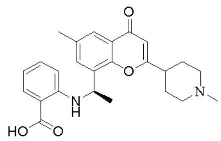
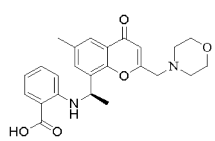
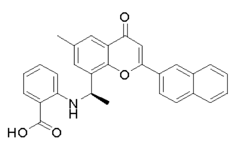

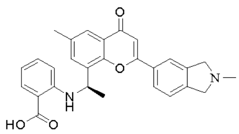
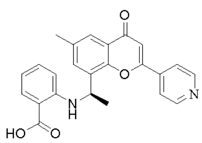
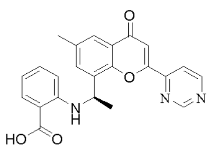
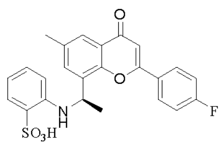
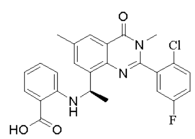

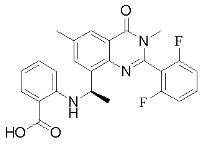 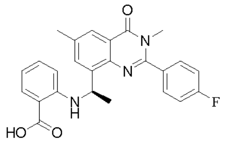 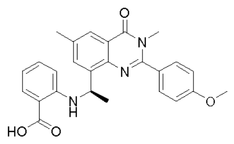 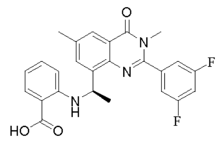 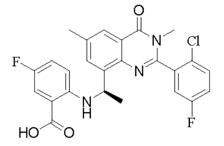

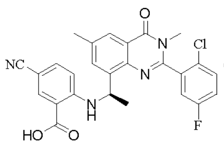 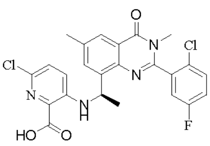 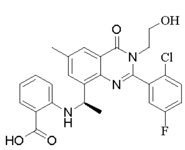 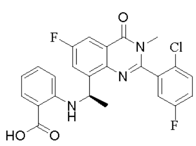 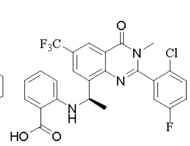 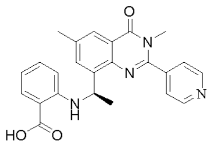

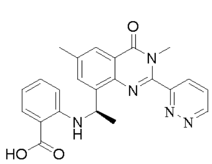 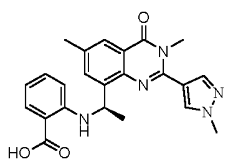 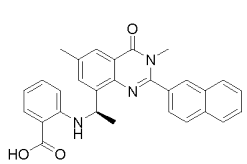 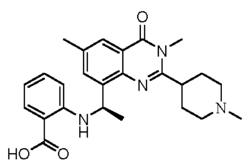 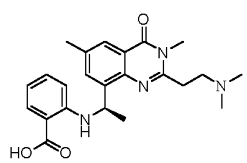

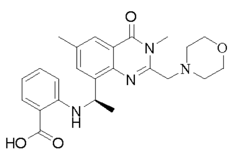 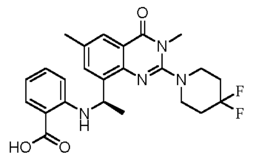 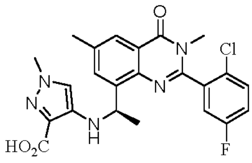 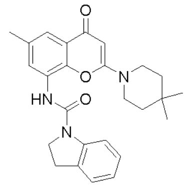 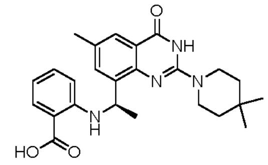

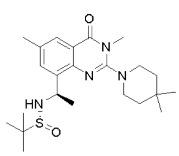 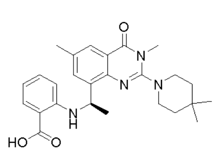 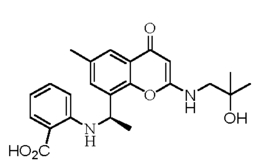 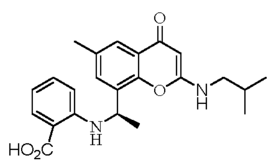 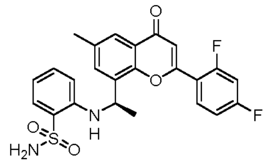

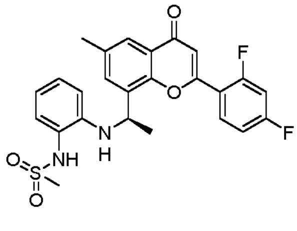 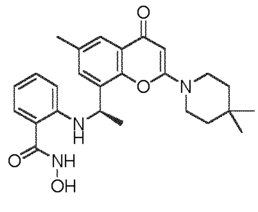 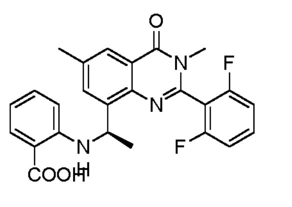 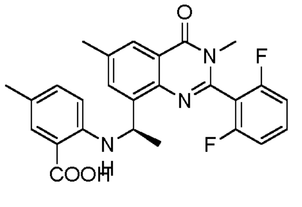

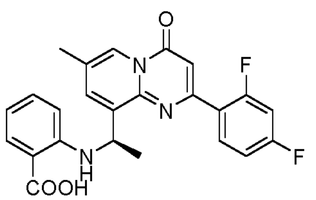

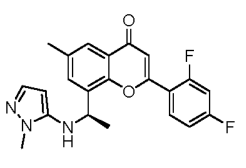 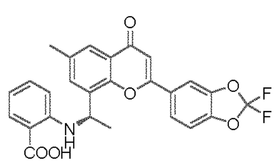 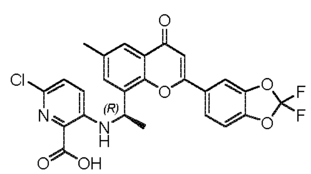 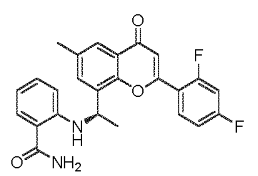

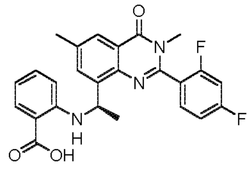
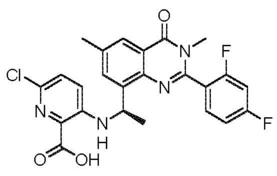
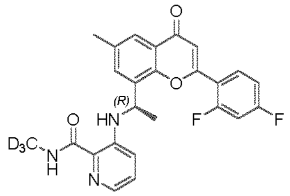
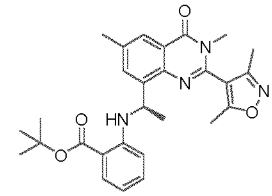

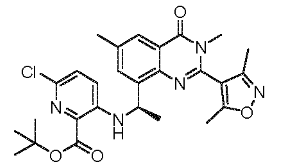
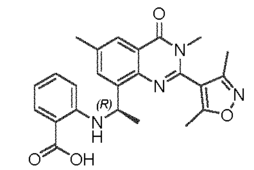
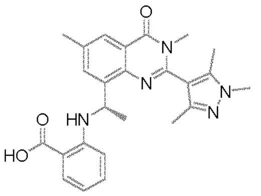
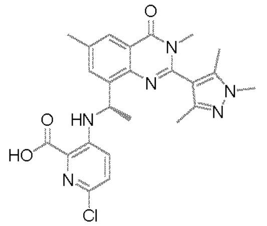

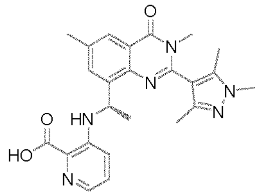
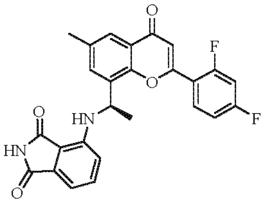
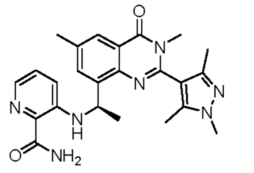
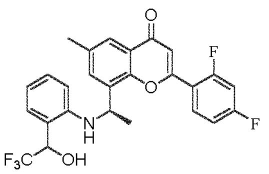

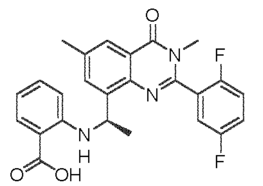
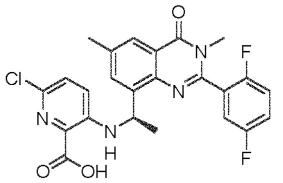
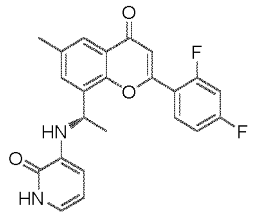
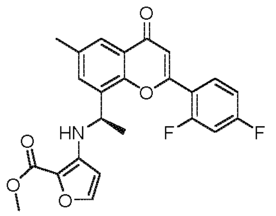

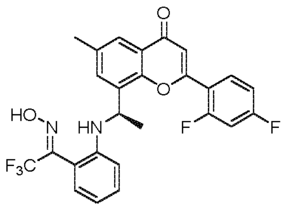
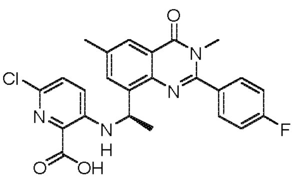
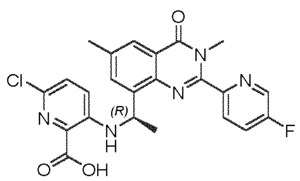
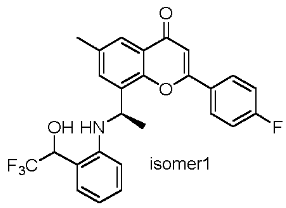
isomer1

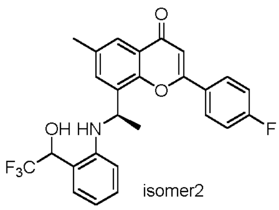
isomer2
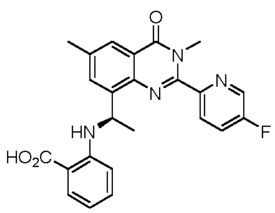
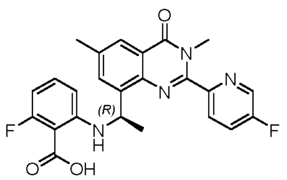
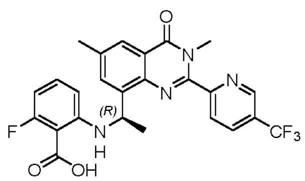

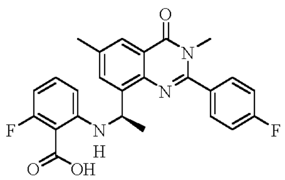
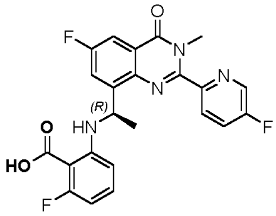
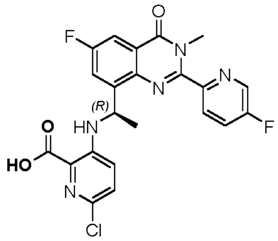
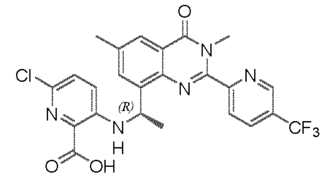

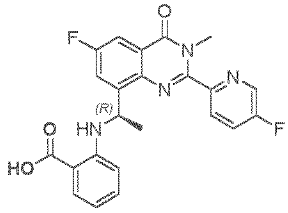 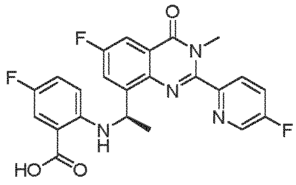 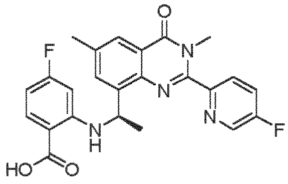 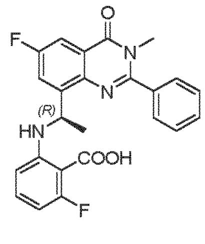

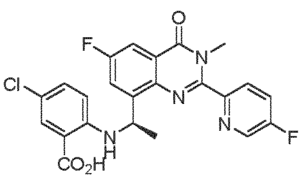 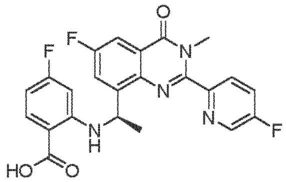 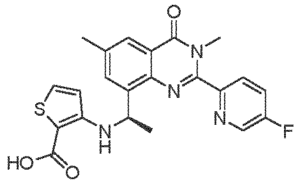 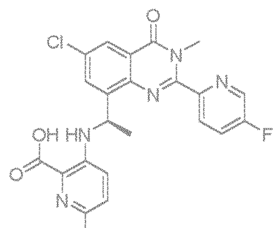

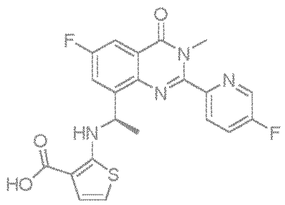 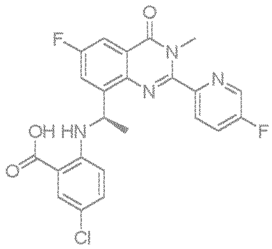 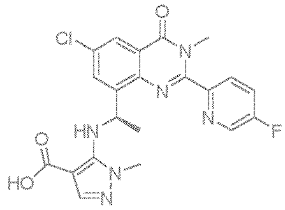 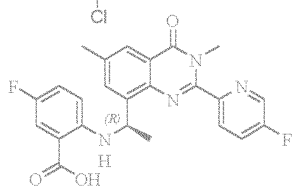

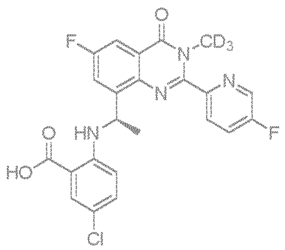 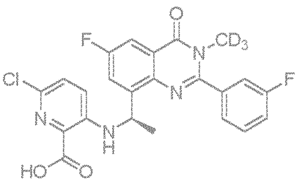 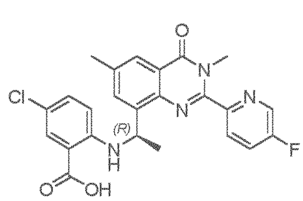 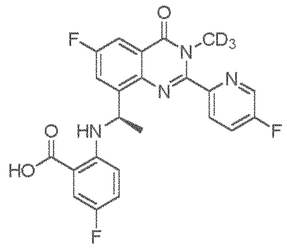

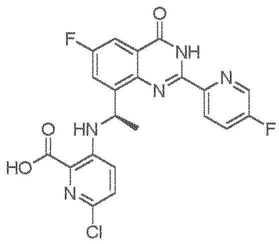 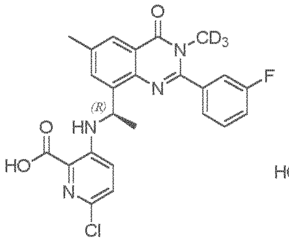 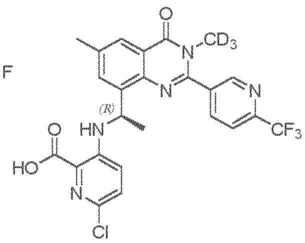 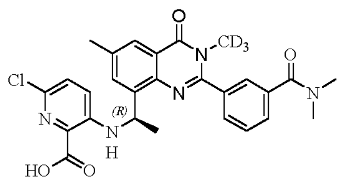

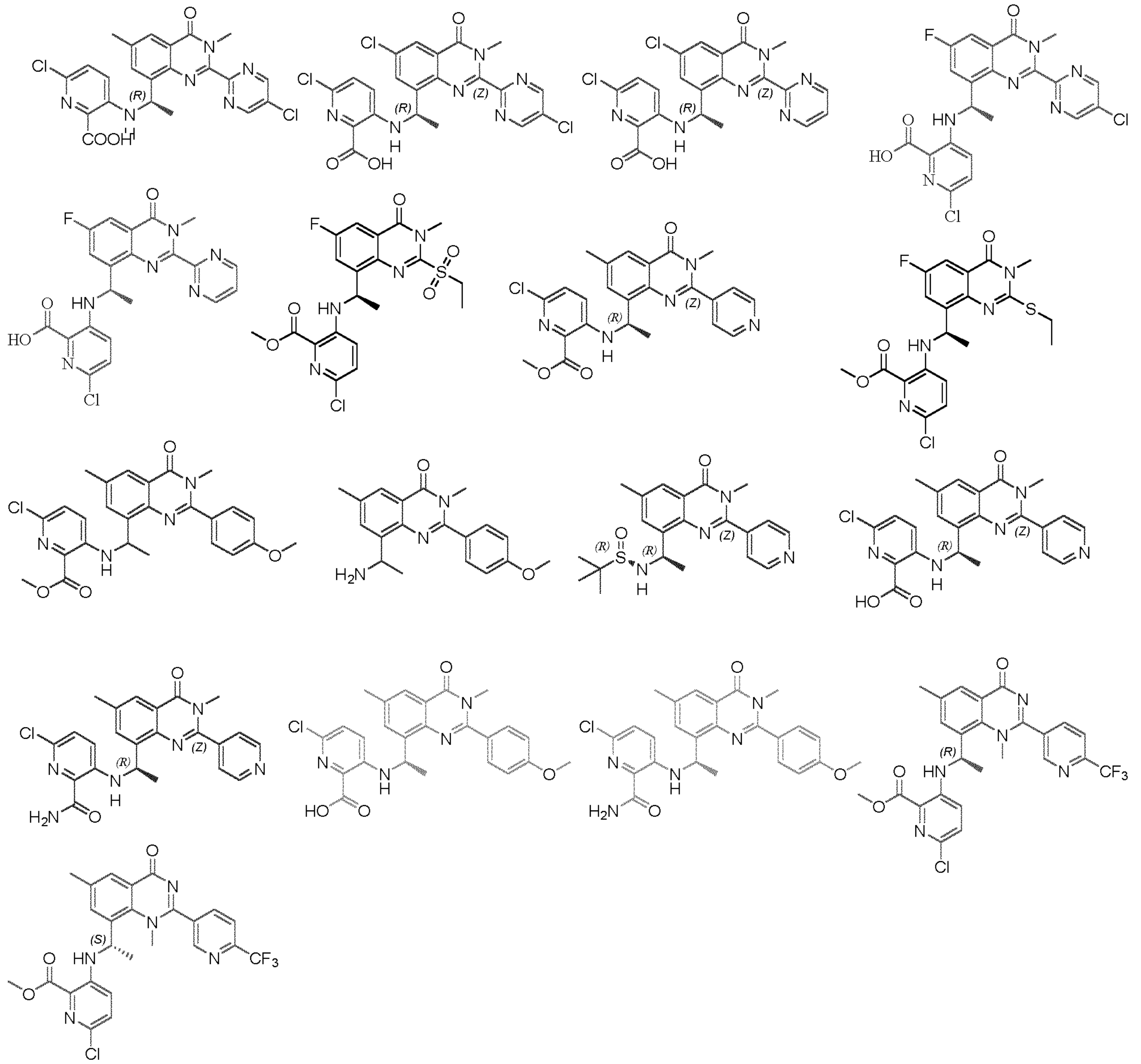

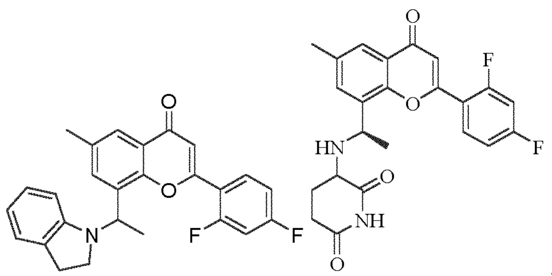

.

19. The compound according to any one of claims 1 to 18, or a salt thereof, or a stereoisomer thereof, or a solvate thereof, or a hydrate thereof, or a prodrug thereof, or a deuterated compound thereof for use in the manufacture of PI3K inhibitors;
preferably, the PI3K inhibitor is a selective PI3K inhibitor.

20. The compound according to any one of claims 1 to 18, or a salt thereof, or a stereoisomer thereof, or a solvate thereof, or a hydrate thereof, or a prodrug thereof, or a deuterated compound thereof for use in the manufacture of medicaments for the prevention and/or treatment of diseases associated with PI3K.

21. The use according to claim 19, **characterized in that** the disease is cancer, inflammation, or cardiovascular diseases

related to PI3K;
preferably, the cancers are breast cancer, colorectal cancer, gastric cancer, colon cancer, rectal cancer, ovarian cancer, and prostate cancer.

**22.** A medicament, **characterized in that** it is prepared from the compound according to any one of claims 1 to 18, or a salt thereof, or a stereoisomer thereof, or a solvate thereof, or a hydrate thereof, or a prodrug thereof, or a deuterated compound thereof as the active ingredient, in combination with pharmaceutically acceptable excipients or auxiliary ingredients.

**INTERNATIONAL SEARCH REPORT**

International application No.
**PCT/CN2023/105915**

**A. CLASSIFICATION OF SUBJECT MATTER**

C07D 471/04(2006.01)i; C07D 487/04(2006.01)i; C07D 413/12(2006.01)i; C07D 405/04(2006.01)i; A61K 31/4433(2006.01)i; A61K 31/352(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, VEN, DWPI, ENTXTC, CNKI, 万方, WANFANG, 百度学术, BAIDU SCHOLAR, ISI web of Science, STN: 海创药业, 樊磊, 于华, 王飞, 艾朝武, 胥珂馨, 刘兴太, 杜静, 彭莹, 罗潼川, 谭斌, 王文仲, 严德俊, 王朝旭, 李亮, 肖代彪, 刘城成, 骆瑶, 李兴海, 陈元伟, PI3K, 抑制剂, 磷酸肌醇, 磷脂酰肌醇, 激酶, phosphoinositide, kinase, structural formula search.

**C. DOCUMENTS CONSIDERED TO BE RELEVANT**

| Category* | Citation of document, with indication, where appropriate, of the relevant passages | Relevant to claim No. |
|---|---|---|
| PX | WO 2023060262 A1 (RELAY THERAPEUTICS, INC.) 13 April 2023 (2023-04-13) abstract, description, paragraphs 48-548 and 566-667, tables 1-2, and claims 1-47 | 1-22 |
| PX | WO 2023078401 A1 (FOCHON BIOSCIENCES, LTD.) 11 May 2023 (2023-05-11) abstract, description, paragraphs 10-22, tables 1-2, and claims 1-48 | 1-22 |
| PX | WO 2023081209 A1 (ZENO MANAGEMENT, INC.) 11 May 2023 (2023-05-11) abstract, description, paragraphs 71-88 and 90-115, tables A, and table 2 | 1-22 |
| PX | WO 2023104111 A1 (NANJING ZENSHINE PHARMACEUTICALS CO., LTD.) 15 June 2023 (2023-06-15) abstract, descriptiont, pages 3-14, tables 1-2, and claims 1-22 | 1-8, 18-22 |
| PX | WO 2022235574 A1 (PETRA PHARMA CORP.) 10 November 2022 (2022-11-10) abstract, description, paragraphs 15-394, embodiments 1-178, and claims 1-74 | 1-8, 18-22 |
| PX | WO 2022251482 A1 (PETRA PHARMA CORP.) 01 December 2022 (2022-12-01) abstract, description, paragraphs 62-264, embodiments 1-266, and claims 51-78 | 1-8, 18-22 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 October 2023** | **31 October 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2023/105915

C. DOCUMENTS CONSIDERED TO BE RELEVANT

| Category* | Citation of document, with indication, where appropriate, of the relevant passages | Relevant to claim No. |
|---|---|---|
| X | CN 1688575 A (KINACIA PTY LTD.) 26 October 2005 (2005-10-26) abstract, claims 1-26, embodiment 1, and embodiment 2i | 1-8, 17-22 |
| X | WO 2021202964 A1 (PETRA PHARMA CORP.) 07 October 2021 (2021-10-07) abstract, description, paragraphs 83-209, table 2, embodiments 77 and 126, and claims 1-36 | 1-8, 18-22 |
| A | CN 107001317 A (SHANDONG XUANZHU PHARMA CO., LTD.) 01 August 2017 (2017-08-01) entire document | 1-22 |

Form PCT/ISA/210 (second sheet) (July 2022)

**INTERNATIONAL SEARCH REPORT**
**Information on patent family members**

International application No.
**PCT/CN2023/105915**

| Patent document cited in search report | Publication date (day/month/year) | Patent family member(s) | Publication date (day/month/year) |
|---|---|---|---|
| WO 2023060262 A1 | 13 April 2023 | None | |
| WO 2023078401 A1 | 11 May 2023 | None | |
| WO 2023081209 A1 | 11 May 2023 | None | |
| WO 2023104111 A1 | 15 June 2023 | None | |
| WO 2022235574 A1 | 10 November 2022 | TW 202309003 A | 01 March 2023 |
| | | US 2023017140 A1 | 19 January 2023 |
| | | JP 2023523095 A | 01 June 2023 |
| | | JP 7308369 B2 | 13 July 2023 |
| WO 2022251482 A1 | 01 December 2022 | TW 202313605 A | 01 April 2023 |
| | | US 2023096175 A1 | 30 March 2023 |
| CN 1688575 A | 26 October 2005 | WO 2004016607 A1 | 26 February 2004 |
| | | EP 1537102 A1 | 08 June 2005 |
| | | JP 2006500361 A | 05 January 2006 |
| | | US 2006500361 A1 | 07 December 2006 |
| | | CN 100430389 C | 05 November 2008 |
| | | US 7872011 B | 18 January 2011 |
| WO 2021202964 A1 | 07 October 2021 | PE 20230824 A1 | 19 May 2023 |
| | | AR 121719 A1 | 29 June 2022 |
| | | US 2023286960 A1 | 14 September 2023 |
| | | CA 3173569 A1 | 07 October 2021 |
| | | CO 2022014160 A2 | 16 February 2023 |
| | | TW 202200567 A | 01 January 2022 |
| | | TWI 794780 B | 01 March 2023 |
| | | CR 20220493 A | 15 December 2022 |
| | | KR 20220163462 A | 09 December 2022 |
| | | ECSP 22077299 A | 31 January 2023 |
| | | BR 112022019918 A2 | 14 February 2023 |
| | | JOP 20220247 A1 | 30 January 2023 |
| | | AU 2021248415 A1 | 20 October 2022 |
| | | US 2022372023 A1 | 24 November 2022 |
| | | US 11649227 B2 | 16 May 2023 |
| | | IL 296918 A | 01 December 2022 |
| | | EP 4126851 A1 | 08 February 2023 |
| | | JP 2023521321 A | 24 May 2023 |
| CN 107001317 A | 01 August 2017 | WO 2016050201 A1 | 07 April 2016 |

Form PCT/ISA/210 (patent family annex) (July 2022)